# EUROPEAN PATENT APPLICATION

(11) **EP 4 434 522 A2**
(43) Date of publication of application: **25.09.2024**
(21) Application number: 24185703.6
(22) Date of filing: 05.01.2022
(51) Int. Cl.: A61K 31/44

(54) **HETEROCYCLIC P2Y14 RECEPTOR ANTAGONISTS**

(30) Priority: 18.01.2021 US 202163138581 P
(62) Divisional of application: 22701105.3
(71) Applicant: The United States of America, as represented by the Secretary, Department of Health and Human Services, Bethesda, Maryland 20892-7788 (US)
(72) Inventor: JACOBSON, Kenneth A., Silver Spring, 20902 (US); WEN, Zhiwei, Rockville, 20852 (US); JUNG, Young-Hwan, Rockville, 20852 (US)
(74) Representative: Pfenning, Meinig & Partner mbB

(57) **Abstract**

Disclosed are compounds for treating or preventing a disease or disorder responsive to antagonism of a P2Y₁₄R receptor agonist in a mammal in need thereof, for example, compounds of formulas (I) and (II), wherein R¹-R⁸, X, Y, Z, X', Y', Z', and A are as defined herein, that are useful in treating an inflammatory such as asthma, cystic fibrosis, and sterile inflammation of the kidney.

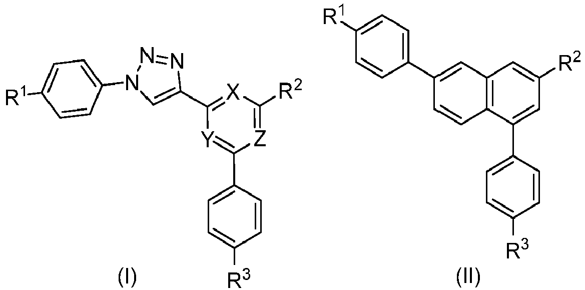

## Description

### CROSS-REFERENCE TO A RELATED APPLICATION

This patent application claims the benefit of United States Provisional Patent Application No. 63/138,581, filed January 18, 2021, the disclosure of which is incorporated herein in its entirety for all purposes.

### STATEMENT REGARDING FEDERALLY SPONSORED RESEARCH OR DEVELOPMENT

This invention was made with Government support under Grant Number ZIADK003116 awarded by the National Institutes of Health. The Government has certain rights in this invention.

### BACKGROUND OF THE INVENTION

Extracellular nucleotides released by tissue and organs during stress or injury activate a class of cell-surface receptors (P2Rs) to boost the innate and adaptive immune responses (1-3). This mechanism acts as a time-dependent component of the signaling purinome, along with the anti-inflammatory adenosine receptors (ARs, also termed P1 receptors), to protect the organism in various challenged circumstances. The P2Y₁₄ receptor (P2Y₁₄R) responds to endogenous agonists uridine-5'-diphosphoglucose and uridine-5'-diphosphate to mediate inflammatory activity, in part by activating neutrophil motility (4-6). Structurally, the P2Y₁₄R belongs to the δ-branch of rhodoposin-like G protein-coupled receptors (GPCRs). Three subtypes of the P2YRs are preferentially coupled to inhibition of adenylate cyclase through guanine nucleotide inhibitory (Gᵢ) protein: P2Y₁₂R, P2Y₁₃R and P2Y₁₄R. Selective P2Y₁₄R antagonists are sought as potential agents for treating asthma, sterile inflammation of the kidney, diabetes and neurodegeneration (7-12). However, only a few antagonists are known, so there is a clear need for more competitive P2Y₁₄R antagonists. Other subtypes of the P2YR family in general, e.g., P2Y₂R and P2Y₆R, are also associated with proinflammatory effects, and their antagonists are desired for providing anti-inflammatory activity (13, 14).

Antagonists of the P2Y₁₄R were first reported by Black and colleagues (58), and of the two classes of antagonists reported, naphthoic acids and pyrido[4,3-*d*]pyrimidines, only the former appeared to be competitive antagonists. Thus, there is an unmet need for diverse competitive P2Y₁₄R antagonists.

### BRIEF SUMMARY OF THE INVENTION

The invention provides a compound of formula (I) or formula (II): 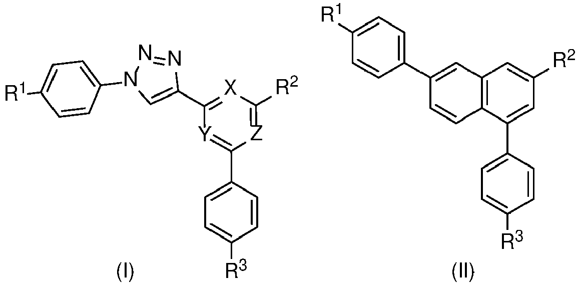
wherein X, Y, and Z are, independently, CH or N,
R¹ is halo or CF₃,
R² is selected from the group consisting of COOH, COOR¹², CONHOH, CH₂CON(R¹¹)₂, CHR⁷OCOR⁸, (CH₂)ₒNR⁹, tetrazolyl, CH₂OPO(OH)₂ and 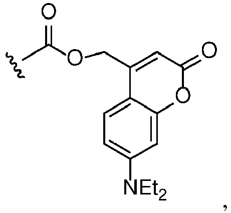 R³ is selected from the group consisting of NHR⁴, COOR⁵, (C≡C)ₙ(CH₂)ₘR⁶, CONH(CH₂)_{q}NH₂, COCF₃, 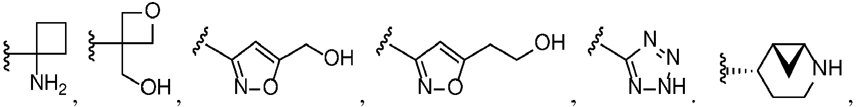 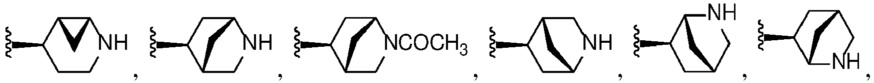 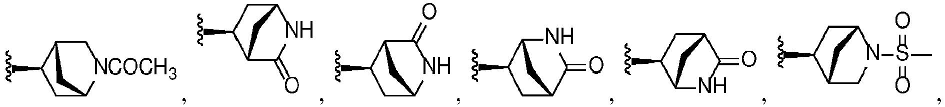 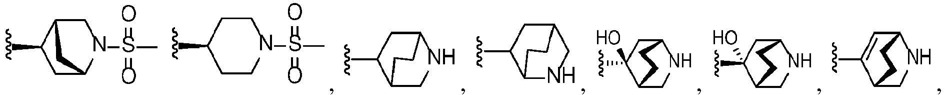 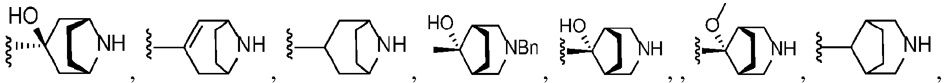 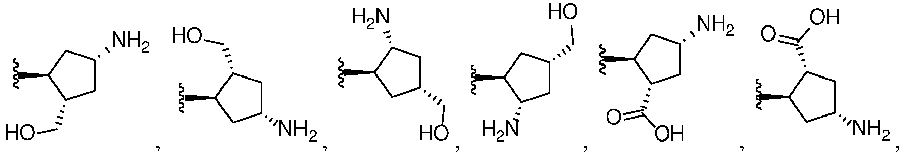 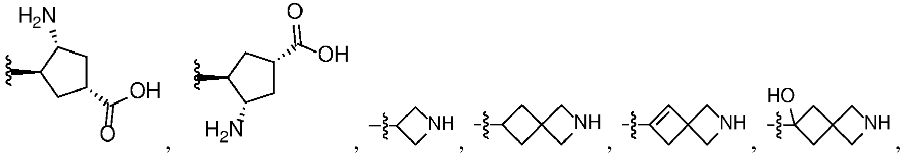 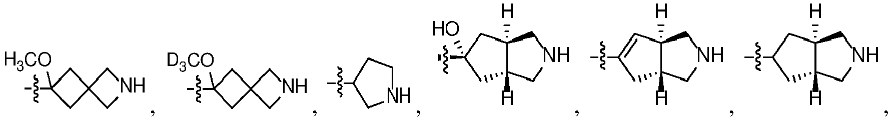 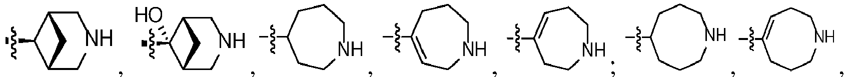 and 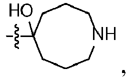
R⁴ is H or COR⁶ wherein R⁶ is C₁-C₆ alkyl, C₆-C₁₀ aryl, or 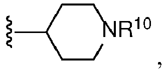
R⁵ is C₁-C₆ alkyl, C₃-C₈ cycloalkyl, or benzyl,
R⁶ is NH₂, or CONH₂,
R⁷-R⁹ are independently hydrogen or C₁-C₆ alkyl,
R¹⁰ is H, C₁-C₁₀ alkylcarbonyl, or C₁-C₁₀ alkyloxycarbonyl,
R¹¹ is hydrogen or C₁-C₆ alkyl,
R¹² is C₁-C₆ alkyl, C₃-C₈ cycloalkyl, or benzyl, and
m, o, and q are independently integers of 1 to about 10, and
n is zero or an integer of 1 to about 10,
or a pharmaceutically acceptable salt thereof, as well as stereoisomers thereof.

The present invention further provides a compound of formula (III) 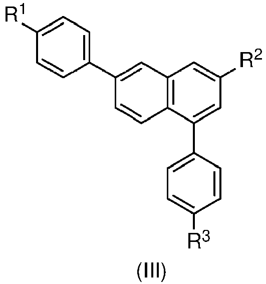 wherein R¹ is CF₃, R² is COOH, and R³ is a 4-8 membered heterocyclic ring having at least one nitrogen atom.

The present invention further provides a compound of formula (IV) or (V): 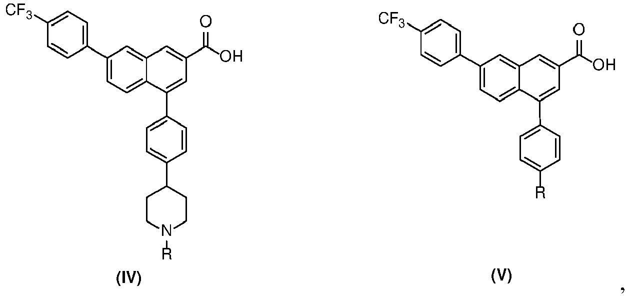 wherein: R is:

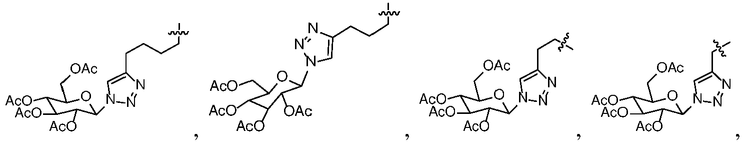

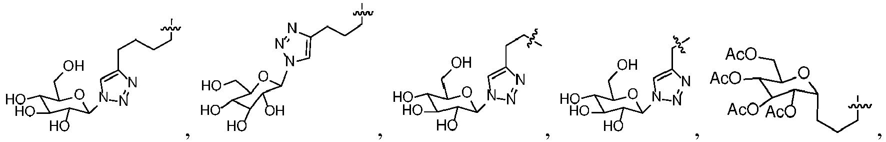

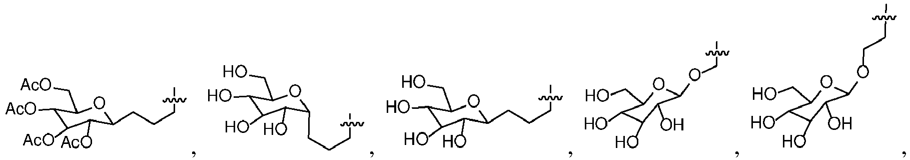

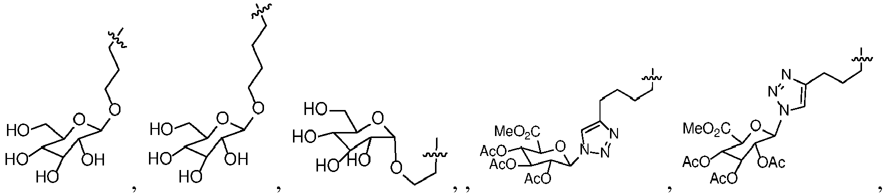

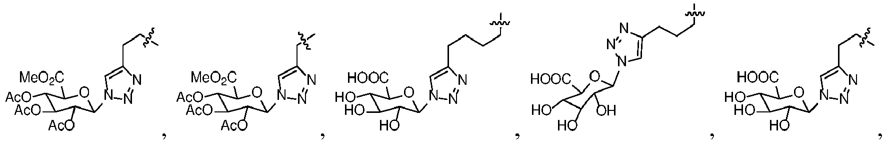

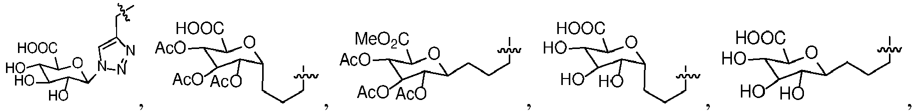

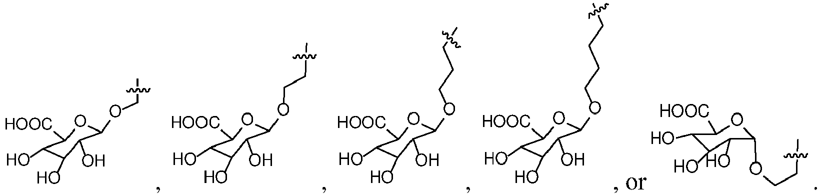

The invention also provides a method of antagonizing a P2Y₁₄R receptor in a mammal in need thereof, comprising administering to the mammal an effective amount of a compound of the invention or a pharmaceutically acceptable salt thereof.

The invention further provides a method of treating or preventing an inflammatory condition in a mammal in need thereof, comprising administering to the mammal an effective amount of a compound of the invention or a pharmaceutically acceptable salt thereof.

The invention additionally provides a method of treating pain in a mammal in need thereof, comprising administering to the mammal an effective amount of a compound of the invention or a pharmaceutically acceptable salt thereof.

### BRIEF DESCRIPTION OF THE SEVERAL VIEWS OF THE DRAWING(S)

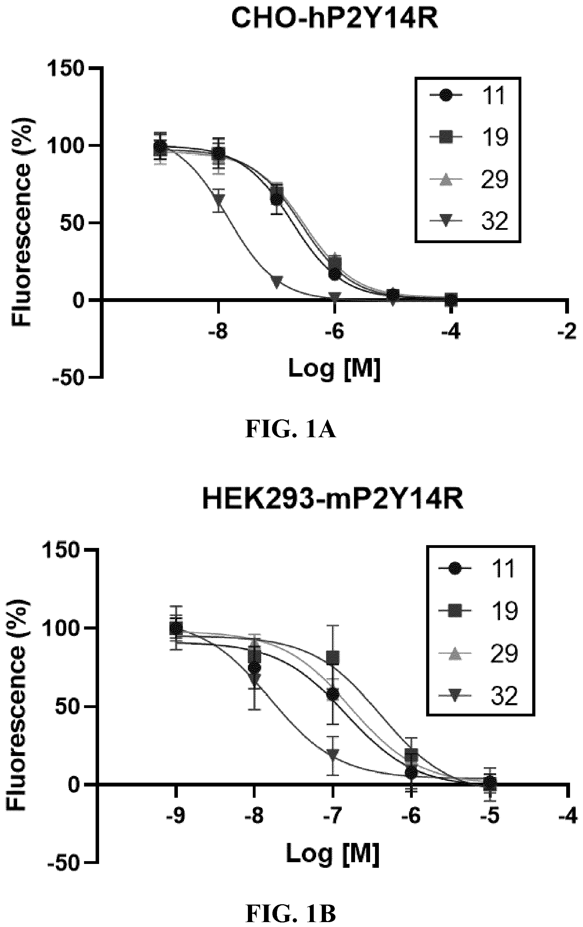

FIG. 1A-B show the inhibition of binding of fluorescent antagonists 11, 19, 29, and 32 at hP2Y₁₄R (FIG. 1A) and at mP2Y₁₄R (FIG. 2B) exhibited by four P2Y₁₄R antagonist analogues in accordance with an aspect of the invention (mean ± SD, n=3-4 independent determinations, performed in triplicate).
FIG. 2 shows reversal of CCI-induced mechano-allodynia in the mouse induced by compound **32** (10 µmol/kg, i.p.). Mean±SD is shown, n=3, ANOVA, *P<0.05 vs. Day 0 and † P<0.05 vs. day 7 post-injury. **32** was dissolved in 5% DMSO in water as vehicle, and a volume of 0.2 mL was injected.
FIG. 3 shows the effects of P2Y₁₄R antagonists and their prodrugs, namely **32**, **37b**, **3a**, **37a**, **1b**, and **37c**, compared to reference antagonist **1a**, during allergen challenge reduces eosinophilic airway inflammation in a protease model of asthma. Effect on BALF leukocytes of i.p. injection of the P2Y₁₄R antagonists 2 d post-challenge of ASP/OVA-sensitized mice. * *P* <0.05, ** *P* <0.01, *** *P* <0.001, **** *P* <0.0001 vs. vehicle by one-way ANOVA followed by Dunnett's test.
FIG. 4 shows the reversal of established mechano-allodynia in the CCI mouse model by compound **14** (10 µmol/kg, i.p.) at the time of peak pain, i.e. seven days post-injury. Mean±SD is shown, n=3, ANOVA, *P<0.05 vs. Day 0 and † P<0.05. The vehicle consisted of 5% aqueous DMSO (0.2 mL injected).

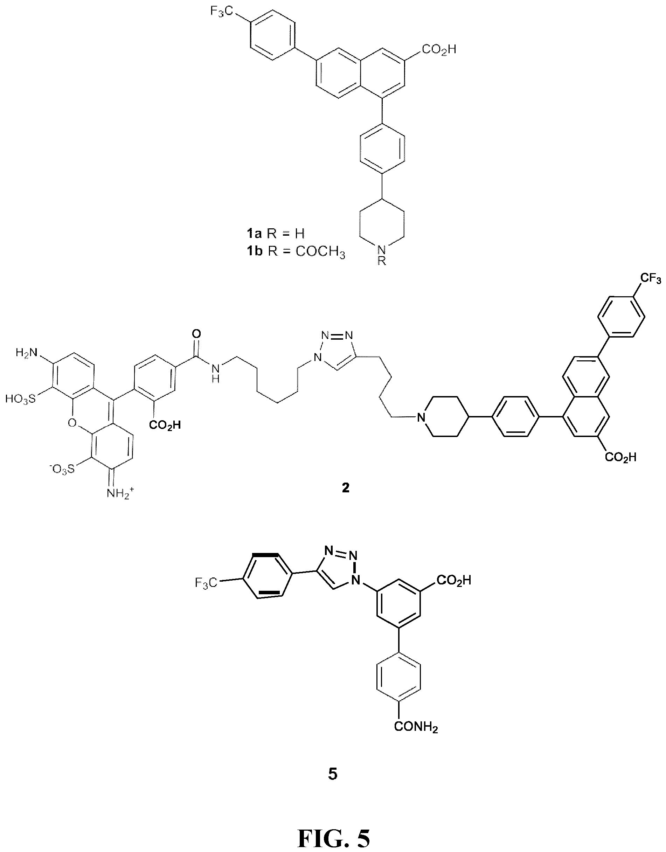

FIG. 5 shows the structures of reference compound **1a**, compound **1b**, compound **5**, and fluorescent inhibitor 2.
FIG. 6 shows that compound **114** (10 µmol/kg, i.p.) reversed the established mechano-allodynia in the CCI mouse model at the time of peak pain, i.e., seven days post-injury. Mean ± SD is shown, n=3, ANOVA, *P<0.05 vs. Day 0 and † P<0.05. The vehicle consisted of 5% aqueous DMSO (0.2 mL injected).
FIG. 7A-E depict the activities of P2Y14R antagonists **114** and its ester prodrug **141**, carbamate prodrug **142**, and double prodrug **143** in a mouse model of asthma (dose: 20 µmol/kg (9.8-12.2 mg/kg); concentration: 2 µmol/mL (2 mM) in 10% DMSO, 30% PEG-400, 60% H₂O; administration: 10 µL/g of body weight). 7-Day sensitization with ovalbumin/aspergillus was followed by a single challenge (ovalbumin, aerosol) at day 14. Antagonists were given 30 min prior to challenge. Harvesting of cells and cell counting were on day 16.

### DETAILED DESCRIPTION OF THE INVENTION

In an aspect, the invention provides a compound of formula (I) or formula (II): 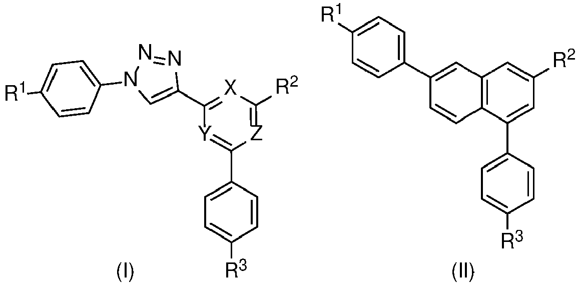
wherein X, Y, Z are CH or N,
R¹ is halo or CF₃,
R² is selected from the group consisting of COOH, COOR¹², CONHOH, CH₂CON(R¹¹)₂, CHR⁷OCOR⁸, (CH₂)ₒNR⁹, tetrazolyl, CH₂OPO(OH)₂, and 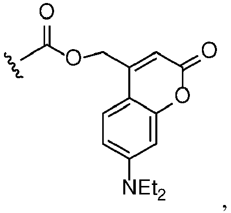
R³ is selected from the group consisting of NHR⁴, COOR⁵, (C≡C)ₙ(CH₂)ₘR⁶, CONH(CH₂)_{q}NH₂, COCF₃, 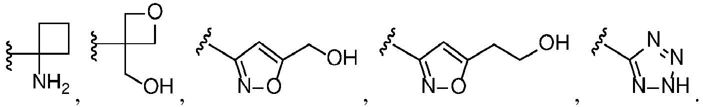 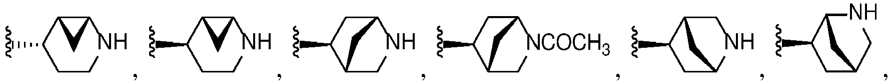 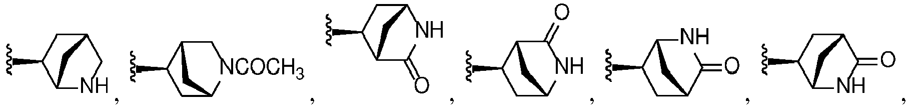 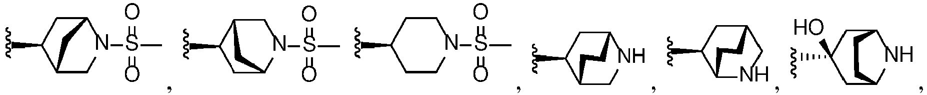 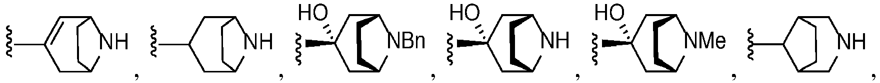 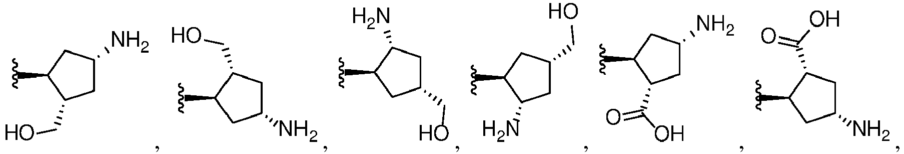 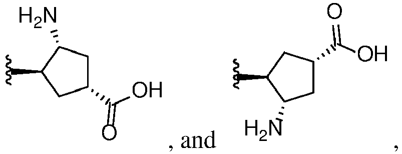
R⁴ is H or COR⁶ wherein R⁶ is C₁-C₆ alkyl, C₆-C₁₀ aryl, or 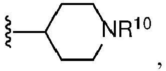
R⁵ is C₁-C₆ alkyl, C₃-C₈ cycloalkyl, or benzyl,
R⁶ is NH₂, or CONH₂,
R⁷-R⁹ are independently hydrogen or C₁-C₆ alkyl,
R¹⁰ is H, C₁-C₁₀ alkylcarbonyl, or C₁-C₁₀ alkyloxycarbonyl,
R¹¹ is hydrogen or C₁-C₆ alkyl,
R¹² is C₁-C₆ alkyl, C₃-C₈ cycloalkyl, or benzyl, and
m, o, and q are independently integers of 1 to about 10, and
n is zero or an integer of 1 to about 10,
or a pharmaceutically acceptable salt thereof, as well as stereoisomers thereof.

In certain aspects, R¹ is CF₃.

In certain aspects, the compound is of formula (I).

In certain aspects of the compound of formula (I), R³ is selected from NH₂, NHCOCH₃, NHCOC₆H₅, NHCOC(CH₃)₃,

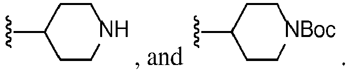

In certain aspects, R³ is COOR⁵.

In certain aspects, R³ is (CµC)ₙ(CH₂)ₘR⁶ or CONH(CH₂)_{q}NH₂.

In certain of these aspects, R³ is selected from CONH(CH₂)₃NH₂, CH₂CONH₂, (CH₂)₃NH₂, and C≡CCH₂NH₂.

In certain aspects, R³ is selected from Br, (CH₂)₂-CN, , 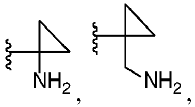 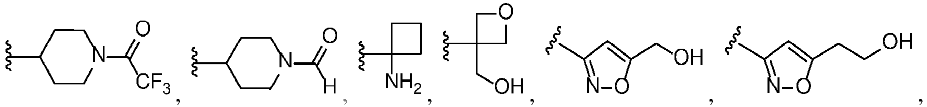 and

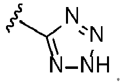

In certain aspects, the compound is of formula (II).

In certain aspects of formula (II), R³ is selected from Br, (CH₂)₂-CN,

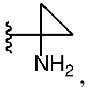

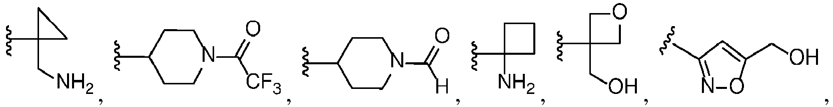

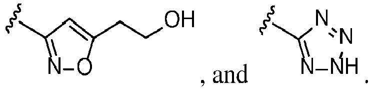

In certain particular aspects, R³ is

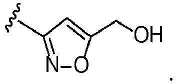

In certain aspects, R² is H.

In certain aspects, R² is COOR¹², CH₂CON(Rⁿ)₂, CHR⁷OCOR⁸, (CH₂)ₒNR⁹, tetrazolyl, CH₂OPO(OH)₂, or

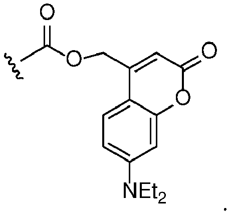

In a particular aspect, the compound is: 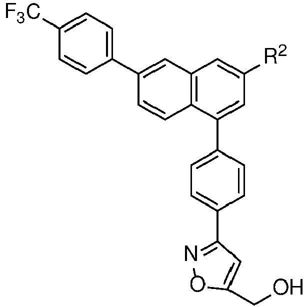 wherein R² is COOH or COOCH₂CON(CH₃)₂.

In certain aspects, R³ is selected from 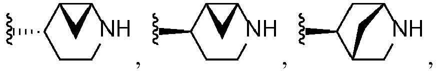 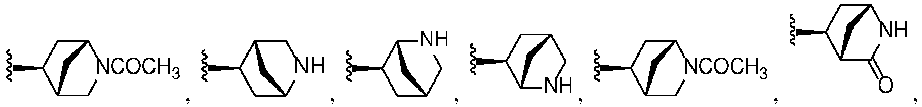 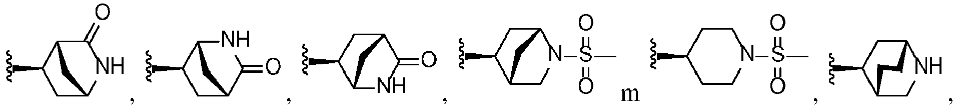 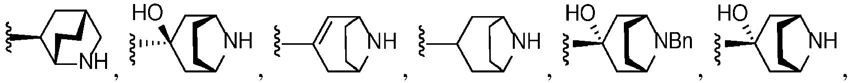 and

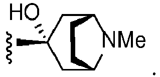

In certain particular aspects, R³ is 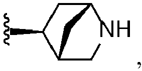 and wherein the configuration of the three chiral centers is (S,S,S).

In certain aspects, R³ is selected from

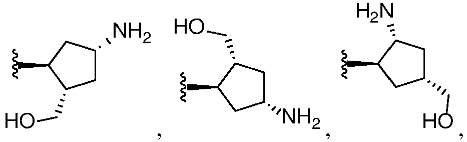

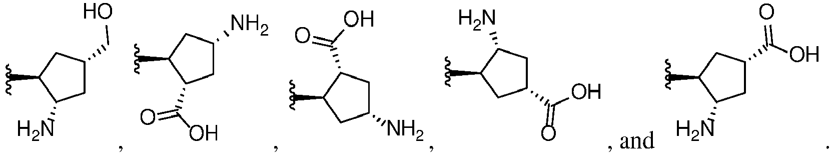

In certain aspects, R² is H.

In certain aspects, R² is COOR¹², CH₂CON(Rⁿ)₂, CHR⁷OCOR⁸, (CH₂)ₒNR⁹, tetrazolyl, CH₂OPO(OH)₂, or

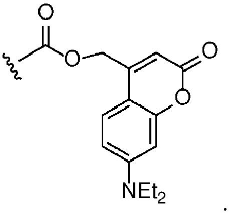

In a particular aspect, the compound is: 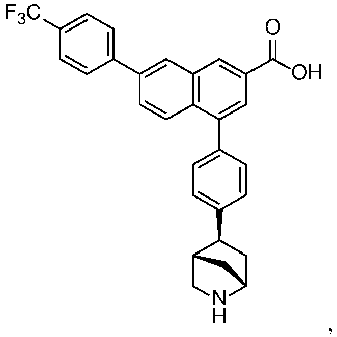 wherein the configuration about the three chiral centers is (S,S,S).

The present invention further provides a compound of formula (III) 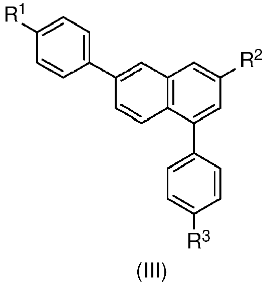 wherein R¹ is CF₃, R² is COOH, and R³ is a 4-8 membered heterocyclic ring having at least one nitrogen atom, wherein the heterocyclic ring comprises 1, 2, or 3 rings, fused or linked at one or more atoms, and wherein the heterocyclic ring is saturated or unsaturated, and wherein the heterocyclic ring is optionally substituted with one or more of substituents selected from the group consisting of alkyl, alkoxy, and hydroxy. Examples of compounds of formula (III) those wherein R³ is:

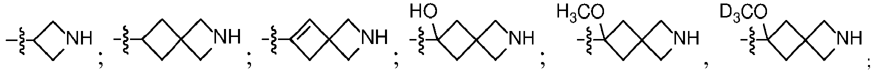

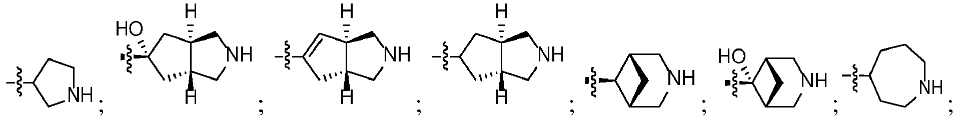

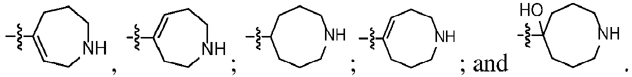

The present invention further provides a compound of formula (IV) or (V): 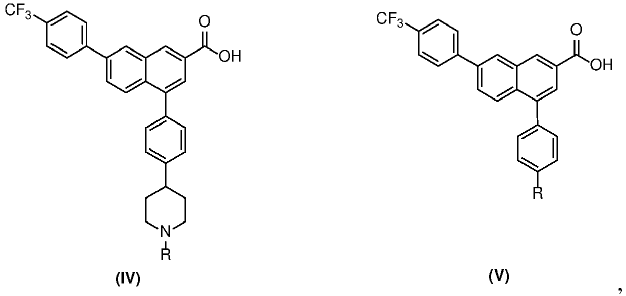 wherein:
R is sugar-triazolylalkyl moiety wherein the sugar moiety can a six membered sugar moiety, particularly an aldohexose, including allose, altrose, glucose, mannose, gulose, idose, galactose, or talose, including derivatives thereof, particularly acetyloxy derivatives thereof; for example,

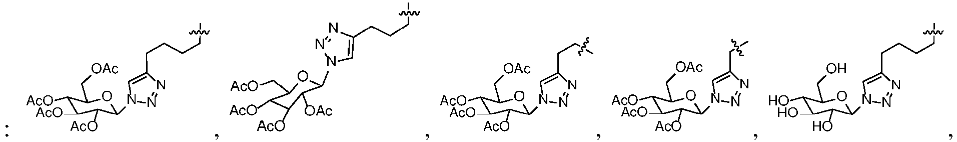

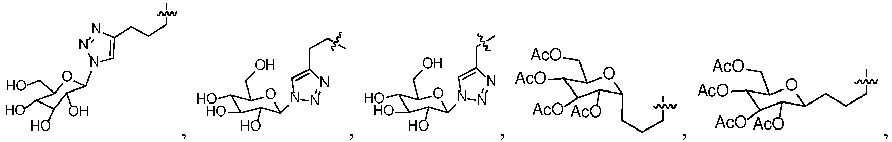

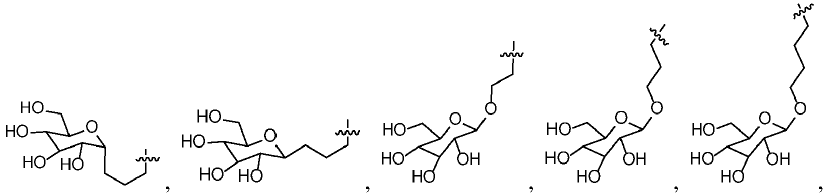

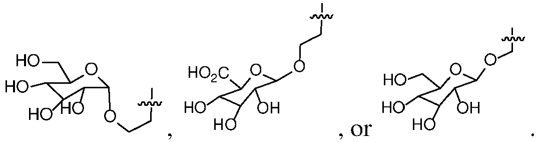

Referring now to terminology used generically herein, the term "alkyl" means a straight-chain or branched alkyl substituent containing from, for example, 1 to about 6 carbon atoms, preferably from 1 to about 4 carbon atoms, more preferably from 1 to 2 carbon atoms. Examples of such substituents include methyl, ethyl, propyl, isopropyl, n-butyl, sec-butyl, isobutyl, tert-butyl, pentyl, isoamyl, hexyl, and the like.

The term "cycloalkyl," as used herein, means a cyclic alkyl substituent containing from, for example, about 3 to about 8 carbon atoms, preferably from about 4 to about 7 carbon atoms, and more preferably from about 4 to about 6 carbon atoms. Examples of such substituents include cyclopropyl, cyclobutyl, cyclopentyl, cyclohexyl, cycloheptyl, cyclooctyl, and the like. The cyclic alkyl groups may be unsubstituted or further substituted with alkyl groups such as methyl groups, ethyl groups, and the like.

The term "alkylcarbonyl," as used herein, refers to an alkyl group linked to a carbonyl group and further linked to a molecule via the carbonyl group, e.g., alkyl-C(=O)-. The term "alkoxycarbonyl," as used herein, refers to an alkoxy group linked to a carbonyl group and further linked to a molecule via the carbonyl group, e.g., alkyl-O-C(=O)-.

The term "halo" or "halogen," as used herein, means a substituent selected from Group VIIA, such as, for example, fluorine, bromine, chlorine, and iodine.

The term "tetrazolyl", as used herein, refers to a group of the formula:

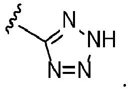

Whenever a range of the number of atoms in a structure is indicated (e.g., a C₁-C₁₂, C₁-C₈, C₁-C₆, C₁-C₄, or C₂-C₁₂, C₂-C₈, C₂-C₆, C₂-C₄ alkyl, alkenyl, alkynyl, etc.), it is specifically contemplated that any sub-range or individual number of carbon atoms falling within the indicated range also can be used. Thus, for instance, the recitation of a range of 1-8 carbon atoms (e.g., C₁-C₈), 1-6 carbon atoms (e.g., C₁-C₆), 1-4 carbon atoms (e.g., C₁-C₄), 1-3 carbon atoms (e.g., C₁-C₃), or 2-8 carbon atoms (e.g., C₂-C₈) as used with respect to any chemical group (e.g., alkyl, alkylamino, etc.) referenced herein encompasses and specifically describes 1, 2, 3, 4, 5, 6, 7, 8, 9, 10, 11, and/or 12 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 1-7 carbon atoms, 1-8 carbon atoms, 1-9 carbon atoms, 1-10 carbon atoms, 1-11 carbon atoms, 1-12 carbon atoms, 2-3 carbon atoms, 2-4 carbon atoms, 2-5 carbon atoms, 2-6 carbon atoms, 2-7 carbon atoms, 2-8 carbon atoms, 2-9 carbon atoms, 2-10 carbon atoms, 2-11 carbon atoms, 2-12 carbon atoms, 3-4 carbon atoms, 3-5 carbon atoms, 3-6 carbon atoms, 3-7 carbon atoms, 3-8 carbon atoms, 3-9 carbon atoms, 3-10 carbon atoms, 3-11 carbon atoms, 3-12 carbon atoms, 4-5 carbon atoms, 4-6 carbon atoms, 4-7 carbon atoms, 4-8 carbon atoms, 4-9 carbon atoms, 4-10 carbon atoms, 4-11 carbon atoms, and/or 4-12 carbon atoms, etc., as appropriate). Similarly, the recitation of a range of 6-10 carbon atoms (e.g., C₆-C₁₀) as used with respect to any chemical group (e.g., aryl) referenced herein encompasses and specifically describes 6, 7, 8, 9, and/or 10 carbon atoms, as appropriate, as well as any sub-range thereof (e.g., 6-10 carbon atoms, 6-9 carbon atoms, 6-8 carbon atoms, 6-7 carbon atoms, 7-10 carbon atoms, 7-9 carbon atoms, 7-8 carbon atoms, 8-10 carbon atoms, and/or 8-9 carbon atoms, etc., as appropriate).

In any of the above aspects, the compound or salt of formula (I) or formula (II) can have at least one asymmetric carbon atom. When the compound or salt has at least one asymmetric carbon atom, the compound or salt can exist in the racemic form, in the form of its pure optical isomers, or in the form of a mixture wherein one isomer is enriched relative to the other. In particular, in accordance with the present invention, when the inventive compounds have a single asymmetric carbon atom, the inventive compounds may exist as racemates, i.e., as mixtures of equal amounts of optical isomers, i.e., equal amounts of two enantiomers, or in the form of a single enantiomer. As used herein, "single enantiomer" is intended to include a compound that comprises more than 50% of a single enantiomer (i.e., enantiomeric excess more than 60%, more than 70%, more than 80%, more than 90%, or up to 100% pure enantiomer).

When the compound or salt has more than one chiral center, the compound or salt can therefore exist as a mixture of diastereomers or in the form of a single diastereomer. As used herein, "single diastereomer" is intended to mean a compound that comprises more than 50% of a single diastereomer (i.e., diastereomeric excess more than 60%, more than 70%, more than 80%, more than 90%, or up to 100% pure diastereomer).

The phrase "pharmaceutically acceptable salt" is intended to include nontoxic salts synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, nonaqueous media such as ether, ethyl acetate, ethanol, isopropanol, or acetonitrile are preferred. Lists of suitable salts are found in Remington's Pharmaceutical Sciences, 18th ed., Mack Publishing Company, Easton, PA, 1990, p. 1445, and Berge, S. M., at al., Journal of Pharmaceutical Science, 66, 1-19 (1977).

Suitable bases include inorganic bases such as alkali and alkaline earth metal bases, e.g., those containing metallic cations such as sodium, potassium, magnesium, calcium and the like. Non-limiting examples of suitable bases include sodium hydroxide, potassium hydroxide, sodium carbonate, and potassium carbonate. Suitable acids include inorganic acids such as hydrochloric acid, hydrobromic acid, hydroiodic acid, sulfuric acid, phosphoric acid, and the like, and organic acids such as p-toluenesulfonic, methanesulfonic acid, benzenesulfonic acid, oxalic acid, p-bromophenylsulfonic acid, carbonic acid, succinic acid, citric acid, benzoic acid, acetic acid, maleic acid, tartaric acid, fatty acids, long chain fatty acids, and the like. Preferred pharmaceutically acceptable salts of inventive compounds having an acidic moiety include sodium and potassium salts. Preferred pharmaceutically acceptable salts of inventive compounds having a basic moiety (e.g., a dimethylaminoalkyl group) include hydrochloride and hydrobromide salts. The compounds of the present invention containing an acidic or basic moiety are useful in the form of the free base or acid or in the form of a pharmaceutically acceptable salt thereof.

It should be recognized that the particular counterion forming a part of any salt of this invention is usually not of a critical nature, so long as the salt as a whole is pharmacologically acceptable and as long as the counterion does not contribute undesired qualities to the salt as a whole.

It is further understood that the above compounds and salts may form solvates, or exist in a substantially uncomplexed form, such as the anhydrous form. As used herein, the term "solvate" refers to a molecular complex wherein the solvent molecule, such as the crystallizing solvent, is incorporated into the crystal lattice. When the solvent incorporated in the solvate is water, the molecular complex is called a hydrate. Pharmaceutically acceptable solvates include hydrates, alcoholates such as methanolates and ethanolates, acetonitrilates and the like. These compounds can also exist in polymorphic forms.

The present invention further provides a pharmaceutical composition comprising a compound as described above and a pharmaceutically acceptable carrier. The present invention provides a pharmaceutical composition comprising a pharmaceutically acceptable carrier and an effective amount, e.g., a therapeutically effective amount, including a prophylactically effective amount, of one or more of the aforesaid compounds, or salts thereof, of the present invention.

The pharmaceutically acceptable carrier can be any of those conventionally used and is limited only by chemico-physical considerations, such as solubility and lack of reactivity with the compound, and by the route of administration. It will be appreciated by one of skill in the art that, in addition to the following described pharmaceutical compositions; the compounds of the present invention can be formulated as inclusion complexes, such as cyclodextrin inclusion complexes, or liposomes.

The pharmaceutically acceptable carriers described herein, for example, vehicles, adjuvants, excipients, or diluents, are well known to those who are skilled in the art and are readily available to the public. It is preferred that the pharmaceutically acceptable carrier be one which is chemically inert to the active compounds and one which has no detrimental side effects or toxicity under the conditions of use.

The choice of carrier will be determined in part by the particular active agent, as well as by the particular method used to administer the composition. Accordingly, there is a wide variety of suitable formulations of the pharmaceutical composition of the present invention. The following formulations for oral, aerosol, parenteral, subcutaneous, intravenous, intraarterial, intramuscular, interperitoneal, intrathecal, rectal, and vaginal administration are merely exemplary and are in no way limiting.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such carriers as are known in the art.

The compounds of the present invention, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene-polypropylene copolymers, (d) amphoteric detergents such as, for example, alkyl-beta-aminopropionates, and 2-alkyl-imidazoline quaternary ammonium salts, and (3) mixtures thereof.

The parenteral formulations will typically contain from about 0.5 to about 25% by weight of the active ingredient in solution. Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampoules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

The compounds of the present invention may be made into injectable formulations. The requirements for effective pharmaceutical carriers for injectable compositions are well known to those of ordinary skill in the art. See Pharmaceutics and Pharmacy Practice, J. B. Lippincott Co., Philadelphia, Pa., Banker and Chalmers, eds., pages 238-250 (1982), andASHP Handbook on Injectable Drugs, Toissel, 4th ed., pages 622-630 (1986).

Topical formulations, including those that are useful for transdermal drug release, are well-known to those of skill in the art and are suitable in the context of the invention for application to skin. Topically applied compositions are generally in the form of liquids, creams, pastes, lotions and gels. Topical administration includes application to the oral mucosa, which includes the oral cavity, oral epithelium, palate, gingival, and the nasal mucosa. In some aspects, the composition contains at least one active component and a suitable vehicle or carrier. It may also contain other components, such as an anti-irritant. The carrier can be a liquid, solid or semisolid. In aspects, the composition is an aqueous solution. Alternatively, the composition can be a dispersion, emulsion, gel, lotion or cream vehicle for the various components. In one aspect, the primary vehicle is water or a biocompatible solvent that is substantially neutral or that has been rendered substantially neutral. The liquid vehicle can include other materials, such as buffers, alcohols, glycerin, and mineral oils with various emulsifiers or dispersing agents as known in the art to obtain the desired pH, consistency and viscosity. It is possible that the compositions can be produced as solids, such as powders or granules. The solids can be applied directly or dissolved in water or a biocompatible solvent prior to use to form a solution that is substantially neutral or that has been rendered substantially neutral and that can then be applied to the target site. In aspects of the invention, the vehicle for topical application to the skin can include water, buffered solutions, various alcohols, glycols such as glycerin, lipid materials such as fatty acids, mineral oils, phosphoglycerides, collagen, gelatin and silicone based materials.

Additionally, the compounds of the present invention may be made into suppositories by mixing with a variety of bases, such as emulsifying bases or water-soluble bases. Formulations suitable for vaginal administration may be presented as pessaries, tampons, creams, gels, pastes, foams, or spray formulas containing, in addition to the active ingredient, such carriers as are known in the art to be appropriate.

The dose administered to a mammal, particularly, a human, in accordance with the present invention should be sufficient to effect the desired response. Such responses include reversal or prevention of the adverse effects of the disease for which treatment is desired or to elicit the desired benefit. One skilled in the art will recognize that dosage will depend upon a variety of factors, including the age, condition, and body weight of the human, as well as the source, particular type of the disease, and extent of the disease in the human. The size of the dose will also be determined by the route, timing and frequency of administration as well as the existence, nature, and extent of any adverse side-effects that might accompany the administration of a particular compound and the desired physiological effect. It will be appreciated by one of skill in the art that various conditions or disease states may require prolonged treatment involving multiple administrations.

Suitable doses and dosage regimens can be determined by conventional range-finding techniques known to those of ordinary skill in the art. Generally, treatment is initiated with smaller dosages that are less than the optimum dose of the compound. Thereafter, the dosage is increased by small increments until the optimum effect under the circumstances is reached. The present inventive method typically will involve the administration of about 0.1 to about 300 mg of one or more of the compounds described above per kg body weight of the animal or mammal.

The therapeutically effective amount of the compound or compounds administered can vary depending upon the desired effects and the factors noted above. Typically, dosages will be between 0.01 mg/kg and 250 mg/kg of the subject's body weight, and more typically between about 0.05 mg/kg and 100 mg/kg, such as from about 0.2 to about 80 mg/kg, from about 5 to about 40 mg/kg or from about 10 to about 30 mg/kg of the subject's body weight. Thus, unit dosage forms can be formulated based upon the suitable ranges recited above and the subject's body weight. The term "unit dosage form" as used herein refers to a physically discrete unit of therapeutic agent appropriate for the subject to be treated.

Alternatively, dosages are calculated based on body surface area and from about 1 mg/m² to about 200 mg/m², such as from about 5 mg/m² to about 100 mg/m² will be administered to the subject per day. In particular aspects, administration of the therapeutically effective amount of the compound or compounds involves administering to the subject from about 5 mg/m² to about 50 mg/m², such as from about 10 mg/m² to about 40 mg/m² per day. It is currently believed that a single dosage of the compound or compounds is suitable, however a therapeutically effective dosage can be supplied over an extended period of time or in multiple doses per day. Thus, unit dosage forms also can be calculated using a subject's body surface area based on the suitable ranges recited above and the desired dosing schedule.

In certain aspects, the invention provides a method of antagonizing a P2Y₁₄R receptor in a mammal in need thereof, comprising administering to the mammal an effective amount of a compound of the invention or a pharmaceutically acceptable salt thereof.

In certain aspects, the invention provides a method of treating or preventing an inflammatory condition in a mammal in need thereof, comprising administering to the mammal an effective amount of a compound of the invention or a pharmaceutically acceptable salt thereof.

In these aspects, the inflammatory condition is selected from the group consisting of asthma, cystic fibrosis, and sterile inflammation of the kidney.

In certain aspects, the invention provides a method of treating pain in a mammal in need thereof, comprising administering to the mammal an effective amount a compound of the invention or a pharmaceutically acceptable salt thereof.

In certain aspects, the invention provides a compound of the invention or a pharmaceutically acceptable salt thereof, for use in antagonizing a P2Y₁₄R receptor in a mammal in need thereof.

In certain aspects, the invention provides a compound of the invention or a pharmaceutically acceptable salt thereof, for use in treating or preventing an inflammatory condition in a mammal in need thereof.

In these aspects, the inflammatory condition is selected from the group consisting of asthma, cystic fibrosis, and sterile inflammation of the kidney.

In certain aspects, the invention provides a compound of the invention or a pharmaceutically acceptable salt thereof, for use in treating pain in a mammal in need thereof.

### Chemistry

Schemes 1-14 depict exemplary syntheses of compounds of the invention.

Schemes 1A and 1B show synthesis of triazole-containing derivatives of the compound of formula (I) with acyl or acylamino substituents for R³. 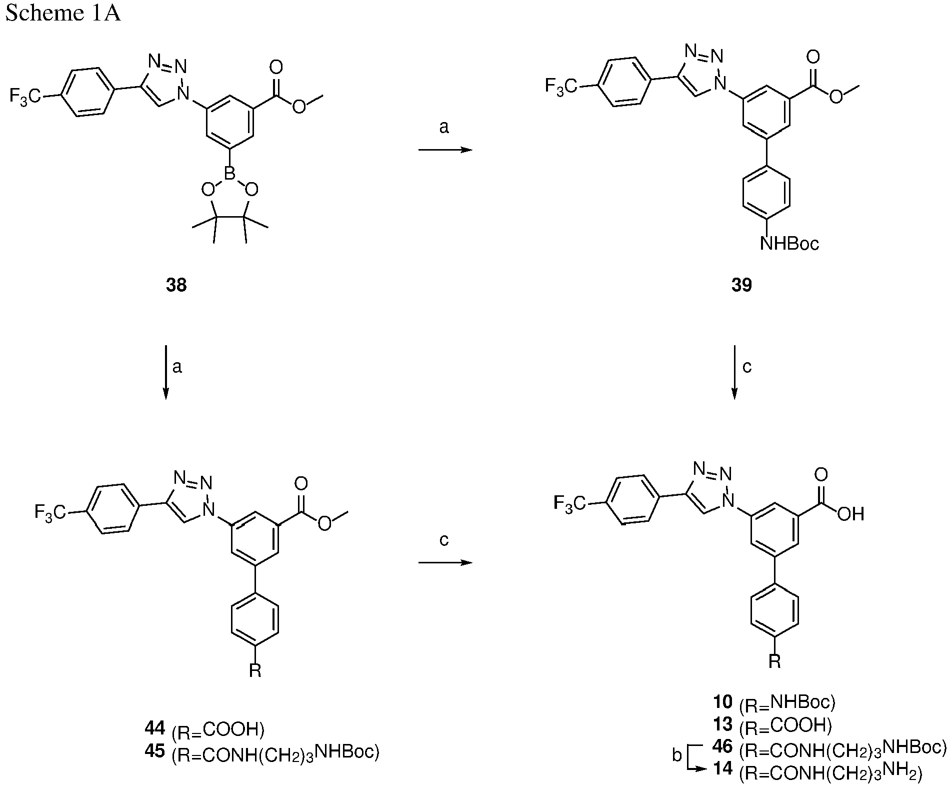 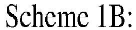 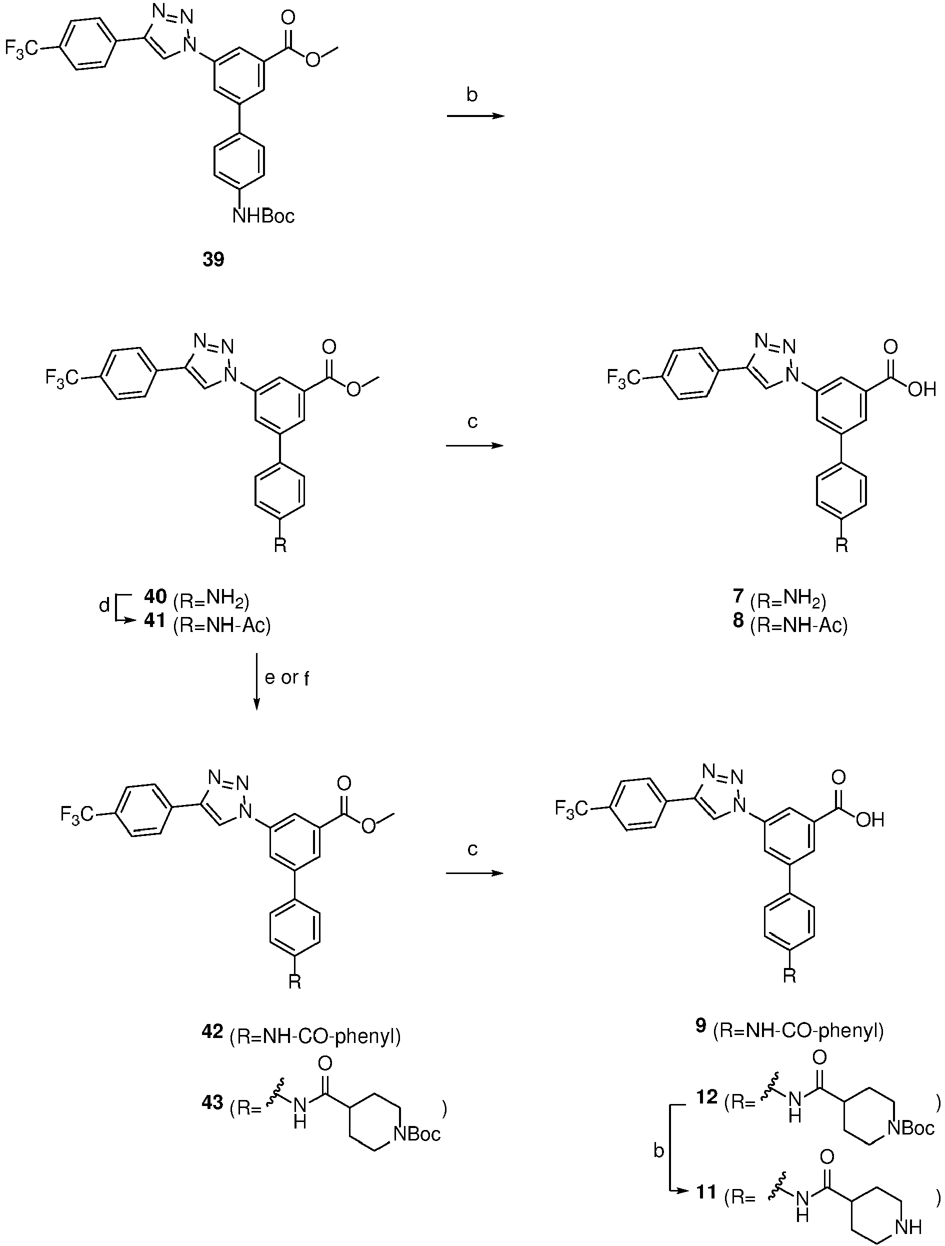 Reagents and conditions: (a) Pd(PPh₃)₄, K₂CO₃, DMF, 85 °C, 5 h, 40-68%; (b) TFA:THF = 2:1, rt, 0.5 h, 57-87%; (c) KOH, MeOH:H₂O = 2:1, 50 °C, 50-82%; (d) acetic anhydride, DCM, rt, 2 h, 76%; (e) benzoyl chloride, TEA, DCM, rt, 18 h, 63%; (f) Boc-isonipecotic acid (Boc-Inp-OH), HATU, DIPEA, DMF, rt, 2 h, 94%.

Scheme 2 shows synthesis of triazole-containing derivatives of the compound of formula (I) with acyl or acylamino substituents for R³.

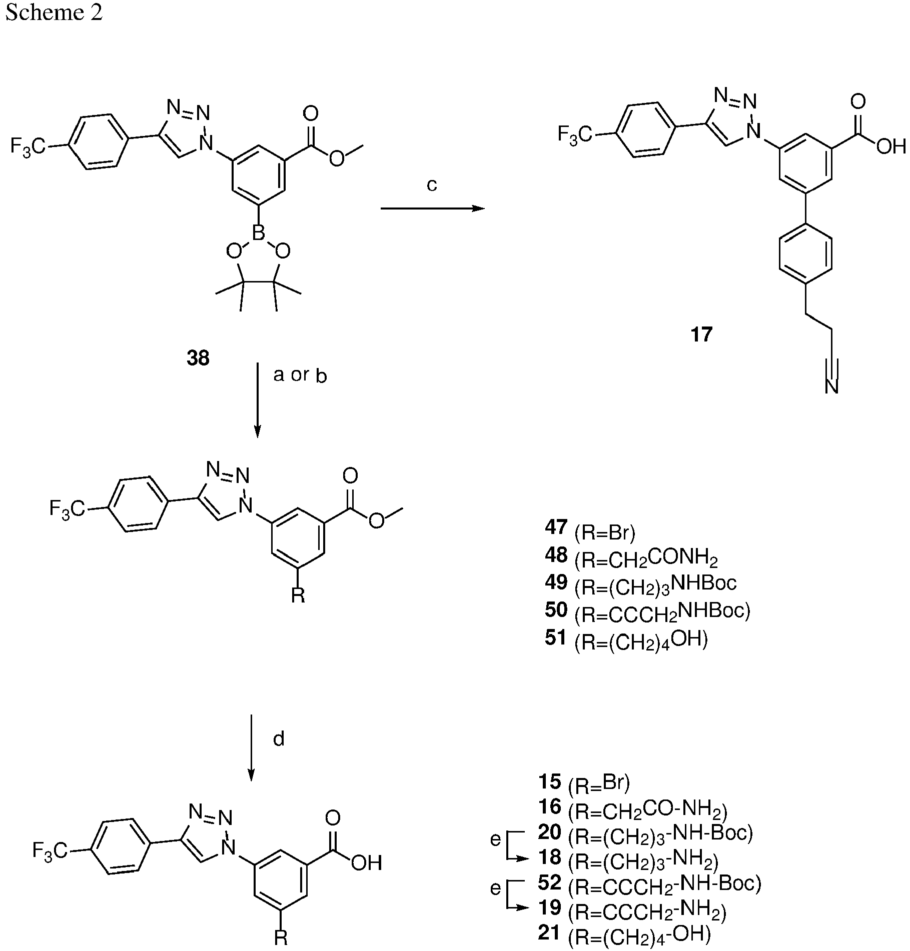

Reagents and conditions: (a) Pd(PPh₃)₄, K₂CO₃, DMF, 85 °C, 5 h, 27-55%; (b) PdCl₂(dppf), dimethoxyethane: Na₂CO₃ (aq, 2M) = 10:1, 60 °C, 4 h, 73%; (c) Pd(PPh₃)₄, K₂CO₃, DMF:Water = 1:1, 90 °C, 12 h, 64%; (d) KOH, MeOH:H₂O = 2:1, 50 °C, 39-88%; (e) TFA:THF = 2:1, rt, 0.5 h, 62-98%.

Scheme 3 shows synthesis of triazole-containing derivatives of the compound of formula (I) with cyclic groups for R³.

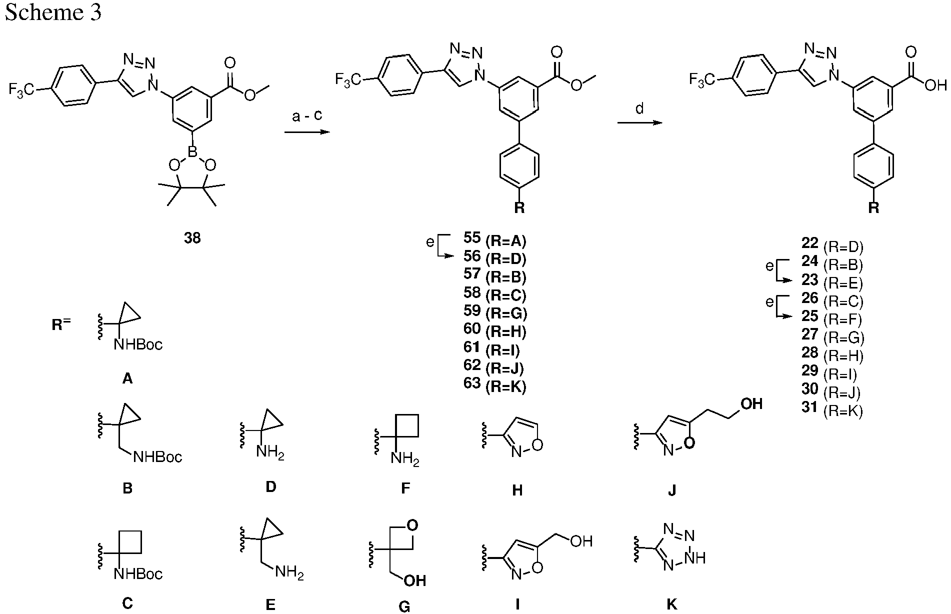

Reagents and conditions: (a) Pd(PPh₃)₄, K₂CO₃, DMF, 85 °C, 5 h, 46-91%; (b) Pd(PPh₃)₄, K₂CO₃, DMF, µW, 100 °C, 0.5 h, 80-91%; (c) Pd(PPh₃)₄, K₂CO₃, DMF:water = 1:1, 90 °C, 12 h, 30-70%; (d) KOH, MeOH:H₂O = 2:1, 50 °C, 43-74%; (e) TFA:THF = 2:1, rt, 0.5 h, 52-90%.

Scheme 4 shows a synthesis of naphthalene-containing isoxazole derivative 32.

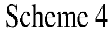

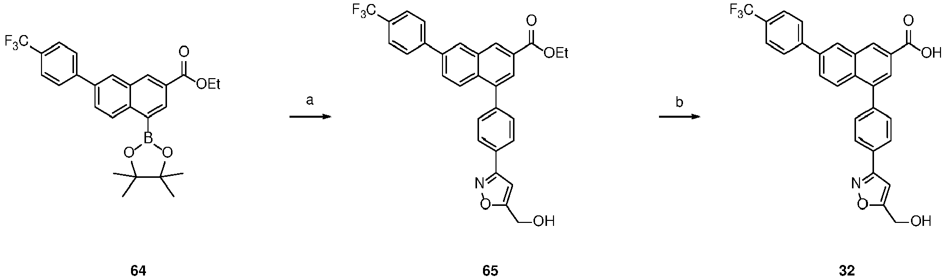

Reagents and conditions: (a) Pd(PPh₃)₄, K₂CO₃, DMF, µW, 100 °C, 1.5 h, 50%; (b) KOH, MeOH:H₂O = 2:1, 50 °C, 98%.

Scheme 5 shows a synthesis of amide-containing isoxazole derivative 33.

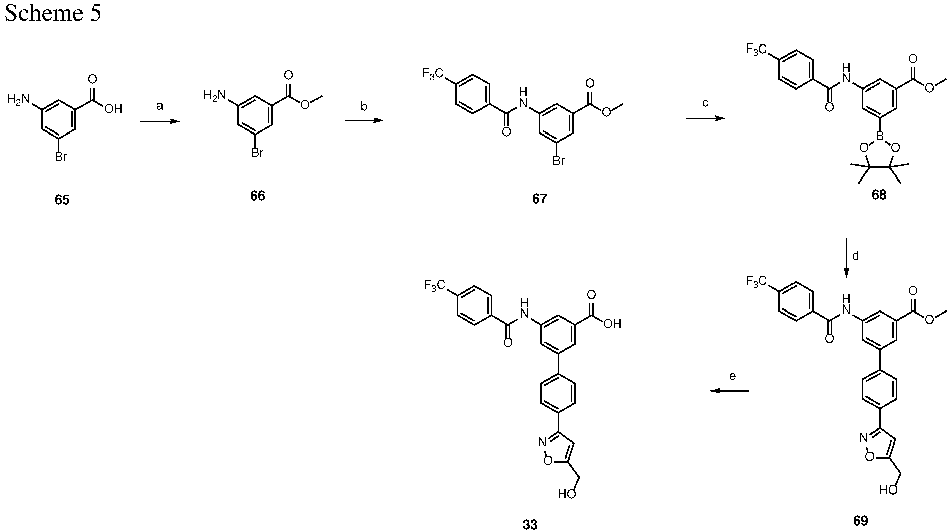

Reagents and Conditions: (a) SOCl₂, MeOH, rt, 15 h, 98%; (b) 4-(trifluoromethyl)benzoic acid, HATU, DIPEA, DMF, rt, 5 h, 70%; (c) B₂pin₂, PdCl₂(dppf), KOAc, dioxane, 70 °C, 6 h, 65%; (d) Pd(PPh₃)₄, K₂CO₃, DMF, 85 °C, 5 h, 45%; (e) KOH, MeOH, H₂O, 50 °C, 3 h, 62%.

Scheme 6 shows a synthesis of triazole-containing derivatives 34 - 36.

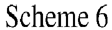

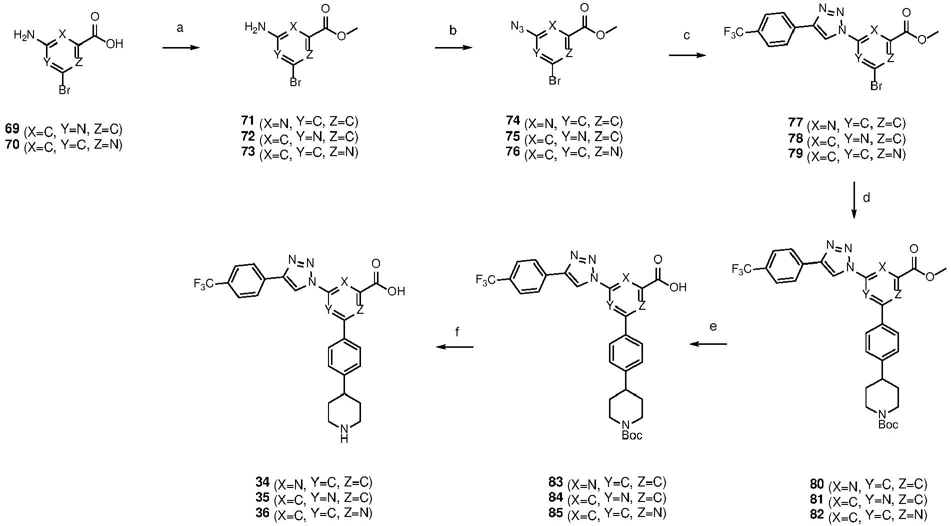

Reagents and conditions: (a) SOCl₂, MeOH, rt, 15 h, 40%; (b) tert-butyl nitrite, azidotrimethylsilane, acetonitrile, rt, 1 h, 37-65%; (c) 4-ethynyl-α,α,α-trifluorotoluene, CuSO₄·5H₂O, Na ascorbate, TBTA, *tert*-BuOH:H₂O=1:1, rt, 45 min, 36-69%; (d) *tert*-butyl 4-(4-(4,4,5,5-tetramethyl-1,3,2-dioxaborolan-2-yl)phenyl)piperidine-1-carboxylate, Pd(PPh₃)₄, K₂CO₃, DMF, 85 °C, 4 h, 35-91%; (e) KOH, MeOH:H₂O = 2:1, 50 °C, 5 h, 68-91%; (f) TFA:THF = 2:1, rt, 0.5 h, 60-65%.

Scheme 7 shows a synthesis of compounds **37a** - **37c.**

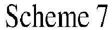

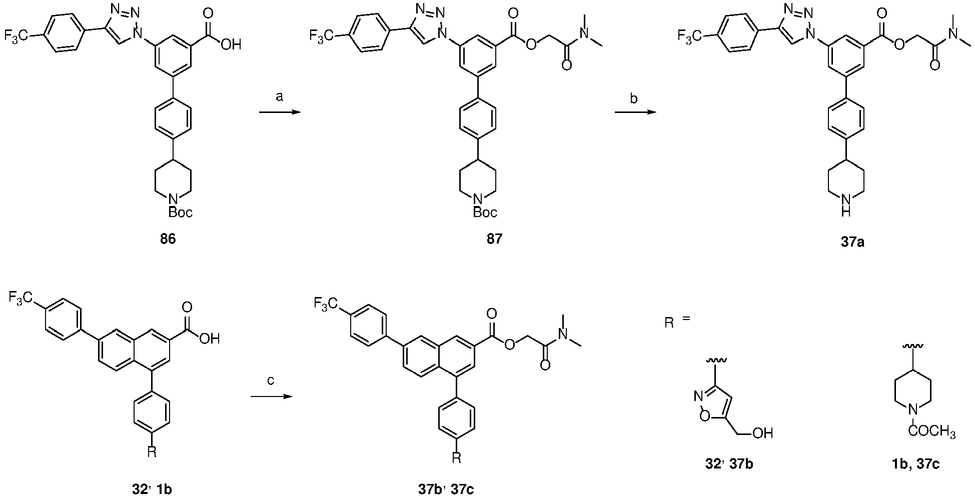

Reagents and conditions: (a) 2-chloro-N,N-dimethylacetamide, K₂CO₃, DMF, 45 °C, 1 h, 70-72%; (b) TFA:THF = 2:1, rt, 0.5 h, 78%; (c) 2-chloro-N,N-dimethylacetamide, Cs₂CO₃, DMF, 45 °C, 1 h, 68%.

Scheme 8 shows the preparation of 4-bromophenyl intermediates containing modified piperidine rings for Suzuki coupling.

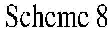

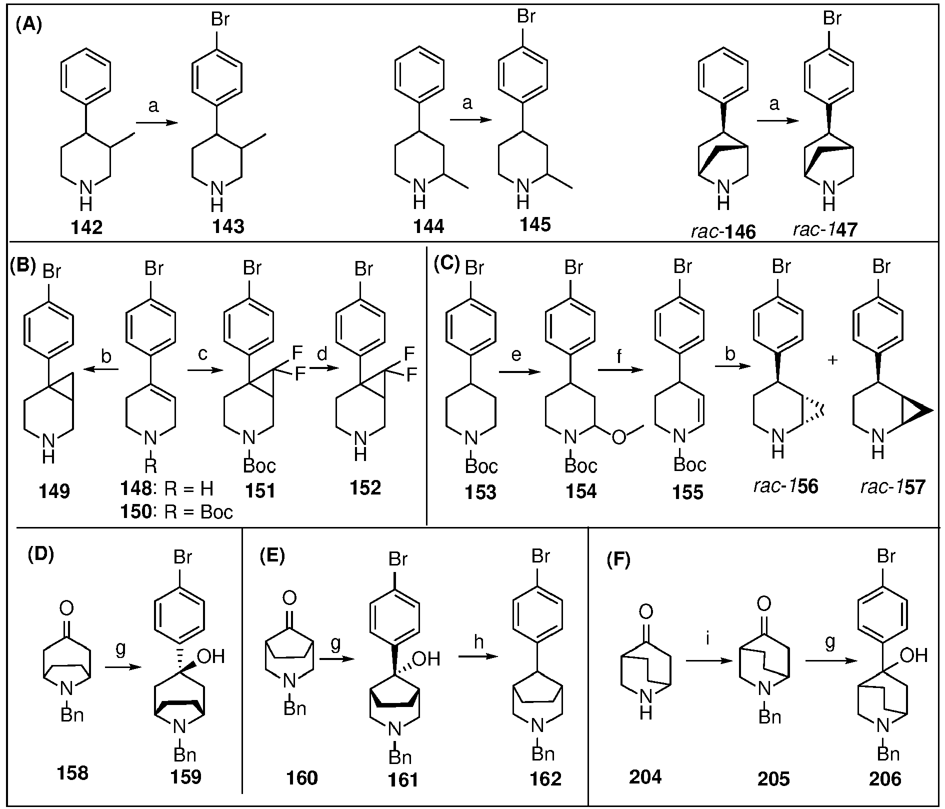

Reagents and conditions: (a) Br₂, AcOH, rt, 10 min, 30-62%; (b) Et₂Zn, CH₂I₂, DCM, Ar, rt, overnight, 60-62%; (c) TMSCF₃, NaI, THF, 65°C, overnight, 26%; (d) TFA, DCM, rt, 1 h, 75%; (e) (+) C-C (-) (4.5 V, 2 F/mol), MeOH, Et₄NOTs, 95%; (f) NH₄Cl, 100 °C, 73%; (g) *i*, dibromobenzene, magnesium, THF, sonication; *ii*, ketone, THF, 0 °C, 2 h; 42-86%; (h) H₂SO₄, 80 °C, 30 min, 15%. (i) K₂CO₃, PhCH₂Br, DMF, 70 °C.

Scheme 9 shows a general scheme for preparation of derivatives of the lead P2Y₁₄R antagonist, piperidine **1,** via Suzuki coupling followed by ester hydrolysis. Compound **63** is 3-(4-bromophenyl)pyrrolidine hydrochloride, starting material for **75.**

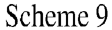

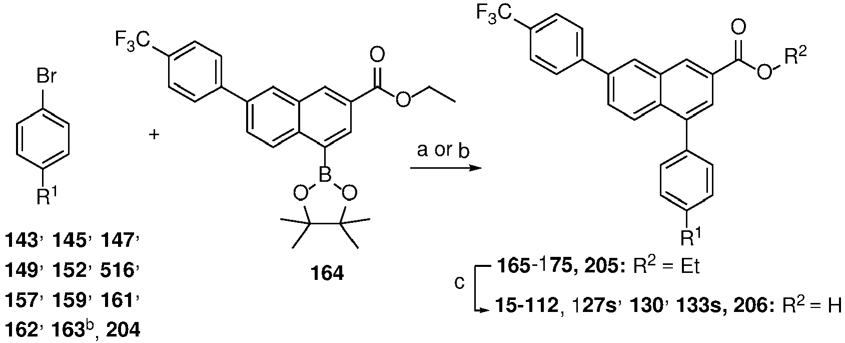

Reagents and conditions: (a) Pd(PPh₃)₄, K₂CO₃, DMF, MW, 150 °C, 30 min, 37-76%; (b) for substrate **56** and **57:** Pd(PPh₃)₄, aq. Na₂CO₃, DME-EtOH, 80 °C, 30 min, 25-40%; (c) MeOH, aq KOH, overnight, 42-76%.

Scheme 10 shows a preparation of bridged piperidine analogues **114** and **116** containing a 2-azanorbornane moiety, **119** and **121** containing a lactam; and ring-opened **134** and **136** containing 1-amino-3-hydroxymethylcyclopentane, and **138** and **140** containing 1-amino-3-carboxylcyclopentane. The synthesis maintained absolute stereochemistry originating from chiral bicyclic precursor **176.** 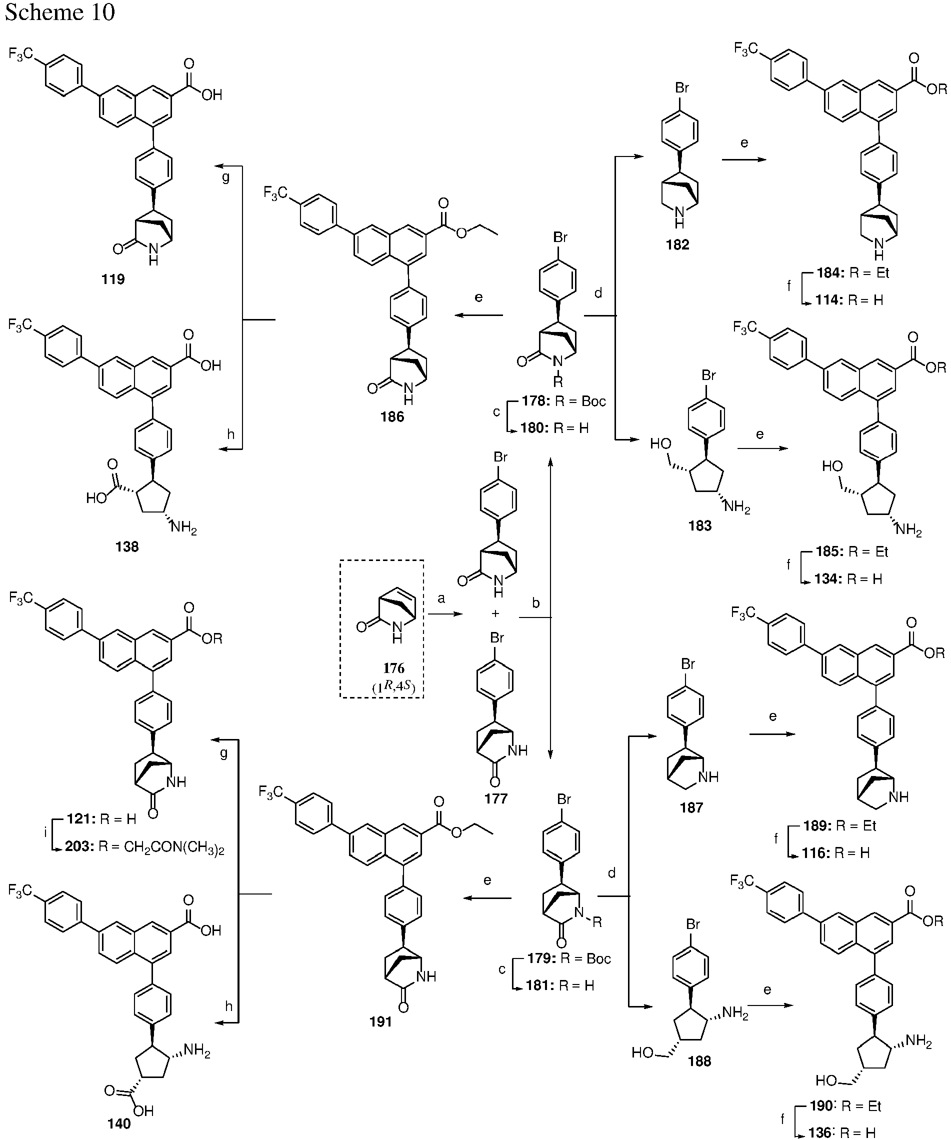 Reagents and conditions: (a) 1-bromo-4-iodobenzene, (PPh₃)₂PdCl₂, formic acid, TEA, THF, 65 °C overnight, quantitative; (b) TEA, DMAP, (Boc)₂O, DCM, rt, overnight. 46% **178** and 36% **179;** (c) TFA, DCM, rt, 1 h, 88-95%; (d) H₃N-BH₃, B(C₆F₅)₃, BF₃Et₂O, DCE, 75 °C, 24 h, 52% **182** and 48% **183;** (e) boronic acid pinacol ester (**164**), Na₂CO₃, Pd(PPh₃)₄, DME-H₂O (4:1), 85 °C, overnight, 68-93%; (f) LiOH, THF-MeOH-H₂O (3:1:1), rt, 3 h, 44-66%; (g) LiOH, THF-MeOH-H₂O (3:1:1), rt, 1.5 h, 40-62%; (h) LiOH, THF-MeOH-H₂O (3:1:1), rt, overnight, 73-77%. (i) 2-chloro-*N,N*-dimethylacetamide, Cs₂CO₃, DMF, 45 °C, 2 h, 86%.

Scheme 11 shows a preparation of analogues **115** and **117** with 2-azanorbornane, **120** and **122** with lactam, **135** and **137** with 1-amino-3-hydroxymethylcyclopentane, and **139** and **141** with 1-amino-3-carboxylcyclopentane with absolute stereochemistry from **192.**^{a}

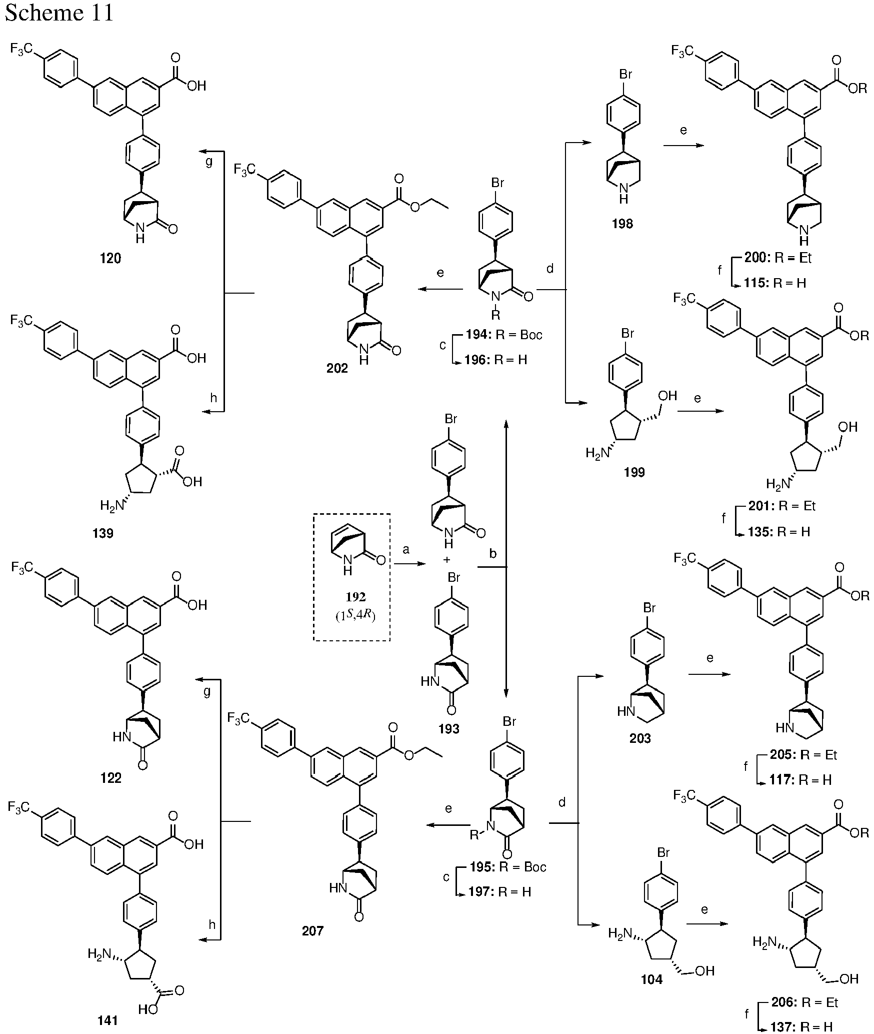

^{a}**Reagents and conditions:** (a) 1-Bromo-4-iodobenzene, (PPh₃)₂PdCl₂, formic acid, TEA, THF, 65 °C overnight, quantitative; (b) TEA, DMAP, (Boc)₂O, DCM, rt, overnight. 55% **194** and 41% **195;** (c) TFA, DCM, rt, 1 h, 78-79%; (d) H₃N-BH₃, B(C₆F₅)₃, BF₃Et₂O, DCE, 75 °C, 24 h, 51% **198** and 44% **199;** (e) **164,** Na₂CO₃, Pd(PPh₃)₄, DME-H₂O (4:1), 85 °C, overnight, 68-93%; (f) LiOH, THF-MeOH-H₂O (3:1:1), rt, 3 h, 44-66%; (g) LiOH, THF-MeOH-H₂O (3:1:1), rt, 1.5 h, 40-62%; (h) LiOH, THF-MeOH-H₂O (3:1:1), rt, overnight, 73-77%. (i) 2-chloro-*N,N-*dimethylacetamide, Cs₂CO₃, DMF, 45 °C, 2 h, 80%.

Scheme 12 shows a preparation of N-acetyl 2-azanorbornane derivatives.

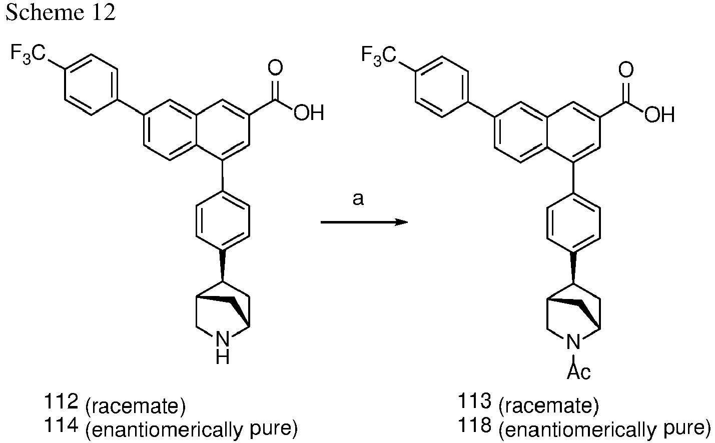

Reagents and Conditions: (a) Acetic anhydride, TEA, DMF, rt. 30 min, 53%.

Scheme 13 shows a preparation of nortropane derivatives from hydroxylated intermediate **127s.**

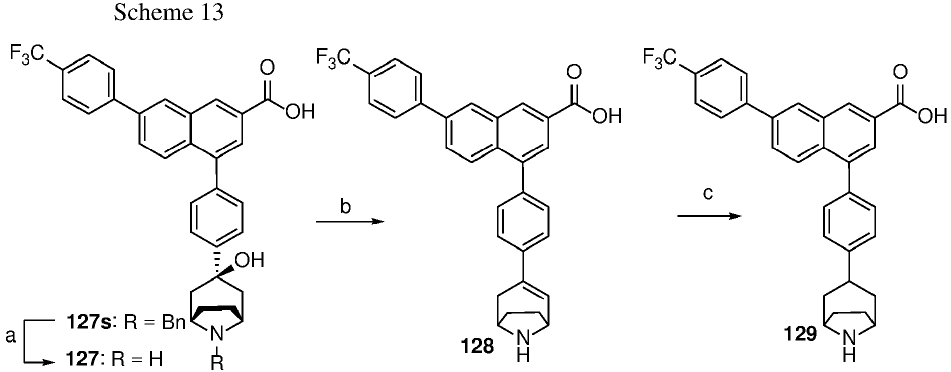

Reagents and conditions: (a) H₂, Pd/C, 150 psi, 7 h, 58%; (b) TFA, 90 °C, 2 h, 27%; (c) H₂, Pd/C, 100 psi, 3 h, overall yield 19% from **127s.**

Scheme 14 shows a preparation of isonortropane derivatives, including both hydroxylated **131, 132** and nonhydroxylated **133** and **133s** analogues.

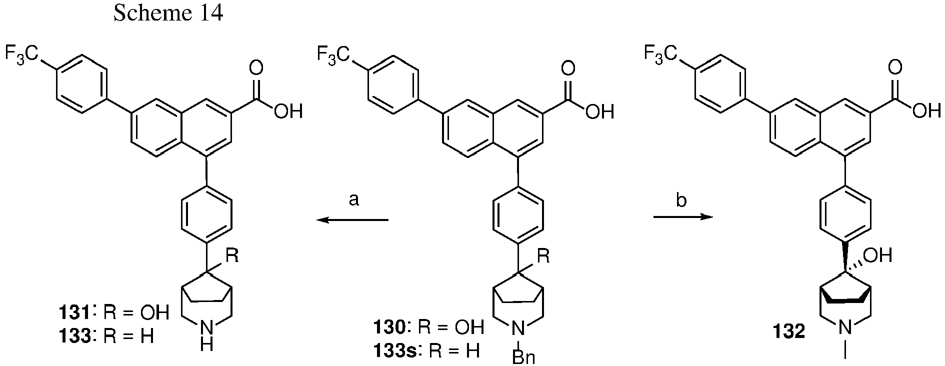

Reagents and conditions: (a) DMF, Pd/C, H₂ (70 psi), overnight, 61%; (b) MeOH, Pd/C, H₂ (125 psi), overnight, 20%.

Scheme 15 shows the preparation of isoquinuclidine derivatives from hydroxylated intermediate **206**.

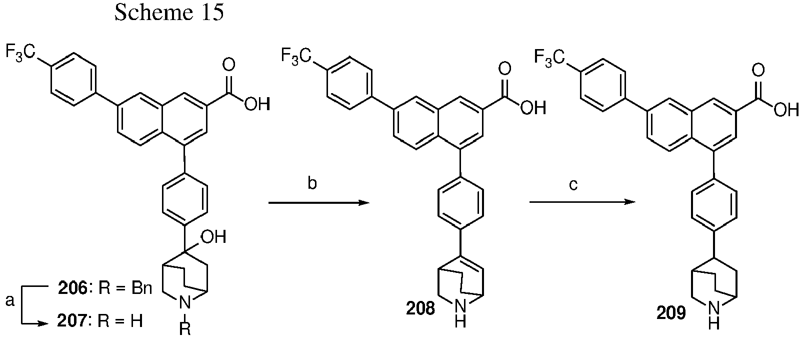

Reagents and conditions: (a) H₂, Pd/C, 150 psi, 7 h,; (b) TFA, 90 °C, 2 h,; (c) H₂, Pd/C, 100 psi, 3 h.

Scheme 16 shows a preparation of compounds **1c** and **1d**.

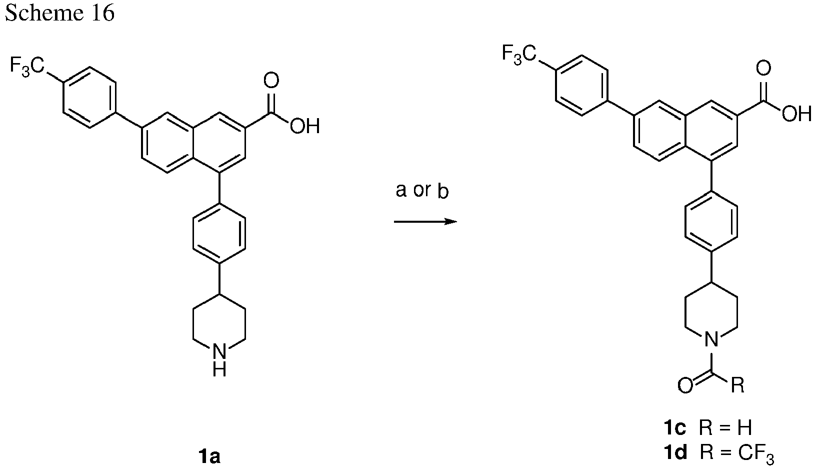

Reagents and conditions: **1c**: (a) acetic formic anhydride (from Ac₂O:formic acid = 2:1, v/v), 0 °C for 3 h → room temperature for 12 h, 71%. **1d**: (b) Trifluoroacetic anhydride, 0 °C for 1hr to rt for 2 hr.

Scheme 17 shows a preparation of phosphate **3b**.

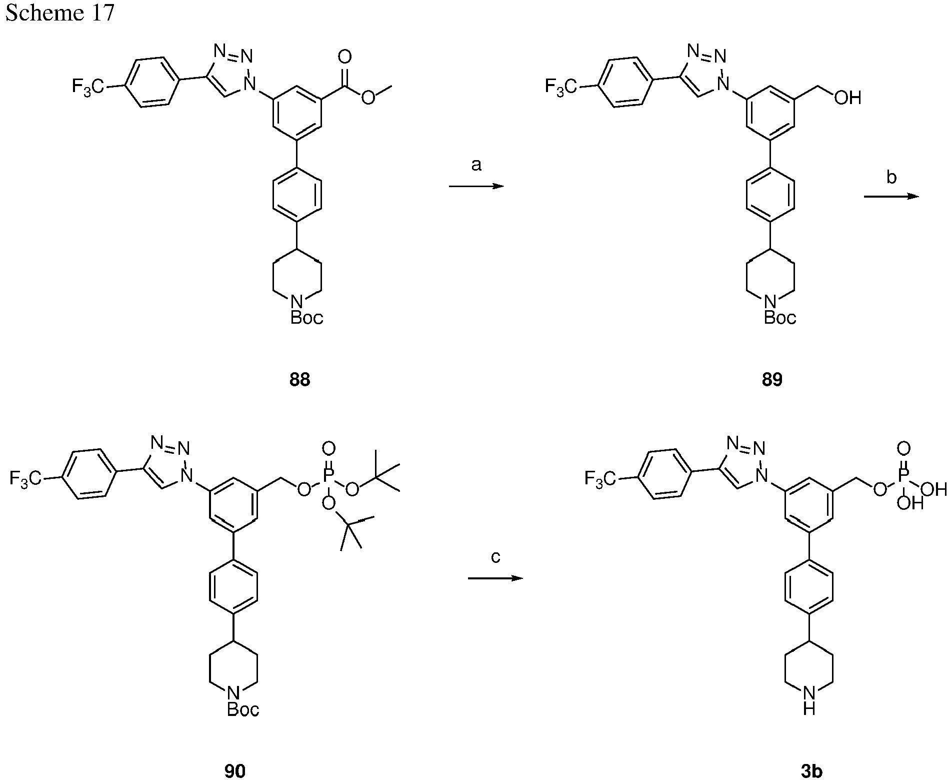

Reagents and Conditions: (a) LAH, THF, 0 °C, 1 h, 45%; (b) Di-*tert*-butyl *N,N-*diethylphosphoramidite, tetrazole, THF, rt, 1 h, then MCPBA, -78 °C, 84%; (c) TFA:THF = 2:1, rt, 2 h, 69%.

### Synthesis of compounds of formula (IV) or (V)b:

PPTN analogues with β-D-glucose bridged with triazole were prepared via click reaction (Scheme 18). The azide **523** was obtained from substitution of bromo starting material **522** with sodium azide. PPTN analogues **525-527** modified with alkynes tethered at the N of piperidine were prepared by reacting PPTN with iodo-substituted alkynes. The click reaction between alkynes **525-527** and azide **523** was catalyzed by CuSO₄/sodium ascorbate at 90 °C overnight and provided acetyl protected ligands **504, 506,** and **507,** which were further hydrolyzed to yield PPTN analogues **508**, **510**, and **511** with β-D-glucose bridged by various-length alkyltriazolyl linker, respectively.

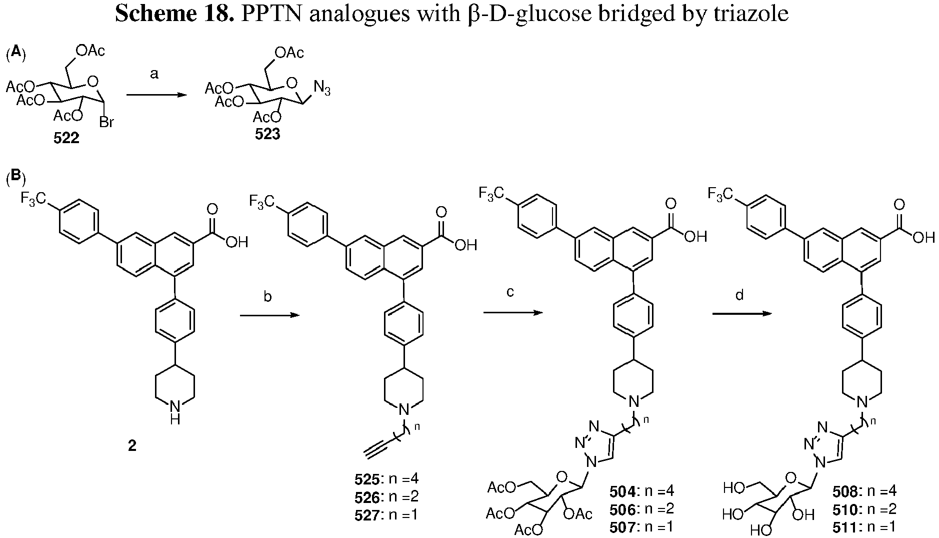

*^{a}*Reagents and conditions: (a) NaN₃, DMF, rt, 2 h, 99%; (b) alkynyl iodide, K₂CO₃, DMF, rt, overnight, 43%; (c) **523,** CuSO4.5H₂O, sodium ascorbate, DMF/H₂O (9:1), 90 °C, overnight, quantitative; (d) NaOH (3 M), MeOH, rt, overnight, 79%.

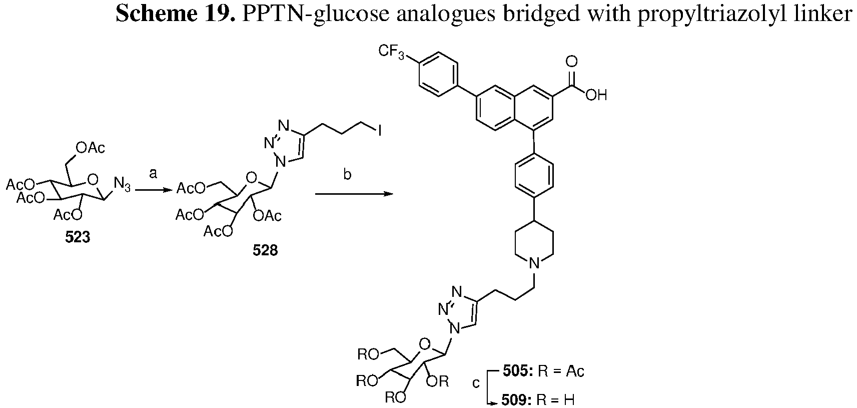

Reagents and conditions: (a) 5-iodo-1-pentyne, CuSO₄.5H₂O, sodium ascorbate, DMF/H₂O (9:1), 90 °C, overnight; (b) **502**, K₂CO₃, DMF, rt, overnight; (c) NaOH (3 M), MeOH, rt, overnight.

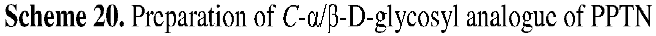

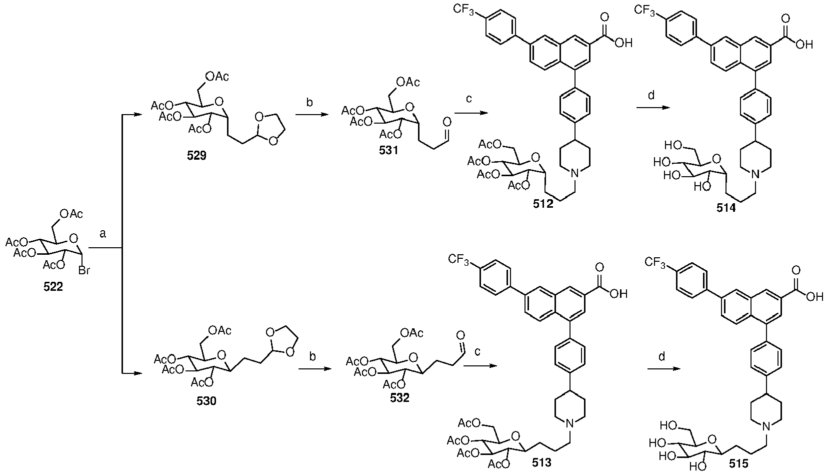

Reagents and conditions: (a) 2-vinyl-1,3-dioxolane, Bu₃SnH, AIBN, benzene, 75 °C; (b) In(OTf)₃, acetone, rt, overnight; (c) NaBH(OAc)₃, HOAc, DCM, 0-5 °C, 2 h; (d) NaOH (3 M), MeOH, rt, overnight.

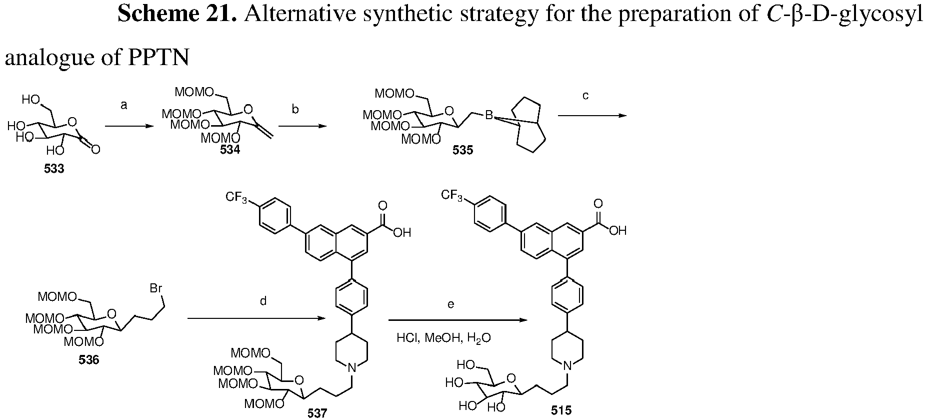

Reagents and conditions: (a) i) chloromethyl methyl ether, DIPEA, Bu₄NI, DCM, 0 °C to rt, 48 h; *ii*) dimethyltitanocene, toluene, 70 °C, 24 h; (b) 9-BBN-H, THF, reflux, 5 h; (c) 1-bromo-2-iodo-ethane, Pd(PPh₃)₄, K₂PO₄, dioxane, 60 °C, 24 h; (d) **2**, K₂CO₃, DMF, rt, overnight; (e) HCl, MeOH, H₂O, rt, overnight.

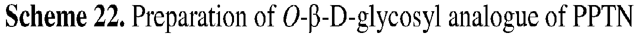

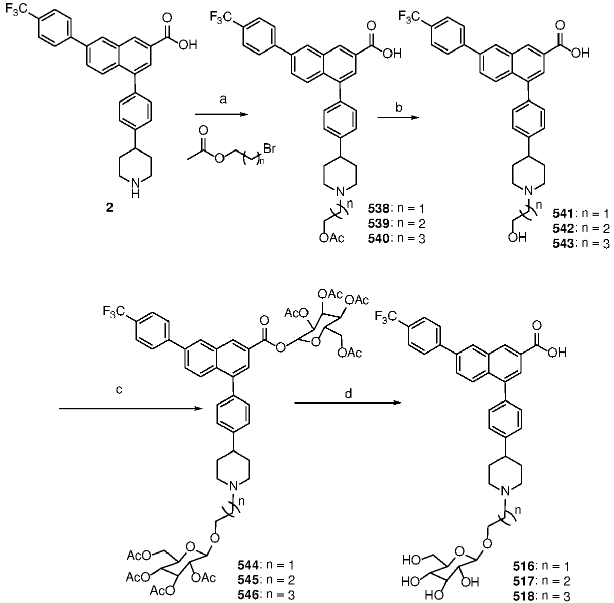

*^{a}*Reagents and conditions: (a) K₂CO₃, DMF, rt, overnight; (b) NaOH (3 M), MeOH, rt, overnight; (c) **522**, Ag₂O, TMSOTf, DMF, rt, 5 min; (d) NaOH (3 M), MeOH, rt, overnight.

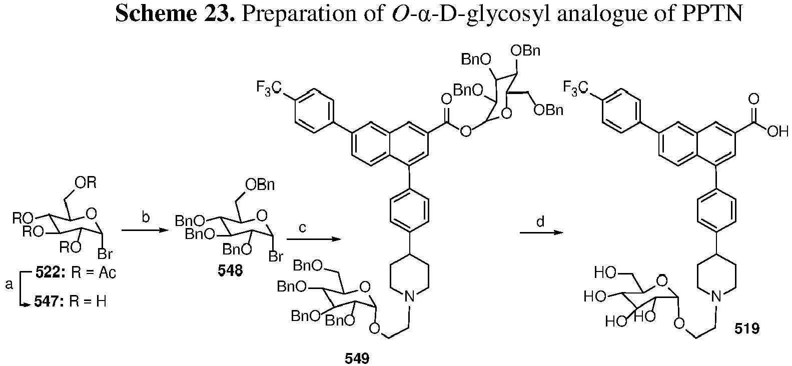

Reagents and conditions: (a) NaOH (3 M), MeOH, rt, overnight, (b) benzyl bromide, acetonitrile, reflux, 5 h; (c) **541**, Ag₂O, TMSOTf, DMF, rt, 5 min; (d) i) H₂, Pd/C, DMF, 5 h; *ii*) NaOH (3 M), MeOH, rt, overnight

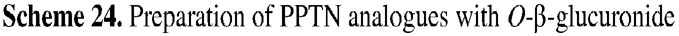

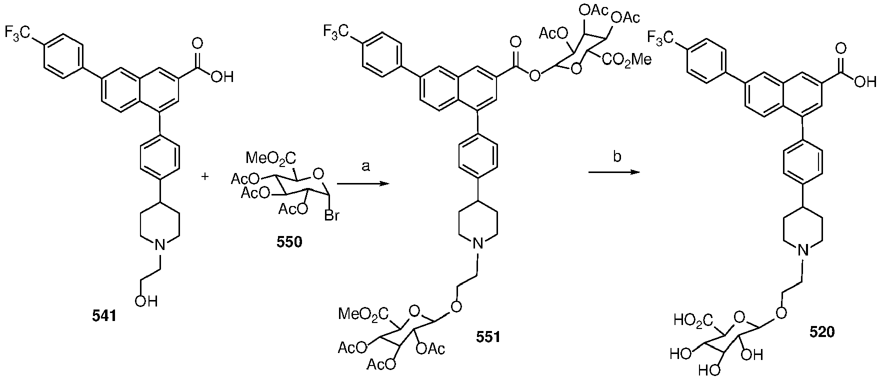

Reagents and conditions: (a) Ag₂O, TMSOTf, DMF, rt, 5 min; (b) NaOH (3 M), MeOH, rt, overnight.

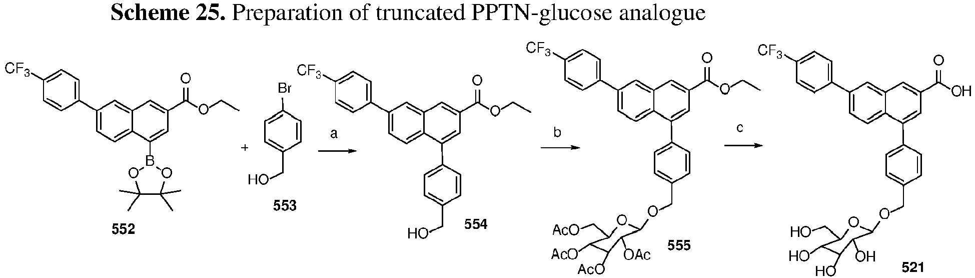

Reagents and conditions: (a) Na₂CO₃, Pd(PPh₃)₄, DME-H₂O (4:1), 85 °C, overnight; (b) **522**, Ag₂O, TMSOTf, DMF, rt, 5 min; (c) NaOH (3 M), MeOH, rt, overnight.

### EXAMPLES

The following examples further illustrate the invention but, of course, should not be construed as in any way limiting its scope.

### Pharmacological Characterization

The inhibition by the compounds in accordance with aspects of the invention of the binding of fluorescent tracer **2** was measured in hP2Y₁₄R-expressing CHO (references 1 and 2).

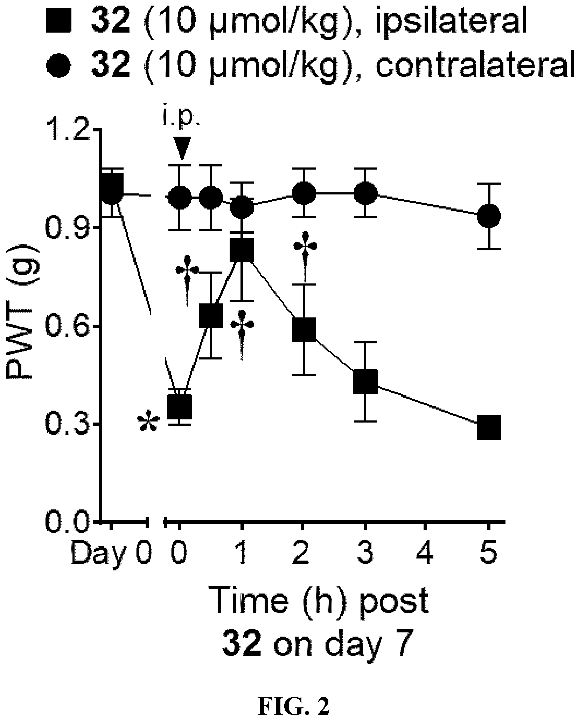

Therefore, several of the compounds (**11**, **19**, **29**, **32**) were compared in affinity at hP2Y₁₄R and mP2Y₁₄R (Figure 2) using the fluorescent binding procedures.²

The solubility (using the pION method)^{2,25,26} and lipophilicity (based on the HPLC retention time)^{2,27} were determined for the 5-(hydroxymethyl)isoxazol-3-yl compounds **29** and **35.**

Absorption, distribution, metabolism, excretion and toxicology (ADMET) properties were determined for compound **32** (Tables 4 and 5), by the same methods as in Jung et al.²

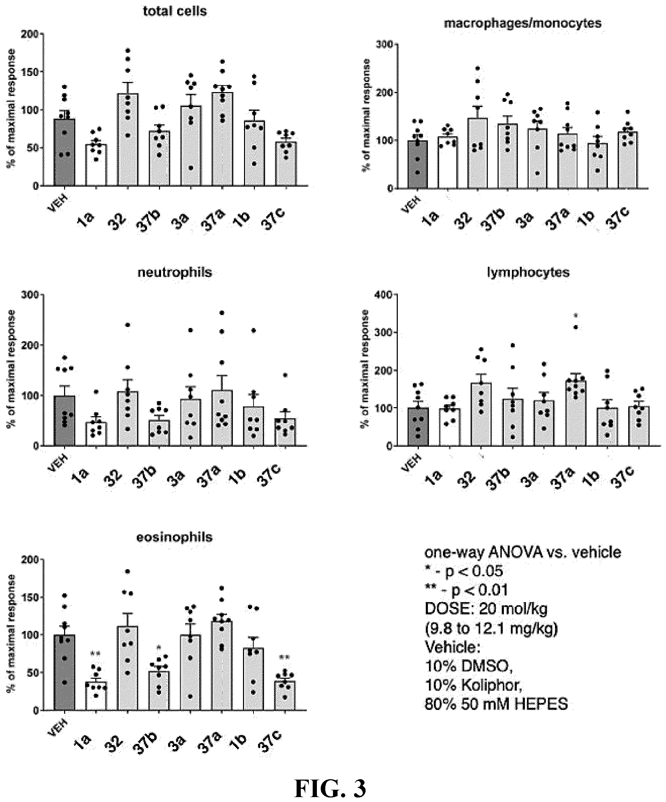

The potent 5-(hydroxymethyl)isoxazol-3-yl derivative **32** was first tested in a well-established mouse model of neuropathic pain caused by chronic constriction of the sciatic nerve (CCI)³² at a dose of 10 µmol/ kg (4.9 mg/kg, i.p., Fig. 3). Previously, it was demonstrated that **1a**, **1b**, **3a** and other P2Y₁₄R antagonists of this series were effective in reducing chronic pain in this model and reached full reversal in some cases (Table S3, Supporting information).^{2,7} A maximal 71.0±17.4% reversal of CCI-induced mechano-allodynia was observed 1 h post-injection of **32**, and the degree of protection declined during the following 2 h and was not statistically significant at 3 or 5 h. No side effects were evident at this dose.

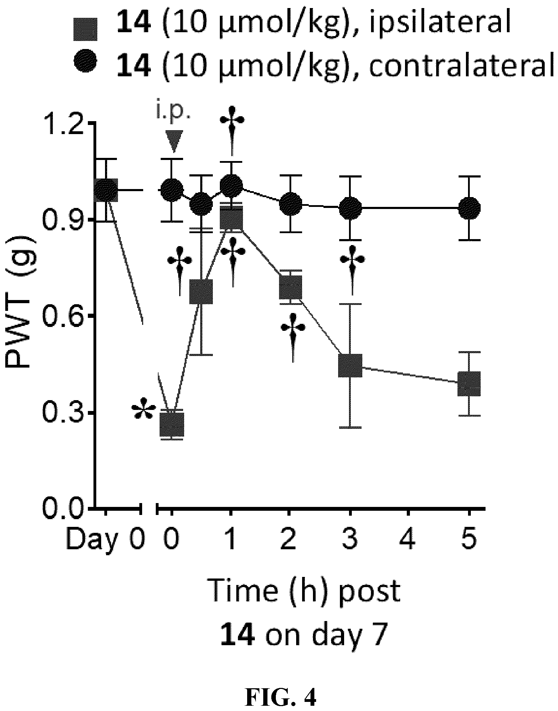

Selected compounds were examined for their ability to reduce eosinophils in the bronchoalveolar lavage fluid (BALF) in an ovalbumin/*Aspergillus* mouse asthma model.^{2,17,33} Compounds **1a**, **1b, 3a** and **32** (10 mg/kg) were administered prior to allergen challenge at a dose of 20 µmol/kg, i.p. Airway inflammation was determined two days post-challenge (Fig. 4). Also, the corresponding prodrug derivatives **37a-37c** of triazole **3a**, 5-(hydroxymethyl)isoxazole **32** and N-acetyl-piperidine **1b** derivatives, respectively, were administered at the same dose. Eosinophil counts in the BALF were reduced following administration of reference antagonist **1a** or several of the prodrugs. In fact, **37b** and **37c** significantly reduced eosinophils to a greater extent than the parent antagonists, which showed no significant reduction. Other immune cells showed no significant effect, except the lymphocyte count following administration of prodrug **37a** trended higher than in vehicle control.

### EXAMPLE 1

This example demonstrates synthesis of compounds, in accordance with aspects of the invention.

### General procedures

### Deprotection reactions:

**Method A**: A mixture of compound (1 eq) and potassium hydroxide (5 eq) in methanol:water (2:1) was stirred at 50 °C. This mixture was neutralized with 1N HCl until pH was 5-6. The slightly acidic mixture was evaporated under reduced pressure and purified by silica gel column chromatography (dichloromethane:methanol:acetic acid=95:5:0.1) or semipreparative HPLC (10 mM triethylammonium acetate buffer:acetonitrile=80:20 to 20:80 in 40 min) to afford the compound as a white solid.

**Method B**: A solution of compound in trifluoroacetic acid:tetrahydrofuran (2:1) was stirred at room temperature. The solvent was evaporated with toluene under reduced pressure. The residue was purified by silica gel column chromatography (dichloromethane:methanol=95:5) or semipreparative HPLC (10 mM triethylammonium acetate buffer:acetonitrile=80:20 to 20:80 in 40 min) to afford the compound as a white solid.

### General procedure: Suzuki reaction

### Suzuki coupling:

**Method C**: A mixture of compound **38** (1 eq), Pd(PPh₃)₄ (0.06 eq) and potassium carbonate (3 eq) in *N,N*-dimethylformamide was purged with nitrogen gas for 15 min, and then various aryl halides (1.2 eq) was added to the mixture. The mixture was stirred at 85 °C for 5 h, and then allowed to be cooled at room temperature. This mixture was partitioned ethyl acetate (5 mL) and water (10 mL). The aqueous layer was extracted with ethyl acetate (5 mL x 2), and the combined organic layer was washed with brine (3 mL), dried (MgSO₄ or Na₂SO₄), filtered and evaporated under reduced pressure. The residue was purified by silica gel column chromatography to afford the compound as a white solid.

**Method D**: The mixture of compound **38** (1 eq) and aryl halide (1.2 eq) in dimethoxyethane/2M Na₂CO₃ aqueous solution (10:1) was purged with nitrogen gas for 30 min, and then PdCl₂(dppf) (0.1 eq) was added to the mixture. The mixture was stirred at 60 °C for 4 h. After cooling at room temperature, this mixture was partitioned ethyl acetate (5 mL) and water (10 mL). The aqueous layer was extracted with ethyl acetate (5 mL x 2), and then the combined organic layer was washed with brine (3 mL), dried (MgSO₄ or Na₂SO₄), filtered and evaporated under reduced pressure. The residue was purified by silica gel column chromatography to afford the compound as a white solid.

**Method E**: A mixture of compound **38** (1 eq) and aryl bromide (1.2 eq) in DMF/H₂O (10:1; 20 mM) was purged with nitrogen gas for 30 min, and then Pd(PPh₃)₄ (0.05 eq) and Na₂CO₃ (3 eq) were added to the mixture, which was stirred for 1 h at 40-120 °C. The aqueous layer was extracted with ethyl acetate twice, and the combined organic layer was washed with brine, dried (MgSO₄ or Na₂SO₄), filtered, and evaporated under reduced pressure. The residue was purified by silica gel column chromatography to afford the compound as white solids.

**Method F**: The mixture of compound **38** (1 eq), Pd(PPh₃)₄ (0.06 eq), potassium carbonate (3 eq) and various aryl halides (1.2 eq) in *N,N*-dimethylformamide was purged with nitrogen gas for 15 min. The mixture was stirred at 100 °C for 30-90 min in microwave. After microwave irradiation, and then allowed to be cooled at room temperature. This mixture was partitioned ethyl acetate (5 mL) and water (10 mL). The aqueous layer was extracted with ethyl acetate (5 mL x 2), and the combined organic layer was washed with brine (3 mL), dried (Na₂SO₄), filtered, and evaporated under reduced pressure. The residue was purified by silica gel column chromatography to afford the compound as a white solid.

4-(4-(1-Formylpiperidin-4-yl)phenyl)-7-(4-(trifluoromethyl)phenyl)-2-naphthoic acid (**1c**).

To a solution of acetic formic anhydride (1 mL from Ac₂O and formic acid = 2:1, v/v) was slowly added compound **1a** (10 mg, 0.019 mmol) at 0 °C. After complete addition, cooling was continued for 3 h, and the mixture was then stirred at rt for 12 h. The reaction mixture was evaporated under reduced pressure. The residue was purified by silica gel column chromatography (dichloromethane:methanol = 50:1) to afford compound **1c** (7.0 mg, 71%) as a white solid; HPLC purity 98% (Rt = 14.62 min); ¹H NMR (400 MHz, DMSO-*d*₆) δ 8.73 (s, 1H), 8.64 (s, 1H), 8.08-7.96 (m, 5H), 7.90-7.88 (m, 3H), 7.50-7.44 (m, 4H), 4.37-4.34 (m, 1H), 3.85-3.81 (m, 1H), 3.22-3.16 (m, 1H), 2.97-2.90 (m, 1H), 2.77-2.70 (m, 1H), 1.96-1.89 (m, 2H), 1.68-1.47 (m, 2H); MS (ESI, m/z) 504.2 [M+1]⁺; ESI-HRMS calcd. m/z for C₃₀H₂₅NO₃F₃ 503.1787, found 504.1792 [M+1]⁺.

4-(4-(1-(2,2,2-trifluoroacetyl)piperidin-4-yl)phenyl)-7-(4-(trifluoromethyl)phenyl)-2-naphthoic acid (**1d**).

To trifluoroacetic anhydride (100 µL) was slowly added compound **1a** (10 mg, 0.019 mmol) at 0 °C. After complete addition, cooling was continued for 1 h, and the mixture was then stirred at rt for 2 h. The reaction mixture was evaporated under reduced pressure. The residue was purified by silica gel column chromatography (dichloromethane:methanol = 50:1) to afford compound **1d** (10.0 mg, 92%) as a white solid; HPLC purity 99% (Rt = 16.92 min); ¹H NMR (400 MHz, , CDCl₃) δ 8.80 (s, 1H), 8.27 (s, 1H), 8.12-8.07 (m, 2H), 7.86-7.77 (m, 5H), 7.53 (d, *J* = 8.00 Hz, 2H), 7.39 (d, *J* = 8.00 Hz, 2H), 4.81-4.78 (m, 1H), 4.25-4.22 (m, 1H), 3.37-3.30 (m, 1H), 3.01-2.92 (m, 2H), 2.15-2.11 (m, 2H), 1.91-1.81 (m, 2H); MS (ESI, m/z) 570.1 [M-1]⁻, ESI-HRMS calcd. m/z for C₃₁H₂₂NO₃F₆ 570.1504, found 570.1493 [M-1]⁻.

(4'-(Piperidin-4-yl)-5-(4-(4-(trifluoromethyl)phenyl)-1*H*-1,2,3-triazol-1-yl)-[1,1'-biphenyl]-3-yl)methyl dihydrogen phosphate (**3b**).

Method B: Yield 69%; HPLC purity 96% (Rt = 8.58 min); ¹H NMR (400 MHz, DMSO-*d*₆) δ 9.20-9.19 (m, 1H), 8.05-8.03 (m, 2H), 7.96 (s, 1H), 7.85 (s, 1H), 7.79 (d, *J* = 8.00 Hz, 2H), 7.74 (s, 1H), 7.67 (d, *J* = 8.40 Hz, 2H), 7.34 (d, *J* = 7.60 Hz, 2H), 4.82 - 4.80 (m, 2H), 2.96 -2.92 (m, 2H), 2.66 - 2.58 (m, 3H), 1.71-1.67 (m, 2H), 1.56-1.49 (m, 2H); MS (ESI, m/z) 559.2 [M+1]⁺; ESI-HRMS calcd. m/z for C₂₇H₂₇N₄O₄F₃P 559.1722, found 559.1716 [M+1]⁺.

4'-Amino-5-(4-(4-(trifluoromethyl)phenyl)-1*H*-1,2,3-triazol-1-yl)-[1,1'-biphenyl]-3-carboxylic acid (7).

Method A: Yield 62%; HPLC purity 97% (Rt = 8.96 min); ¹H NMR (400 MHz, CD₃OD) δ 9.25 (s, 1H), 8.48 (s, 1H), 8.37 (s, 2H), 8.16 (d, *J* = 8.00 Hz, 2H), 7.79 (d, *J* = 8.00 Hz, 2H), 7.73 (d, *J* = 8.40 Hz, 2H), 7.62 (d, *J* = 8.40 Hz, 2H); MS (ESI, m/z) 425.1 [M+1]⁺; ESI-HRMS calcd. m/z for C₂₂H₁₆N₄O₂F₃ 425.1225, found 425.1221 [M+1]⁺.

4'-Acetamido-5-(4-(4-(trifluoromethyl)phenyl)-1*H*-1,2,3-triazol-1-yl)-[1,1'-biphenyl]-3-carboxylic acid (**8**).

Method A: Yield 76%; HPLC purity 99% (Rt = 7.95 min); ¹H NMR (400 MHz, CD₃OD) δ 9.21 (s, 1H), 8.46 (s, 1H), 8.34 (d, *J* =7.20 Hz, 2H), 8.13 (d, *J* =8.00 Hz, 2H), 7.76 (d, *J* =8.40 Hz, 2H), 7.72 (s, 4H), 2.15 (s, 3H); MS (ESI, m/z) 467.1 [M+1]⁺; ESI-HRMS calcd. m/z for C₂₄H₁₈N₄O₃F₃ 467.1331, found 467.1330 [M+1]⁺.

4'-Benzamido-5-(4-(4-(trifluoromethyl)phenyl)-1*H*-1,2,3-triazol-1-yl)-[1,1'-biphenyl]-3-carboxylic acid (**9**).

Method A: Yield 73%; HPLC purity 99% (Rt = 9.48 min); ¹H NMR (400 MHz, DMSO-*d*₆) δ 9.70 (s, 1H), 8.41 (s, 1H), 8.29 (broad s, 2H), 8.21 (d, *J* = 7.60 Hz, 2H), 8.00-7.97 (m, 4H), 7.90-7.83 (m, 4H), 7.62-7.54 (m, 3H); MS (ESI, m/z) 529.1 [M+1]⁺; ESI-HRMS calcd. m/z for C₂₉H₂₀N₄O₃F₃ 529.1488, found 529.1489 [M+1]⁺.

4'-((*tert*-Butoxycarbonyl)amino)-5-(4-(4-(trifluoromethyl)phenyl)-1*H*-1,2,3-triazol-1-yl)-[1,1'-biphenyl]-3-carboxylic acid (**10**).

Method A: Yield 50%; HPLC purity 99% (Rt = 10.36 min); ¹H NMR (400 MHz, CD₃OD) δ 9.25 (s, 1H), 8.48 (s, 1H), 8.37-8.35 (m, 2H), 8.16 (d, *J* =8.40 Hz, 2H), 7.78 (d, *J* =8.00 Hz, 2H), 7.71 (d, *J* =8.40 Hz, 2H), 7.58 (d, *J* = 8.40 Hz, 2H), 1.54 (s, 9H); MS (ESI, m/z) 525.2 [M+1]⁺; ESI-HRMS calcd. m/z for C₂₇H₂₄N₄O₄F₃ 525.1750, found 525.1755 [M+1]⁺.

4'-(Piperidine-4-carboxamido)-5-(4-(4-(trifluoromethyl)phenyl)-1H-1,2,3-triazol-1-yl)-[1,1'-biphenyl]-3-carboxylic acid (**11**).

Method B: Yield 87%; HPLC purity 99% (Rt = 21.20 min); ¹H NMR (400 MHz, DMSO-*d*₆) δ 8.03-8.01 (m, 4H), 7.83 (s, 1H), 7.68 (d, *J* =8.40 Hz, 2H), 7.30-7.25 (m, 5H), 2.70-2.68 (m, 2H), 2.23-2.20 (m, 2H), 2.00-1.93 (m, 1H), 1.44-1.27 (m, 4H); MS (ESI, m/z) 536.2 [M+Na]⁺; ESI-HRMS calcd. m/z for C₂₈H₂₅N₅O₃F₃ 536.1909, found 536.1913 [M+1]⁺.

4'-(1-(tert-Butoxycarbonyl)piperidine-4-carboxamido)-5-(4-(4-(trifluoromethyl)phenyl)-1*H*-1,2,3-triazol-1-yl)-[1,1'-biphenyl]-3-carboxylic acid (12).

Method A: Yield 82%; HPLC purity 98% (Rt = 24.10 min); ¹H NMR (400 MHz, DMSO-*d*₆) δ 9.73 (s, 1H), 8.43 (s, 1H), 8.38 (s, 1H), 8.27 (s, 1H), 8.20 (d, *J* =8.00 Hz, 2H), 7.90 (d, *J* =8.40 Hz, 2H), 7.91-7.77 (m, 4H), 4.05-3.97 (m, 2H), 2.92-2.75 (m, 3H), 1.85-1.76 (m, 2H), 1.56-1.45 (m, 2H), 1.41 (s, 9H); MS (ESI, m/z) 658.2 [M+Na]⁺; ESI-HRMS calcd. m/z for C₃₃H₃₂N₅O₅F₃Na 658.2253, found 658.2256 [M+Na]⁺.

5-(4-(4-(Trifluoromethyl)phenyl)-1*H*-1,2,3-triazol-1-yl)-[1,1'-biphenyl]-3,4'-dicarboxylic acid (**13**).

Method A: Yield 68%; HPLC purity 99% (Rt = 13.46 min); ¹H NMR (400 MHz, DMSO-*d*₆) δ 9.77 (s, 1H), 8.56-8.53 (m, 2H), 8.36 (broad s, 1H), 8.20 (d, *J* =8.00 Hz, 2H), 8.11 (d, *J* =8.40 Hz, 2H), 8.00 (d, *J* =8.40 Hz, 2H), 7.91 (d, *J* =8.40 Hz, 2H); MS (ESI, m/z) 454.1 [M+1]⁺; ESI-HRMS calcd. m/z for C₂₃H₁₅N₃O₄F₃ 454.1015, found 454.1017 [M+1]⁺.

4'-((3-Aminopropyl)carbamoyl)-5-(4-(4-(trifluoromethyl)phenyl)-1H-1,2,3-triazol-1-yl)-[1,1'-biphenyl]-3-carboxylic acid (**14**).

Method B: Yield 63%; HPLC purity 97% (Rt = 12.68 min); ¹H NMR (400 MHz, DMSO-*d*₆) δ 9.73 (s, 1H), 8.55 (s, 1H), 8.51 (s, 1H), 8.37 (s, 1H), 8.23-8.20 (m, 4H), 8.00 (d, *J* =8.40 Hz, 2H), 7.90 (d, *J* =8.00 Hz, 2H), 3.51-3.46 (m, 2H), 3.00-2.99 (m, 2H), 1.93-1.90 (m, 2H); MS (ESI, m/z) 510.2 [M+1]⁺; ESI-HRMS calcd. m/z for C₂₆H₂₃N₅O₃F₃ 510.1753, found 510.1754 [M+1]⁺.

4'-Bromo-5-(4-(4-(trifluoromethyl)phenyl)-1*H*-1,2,3-triazol-1-yl)-[1,1'-biphenyl]-3-carboxylic acid (**15**).

Method A: Yield 77%; HPLC purity 99% (Rt = 12.93 min); ¹H NMR (400 MHz, CD₃OD) δ 9.17 (s, 1H), 8.43 (s, 1H), 8.35 (s, 1H), 8.25 (s, 1H), 8.16 (d, *J* =8.00 Hz, 2H), 7.79 (d, *J* =8.00 Hz, 2H), 7.72 (d, *J* =8.00 Hz, 2H), 7.66 (d, *J* =8.40 Hz, 2H); MS (ESI, m/z) 488.0, 490.0 [M+1]⁺; ESI-HRMS calcd. m/z for C₂₂H₁₄N₃O₂F₃Br 488.0221, found 488.0218 [M+1]⁺.

4'-(2-Amino-2-oxoethyl)-5-(4-(4-(trifluoromethyl)phenyl)-1*H*-1,2,3-triazol-1-yl)-[1,1'-biphenyl]-3-carboxylic acid (**16**).

Method A: Yield 64%; HPLC purity 99% (Rt = 8.55 min); ¹H NMR (400 MHz, CD₃OD) δ 9.24 (s, 1H), 8.50 (s, 1H), 8.38 (s, 2H), 8.16 (d, *J* =8.00 Hz, 2H), 7.79-7.74 (m, 4H), 7.48 (d, *J* =7.60 Hz, 2H), 3.60 (s, 2H); MS (ESI, m/z) 467.1 [M+1]⁺; ESI-HRMS calcd. m/z for C₂₄H₁₈N₄O₃F₃ 467.1331, found 467.1332 [M+1]⁺.

### 4'-(2-Cyanoethyl)-5-(4-(4-(trifluoromethyl)phenyl)-1H-1,2,3-triazol-1-yl)-[1,1'-biphenyl]-3-carboxylic acid (17):

The mixture of compound **38** (30 mg, 0.063 mmol), Pd(PPh₃)₄ (4 mg, 0.003 mmol), and 2 M K₂CO₃ aqueous solution (90 µL, 0.180 mmol) in *N,N*-dimethylformamide:water (1:1, 3mL) was purged with nitrogen gas for 15 min, and then 3-(4-bromophenyl)propionitrile (16 mg, 0.076 mmol) was added to the mixture. The mixture was stirred at 90 °C for 12 h, and then allowed to be cooled at room temperature. This mixture was partitioned ethyl acetate (5 mL) and water (10 mL). The aqueous layer was extracted with ethyl acetate (5 mL x 2), and then the combined organic layer was washed with brine (3 mL), dried (MgSO₄), filtered and evaporated under reduced pressure. The residue was purified by silica gel column chromatography (chloroform:methanol = 10:1) to afford the compound **17** (18 mg, 61%) as a white solid; HPLC purity 99% (Rt = 7.63 min); ¹H NMR (400 MHz, CD₃OD) δ 9.27 (s, 1H), 8.53 (s, 1H), 8.40 (m, 2H), 8.17 (d, *J* =8.00 Hz, 2H), 7.80-7.77 (m, 4H), 7.48 (d, *J* =8.00 Hz, 2H), 3.03 (t, *J* =7.20 Hz, 2H), 2.81 (t, *J* =7.20 Hz, 2H); MS (ESI, m/z) 463.1 [M+1]⁺; ESI-HRMS calcd. m/z for C₂₅H₁₈N₄O₂F₃ 463.1382, found 467.1381 [M+1]⁺.

4'-(3-Aminopropyl)-5-(4-(4-(trifluoromethyl)phenyl)-1H-1,2,3-triazol-1-yl)-[1,1'-biphenyl]-3-carboxylic acid (**18**).

Method B: Yield 98%; HPLC purity 98% (Rt = 8.14 min); ¹H NMR (400 MHz, CD₃OD) δ 9.27 (s, 1H), 8.52 (s, 1H), 8.42 (s, 1H), 8.38 (s, 1H), 8.17 (d, *J* =7.60 Hz, 2H), 7.80-7.75 (m, 4H), 7.43 (d, *J* =8.00 Hz, 2H), 2.99 (t, *J* =7.60 Hz, 2H), 2.82 (t, *J* =7.60 Hz, 2H), 2.05-2.02 (m, 2H); MS (ESI, m/z) 467.2 [M+1]⁺; ESI-HRMS calcd. m/z for C₂₅H₂₂N₄O₂F₃ 467.1695, found 467.1689 [M+1]⁺.

4'-(3-Aminoprop-1-yn-1-yl)-5-(4-(4-(trifluoromethyl)phenyl)-1H-1,2,3-triazol-1-yl)-[1,1'-biphenyl]-3-carboxylic acid (**19**).

Method B: Yield 62%; HPLC purity 97% (Rt = 10.49 min); ¹H NMR (400 MHz, DMSO-*d*₆) δ 9.67 (s, 1H), 8.42 (s, 1H), 8.26 (s, 1H), 8.21 (d, *J* =8.00 Hz, 2H), 8.16 (s, 1H), 7.88 (d, *J* =8.40 Hz, 2H), 7.79 (d, *J* =8.00 Hz, 2H), 7.53 (d, *J* =8.00 Hz, 2H), 3.53 (s, 2H); MS (ESI, m/z) 463.1 [M+1]⁺; ESI-HRMS calcd. m/z for C₂₅H₁₈N₄O₂F₃ 463.1382, found 463.1380 [M+1]⁺.

4'-(3-((*tert*-Butoxycarbonyl)amino)propyl)-5-(4-(4-(trifluoromethyl)phenyl)-1*H-*1,2,3-triazol-1-yl)-[1,1'-biphenyl]-3-carboxylic acid (**20**).

Method A: Yield 66%; HPLC purity 99% (Rt = 10.56 min); ¹H NMR (400 MHz, CD₃OD) δ 9.24 (s, 1H), 8.49 (s, 1H), 8.38-8.36 (m, 2H), 8.16 (d, *J* =8.00 Hz, 2H), 7.78 (d, *J* =8.00 Hz, 2H), 7.70 (d, *J* =8.40 Hz, 2H), 7.37 (d, *J* =8.00 Hz, 2H), 3.10 (t, *J* =7.20 Hz, 2H), 2.71 (t, *J* =7.20 Hz, 2H), 1.87-1.78 (m, 2H), 1.45 (s, 9H); MS (ESI, m/z) 589.2 [M+Na]⁺; ESI-HRMS calcd. m/z for C₃₀H₂₉N₄O₄F₃Na 589.2039, found 589.2043 [M+1]⁺.

4'-(4-Hydroxybutyl)-5-(4-(4-(trifluoromethyl)phenyl)-1*H*-1,2,3-triazol-1-yl)-[1,1'-biphenyl]-3-carboxylic acid (**21**).

Method A: Yield 39%; HPLC purity 99% (Rt = 7.40 min); ¹H NMR (400 MHz, CD₃OD) δ 9.25 (s, 1H), 8.50 (s, 1H), 8.38 (s, 2H), 8.16 (d, *J* =8.00 Hz, 2H), 7.79 (d, *J* =8.40 Hz, 2H), 7.70 (d, *J* =8.00 Hz, 2H), 7.37 (d, *J* =8.00 Hz, 2H), 3.59 (t, *J* =6.40 Hz, 2H), 2.72 (t, *J* =7.60 Hz, 2H), 1.78-1.71 (m, 2H), 1.64-1.57 (m, 2H); MS (ESI, m/z) 482.2 [M+1]⁺; ESI-HRMS calcd. m/z for C₂₆H₂₃N₃O₃F₃ 482.1692, found 482.1694 [M+1]⁺.

4'-(1-Aminocyclopropyl)-5-(4-(4-(trifluoromethyl)phenyl)-1H-1,2,3-triazol-1-yl)-[1,1'-biphenyl]-3-carboxylic acid (**22**).

Method A: Yield 69%; HPLC purity 98% (Rt = 6.80 min); ¹H NMR (400 MHz, CD₃OD) δ 9.27 (s, 1H), 8.55-8.39 (m, 3H), 8.15 (broad s, 2H), 7.88 (broad s, 2H), 7.78 (broad s, 2H), 7.63 (broad s, 2H), 1.43-1.38 (m, 4H); MS (ESI, m/z) 465.2 [M+1]⁺; ESI-HRMS calcd. m/z for C₂₅H₂₀N₄O₂F₃ 465.1538, found 465.1539 [M+1]⁺.

4'-(1-(Aminomethyl)cyclopropyl)-5-(4-(4-(trifluoromethyl)phenyl)-1*H*-1,2,3-triazol-1-yl)-[1,1'-biphenyl]-3-carboxylic acid (**23**).

Method B: Yield 52%; HPLC purity 97% (Rt = 10.85 min); ¹H NMR (400 MHz, CD₃OD) δ 9.08 (s, 1H), 8.38 (s, 1H), 8.30 (s, 1H), 8.16 (s, 1H), 8.09 (d, *J* =8.00 Hz, 2H), 7.77-7.73 (m, 4H), 7.55 (d, *J* =8.00 Hz, 2H), 3.22 (s, 2H), 1.09-1.06 (m, 4H); MS (ESI, m/z) 479.2 [M+1]⁺; ESI-HRMS calcd. m/z for C₂₆H₂₂N₄O₂F₃ 479.1695, found 479.1693 [M+1]⁺.

4'-(1-(((*tert*-Butoxycarbonyl)amino)methyl)cyclopropyl)-5-(4-(4-(trifluoromethyl)phenyl)-1*H*-1,2,3-triazol-1-yl)-[1,1'-biphenyl]-3-carboxylic acid (**24**).

Method A: Yield 51%; HPLC purity 99% (Rt = 21.57 min); ¹H NMR (400 MHz, CD₃OD) δ 9.26 (s, 1H), 8.51 (s, 1H), 8.40-8.38 (m, 2H), 8.17 (d, *J* =8.00 Hz, 2H), 7.80 (d, *J* =8.00 Hz, 2H), 7.73 (d, *J* =8.00 Hz, 2H), 7.51 (d, *J* =8.00 Hz, 2H), 3.34 (s, 2H), 1.39-1.28 (m, 9H), 0.94-0.86 (m, 4H); MS (ESI, m/z) 601.2 [M+Na]⁺; ESI-HRMS calcd. m/z for C₃₁H₂₉N₄O₄F₃Na 601.2039, found 601.2040 [M+Na]⁺.

4'-(1-Aminocyclobutyl)-5-(4-(4-(trifluoromethyl)phenyl)-1*H*-1,2,3-triazol-1-yl)-[1,1'-biphenyl]-3-carboxylic acid (**25**).

Method B: Yield 65%; HPLC purity 99% (Rt = 8.69 min); ¹H NMR (400 MHz, CD₃OD) δ 9.27 (s, 1H), 8.55 (s, 1H), 8.45 (s, 1H), 8.41 (s, 1H), 8.16 (d, *J* =8.00 Hz, 2H), 7.93 (d, *J* =8.40 Hz, 2H), 7.79 (d, *J* = 8.40 Hz, 2H), 7.69 (d, *J* =8.40 Hz, 2H), 2.89-2.82 (m, 2H), 2.70-2.63 (m, 2H), 2.33-2.24 (m, 1H), 2.07-1.97 (m, 1H); MS (ESI, m/z) 479.3 [M+1]⁺; ESI-HRMS calcd. m/z for C₂₆H₂₂N₄O₂F₃ 479.1695, found 479.1696 [M+1]⁺.

4'-(1-((*tert*-Butoxycarbonyl)amino)cyclobutyl)-5-(4-(4-(trifluoromethyl)phenyl)-1*H-*1,2,3-triazol-1-yl)-[1,1'-biphenyl]-3-carboxylic acid (**26**).

Method A: Yield 47%; HPLC purity 99% (Rt = 10.76 min); ¹H NMR (400 MHz, CD₃OD) δ 9.18 (s, 1H), 8.40-8.39 (m, 2H), 8.26 (s, 1H), 8.17 (d, *J* =8.00 Hz, 2H), 7.80-7.76 (m, 4H), 7.59 (d, *J* = 8.40 Hz, 2H), 2.60-2.46 (m, 4H), 2.16-2.07 (m, 1H), 1.99-1.88 (m, 1H), 1.40-1.25 (m, 9H); MS (ESI, m/z) 579.2 [M+1]⁺; ESI-HRMS calcd. m/z for C₃₁H₃₀N₄O₄F₃ 579.2219, found 579.2219 [M+1]⁺.

4'-(3-(Hydroxymethyl)oxetan-3-yl)-5-(4-(4-(trifluoromethyl)phenyl)-1*H*-1,2,3-triazol-1-yl)-[1,1'-biphenyl]-3-carboxylic acid (**27**).

Method A: Yield 47%; HPLC purity 99% (Rt = 8.71 min); ¹H NMR (400 MHz, CD₃OD) δ 9.25 (s, 1H), 8.51 (s, 1H), 8.41 (s, 1H), 8.39 (s, 1H), 8.15 (d, *J* =8.40 Hz, 2H), 7.80-7.77 (m, 4H), 7.32 (d, *J* =8.00 Hz, 2H), 4.97 (d, *J* = 5.60 Hz, 2H), 4.89 (d, *J* = 6.00 Hz, 2H), 3.94 (s, 2H); MS (ESI, m/z) 496.2 [M+1]⁺; ESI-HRMS calcd. m/z for C₂₆H₂₁N₃O₄F₃ 496.1484, found 496.1488 [M+1]⁺.

4'-(Isoxazol-3-yl)-5-(4-(4-(trifluoromethyl)phenyl)-1*H*-1,2,3-triazol-1-yl)-[1,1'-biphenyl]-3-carboxylic acid (**28**).

Method A: Yield 74%; HPLC purity 98% (Rt = 11.86 min); ¹H NMR (400 MHz, CD₃OD) δ 9.28 (s, 1H), 8.75 (s, 1H), 8.57 (s, 1H), 8.46 (s, 2H), 8.17 (d, *J* = 7.60 Hz, 2H), 8.05 (d, *J* =8.00 Hz, 2H), 7.94 (d, *J* = 8.40 Hz, 2H), 7.79 (d, *J* =8.00 Hz, 2H), 7.00 (s, 1H); MS (ESI, m/z) 477.1 [M+1]⁺; ESI-HRMS calcd. m/z for C₂₅H₁₆N₄O₃F₃ 477.1175, found 477.1177 [M+1]⁺.

4'-(5-(Hydroxymethyl)isoxazol-3-yl)-5-(4-(4-(trifluoromethyl)phenyl)-1*H*-1,2,3-triazol-1-yl)-[1,1'-biphenyl]-3-carboxylic acid (**29**).

Method A: Yield 43%; HPLC purity 98% (Rt = 20.10 min); ¹H NMR (400 MHz, DMSO-*d*₆) δ 9.77 (s, 1H), 8.57 (s, 1H), 8.54 (s, 1H), 8.36 (s, 1H), 8.20 (d, *J* =8.00 Hz, 2H), 8.08-8.01 (m, 4H), 7.91 (d, *J* = 8.00 Hz, 2H), 7.05 (s, 1H), 5.75 (t, *J* = 6.00 Hz, 1H), 4.64 (d, *J* = 6.00 Hz, 2H); MS (ESI, m/z) 507.1 [M+1]⁺; ESI-HRMS calcd. m/z for C₂₆H₁₈N₄O₄F₃ 507.1280, found 507.1271 [M+1]⁺.

4'-(5-(2-Hydroxyethyl)isoxazol-3-yl)-5-(4-(4-(trifluoromethyl)phenyl)-1*H*-1,2,3-triazol-1-yl)-[1,1'-biphenyl]-3-carboxylic acid (**30**).

Method A: Yield 55%; HPLC purity 95% (Rt = 10.69 min); ¹H NMR (400 MHz, DMSO-*d*₆) δ 9.74 (s, 1H), 8.51 (s, 1H), 8.46 (s, 1H), 8.35 (s, 1H), 8.20 (d, *J* =8.00 Hz, 2H), 8.03-7.98 (m, 4H), 7.90 (d, *J* = 8.00 Hz, 2H), 6.93 (s, 1H), 3.77 (t, *J* = 6.40 Hz, 2H), 2.97 (d, *J* =6.40 Hz, 2H); MS (ESI, m/z) 521.1 [M+1]⁺; ESI-HRMS calcd. m/z for C₂₇H₂₀N₄O₄F₃ 521.1437, found 521.1435 [M+1]⁺.

4'-(2*H*-Tetrazol-5-yl)-5-(4-(4-(trifluoromethyl)phenyl)-1*H*-1,2,3-triazol-1-yl)-[1,1'-biphenyl]-3-carboxylic acid (**31**).

Method A: Yield 43%; HPLC purity 98% (Rt = 15.95 min); ¹H NMR (400 MHz, CD₃OD) δ 9.24 (s, 1H), 8.49 (s, 1H), 8.45 (s, 1H), 8.35 (s, 1H), 8.22-8.17 (m, 4H), 7.92 (d, *J* =8.40 Hz, 2H), 7.80 (d, *J* = 8.00 Hz, 2H); MS (ESI, m/z) 478.1 [M+1]⁺; ESI-HRMS calcd. m/z for C₂₃H₁₅N₇O₂F₃ 478.1239, found 478.1242 [M+1]⁺.

4-(4-(5-(Hydroxymethyl)isoxazol-3-yl)phenyl)-7-(4-(trifluoromethyl)phenyl)-2-naphthoic acid (**32**).

Method A: Yield 93%; HPLC purity 98% (Rt = 12.07 min); ¹H NMR (400 MHz, DMSO-*d*₆) δ 8.79 (s, 1H), 8.68 (s, 1H), 8.09-8.05 (m, 5H), 8.01-7.98 (m, 1H), 7.95 (m, 1H), 7.91-7.89 (m, 2H), 7.70 (d, *J* = 8.40 Hz, 2H), 7.04 (s, 1H), 5.80-5.73 (m, 1H), 4.66-4.63 (m, 2H); MS (ESI, m/z) 490.1 [M+1]⁺; ESI-HRMS calcd. m/z for C₂₈H₁₉NO₄F₃ 490.1266, found 490.1269 [M+1]⁺.

4'-(5-(Hydroxymethyl)isoxazol-3-yl)-5-(4-(trifluoromethyl)benzamido)-[1,1'-biphenyl]-3-carboxylic acid (**33**).

Method A: Yield 62%; HPLC purity 99% (Rt = 10.29 min); ¹H NMR (400 MHz, CD₃OD) δ 8.35 (broad s, 2H), 8.17-8.13 (m, 3H), 7.97 (d, *J* =8.40 Hz, 2H), 7.86-7.81 (m, 4H), 6.82 (s, 1H), 4.74 (s, 2H); MS (ESI, m/z) 483.1 [M+1]⁺; ESI-HRMS calcd. m/z for C₂₅H₁₈N₂O₅F₃ 483.1168, found 483.1170 [M+1]⁺.

4-(4-(Piperidin-4-yl)phenyl)-6-(4-(4-(trifluoromethyl)phenyl)-1*H*-1,2,3-triazol-1-yl)picolinic acid (**34**).

Method B: Yield 60%; HPLC purity 99% (Rt = 7.87 min); ¹H NMR (400 MHz, DMSO-*d*₆) δ 9.55 (s, 1H), 8.31-8.26 (m, 4H), 7.91-7.85 (m, 4H), 7.47 (d, *J* =8.00 Hz, 2H), 3.68-3.65 (m, 1H), 3.02-2.88 (m, 4H), 1.95-1.83 (m, 4H); MS (ESI, m/z) 494.2 [M+1]⁺; ESI-HRMS calcd. m/z for C₂₆H₂₃N₅O₂F₃ 494.1804, found 494.1805 [M+1]⁺.

2-(4-(Piperidin-4-yl)phenyl)-6-(4-(4-(trifluoromethyl)phenyl)-1*H*-1,2,3-triazol-1-yl)isonicotinic acid (**35**).

Method B: Yield 65%; HPLC purity 95% (Rt = 10.72 min); ¹H NMR (400 MHz, DMSO-*d*₆) δ 9.79 (s, 1H), 8.42 (s, 2H), 8.34-8.33 (m, 4H), 7.89 (d, *J* =8.00 Hz, 2H), 7.44 (d, *J* =8.00 Hz, 2H), 3.62-3.59 (m, 1H), 3.07-2.92 (m, 4H), 2.03-1.89 (m, 4H); MS (ESI, m/z) 494.2 [M+1]⁺; ESI-HRMS calcd. m/z for C₂₆H₂₃N₅O₂F₃ 494.1804, found 494.1805 [M+1]⁺.

6-(4-(Piperidin-4-yl)phenyl)-4-(4-(4-(trifluoromethyl)phenyl)-1*H*-1,2,3-triazol-1-yl)picolinic acid (**36**).

Method B: Yield 65%; HPLC purity 99% (Rt = 11.24 min); ¹H NMR (400 MHz, DMSO-*d*₆) δ 9.94 (s, 1H), 8.71 (s, 1H), 8.56 (s, 1H), 8.28 (d, *J* =7.20 Hz, 2H), 8.19 (d, *J* =7.60 Hz, 2H), 7.93 (d, *J* =7.20 Hz, 2H), 7.46 (d, *J* =7.20 Hz, 2H), 3.43-3.40 (m, 2H), 3.07-2.92 (m, 3H), 2.03-2.00 (m, 2H), 1.91-1.84 (m, 2H); MS (ESI, m/z) 494.2 [M+1]⁺; ESI-HRMS calcd. m/z for C₂₆H₂₃N₅O₂F₃ 494.1804, found 494.1808 [M+1]⁺.

2-(Dimethylamino)-2-oxoethyl 4'-(piperidin-4-yl)-5-(4-(4-(trifluoromethyl)phenyl)-1*H*-1,2,3-triazol-1-yl)-[1,1'-biphenyl]-3-carboxylate (**37a**).

Method B: Yield 78%; HPLC purity 97% (Rt = 20.45 min); ¹H NMR (400 MHz, CD₃OD) δ 9.29 (s, 1H), 8.59 (s, 1H), 8.47 (s, 1H), 8.45 (s, 1H), 8.17 (d, *J* =8.00 Hz, 2H), 7.82-7.79 (m, 4H), 7.47 (d, *J* =8.00 Hz, 2H), 5.18 (s, 2H), 3.56-3.53 (m, 2H), 3.23-3.16 (s, 2H), 3.13 (s, 3H), 3.05-2.99 (m, 1H), 3.02 (s, 3H), 2.18-2.14 (m, 2H), 2.03-1.92 (m, 2H); MS (ESI, m/z) 578.3 [M+1]⁺; ESI-HRMS calcd. m/z for C₃₁H₃₁N₅O₃F₃ 578.2379, found 578.2390 [M+1]⁺.

2-(Dimethylamino)-2-oxoethyl 4-(4-(5-(hydroxymethyl)isoxazol-3-yl)phenyl)-7-(4-(trifluoromethyl)phenyl)-2-naphthoate (**37b**).

To a solution of **32** (3.5 mg, 0.007 mmol) in DMF (1 mL) was added Cs₂CO₃ (3.0 mg, 0.010 mmol). 2-Chloro-*N,N*-dimethylacetamide (1.0 mg, 0.007 mmol) was added to the reaction mixture under N₂ atmosphere, and the reaction was stirred at 45 °C for 1 h under N₂ atmosphere. The reaction mixture was partitioned ethyl acetate (5 mL) and water (5 mL), and the aqueous phase was extracted with ethyl acetate (2 x 5 mL). The combined organic layers were dried over Na₂SO₄, filtered, and concentrated under pressure. The residue was purified by silica gel chromatography (chloroform:methanol = 30:1) to afford compound **37b** (2.7 mg, 68%) as a white solid; HPLC purity 99% (Rt = 17.73 min); ¹H NMR (400 MHz, CD₃OD) δ 8.85-8.84 (m, 1H), 8.45-8.44 (m, 1H), 8.08-7.97 (m, 7H), 7.81 (d, *J* =8.40 Hz, 2H), 7.66 (d, *J* =8.00 Hz, 2H), 6.88 (s, 1H), 5.14 (s, 2H), 4.76 (s, 2H), 3.12 (s, 3H), 3.00 (s, 3H); MS (ESI, m/z) 575.2 [M+1]⁺; ESI-HRMS calcd. m/z for C₃₂H₂₆N₂O₅F₃ 575.1794, found 575.1786 [M+1]⁺.

2-(Dimethylamino)-2-oxoethyl 4-(4-(1-acetylpiperidin-4-yl)phenyl)-7-(4-(trifluoromethyl)phenyl)-2-naphthoate (**37c**).

Compound **1b** (24 mg, 0.046 mmol) was converted to compound 37c (20 mg, 72%) as a white solid, using a similar procedure to that used in the preparation of compound 87; HPLC purity 99% (Rₜ = 16.43 min); ¹H NMR (400 MHz, CD₃OD) δ 8.80 (s, 1H), 8.42 (s, 1H), 8.03-7.98 (m, 4H), 7.93-7.91 (m, 1H), 7.80 (d, *J =* 7.60 Hz, 2H), 7.47-7.43 (m, 4H), 5.14 (s, 2H), 4.75-4.72 (m, 1H), 4.12-4.08 (m, 1H), 3.27-3.24 (m, 1H), 3.12 (s, 3H), 3.00 (s, 3H), 2.95-2.92 (m, 1H), 2.81-2.75 (m, 1H), 2.18 (s, 3H), 2.04-1.97 (m, 2H), 1.84-1.68 (m, 2H); MS (ESI, m/z) 603.2 [M+1]⁺; ESI-HRMS calcd. m/z for C₃₅H₃₄N₂O₄F₃ 603.2471, found 603.2479 [M+1]⁺.

Methyl 4'-((*tert*-butoxycarbonyl)amino)-5-(4-(4-(trifluoromethyl)phenyl)-1H-1,2,3-triazol-1-yl)-[1,1'-biphenyl]-3-carboxylate (**39**).

Method C: Yield 40%; ¹H NMR (400 MHz, CDCl₃) δ 8.40 (s, 1H), 8.33 (s, 1H), 8.29 (broad s, 2H), 8.06 (d, *J* =8.00 Hz, 2H), 7.74 (d, *J* =8.40 Hz, 2H), 7.64 (d, *J* =8.40 Hz, 2H), 7.52 (d, *J* =8.40 Hz, 2H), 4.01 (s, 3H), 1.54 (s, 9H); MS (ESI, m/z) 539.2 [M+1]⁺; ESI-HRMS calcd. m/z for C₂₈H₂₆N₄O₄F₃ 539.1906, found 539.1916 [M+1]⁺.

Methyl 4'-amino-5-(4-(4-(trifluoromethyl)phenyl)-1*H*-1,2,3-triazol-1-yl)-[1,1'-biphenyl]-3-carboxylate (**40**).

Method B: Yield 57%; ¹H NMR (400 MHz, CDCl₃) δ 8.38 (s, 1H), 8.32 (s, 1H), 8.26-8.24 (m, 2H), 8.06 (d, *J* =8.00 Hz, 2H), 7.74 (d, *J* =8.00 Hz, 2H), 7.54 (d, *J* =8.40 Hz, 2H), 6.81 (d, *J* =8.40 Hz, 2H), 4.00 (s, 3H); MS (ESI, m/z) 439.1 [M+1]⁺; ESI-HRMS calcd. m/z for C₂₃H₁₈N₄O₂F₃ 439.1382, found 439.1378 [M+1]⁺.

Methyl 4'-acetamido-5-(4-(4-(trifluoromethyl)phenyl)-1*H*-1,2,3-triazol-1-yl)-[1,1'-biphenyl]-3-carboxylate (**41**).

**40** (12 mg, 0.027 mmol) was dissolved in dichloromethane (1 mL) and degassed for 30 min with nitrogen gas. Acetic anhydride (3.2 µL, 0.033 mmol) was added to the reaction and was stirred for 2 h at room temperature. The reaction mixture was partitioned dichloromethane (10 mL) and saturated aqueous Na₂CO₃ solution (10 mL), and the aqueous phase was extracted with dichloromethane (10 mL x 2). The combined organic layers were dried over Na₂SO₄, filtered, and concentrated under pressure. The residue was purified by silica gel chromatography (hexane:ethyl acetate = 2:1) to afford compound **41** (10 mg, 76%) as a white solid; ¹H NMR (400 MHz, CDCl₃) δ 8.40 (s, 1H), 8.34 (s, 1H), 8.32-8.30 (m, 2H), 8.06 (d, *J* =8.00 Hz, 2H), 7.74 (d, *J* =8.40 Hz, 2H), 7.67 (s, 4H), 4.01 (s, 3H), 2.23 (s, 3H); MS (ESI, m/z) 481.2 [M+1]⁺; ESI-HRMS calcd. m/z for C₂₅H₂₀N₄O₃F₃ 481.1488, found 481.1487 [M+1]⁺.

Methyl 4'-benzamido-5-(4-(4-(trifluoromethyl)phenyl)-1*H*-1,2,3-triazol-1-yl)-[1,1'-biphenyl]-3-carboxylate (**42**).

**40** (12 mg, 0.027 mmol) was dissolved in dichloromethane (1 mL) at 0 °C. Triethylamine (4.2 µL, 0.030 mmol) and benzoyl chloride (3.2 µL, 0.027 mmol) were added, and the mixture was stirred for 18 h at room temperature. The reaction mixture was partitioned dichloromethane (10 mL) and water (10 mL), and the aqueous phase was extracted with dichloromethane (10 mL x 2). The combined organic layers were dried over Na₂SO₄, filtered, and concentrated under pressure. The residue was purified by silica gel chromatography (hexane:ethyl acetate = 3:1) to afford compound **42** (9 mg, 63%) as a white solid; ¹H NMR (400 MHz, CDCl₃) δ 8.41 (s, 1H), 8.37 (s, 1H), 8.33 (broad s, 2H), 8.10-8.05 (m, 2H), 7.94-7.90 (m, 3H), 7.82 (d, *J* =8.40 Hz, 2H), 7.75-7.72 (m, 3H), 7.61-7.45 (m, 3H), 4.02 (s, 3H); MS (ESI, m/z) 543.2 [M+1]⁺; ESI-HRMS calcd. m/z for C₃₀H₂₁N₄O₃F₃ 543.1644, found 543.1647 [M+1]⁺.

*tert*-Butyl 4-((3'-(methoxycarbonyl)-5'-(4-(4-(trifluoromethyl)phenyl)-1*H*-1,2,3-triazol-1-yl)-[1,1'-biphenyl]-4-yl)carbamoyl)piperidine-1-carboxylate (**43**).

To a solution of compounds **40** (27 mg, 0.061 mmol) in *N,N*-dimethylformamide (2 mL) were added Boc-Inp-OH (15 mg, 0.067 mmol), HATU (25 mg, 0.067 mmol) and*N,N-*diisopropylethylamine (13 µL, 0.080 mmol), and then this reaction mixture was stirred at room temperature for 2 h. The reaction mixture was partitioned ethyl acetate (5 mL) and water (5 mL), and the aqueous layer was extracted with ethyl acetate (5 mL x 2). The combined organic layer was washed brine (3 mL), dried over MgSO₄, filtered and evaporated under reduced pressure. The residue was purified by silica gel column chromatography (hexane:ethyl acetate = 3:1) to afford compound **43** (37 mg, 94%) as a white solid; ¹H NMR (400 MHz, CDCl₃) δ 8.40 (s, 1H), 8.34-8.33 (m, 1H), 8.32-8.30 (m, 2H), 8.06 (d, *J* =8.40 Hz, 2H), 7.74 (d, *J* =8.00 Hz, 2H), 7.68 (s, 4H), 4.25-4.18 (m, 2H), 4.01 (s, 3H), 2.84-2.78 (m, 2H), 2.46-2.40 (m, 1H), 1.95-1.92 (m, 2H), 1.78-1.76 (m, 2H), 1.47 (s, 9H); MS (ESI, m/z) 672.2 [M+ Na]⁺; ESI-HRMS calcd. m/z for C₃₄H₃₄N₅O₅F₃Na 672.2410, found 672.2413 [M+Na]⁺.

3'-(Methoxycarbonyl)-5'-(4-(4-(trifluoromethyl)phenyl)-1*H*-1,2,3-triazol-1-yl)-[1,1'-biphenyl]-4-carboxylic acid (**44**).

Method C: Yield 45%; ¹H NMR (400 MHz, DMSO-*d*₆) δ 9.78 (s, 1H), 8.59-8.56 (m, 2H), 8.36 (s, 1H), 8.19 (d, *J* =8.00 Hz, 2H), 8.10 (d, *J* =8.40 Hz, 2H), 8.00 (d, *J* =8.40 Hz, 2H), 7.91 (d, *J* =8.40 Hz, 2H), 3.98 (s, 3H); MS (ESI, m/z) 468.1 [M+1]⁺; ESI-HRMS calcd. m/z for C₂₄H₁₇N₃O₄F₃ 468.1171, found 468.1169 [M+1]⁺.

Methyl 4'-((3-((tert-butoxycarbonyl)amino)propyl)carbamoyl)-5-(4-(4-(trifluoromethyl)phenyl)-1*H*-1,2,3-triazol-1-yl)-[1,1'-biphenyl]-3-carboxylate (**45**).

Method C: Yield 68%; ¹H NMR (400 MHz, CD₃OD) δ 9.15 (s, 1H), 8.41 (s, 1H), 8.32 (s, 1H), 8.25 (s, 1H), 8.04 (d, *J* =8.00 Hz, 2H), 7.93 (d, *J* =8.40 Hz, 2H), 7.78 (d, *J* =8.40 Hz, 2H), 7.71 (d, *J* =8.00 Hz, 2H), 3.95 (s, 3H), 3.44 (t, *J* =6.80 Hz, 2H), 3.17-3.14 (m, 2H), 1.80-1.76 (m, 2H); MS (ESI, m/z) 646.2 [M+Na]⁺; ESI-HRMS calcd. m/z for C₃₂H₃₂N₅O₅F₃Na 646.2253, found 646.2255 [M+Na]⁺.

4'-((3-((*tert*-Butoxycarbonyl)amino)propyl)carbamoyl)-5-(4-(4-(trifluoromethyl)phenyl)-1*H*-1,2,3-triazol-1-yl)-[1,1'-biphenyl]-3-carboxylic acid (**46**).

Method A: Yield 77%; ¹H NMR (400 MHz, CD₃OD) δ 9.27 (s, 1H), 8.57 (s, 1H), 8.46 (s, 1H), 8.41 (s, 1H), 8.15 (d, *J* =8.00 Hz, 2H), 7.99 (d, *J* =7.60 Hz, 2H), 7.89 (d, *J* =8.00 Hz, 2H), 7.78 (d, *J* =8.00 Hz, 2H), 3.47-3.43 (m, 2H), 3.17-3.14 (m, 2H), 1.80-1.77 (m, 2H); MS (ESI, m/z) 610.2 [M+1]⁺; ESI-HRMS calcd. m/z for C₃₁H₃₁N₅O₅F₃ 610.2277, found 610.2270 [M+1]⁺.

Methyl 4'-bromo-5-(4-(4-(trifluoromethyl)phenyl)-1*H*-1,2,3-triazol-1-yl)-[1,1'-biphenyl]-3-carboxylate (**47**).

Method C: Yield 53%; ¹H NMR (400 MHz, CDCl₃) δ 8.40 (s, 1H), 8.35-8.32 (m, 3H), 8.06 (d, *J* =8.00 Hz, 2H), 7.74 (d, *J* =8.40 Hz, 2H), 7.65 (d, *J* =8.40 Hz, 2H), 7.57 (d, *J* =8.40 Hz, 2H), 4.02 (s, 3H); MS (ESI, m/z) 502.0, 504.0 [M+1]⁺; ESI-HRMS calcd. m/z for C₂₃H₁₆N₃O₂F₃Br 502.0378, found 502.0382 [M+1]⁺.

Methyl 4'-(2-amino-2-oxoethyl)-5-(4-(4-(trifluoromethyl)phenyl)-1*H*-1,2,3-triazol-1-yl)-[1,1'-biphenyl]-3-carboxylate (**48**).

Method D: Yield 73%; ¹H NMR (400 MHz, CDCl₃) δ 8.41 (s, 1H), 8.36-8.33 (m, 3H), 8.06 (d, *J* =8.00 Hz, 2H), 7.75-7.69 (m, 2H), 7.50-7.44 (m, 2H), 7.16 (d, *J* =8.40 Hz, 2H), 4.02 (s, 3H), 3.67 (s, 2H); MS (ESI, m/z) 481.2 [M+1]⁺; ESI-HRMS calcd. m/z for C₂₅H₂₀N₄O₃F₃ 481.1488, found 481.1492 [M+1]⁺.

Methyl 4'-(3-((tert-butoxycarbonyl)amino)propyl)-5-(4-(4-(trifluoromethyl)phenyl)-1H-1,2,3-triazol-1-yl)-[1,1'-biphenyl]-3-carboxylate (**49**).

Method E: Yield 55%; ¹H NMR (400 MHz, CDCl₃) δ 8.40 (s, 1H), 8.35 (s, 1H), 8.33-8.31 (m, 2H), 8.06 (d, *J* =8.00 Hz, 2H), 7.74 (d, *J* =8.40 Hz, 2H), 7.62 (d, *J* =8.40 Hz, 2H), 7.33 (d, *J* =8.00 Hz, 2H), 4.01 (s, 3H), 3.20-3.18 (m, 2H), 2.72 (t, *J* =7.60 Hz, 2H), 1.90-1.82 (m, 2H), 1.45 (s, 9H); MS (ESI, m/z) 525.2 [M+1-*tert*-butyl]⁺.

Methyl 4'-(3-((tert-butoxycarbonyl)amino)prop-1-yn-1-yl)-5-(4-(4-(trifluoromethyl)phenyl)-1*H*-1,2,3-triazol-1-yl)-[1,1'-biphenyl]-3-carboxylate (**50**).

Method C: Yield 35%; ¹H NMR (400 MHz, CD₃OD) δ 9.24 (s, 1H), 8.51 (s, 1H), 8.40 (s, 1H), 8.34 (s, 1H), 8.13 (d, *J* =8.00 Hz, 2H), 7.78-7.74 (m, 4H), 7.56 (d, *J* =8.40 Hz, 2H), 4.10-4.08 (m, 2H), 4.00 (s, 3H), 1.48 (s, 9H); MS (ESI, m/z) 577.2 [M+1]⁺; ESI-HRMS calcd. m/z for C₃₁H₂₈N₄O₄F₃ 577.2063, found 577.2057 [M+1]⁺.

Methyl 4'-(4-hydroxybutyl)-5-(4-(4-(trifluoromethyl)phenyl)-1*H*-1,2,3-triazol-1-yl)-[1,1'-biphenyl]-3-carboxylate (**51**).

Method E: Yield 27%; ¹H NMR (400 MHz, CDCl₃) δ 8.48 (s, 1H), 8.40 (s, 1H), 8.32 (s, 2H), 8.06 (d, *J* =8.00 Hz, 2H), 7.74 (d, *J* =8.00 Hz, 2H), 7.62 (d, *J* =8.00 Hz, 2H), 7.33 (d, *J* =8.00 Hz, 2H), 4.01 (s, 3H), 3.72-3.68 (m, 2H), 2.73 (t, *J* =7.60 Hz, 2H), 1.80-1.72 (m, 2H), 1.68-1.58 (m, 2H); MS (ESI, m/z) 496.2 [M+1]⁺; ESI-HRMS calcd. m/z for C₂₇H₂₅N₃O₃F₃ 496.1848, found 496.1855 [M+1]⁺.

4'-(3-((*tert*-Butoxycarbonyl)amino)prop-1-yn-1-yl)-5-(4-(4-(trifluoromethyl)phenyl)-1*H*-1,2,3-triazol-1-yl)-[1,1'-biphenyl]-3-carboxylic acid (**52**).

Method A: Yield 88%; ¹H NMR (400 MHz, DMSO-*d*₆) δ 9.76 (s, 1H), 8.50 (d, *J* =6.40 Hz, 2H), 8.30 (s, 1H), 8.19 (d, *J* =8.00 Hz, 2H), 7.91-7.88 (m, 4H), 7.58 (d, *J* =8.40 Hz, 2H), 4.02 (s, 2H), 1.41 (s, 9H); MS (ESI, m/z) 563.2 [M+1]⁺; ESI-HRMS calcd. m/z for C₃₀H₂₆N₄O₄F₃ 563.1906, found 563.1897 [M+1]⁺.

*tert*-Butyl (3-(4-bromobenzamido)propyl)carbamate (**54a**).

4-Bromobenzoic acid **53a** (Scheme S2, Supporting information, 100 mg, 0.497 mmol) was converted to compound **54a** (54 mg, 31%) as a white solid, using a similar procedure to that used in the preparation of compound **43**; ¹H NMR (400 MHz, CDCl₃) δ 7.73 (d, *J* =8.00 Hz, 2H), 7.57 (d, *J* =8.40 Hz, 2H), 3.51-3.46 (m, 2H), 3.26-3.22 (m, 2H), 1.71-1.68 (m, 2H), 1.44 (s, 9H).

*tert*-Butyl (3-(4-bromophenyl)prop-2-yn-1-yl)carbamate (**54b**).

To a mixture of 1-bromo-4-iodobenzene **53b** (Scheme S2, Supporting information, 300 mg, 1.06 mmol), PdCl₂(PPh₃)₂ (37 mg, 0.053 mmol) and copper iodide (20 mg, 0.106 mmol) in THF were added triethylamine (295 µL, 2.12 mmol) and *N*-Boc-propargylamine (247 mg, 1.59 mmol) at room temperature under N₂ atmosphere, and then this reaction mixture was stirred at room temperature for 12 h under N₂ atmosphere. The reaction mixture was partitioned with ethyl acetate (100 mL) and aqueous saturated NH₄Cl solution (50 mL), and then the aqueous layer was extracted with ethyl acetate (100 mL x 2). The combined organic layer was dried (Na₂SO₄), filtered and evaporated under reduced pressure. The residue was purified by silica gel column chromatography (hexane:ethyl acetate = 5:1) to afford the compound **54b** (310 mg, 94%) as a white solid; ¹H NMR (400 MHz, CDCl₃) δ 7.45 (d, *J* =8.40 Hz, 2H), 7.28 (d, *J* =8.40 Hz, 2H), 4.16-4.15 (m, 2H), 1.48 (s, 9H).

Methyl 4'-(1-((tert-butoxycarbonyl)amino)cyclopropyl)-5-(4-(4-(trifluoromethyl)phenyl)-1*H*-1,2,3-triazol-1-yl)-[1,1'-biphenyl]-3-carboxylate (**55**).

Method E: Yield 30%; ¹H NMR (400 MHz, CDCl₃) δ 8.39 (s, 1H), 8.35-8.30 (m, 3H), 8.06 (d, *J* =8.00 Hz, 2H), 7.74 (d, *J* =8.00 Hz, 2H), 7.63 (d, *J* =8.40 Hz, 2H), 7.34 (d, *J* =8.00 Hz, 2H), 4.01 (s, 3H), 1.46 (s, 9H), 1.35-1.30 (m, 4H); MS (ESI, m/z) 579.2 [M+1]⁺; ESI-HRMS calcd. m/z for C₃₁H₃₀N₄O₄F₃ 579.2219, found 579.2220 [M+1]⁺.

Methyl 4'-(1-aminocyclopropyl)-5-(4-(4-(trifluoromethyl)phenyl)-1*H*-1,2,3-triazol-1-yl)-[1,1'-biphenyl]-3-carboxylate (**56**).

Method B: Yield 90%; ¹H NMR (400 MHz, CD₃OD) δ 9.29 (s, 1H), 8.56 (s, 1H), 8.49 (s, 1H), 8.41 (s, 1H), 8.17 (d, *J* =8.00 Hz, 2H), 7.90 (d, *J* =8.40 Hz, 2H), 7.80 (d, *J* =8.00 Hz, 2H), 7.64 (d, *J* =8.00 Hz, 2H), 4.02 (s, 3H), 1.43-1.40 (m, 4H); MS (ESI, m/z) 479.1 [M+1]⁺; ESI-HRMS calcd. m/z for C₂₆H₂₂N₄O₂F₃ 479.1695, found 479.1699 [M+1]⁺.

Methyl 4'-(1-(((*tert*-Butoxycarbonyl)amino)methyl)cyclopropyl)-5-(4-(4-(trifluoromethyl)phenyl)-1*H*-1,2,3-triazol-1-yl)-[1,1'-biphenyl]-3-carboxylate (57).

Method E: Yield 30%; ¹H NMR (400 MHz, CDCl₃) δ 8.40 (s, 1H), 8.35-8.30 (m, 3H), 8.06 (d, *J* =8.40 Hz, 2H), 7.74 (d, *J* =8.40 Hz, 2H), 7.64 (d, *J* =8.00 Hz, 2H), 7.44 (d, *J* =8.00 Hz, 2H), 4.01 (s, 3H), 3.39-3.35 (m, 2H), 1.40 (s, 9H), 0.93-0.79 (m, 4H); MS (ESI, m/z) 615.2 [M+Na]⁺; ESI-HRMS calcd. m/z for C₃₂H₃₁N₄O₄F₃Na 615.2195, found 615.2199 [M+Na]⁺.

Methyl 4'-(1-((*tert*-butoxycarbonyl)amino)cyclobutyl)-5-(4-(4-(trifluoromethyl)phenyl)-1*H*-1,2,3-triazol-1-yl)-[1,1'-biphenyl]-3-carboxylate (**58**).

Method E: Yield 70%; ¹H NMR (400 MHz, CDCl₃) δ 8.40 (s, 1H), 8.38 (s, 1H), 8.33 (s, 2H), 8.06 (d, *J* =8.00 Hz, 2H), 7.74 (d, *J* =8.00 Hz, 2H), 7.68 (d, *J* =8.00 Hz, 2H), 7.57 (d, *J* =7.60 Hz, 2H), 4.01 (s, 3H), 2.63-2.44 (m, 4H), 2.20-2.09 (m, 1H), 1.97-1.85 (m, 1H), 1.47-1.29 (m, 9H); MS (ESI, m/z) 593.3 [M+1]⁺; ESI-HRMS calcd. m/z for C₃₂H₃₂N₄O₄F₃ 593.2376, found 593.2383 [M+1]⁺.

Methyl 4'-(3-(hydroxymethyl)oxetan-3-yl)-5-(4-(4-(trifluoromethyl)phenyl)-1*H-*1,2,3-triazol-1-yl)-[1,1'-biphenyl]-3-carboxylate (**59**).

Method E: Yield 44%; ¹H NMR (400 MHz, CDCl₃) δ 8.41 (s, 1H), 8.36 (s, 1H), 8.34 (s, 2H), 8.06 (d, *J* =8.00 Hz, 2H), 7.75-7.64 (m, 4H), 7.54 (d, *J* =8.00 Hz, 2H), 5.01 (d, *J* =5.60 Hz, 2H), 4.83 (d, *J* =5.60 Hz, 2H), 4.11 (d, *J* =5.60 Hz, 2H), 4.02 (s, 3H); MS (ESI, m/z) 510.2 [M+1]⁺; ESI-HRMS calcd. m/z for C₂₇H₂₃N₃O₄F₃ 510.1641, found 510.1640 [M+1]⁺.

Methyl 4'-(isoxazol-3-yl)-5-(4-(4-(trifluoromethyl)phenyl)-1*H*-1,2,3-triazol-1-yl)-[1,1'-biphenyl]-3-carboxylate (**60**).

Method F: Yield 91%; ¹H NMR (400 MHz, acetone-*d*₆) δ 9.48 (s, 1H), 8.88 (d, *J* =1.60 Hz, 1H), 8.62-8.61 (m, 2H), 8.46-8.45 (m, 1H), 8.26 (d, *J* =8.00 Hz, 2H), 8.14 (d, *J* =8.40 Hz, 2H), 8.04 (d, *J* =8.40 Hz, 2H), 7.88 (d, *J* =8.00 Hz, 2H), 7.13 (d, *J* =1.60 Hz, 1H), 4.03 (s, 3H); MS (ESI, m/z) 491.1 [M+1]⁺; ESI-HRMS calcd. m/z for C₂₆H₁₈N₄O₃F₃ 491.1331, found 491.1328 [M+1]⁺.

Methyl 4'-(5-(hydroxymethyl)isoxazol-3-yl)-5-(4-(4-(trifluoromethyl)phenyl)-1*H-*1,2,3-triazol-1-yl)-[1,1'-biphenyl]-3-carboxylate (**61**).

Method E: Yield 46%; ¹H NMR (400 MHz, CDCl₃) δ 8.40 (s, 1H), 8.38 (s, 1H), 8.36-8.35 (m, 2H), 8.04 (d, *J* =8.40 Hz, 2H), 7.93 (d, *J* =8.00 Hz, 2H), 7.78 (d, *J* =8.40 Hz, 2H), 7.72 (d, *J* =8.00 Hz, 2H), 6.62 (s, 1H), 4.84 (s, 2H), 4.00 (s, 3H); MS (ESI, m/z) 521.2 [M+1]⁺; ESI-HRMS calcd. m/z for C₂₇H₂₀N₄O₄F₃ 521.1437, found 521.1430 [M+1]⁺.

Methyl 4'-(5-(2-hydroxyethyl)isoxazol-3-yl)-5-(4-(4-(trifluoromethyl)phenyl)-1*H-*1,2,3-triazol-1-yl)-[1,1'-biphenyl]-3-carboxylate (**62**).

Method F: Yield 80%; ¹H NMR (400 MHz, DMSO-*d*₆) δ 9.76 (s, 1H), 8.58 (s, 1H), 8.54 (s, 1H), 8.35 (s, 1H), 8.18 (d, *J* =8.40 Hz, 2H), 8.04-8.00 (m, 4H), 7.90 (d, *J* =8.40 Hz, 2H), 6.94 (s, 1H), 3.97 (s, 3H), 3.77 (q, *J* =6.40 Hz, 2H), 2.97 (d, *J* =6.40 Hz, 2H); MS (ESI, m/z) 535.2 [M+1]⁺; ESI-HRMS calcd. m/z for C₂₈H₂₂N₄O₄F₃ 535.1593, found 535.1592 [M+1]⁺.

Methyl 4'-(2*H*-tetrazol-5-yl)-5-(4-(4-(trifluoromethyl)phenyl)-1*H*-1,2,3-triazol-1-yl)-[1,1'-biphenyl]-3-carboxylate (**63**).

Method C: Yield 53%; ¹H NMR (400 MHz, CD₃OD) δ 9.26 (s, 1H), 8.52 (s, 1H), 8.44 (s, 1H), 8.39 (s, 1H), 8.18 (d, *J* =8.40 Hz, 2H), 8.12 (d, *J* =8.40 Hz, 2H), 7.90 (d, *J* =8.40 Hz, 2H), 7.76 (d, *J* =8.40 Hz, 2H), 4.00 (s, 3H); MS (ESI, m/z) 492.1 [M+1]⁺; ESI-HRMS calcd. m/z for C₂₄H₁₇N₇O₂F₃ 492.1396, found 492.1399 [M+1]⁺.

Ethyl 4-(4-(5-(hydroxymethyl)isoxazol-3-yl)phenyl)-7-(4-(trifluoromethyl)phenyl)-2-naphthoate (**65**).

Method F: Yield 50%; ¹H NMR (400 MHz, DMSO-*d*₆) δ 8.81 (s, 1H), 8.71 (s, 1H), 8.09-8.05 (m, 5H), 7.99-7.97 (m, 1H), 7.94-7.93 (m, 1H), 7.89 (d, *J* =8.00 Hz, 2H), 7.68 (d, *J* =8.00 Hz, 2H), 7.04 (s, 1H), 5.77 (t, *J* =6.00 Hz, 1H), 4.66 (d, *J* =6.00 Hz, 2H), 4.41 (q, *J* =7.20 Hz, 2H), 1.38 (t, *J* =7.20 Hz, 3H); MS (ESI, m/z) 518.2 [M+1]⁺; ESI-HRMS calcd. m/z for C₃₀H₂₃NO₄F₃ 518.1579, found 518.1582 [M+1]⁺.

Methyl 3-amino-5-bromobenzoate (**66**).

3-Bromo-5-aminobenzoic acid (**65**, 1.01 g, 4.62 mmol) was stirred in methanol (15 mL) with ice cooling, and the yellow solution was treated with thionyl chloride (4.00 mL, 55.0 mmol) dropwise over 20 min. The resulting mixture was warm up to room temperature and left stirring for 15 h. The reaction mixture was quenched with aqueous saturated NaHCO₃ solution at 0 °C. The solvent was then removed under vacuum, and the residue was suspended in ethyl acetate (200 mL). The organic phase was washed with brine (100 mL), dried (Na₂SO₄) and concentrated in vacuo to afford the title compound **66** (1.08 g, 98%) as a yellow solid; ¹H NMR (400 MHz, DMSO-*d*₆) δ 7.16 (dd, *J* =1.48, 2.12 Hz, 1H), 7.13 (t, *J* =1.64 Hz, 1H), 6.96 (t, *J* =2.00 Hz, 1H), 5.74 (s, 2H), 3.81 (s, 3H); MS (ESI, m/z) 231 [M+1]⁺; ESI-HRMS calcd. m/z for C₈H₈BrNO₂ 229.9817, found 229.9818 [M+1]⁺.

Methyl 3-bromo-5-(4-(trifluoromethyl)benzamido)benzoate (**67**).

To a solution of compounds **66** (70 mg, 0.304 mmol) in N,N-dimethylformamide (6 mL) were added 4-(Trifluoromethyl)benzoic acid (136 mg, 0.717 mmol), HATU (218 mg, 0.573 mmol) and *N,N*-diisopropylethylamine (250 µL, 1.43 mmol), and then this reaction mixture was stirred at room temperature for 5 h. The reaction mixture was partitioned ethyl acetate (20 mL) and water (10 mL), and the aqueous layer was extracted with ethyl acetate (20 mL x 2). The combined organic layer was washed brine (10 mL), dried over Na₂SO₄, filtered and evaporated under reduced pressure. The residue was purified by silica gel column chromatography (hexane:ethyl acetate=7:1) to afford compound **67** (86 mg, 70%) as a white solid; ¹H NMR (400 MHz, CDCl₃) δ 8.32-8.31 (m, 1H), 8.05-8.04 (m, 1H), 7.99-7.98 (m, 3H), 7.79 (d, *J* =8.40 Hz, 2H), 3.93 (s, 3H); MS (ESI, m/z) 402.0 [M+1]⁺; ESI-HRMS calcd. m/z for C₁₆H₁₂NO₃F₃Br 401.9953, found 401.9948 [M+1]⁺.

Methyl 3-(4,4,5,5-tetramethyl-1,3,2-dioxaborolan-2-yl)-5-(4-(trifluoromethyl)benzamido)benzoate (**68**).

To a solution of compound **67** (40 mg, 0.099 mmol) in 1,4-dioxane (3 mL) were added bis(pinacolato)diboron (63 mg, 0.248 mmol), PdCl₂(dppf) (2.0 mg, 0.002 µmol) and potassium acetate (30 mg, 0.298 mmol), and then this reaction mixture was stirred at 70 °C for 6 h. The reaction mixture was partitioned ethyl acetate (20 mL) and water (10 mL), and the aqueous layer was extracted with ethyl acetate (10 mL x 2). The combined organic layer was washed brine (5 mL), dried over Na₂SO₄, filtered and evaporated under reduced pressure. The residue was purified by silica gel column chromatography (hexane:ethyl acetate=2:1) to afford compound **68** (29.0 mg, 65%) as a white solid; ¹H NMR (400 MHz, CDCl₃) δ 8.50 (s, 1H), 8.29 (s, 1H), 8.15 (s, 1H), 7.99 (d, *J* =8.00 Hz, 2H), 7.78 (d, *J* =8.40 Hz, 2H), 3.93 (s, 3H), 1.35 (s, 12H); MS (ESI, m/z) 450.2 [M+1]⁺; ESI-HRMS calcd. m/z for C₂₂H₂₄NO₅F₃B 450.1700, found 450.1701 [M+1]⁺.

Methyl 4'-(5-(hydroxymethyl)isoxazol-3-yl)-5-(4-(trifluoromethyl)benzamido)-[1,1'-biphenyl]-3-carboxylate (**69**).

Method C: Yield 45%; ¹H NMR (400 MHz, CD₃OD) δ 8.44 (m, 1H), 8.36-8.35 (m, 1H), 8.17-8.13 (m, 3H), 7.98 (d, *J* =8.40 Hz, 2H), 7.87-7.82 (m, 4H), 6.83 (s, 1H), 4.74 (s, 2H), 3.97 (s, 3H); MS (ESI, m/z) 497.1 [M+1]⁺; ESI-HRMS calcd. m/z for C₂₆H₂₀N₂O₅F₃ 497.1324, found 497.1323 [M+1]⁺.

Methyl 2-amino-6-bromoisonicotinate (**72**).

Compound **69** (200 mg, 0.922 mmol) was converted to compound **72** (83 mg, 40%) as a white solid, using a similar procedure to that used in the preparation of compound **66**; ¹H NMR (400 MHz, CD₃OD) δ 7.13 (d, 1H), 7.00 (d, 1H), 3.89 (s, 3H); MS (ESI, m/z) 231.0 [M+1]⁺; ESI-HRMS calcd. m/z for C₇H₈BrN₂O₂ 230.9769, found 230.9769 [M+1]⁺.

Methyl 4-amino-6-bromopicolinate (**73**).

Compound **70** (320 mg, 1.47 mmol) was converted to compound **73** (136 mg, 40%) as a white solid, using a similar procedure to that used in the preparation of compound **66**; ¹H NMR (400 MHz, DMSO-*d*₆) δ 7.23 (d, 1H), 6.79 (d, 1H), 3.81 (s, 3H); MS (ESI, m/z) 231.0 [M+1]⁺; ESI-HRMS calcd. m/z for C₇H₈BrN₂O₂ 230.9769, found 230.9765 [M+1]⁺.

Methyl 6-azido-4-bromopicolinate (**74**).

Compound **71** (121 mg, 0.523 mmol) was dissolved in acetonitrile (4 mL) and cooled to 0 °C in an ice bathe. *tert*-Butyl nitrite (124 µL, 1.047 mmol) was added to the reaction mixture and stirred at 0 °C for 30 min. After that, azidotrimethylsilane (206 µL, 1.57 mmol) was added to the reaction mixture at 0 °C by dropwise. The resulting solution was stirred at room temperature for 12 h. The reaction mixture was concentrated under reduced pressure. The residue was purified by silica gel column chromatography (hexane:ethyl acetate=3:1) to afford compound **74** (50 mg, 37%) as a white solid; ¹H NMR (400 MHz, CDCl₃) δ 7.98 (s, 1H), 7.26 (s, 1H), 3.99 (s, 3H).

Methyl 2-azido-6-bromoisonicotinate (**75**).

Compound **72** (27 mg, 0.107 mmol) was converted to compound **75** (30 mg, 65%) as a white solid, using a similar procedure to that used in the preparation of compound **74**; ¹H NMR (400 MHz, CDCl₃) δ 7.76 (d, 1H), 7.29 (d, 1H), 3.94 (s, 3H).

Methyl 4-azido-6-bromopicolinate (**76**).

Compound **73** (21 mg, 0.090 mmol) was converted to compound **76** (12 mg, 52%) as a white solid, using a similar procedure to that used in the preparation of compound **74;** ¹H NMR (400 MHz, CDCl₃) δ 7.75 (broad s, 1H), 7.31 (broad s, 1H), 4.02 (s, 3H).

Methyl 4-bromo-6-(4-(4-(trifluoromethyl)phenyl)-1*H*-1,2,3-triazol-1-yl)picolinate (77).

To a solution of compound **74** (50 mg, 0.195 mmol) in *tert*-BuOH:water (5 mL, 1:1) were added 4-Ethynyl-*α*,*α*,*α*-trifluorotoluene (50 µL, 0.292 mmol) and Tris[(1-benzyl-1*H*-1,2,3-triazol-4-yl)methyl]amine (TBTA, 7 mg, 0.014 mmol). Subsequently, sodium ascorbate (58 mg, 0.292 mmol, freshly prepared 1 M aqueous solution) and CuSO₄·5H₂O (37 mg, 0.146 mmol, freshly prepared 7.5% aqueous solution) were added into the reaction mixture, and then this reaction mixture was stirred at room temperature for 1.5 h. The solvent of reaction mixture was evaporated under reduced pressure. The residue was purified by silica gel column chromatography (hexane:ethyl acetate=3:1) to afford compound **77** (30 mg, 36%) as a white solid; ¹H NMR (400 MHz, CDCl₃) δ 8.98 (s, 1H), 8.65 (d, 1H), 8.31 (d, 1H), 8.08 (d, *J* =8.00 Hz, 2H), 7.74 (d, *J* =8.00 Hz, 2H), 4.06 (s, 3H); MS (ESI, m/z) 427.0 [M+1]⁺; ESI-HRMS calcd. m/z for C₁₆H₁₁BrNaO₂F₃ 427.0017, found 427.0013 [M+1]⁺.

Methyl 2-bromo-6-(4-(4-(trifluoromethyl)phenyl)-1*H*-1,2,3-triazol-1-yl)isonicotinate (**78**).

Compound **75** (27 mg, 0.107 mmol) was converted to compound **78** (30 mg, 65%) as a white solid, using a similar procedure to that used in the preparation of compound **77;** ¹H NMR (400 MHz, CD₃OD) δ 9.28 (s, 1H), 8.65 (d, 1H), 8.20-8.18 (m, 3H), 7.79 (d, *J* =8.00 Hz, 2H), 4.03 (s, 3H); MS (ESI, m/z) 427.0 [M+1]⁺; ESI-HRMS calcd. m/z for C₁₆H₁₁BrN₄O₂F₃ 427.0017, found 427.0018 [M+1]⁺.

Methyl 6-bromo-4-(4-(4-(trifluoromethyl)phenyl)-1H-1,2,3-triazol-1-yl)picolinate (**79**).

Compound **76** (7.0 mg, 0.027 mmol) was converted to compound **79** (8.0 mg, 69%) as a white solid, using a similar procedure to that used in the preparation of compound **77;** ¹H NMR (400 MHz, CD₃OD) δ 9.24 (s, 1H), 8.68 (s, 1H), 8.42 (s, 1H), 8.08 (d, *J* =8.00 Hz, 2H), 7.73 (d, *J* =8.00 Hz, 2H), 4.04 (s, 3H); MS (ESI, m/z) 427.0 [M+1]⁺; ESI-HRMS calcd. m/z for C₁₆H₁₁BrN₄O₂F₃ 427.0017, found 427.0022 [M+1]⁺.

Methyl 4-(4-(1-(*tert*-butoxycarbonyl)piperidin-4-yl)phenyl)-6-(4-(4-(trifluoromethyl)phenyl)-1*H*-1,2,3-triazol-1-yl)picolinate (**80**).

Method C: Yield 35%; ¹H NMR (400 MHz, CDCl₃) δ 9.05 (s, 1H), 8.67 (d, 1H), 8.41 (d, 1H), 8.10 (d, *J* =8.00 Hz, 2H), 7.78-7.73 (m, 4H), 7.41 (d, *J* =8.40 Hz, 2H), 4.35-4.21 (m, 2H), 2.90-2.79 (m, 3H), 1.93-1.89 (m, 2H), 1.75-1.63 (m, 2H), 1.49 (s, 9H); MS (ESI, m/z) 608.3 [M+1]⁺.

Methyl 2-(4-(1-(*tert*-butoxycarbonyl)piperidin-4-yl)phenyl)-6-(4-(4-(trifluoromethyl)phenyl)-1*H*-1,2,3-triazol-1-yl)isonicotinate (**81**).

Method C: Yield 63%; ¹H NMR (400 MHz, CD₃OD) δ 9.36 (s, 1H), 8.49 (s, 1H), 8.38 (s, 1H), 8.20-8.16 (m, 4H), 7.78 (d, *J* =8.40 Hz, 2H), 7.41 (d, *J* =8.40 Hz, 2H), 4.26-4.16 (m, 2H), 4.04 (s, 3H), 2.95-2.77 (m, 3H), 1.88-1.85 (m, 2H), 1.69-1.59 (m, 2H), 1.49 (s, 9H); MS (ESI, m/z) 608.3 [M+1]⁺; ESI-HRMS calcd. m/z for C₃₂H₃₃N₅O₄F₃ 608.2485, found 608.2492 [M+1]⁺.

Methyl 6-(4-(1-(tert-butoxycarbonyl)piperidin-4-yl)phenyl)-4-(4-(4-(trifluoromethyl)phenyl)-1*H*-1,2,3-triazol-1-yl)picolinate (**82**).

Method C: Yield 91%; ¹H NMR (400 MHz, CD₃OD) δ 9.44-9.36 (m, 1H), 8.57-8.46 (m, 2H), 8.15-8.10 (m, 4H), 7.81-7.76 (m, 2H), 7.40-7.35 (m, 2H), 4.24-4.21 (m, 2H), 4.04 (s, 3H), 2.88-2.77 (m, 3H), 1.86-1.84 (m, 2H), 1.65-1.62 (m, 2H), 1.49 (s, 9H); MS (ESI, m/z) 608.2 [M+1]⁺; ESI-HRMS calcd. m/z for C₃₂H₃₃N₅O₄F₃ 608.2485, found 608.2474 [M+1]⁺.

4-(4-(1-(*tert*-Butoxycarbonyl)piperidin-4-yl)phenyl)-6-(4-(4-(trifluoromethyl)phenyl)-1*H*-1,2,3-triazol-1-yl)picolinic acid (**83**).

Method A: Yield 68%; ¹H NMR (400 MHz, DMSO-*d*₆) δ 9.64 (s, 1H), 8.57 (s, 1H), 8.41 (s, 1H), 8.30 (d, *J* =8.00 Hz, 2H), 7.94 (d, *J* =8.40 Hz, 2H), 7.87 (d, *J* =8.00 Hz, 2H), 7.48 (d, *J* =8.40 Hz, 2H), 4.11-4.05 (m, 2H), 2.90-2.73 (m, 3H), 1.82-1.79 (m, 2H), 1.60-1.50 (m, 2H), 1.42 (s, 9H); MS (ESI, m/z) 594.2 [M+1]⁺; ESI-HRMS calcd. m/z for C₃₁H₃₁N₅O₄F₃ 594.2328, found 594.2320 [M+1]⁺.

2-(4-(1-(*tert*-Butoxycarbonyl)piperidin-4-yl)phenyl)-6-(4-(4-(trifluoromethyl)phenyl)-1*H*-1,2,3-triazol-1-yl)isonicotinic acid (**84**).

Method A: Yield 91%; ¹H NMR (400 MHz, CD₃OD) δ 9.38 (s, 1H), 8.53 (s, 1H), 8.42 (s, 1H), 8.19 (t, *J* =8.40 Hz, 4H), 7.79 (d, *J* =8.40 Hz, 2H), 7.42 (d, *J* =8.00 Hz, 2H), 4.26-4.15 (m, 2H), 2.96-2.73 (m, 3H), 1.89-1.76 (m, 2H), 1.70-1.55 (m, 2H), 1.49 (s, 9H); MS (ESI, m/z) 616.3 [M+Na]⁺; ESI-HRMS calcd. m/z for C₃₁H₃₀N₅O₄F₃Na 616.2148, found 616.2158 [M+Na]⁺.

6-(4-(1-(tert-Butoxycarbonyl)piperidin-4-yl)phenyl)-4-(4-(4-(trifluoromethyl)phenyl)-1H-1,2,3-triazol-1-yl)picolinic acid (85).

Method A: Yield 77%; ¹H NMR (400 MHz, CD₃OD) δ 9.27 (s, 1H), 8.58-8.52 (m, 2H), 8.10 (m, 4H), 7.74 (d, *J* =7.60 Hz, 2H), 7.39 (broad s, 2H), 4.24-4.21 (m, 2H), 2.89-2.73 (m, 3H), 1.89-1.86 (m, 2H), 1.70-1.60 (m, 2H), 1.47 (s, 9H); MS (ESI, m/z) 594.2 [M+1]⁺; ESI-HRMS calcd. m/z for C₃₁H₃₁N₅O₄F₃ 594.2328, found 594.2318 [M+1]⁺.

tert-Butyl 4-(3'-((2-(dimethylamino)-2-oxoethoxy)carbonyl)-5'-(4-(4-(trifluoromethyl)phenyl)-1*H*-1,2,3-triazol-1-yl)-[1,1'-biphenyl]-4-yl)piperidine-1-carboxylate (**87**).

To a solution of **86** (166 mg, 0.280 mmol), which was synthesized according to literature procedures reported,¹ in DMF (10 mL) was added K₂CO₃ (116 mg, 0.840 mmol). 2-Chloro-*N*,*N*-dimethylacetamide (68 mg, 0.560 mmol) was added to the reaction mixture under N₂ atmosphere, and the reaction was stirred at 45 °C for 1 h under N₂ atmosphere. The reaction mixture was partitioned ethyl acetate (10 mL) and water (10 mL), and the aqueous phase was extracted with ethyl acetate (2 x 10 mL). The combined organic layers were dried over Na₂SO₄, filtered, and concentrated under pressure. The residue was purified by silica gel chromatography (hexane:ethyl acetate = 3:1) to afford compound **87** (205 mg, 70%) as a white solid; ¹H NMR (400 MHz, CDCl₃) δ 8.43-8.41 (m, 2H), 8.38-8.36 (m, 2H), 8.05 (d, *J* =8.00 Hz, 2H), 7.73 (d, *J* =8.40 Hz, 2H), 7.65 (d, *J* =8.40 Hz, 2H), 7.34 (d, *J* =8.40 Hz, 2H), 5.05 (s, 2H), 3.76-3.72 (m, 2H), 3.07 (s, 3H), 3.02 (s, 3H), 2.87-2.77 (m, 2H), 2.76-2.67 (m, 1H), 1.89-1.83 (m, 2H), 1.72-1.62 (m, 2H), 1.49 (s, 9H); MS (ESI, m/z) 622.2 [M+1-*tert*-butyl]⁺.

*tert*-Butyl 4-(3'-(hydroxymethyl)-5'-(4-(4-(trifluoromethyl)phenyl)-1*H*-1,2,3-triazol-1-yl)-[1,1'-biphenyl]-4-yl)piperidine-1-carboxylate (**89**).

To a solution of **88** (35 mg, 0.058 mmol), which was synthesized according to literature procedures reported,¹ in THF at 0 °C, 2 M lithium aluminum hydride solution in THF (2.6 mg, 0.070 mmol) was added dropwise over a period of 0.5 h under N₂ atmosphere. After the addition was complete, the reaction mixture was stirred at 0 °C under N₂ atmosphere for 1 h. The unreacted LAH was quenched with saturated aqueous NH₄Cl and the reaction mixture was extracted with dichloromethane 3 times. The combined organic extracts were dried with anhydrous Na₂SO₄, and the mixture was filtered through celite pad. The filtered mixture was concentrated in vacuum and chromatographed on silica gel with hexanes/ethyl acetate = 3:1 to afford **89** (15 mg, 45%) as a white solid; ¹H NMR (400 MHz, CD₃OD) δ 9.16 (s, 1H), 8.15 (d, *J* =8.00 Hz, 2H), 8.05 (s, 1H), 7.90 (s, 1H), 7.80-7.76 (m, 3H), 7.70 (d, *J* =8.40 Hz, 2H), 7.38 (d, *J* =8.40 Hz, 2H), 4.80 (s, 2H), 4.25-4.22 (m, 2H), 2.96-2.77 (m, 3H), 1.88-1.85 (m, 2H), 1.68-1.59 (m, 2H), 1.49 (s, 9H); MS (ESI, m/z) 579.2 [M+1]+; ESI-HRMS calcd. m/z for C₃₂H₃₄N₄O₃F₃ 579.2583, found 579.2588 [M+1]+.

*tert*-Butyl 4-(3'-(((di-tert-butoxyphosphoryl)oxy)methyl)-5'-(4-(4-(trifluoromethyl)phenyl)-1*H-*1,2,3-triazol-1-yl)-[1,1'-biphenyl]-4-yl)piperidine-1-carboxylate (**90**).

A stirred solution of **89** (12 mg, 0.020 mmol) in dry THF (1.0 mL) was treated successively with tetrazole (17 mg, 0.242 mmol) and di-*tert*-butyl *N,N*-diethylphosphoramidite (115 µL, 0.414 mmol) at room temperature under N₂ atmosphere. The resulting solution was stirred at room temperature for 1 h under N₂ atmosphere, and the reaction mixture was cooled to -78 °C. 3-Chloroperbenzoic acid (MCPBA, contains 50%, 78 mg) was added, and the reaction mixture was warmed to 0 °C and stirred for 0.5 h. The reaction mixture was partitioned ethyl acetate (10 mL) and aqueous saturated NaHCO₃ solution (10 mL), and the aqueous phase was extracted with ethyl acetate (2 x 10 mL). The combined organic layers were dried over Na₂SO₄, filtered, and concentrated under pressure. The residue was purified by silica gel chromatography (chloroform:methanol = 30:1) to afford compound **90** (13 mg, 84%) as a white solid; ¹H NMR (400 MHz, CD₃OD) δ 9.22 (s, 1H), 8.18-8.16 (m, 3H), 8.01 (s, 1H), 7.83-7.79 (m, 3H), 7.73 (d, *J* =8.40 Hz, 2H), 7.41 (d, *J* =8.40 Hz, 2H), 5.23-5.22 (m, 2H), 4.27-4.23 (m, 2H), 2.96-2.77 (m, 3H), 1.91-1.87 (m, 2H), 1.71-1.59 (m, 2H), 1.52-1.50 (m, 27H); MS (ESI, m/z) 793.3 [M+Na]+; ESI-HRMS calcd. m/z for C₄₀H₅₀N₄O₆F₃PNa 793.3318, found 793.3303 [M+Na]+.

### EXAMPLE 2

4-(4-(Pyrrolidin-3-yl)phenyl)-7-(4-(trifluoromethyl)phenyl)-2-naphthoic acid (**105**). **Procedure A.** Into a solution of **175** (15.7 mg, 0.032 mmol) in MeOH (2 mL) was added aq. potassium hydroxide (0.2 M, 1 mL). The resulting mixture was stirred at 50 °C overnight. The reaction was quenched and neutralized by 1 M HCl to pH 6. Volatiles were evaporated, and the residue was purified by RP-HPLC (C18, A: ACN, B: 10 mM TEAA, 45% → 60% A in 40 min, flow rate = 5 mL/min, t_{R} = 26 min) to give stereoisomeric mixture **105** as a white powder (8.6 mg, 58%): ¹H NMR (400 MHz, DMSO) δ 8.48 (s, 1H), 8.41 (d, *J =* 2.0 Hz, 1H), 8.06 (d, *J =* 8.1 Hz, 2H), 7.98 (d, *J =* 1.5 Hz, 1H), 7.94 - 7.80 (m, 4H), 7.48 - 7.39 (m, 4H), 3.30 - 3.15 (m, 2H), 3.06 - 2.97 (m, 1H), 2.97 - 2.89 (m, 1H), 2.77 - 2.68 (m, 1H), 2.27 - 2.15 (m, 1H), 1.85 - 1.72 (m, 1H). HRMS m/z [M+H]⁺ for C₂₈H₂₃NO₂F₃ calculated 462.1681, found 462.1689.

4-(4-(3-Methylpiperidin-4-yl)phenyl)-7-(4-(trifluoromethyl)phenyl)-2-naphthoic acid (**106**). Treatment of **165** (13.2 mg, 0.026 mmol) by using Procedure A gave stereoisomeric mixture **106** (5.4 mg, 42%) as a white powder: ¹H NMR (400 MHz, methanol-*d*₄) δ 8.65 (s, 1H), 8.34 (d, *J=* 1.9 Hz, 1H), 8.02 - 7.92 (m, 4H), 7.84 - 7.74 (m, 3H), 7.48 - 7.36 (m, 4H), 3.61 - 3.36 (m, 2H), 3.25 - 2.54 (m, 4H), 2.31 - 2.07 (m, 1H), 1.34 - 1.29 (m, 1H), 1.20 - 1.13 (m, 3H). HRMS m/z [M+H]⁺ for C₃₀H₂₇NO₂F₃ calculated 490.1994, found 490.1990.

4-(4-(2-Methylpiperidin-4-yl)phenyl)-7-(4-(trifluoromethyl)phenyl)-2-naphthoic acid (**107**). Treatment of **166** (11 mg, 0.021 mmol) by using Procedure A gave stereoisomeric mixture **7** (8.0 mg, 76%) as a white powder: ¹H NMR (400 MHz, methanol-*d*₄) δ 8.62 (s, 1H), 8.30 (d, *J* = 2.0 Hz, 1H), 8.03 (s, 1H), 7.96 - 7.91 (m, 2H), 7.77 (t, *J =* 9.3 Hz, 2H), 7.69 - 7.50 (m, 3H), 7.49 - 7.37 (m, 3H), 3.52 (t, *J =* 15.3 Hz, 1H), 3.45 - 3.25 (m, 1H), 3.25 - 3.10 (m, 1H), 3.09 - 2.98 (m, 1H), 2.22 - 2.04 (m, 2H), 1.90 - 1.62 (m, 1H), 1.41 (d, *J* = 6.5 Hz, 2.2H), 1.36 (d, *J =* 6.5 Hz, 0.8H), 0.97 - 0.84 (m, 1H). HRMS m/z [M+H]⁺ for C₃₀H₂₇NO₂F₃ calculated 490.1994, found 490.1996.

4-(4-(3-Azabicyclo[4.1.0]heptan-6-yl)phenyl)-7-(4-(trifluoromethyl)phenyl)-2-naphthoic acid (**108**). Treatment of **168** (8 mg, 0.0156 mmol) by using Procedure A gave diastereomeric mixture **108** (5.6 mg, 74%) as a white powder: ¹H NMR (400 MHz, methanol-*d*₄) δ 8.75 - 8.70 (m, 1H), 8.40 (d, *J =* 1.9 Hz, 1H), 8.02 - 7.87 (m, 5H), 7.80 (d, *J =* 8.2 Hz, 2H), 7.58 (d, *J =* 8.1 Hz, 2H), 7.49 (d, *J =* 8.2 Hz, 2H), 3.85 (dd, *J =* 13.5, 7.8 Hz, 1H), 3.36 - 3.32 (m, 2H), 2.97 (ddd, *J =* 13.2, 9.4, 5.9 Hz, 1H), 2.51 - 2.40 (m, 2H), 1.75 - 1.64 (m, 1H), 1.37 (dd, *J* = 9.1, 5.6 Hz, 1H), 1.18 (t, *J =* 5.6 Hz, 1H). HRMS m/z [M+H]⁺ for C₃₀H₂₅NO₂F₃ calculated 488.1837, found 488.1840.

4-(4-(7,7-Difluoro-3-azabicyclo[4.1.0]heptan-6-yl)phenyl)-7-(4-(trifluoromethyl)phenyl)-2-naphthoic acid (**109**). Treatment of **169** (5.6 mg, 0.01 mmol) by using Procedure A gave diastereomeric mixture **109** (2.7 mg, 52%) as a white powder: ¹H NMR (400 MHz, methanol-*d*₄) δ 8.63 (s, 1H), 8.37 (s, 1H), 8.02 (s, 1H), 7.96 (d, *J =* 8.1 Hz, 2H), 7.84 (d, *J* = 8.9 Hz, 1H), 7.78 (d, *J =* 8.1 Hz, 2H), 7.72 (d, *J =* 8.8 Hz, 1H), 7.54 (d, *J =* 7.7 Hz, 2H), 7.41 (d, *J =* 7.8 Hz, 2H), 3.82 (t, *J =* 11.6 Hz, 1H), 3.33 - 3.25 (m, 1H), 3.22 - 3.14 (m, 1H), 2.95 - 2.82 (m, 1H), 2.51 (d, *J* = 14.9 Hz, 1H), 2.38 (t, *J* = 11.0 Hz, 1H), 2.27 - 2.15 (m, 1H). HRMS m/z [M+H]⁺ for C₃₀H₂₃NO₂F₅ calculated 524.1649, found 524.1655.

*rac*-4-(4-((1*S*,5*R*,6*S*)-2-Azabicyclo[4.1.0]heptan-5-yl)phenyl)-7-(4-(trifluoromethyl)phenyl)-2-naphthoic acid (**110**). Treatment of **170** (9.8 mg, 0.02 mmol) by using Procedure A gave racemate **110** (2.4 mg, 25%) as a white powder: ¹H NMR (400 MHz, methanol-*d*₄) δ 8.56 (s, 1H), 8.35 (d, *J =* 2.0 Hz, 1H), 8.06 - 8.02 (m, 1H), 7.99 (d, *J =* 8.9 Hz, 3H), 7.86 - 7.76 (m, 3H), 7.57 (d, *J* = 8.1 Hz, 2H), 7.52 (d, *J* = 8.2 Hz, 2H), 3.26 (t, *J* = 6.6 Hz, 1H), 2.83 - 2.73 (m, 1H), 2.61 - 2.49 (m, 2H), 1.87 - 1.81 (m, 1H), 1.62 - 1.52 (m, 1H), 1.24 - 1.16 (m, 1H), 0.85 (ddd, *J =* 9.5, 7.3, 5.5 Hz, 1H), 0.67 - 0.59 (m, 1H). HRMS m/z [M+H]⁺ for C₃₀H₂₅NO₂F₃ calculated 488.1837, found 488.1831.

*rac-*4-(4-((1*R*,5*R*,6*R*)-2-Azabicyclo[4.1.0]heptan-5-yl)phenyl)-7-(4-(trifluoromethyl)phenyl)-2-naphthoic acid (**111**). Treatment of **171** (15.4 mg, 0.03 mmol) by using Procedure A gave racemate **111** (0.16 mg, 1%) as a white powder: ¹H NMR (400 MHz, methanol-*d*₄) δ 8.56 (s, 1H), 8.35 (s, 1H), 8.06 - 7.96 (m, 4H), 7.86 - 7.76 (m, 3H), 7.57 (d, *J* = 8.1 Hz, 1H), 7.52 (d, *J =* 8.1 Hz, 1H), 3.27 - 3.16 (m, 1H), 2.85 - 2.67 (m, 2H), 2.60 (s, 1H), 1.91 - 1.86 (m, 1H), 1.57 (d, *J =* 27.1 Hz, 1H), 1.25 (s, 1H), 0.89 (s, 1H), 0.73 - 0.62 (m, 1H). HRMS m/z [M+H]⁺ for C₃₀H₂₅NO₂F₃ calculated 488.1837, found 488.1833.

*rac*-4-(4-((1*S*,4*S*,5*S*)-2-Azabicyclo[2.2.1]heptan-5-yl)phenyl)-7-(4-(trifluoromethyl)phenyl)-2-naphthoic acid (**112**). Treatment of **167** (9.3 mg, 0.018 mmol) by using Procedure A gave enantiomeric mixture **112** (4.1 mg, 47%) as a white powder: ¹H NMR (400 MHz, DMSO) δ 8.46 (s, 1H), 8.39 (s, 1H), 8.04 (d, *J =* 8.0 Hz, 2H), 7.96 (s, 1H), 7.94 - 7.78 (m, 4H), 7.45 - 7.35 (m, 4H), 3.41 - 3.37 (m, 1H), 2.97 (dd, *J* = 9.1, 5.4 Hz, 1H), 2.79 (dd, *J =* 9.2, 3.5 Hz, 1H), 2.63 (d, *J =* 9.4 Hz, 1H), 2.43 - 2.35 (m, 1H), 2.00 (t, *J =* 10.8 Hz, 1H), 1.75 (dt, *J =* 12.3, 4.6 Hz, 1H), 1.64 - 1.57 (m, 1H), 1.37 (d, *J =* 9.6 Hz, 1H). HRMS m/z [M+H]⁺ for C₃₀H₂₅NO₂F₃ calculated 488.1837, found 488.1842.

*rac*-4-(4-((1*S*,4*S*,5*S*)-2-Acetyl-2-azabicyclo[2.2.1]heptan-5-yl)phenyl)-7-(4-(trifluoromethyl)phenyl)-2-naphthoic acid (**113**). Into the solution of **112** (0.9 mg, 0.0019 mmol) and TEA (3.2 µL, 2.3 mg, 0.023 mmol) in DMF (0.5 mL) was added acetic anhydride (1.8 µL, 2 mg, 0.019 mmol). The solution was stirred at rt for 90 min. Water (10 µL, 10 mg, 0.56 mmol) was added and the reaction mixture was stirred for another 1 h. The reaction residue was purified by RP-HPLC (C18, A: ACN, B: 10 mM TEAA, 45% → 60% A in 40 min, flow rate = 5 mL/min, t_{R} = 26 min) to give stereoisomeric mixture **113** as a white powder (0.5 mg, 53%): ¹H NMR (400 MHz, methanol-*d*₄) δ 8.58 (s, 1H), 8.36 (d, *J =* 1.9 Hz, 1H), 8.05 - 7.96 (m, 4H), 7.83 (dd, *J* = 8.9, 2.1 Hz, 1H), 7.80 (d, *J* = 8.2 Hz, 2H), 7.51 (d, *J* = 8.1 Hz, 2H), 7.46 (d, *J* = 8.0 Hz, 2H), 4.69 (s, 0.4H), 4.46 (s, 0.6H), 3.60 (dd, *J* = 9.3, 3.4 Hz, 0.4H), 3.50 - 3.39 (m, 1H), 3.38 - 3.33 (m, 0.6H), 3.25 - 3.14 (m, 1H), 2.85 (d, *J =* 9.1 Hz, 1H), 2.31 (dt, *J =* 19.5, 10.3 Hz, 1H), 2.15 (s, 1.8H), 2.10 - 2.06 (m, 0.6H), 2.05 (s, 1.2H), 2.04 - 1.90 (m, 1.4H), 1.81 (d, *J* = 10.3 Hz, 0.6H), 1.71 (d, *J* = 10.5 Hz, 0.4H). HRMS m/z [M+H]⁺ for C₃₂H₂₇NO₂F₃ calculated 530.1943, found 530.1939.

4-(4-((1*S*,4*S*,5*S*)-2-Azabicyclo[2.2.1]heptan-5-yl)phenyl)-7-(4-(trifluoromethyl)phenyl)-2-naphthoic acid (**114**). **Procedure B.** LiOH (14.8 mg, 0.62 mmol) was added to a solution of **184** (12.7 mg, 0.025 mmol) in THF-H₂O-MeOH (3:1:1, 2 mL). The resulting mixture was sonicated for 30 s and stirred at rt for 3 h. The reaction was quenched and neutralized by 1 M HCl. Volatiles were evaporated, and the residue was purified by RP-HPLC (C18, A: ACN, B: 10 mM TEAA, 45% → 60% A in 40 min, flow rate = 5 mL/min, t_{R} = 28 min) to give **114** as a white powder (5.3 mg, 44%): ¹H NMR (400 MHz, methanol-*d*₄) δ 8.74 (s, 1H), 8.42 (s, 1H), 8.04 - 7.95 (m, 4H), 7.95 - 7.88 (m, 1H), 7.80 (d, *J* = 8.0 Hz, 2H), 7.56 - 7.46 (m, 4H), 4.25 (s, 1H), 3.35 - 3.26 (m, 3H), 2.96 (s, 1H), 2.46 - 2.35 (m, 1H), 2.18 (d, *J =* 15.1 Hz, 1H), 2.11 (d, *J =* 11.7 Hz, 1H), 1.81 (d, *J =* 11.8 Hz, 1H). HRMS m/z [M+H]⁺ for C₃₀H₂₅NO₂F₃ calculated 488.1837, found 488.1846.

4-(4-((1*R*,4*R*,5*R*)-2-Azabicyclo[2.2.1]heptan-5-yl)phenyl)-7-(4-(trifluoromethyl)phenyl)-2-naphthoic acid (**115**). Treatment of **300** (7.56 mg, 0.0147 mmol) by using Procedure B gave **115** (3.9 mg, 54%) as a white powder: ¹H NMR (400 MHz, methanol-*d*₄) δ 8.74 (s, 1H), 8.42 (s, 1H), 8.04 - 7.95 (m, 4H), 7.95 - 7.89 (m, 1H), 7.80 (d, *J=* 8.1 Hz, 2H), 7.56 - 7.46 (m, 4H), 4.25 (s, 1H), 3.32 - 3.26 (m, 3H), 2.96 (s, 1H), 2.40 (dd, *J* = 14.6, 9.4 Hz, 1H), 2.22 - 2.14 (m, 1H), 2.11 (d, *J =* 11.8 Hz, 1H), 1.81 (d, *J =* 11.7 Hz, 1H). HRMS m/z [M+H]⁺ for C₃₀H₂₅NO₂F₃ calculated 488.1837, found 488.1835.

4-(4-((1*R*,4*R*,6*S*)-2-Azabicyclo[2.2.1]heptan-6-yl)phenyl)-7-(4-(trifluoromethyl)phenyl)-2-naphthoic acid (**116**). Treatment of **189** (3.46 mg, 0.0067 mmol) by using Procedure B gave **116** (1 mg, 30%) as a white powder: ¹H NMR (400 MHz, methanol-*d*₄) δ 8.77 (d, *J =* 1.5 Hz, 1H), 8.45 (d, *J =* 1.9 Hz, 1H), 8.02 (d, *J =* 7.3 Hz, 3H), 7.99 (d, *J =* 1.7 Hz, 1H), 7.95 (dd, *J =* 8.9, 2.0 Hz, 1H), 7.83 (d, *J =* 8.2 Hz, 2H), 7.57 (d, *J =* 8.2 Hz, 2H), 7.52 (d, J = 8.2 Hz, 2H), 4.25 (s, 1H), 3.48 (t, *J* = 7.6 Hz, 1H), 3.28 (d, *J =* 11.0 Hz, 1H), 3.22 - 3.16 (m, 1H), 2.95 (s, 1H), 2.29 - 2.17 (m, 2H), 2.14 (d, *J =* 11.8 Hz, 1H), 1.83 (d, *J =* 11.7 Hz, 1H). HRMS m/z [M+H]⁺ for C₃₀H₂₅NO₂F₃ calculated 488.1837, found 488.1844.

4-(4-((1*S*,4*S*,6*R*)-2-Azabicyclo[2.2.1]heptan-6-yl)phenyl)-7-(4-(trifluoromethyl)phenyl)-2-naphthoic acid (**117**). Treatment of **105** (23 mg, with 17% TEA;0.037 mmol) by using Procedure B gave **117** (6.5 mg, 36%) as a white powder: ¹H NMR (400 MHz, methanol-*d*₄) δ 8.77 (d, *J* = 1.6 Hz, 1H), 8.45 (d, *J* = 2.0 Hz, 1H), 8.04 - 7.92 (m, 5H), 7.82 (d, *J* = 8.0 Hz, 2H), 7.56 (d, *J =* 8.0 Hz, 2H), 7.51 (d, *J =* 8.0 Hz, 2H), 4.24 (s, 1H), 3.47 (t, *J =* 7.6 Hz, 1H), 3.27 - 3.24 (m, 1H), 3.18 (d, *J =* 10.9 Hz, 1H), 2.94 (s, 1H), 2.27 - 2.18 (m, 2H), 2.13 (d, *J* = 12.0 Hz, 1H), 1.83 (d, *J =* 11.8 Hz, 1H). HRMS m/z [M+H]⁺ for C₃₀H₂₅NO₂F₃ calculated 488.1837, found 488.1837.

4-(4-((1*S*,4*S*,5*S*)-2-Acetyl-2-azabicyclo[2.2.1]heptan-5-yl)phenyl)-7-(4-(trifluoromethyl)phenyl)-2-naphthoic acid (**118**). Acetic anhydride (4 µL, 4.2 mg, 0.07 mmol) was added to a solution of **114** (3.5 mg, 0.007 mmol) and TEA (11.6 µL, 8.5 mg, 0.084 mmol) in DMF (0.5 mL). The solution was stirred at rt for 90 min. Water (10 µL, 10 mg, 0.56 mmol) was added and the reaction mixture was stirred for another 1 h. The reaction residue was purified by RP-HPLC (C18, A: ACN, B: 10 mM TEAA, 45% → 60% A in 40 min, flow rate = 5 mL/min, t_{R} = 26 min) to give **118** as a white powder (3 mg, 81%): ¹H NMR (400 MHz, methanol-*d*₄) δ 8.63 (s, 1H), 8.39 (d, *J =* 2.0 Hz, 1H), 8.06 - 7.98 (m, 4H), 7.87 (dd, *J =* 8.9, 2.0 Hz, 1H), 7.81 (d, *J* = 8.2 Hz, 2H), 7.52 (d, *J =* 8.3 Hz, 2H), 7.48 (d, *J =* 8.1 Hz, 2H), 4.71 (s, 0.5H), 4.47 (s, 0.5H), 3.62 (dd, *J =* 9.3, 3.4 Hz, 0.5H), 3.48 - 3.39 (m, 1H), 3.38 - 3.34 (m, 0.5H), 3.29 - 3.23 (m, 1H), 2.87 (d, *J=* 9.7 Hz, 1H), 2.40 - 2.26 (m, 1H), 2.17 (s, 1.5H), 2.11 - 2.07 (m, 0.5H), 2.06 (s, 1.5H), 2.03 - 1.90 (m, 1.5H), 1.82 (d, *J=* 10.4 Hz, 0.5H), 1.73 (d, *J=* 10.5 Hz, 0.5H). HRMS m/z [M+H]⁺ for C₃₂H₂₇NO₂F₃ calculated 530.1943, found 530.1945.

4-(4-((1*S*,4*S*,5*S*)-3-Oxo-2-azabicyclo[2.2.1]heptan-5-yl)phenyl)-7-(4-(trifluoromethyl)phenyl)-2-naphthoic acid (**119**). Treatment of **186** (5.3 mg, 0.01 mmol) by using Procedure B (reaction time, 90 min) gave **119** (3.1 mg, 62%) as a white powder: ¹H NMR (400 MHz, methanol-*d*₄) δ 8.58 (s, 1H), 8.35 (d, *J* = 1.9 Hz, 1H), 8.05 - 7.96 (m, 4H), 7.87 - 7.81 (m, 1H), 7.79 (d, *J* = 8.1 Hz, 2H), 7.56 - 7.46 (m, 4H), 4.08 (s, 1H), 3.35 - 3.31 (m, 1H), 2.89 (d, *J* = 2.2 Hz, 1H), 2.32 (t, *J =* 10.5 Hz, 1H), 2.23 - 2.13 (m, 1H), 2.03 - 1.93 (m, 2H). HRMS m/z [M+H]⁺ for C₃₀H₂₃NO₃F₃ calculated 502.1630, found 502.1649.

4-(4-((1*R*,4*R*,5*R*)-3-Oxo-2-azabicyclo[2.2.1]heptan-5-yl)phenyl)-7-(4-(trifluoromethyl)phenyl)-2-naphthoic acid (**120**). Treatment of **302** (10.6 mg, 0.02 mmol) by using Procedure B (reaction time, 90 min) gave **120** (7.6 mg, 76%) as a white powder: ¹H NMR (400 MHz, methanol-*d*₄) δ 8.63 (s, 1H), 8.37 (s, 1H), 8.04 - 7.96 (m, 4H), 7.86 (d, *J =* 8.9 Hz, 1H), 7.79 (d, *J =* 8.0 Hz, 2H), 7.51 (s, 4H), 4.08 (s, 1H), 3.34 (s, 1H), 2.89 (s, 1H), 2.33 (t, *J =* 10.8 Hz, 1H), 2.18 (d, *J=* 12.9 Hz, 1H), 1.98 (q, *J=* 9.9, 9.3 Hz, 2H). HRMS m/z [M+H]⁺ for C₃₀H₂₃NO₃F₃ calculated 502.1630, found 502.1634.

4-(4-((1R,4R,6S)-3-Oxo-2-azabicyclo[2.2.1]heptan-6-yl)phenyl)-7-(4-(trifluoromethyl)phenyl)-2-naphthoic acid (**121**). Treatment of **191** (5.3 mg, 0.01 mmol) by using Procedure B (reaction time, 90 min) gave **21** (2.02 mg, 40%) as a white powder: ¹H NMR (400 MHz, methanol-*d*₄) δ 8.57 (s, 1H), 8.35 (d, *J* = 2.0 Hz, 1H), 8.05 - 7.96 (m, 4H), 7.86 - 7.76 (m, 3H), 7.53 (d, *J =* 8.3 Hz, 2H), 7.49 (d, *J =* 8.3 Hz, 2H), 4.00 (s, 1H), 3.38 (dd, *J =* 9.1, 5.2 Hz, 1H), 2.82 (dd, *J =* 3.9, 1.8 Hz, 1H), 2.26 (dt, *J =* 12.9, 4.6 Hz, 1H), 2.19 - 2.09 (m, 1H), 1.98 - 1.88 (m, 2H). HRMS m/z [M+H]⁺ for C₃₀H₂₃NO₃F₃ calculated 502.1630, found 502.1634.

4-(4-((1*S*,4*S*,6*R*)-3-Oxo-2-azabicyclo[2.2.1]heptan-6-yl)phenyl)-7-(4-(trifluoromethyl)phenyl)-2-naphthoic acid (**122**). Treatment of **307** (5.4 mg, 0.01 mmol) by using Procedure B (reaction time, 90 min) gave **122** (2.25 mg, 45%) as a white powder: ¹H NMR (400 MHz, methanol-*d*₄) δ 8.59 (s, 1H), 8.36 (d, *J* = 1.9 Hz, 1H), 8.05 - 7.95 (m, 4H), 7.83 (dd, *J* = 8.9, 1.9 Hz, 1H), 7.79 (d, *J* = 8.1 Hz, 2H), 7.52 (d, *J* = 8.4 Hz, 2H), 7.49 (d, *J* = 8.3 Hz, 3H), 4.00 (s, 1H), 3.37 (dd, *J =* 9.1, 5.1 Hz, 1H), 2.82 (d, *J =* 3.8 Hz, 1H), 2.25 (dt, *J =* 13.0, 4.6 Hz, 1H), 2.19 - 2.09 (m, 1H), 2.01 - 1.88 (m, 2H). HRMS m/z [M+H]⁺ for C₃₀H₂₃NO₃F₃ calculated 502.1630, found 502.1628.

4-(4-((1*R*,3*r*,5*S*)-8-Benzyl-3-hydroxy-8-azabicyclo[3.2.1]octan-3-yl)phenyl)-7-(4-(trifluoromethyl)phenyl)-2-naphthoic acid (127s). Treatment of 172 (61.2 mg, 0.096 mmol) by using Procedure A gave 27s (48.3 mg, 83%) of sufficient purity to be used in next step: HRMS m/z [M+H]⁺ for C₃₈H₃₃NO₃F₃ calculated 608.2413, found 608.2421.

4-(4-((1*R*,3*r*,5*S*)-3-Hydroxy-8-azabicyclo[3.2.1]octan-3-yl)phenyl)-7-(4-(trifluoromethyl)phenyl)-2-naphthoic acid (127). Pd/C (5%, 30 mg) was added to a solution of 127s (42.5 mg, 0.07 mmol) in MeOH. The resulting mixture was stirred under H₂ atmosphere (150 psi) at rt for 7 h. The Pd/C was filtered. Volatiles were evaporated, and the residue was purified by RP-HPLC (C18, A: ACN, B: 10 mM TEAA, 10% → 100% A in 40 min, flow rate = 5 mL/min, t_{R} = 32 min) to give **27** as a white powder (20.9 mg, 58%): ¹H NMR (400 MHz, MeOD) δ 8.64 (s, 1H), 8.32 (d, *J =* 1.9 Hz, 1H), 7.94 (d, *J* = 1.6 Hz, 1H), 7.86 (d, *J* = 8.1 Hz, 2H), 7.73 (dd, *J =* 12.1, 8.5 Hz, 3H), 7.62 (d, *J =* 8.2 Hz, 3H), 7.28 (d, *J =* 7.9 Hz, 2H), 4.23 - 4.17 (m, 2H), 2.69 (q, *J =* 9.1, 7.5 Hz, 2H), 2.59 (dd, *J =* 15.5, 3.5 Hz, 2H), 2.17 - 2.09 (m, 4H). HRMS m/z [M+H]⁺ for C₃₁H₂₇NO₃F₃ calculated 518.1943, found 518.1947.

4-(4-(8-Azabicyclo[3.2.1]oct-2-en-3-yl)phenyl)-7-(4-(trifluoromethyl)phenyl)-2-naphthoic acid (**128**). A solution of **127** (14.9 mg, 0.0288 mmol) in TFA (2 mL) was refluxed in a 90 °C oil bath for 1.5 h. Volatiles were evaporated, and the residue was purified by RP-HPLC (C18, A: ACN, B: 10 mM TEAA, 45% → 60% A in 40 min, flow rate = 5 mL/min, t_{R} = 27 min) to give **128** as a white powder (3.9 mg, 27%): ¹H NMR (400 MHz, MeOD) δ 8.69 (s, 1H), 8.35 (d, *J =* 1.8 Hz, 1H), 7.93 (q, *J =* 4.1 Hz, 4H), 7.85 (dd, *J =* 8.9, 1.9 Hz, 1H), 7.74 (d, *J =* 8.1 Hz, 2H), 7.61 (d, *J =* 7.9 Hz, 2H), 7.47 (d, *J =* 7.9 Hz, 2H), 6.51 (d, *J =* 5.7 Hz, 1H), 4.39 (t, *J =* 5.7 Hz, 1H), 4.36 - 4.32 (m, 1H), 3.28 - 3.18 (m, 1H), 2.75 (d, *J* = 18.0 Hz, 1H), 2.41 - 2.25 (m, 2H), 2.25 - 2.12 (m, 1H), 2.08 - 1.96 (m, 1H). HRMS m/z [M+H]⁺ for C₃₁H₂₅NO₂F₃ calculated 500.1837, found 500.1841.

4-(4-(3-Azabicyclo[3.2.1]octan-8-yl)phenyl)-7-(4-(trifluoromethyl)phenyl)-2-naphthoic acid (**129**). Pd/C (5%, 1 mg) was added to a solution of **128** (1 mg, 0.0019) in MeOH. The resulting mixture was stirred under H₂ atmosphere (100 psi) at rt for 3 h. The Pd/C was filtered. Volatiles were evaporated, and the residue was purified by RP-HPLC (C18, A: ACN, B: 10 mM TEAA, 45% → 60% A in 40 min, flow rate = 5 mL/min, t_{R} = 29 min) to give **129** as a white powder (0.88 mg, 92%): ¹H NMR (400 MHz, MeOD) δ 8.75 (s, 1H), 8.44 (s, 1H), 8.05 - 7.96 (m, 4H), 7.96 - 7.90 (m, 1H), 7.81 (d, *J* = 8.1 Hz, 2H), 7.67 (d, *J* = 8.0 Hz, 2H), 7.55 (d, *J* = 8.1 Hz, 2H), 4.13 (s, 2H), 3.32 - 3.29 (m, 1H), 2.62 - 2.52 (m, 2H), 2.46 (d, *J* = 15.4 Hz, 2H), 2.09 - 2.00 (m, 4H). HRMS m/z [M+H]⁺ for C₃₁H₂₇NO₂F₃ calculated 502.1994, found 502.1998.

4-(4-((1*R*,5*S*,8*r*)-3-Benzyl-8-hydroxy-3-azabicyclo[3.2.1]octan-8-yl)phenyl)-7-(4-(trifluoromethyl)phenyl)-2-naphthoic acid (**130**). Treatment of **173** (5 mg, 0.008 mmol) by using Procedure A gave stereoisomeric mixture **130** (1.9 mg, 38%) as a white powder: ¹H NMR (400 MHz, DMSO) δ 8.63 (s, 1H), 8.55 (s, 1H), 8.07 (d, *J* = 8.1 Hz, 2H), 7.98 - 7.92 (m, 3H), 7.88 (d, *J* = 8.2 Hz, 2H), 7.66 (d, *J* = 8.0 Hz, 2H), 7.50 (d, *J* = 7.9 Hz, 2H), 7.41 - 7.30 (m, 4H), 7.24 (t, *J* = 7.0 Hz, 1H), 5.13 (s, 1H), 3.58 (s, 2H), 2.93 (d, *J* = 9.6 Hz, 2H), 2.58 - 2.52 (m, 2H), 2.48 - 2.42 (m, 2H), 1.74 (q, *J* = 5.2 Hz, 2H), 1.46 - 1.39 (m, 2H). HRMS m/z [M+H]⁺ for C₃₈H₃₃NO₃F₃ calculated 608.2413, found 608.2421.

4-(4-((1*R*,5*S*,8*r*)-8-Hydroxy-3-azabicyclo[3.2.1]octan-8-yl)phenyl)-7-(4-(trifluoromethyl)phenyl)-2-naphthoic acid (**131**). Pd/C (5%, 30 mg) was added to a solution of **130** (16.5 mg, 0.027 mmol) in DMF (5 mL). The resulting mixture was stirred under H₂ atmosphere (70 psi) at rt for 4 h. The Pd/C was filtered. Volatiles were evaporated, and the residue was purified by RP-HPLC (C18, A: ACN, B: 10 mM TEAA, 35% → 55% A in 40 min, flow rate = 5 mL/min, t_{R} = 26 min) to give **131** as a white powder (12 mg, 85%): ¹H NMR (400 MHz, MeOD) δ 8.77 (d, *J =* 1.7 Hz, 1H), 8.45 (d, *J =* 1.9 Hz, 1H), 8.08 - 7.97 (m, 4H), 7.94 (dd, *J* = 8.9, 1.9 Hz, 1H), 7.82 (d, *J* = 8.2 Hz, 2H), 7.77 (d, *J* = 8.2 Hz, 2H), 7.61 (d, *J* = 8.2 Hz, 2H), 3.93 (d, *J =* 12.0 Hz, 2H), 3.23 (dd, *J =* 12.4, 3.0 Hz, 2H), 2.84 (s, 2H), 1.94 - 1.80 (m, 4H). HRMS m/z [M+H]⁺ for C₃₁H₂₇NO₃F₃ calculated 518.1943, found 518.1942.

4-(4-((1*R*,5*S*,8*r*)-8-Hydroxy-3-methyl-3-azabicyclo[3.2.1]octan-8-yl)phenyl)-7-(4-(trifluoromethyl)phenyl)-2-naphthoic acid (**132**). Pd/C (5%, 3 mg) was added to a solution of **130** (2.8 mg, 0.0046 mmol) in MeOH (1 mL). The resulting mixture was stirred under H₂ atmosphere (125 psi) at rt overnight. The Pd/C was filtered. Volatiles were evaporated, and the residue was purified by RP-HPLC (C18, A: ACN, B: 10 mM TEAA, 45% → 60% A in 40 min, flow rate = 5 mL/min, t_{R} = 24 min) to give **132** as a white powder (0.46 mg, 19%): ¹H NMR (400 MHz, MeOD) δ 8.78 (d, *J =* 1.5 Hz, 1H), 8.46 (d, *J =* 1.9 Hz, 1H), 8.08 - 7.99 (m, 4H), 7.95 (dd, *J =* 8.9, 2.0 Hz, 1H), 7.83 (d, *J =* 8.2 Hz, 2H), 7.78 (d, *J =* 8.1 Hz, 2H), 7.62 (d, *J =* 8.1 Hz, 2H), 3.88 (d, *J =* 11.9 Hz, 2H), 3.50 - 3.42 (m, 2H), 2.98 (s, 3H), 2.86 (s, 2H), 1.96 - 1.82 (m, 4H). HRMS m/z [M+H]⁺ for C₃₂H₂₉NO₃F₃ calculated 532.2100, found 532.2110.

4-(4-(3-Benzyl-3-azabicyclo[3.2.1]octan-8-yl)phenyl)-7-(4-(trifluoromethyl)phenyl)-2-naphthoic acid (**133s**). Treatment of **174** (5.3 mg, 0.0085 mmol) by using Procedure A gave **133s** (2.3 mg, 45%) of sufficient purity to be used in next step: HRMS m/z [M+H]⁺ for C₃₈H₃₃NO₂F₃ calculated 592.2458, found 582.2464.

4-(4-(3-Azabicyclo[3.2.1]octan-8-yl)phenyl)-7-(4-(trifluoromethyl)phenyl)-2-naphthoic acid (**133**). Pd/C (5%, 1 mg) was added to a solution of **133s** (1.1 mg, 0.0018 mmol) in MeOH/THF (1:1, 1 mL). The resulting mixture was stirred under H₂ atmosphere (100 psi) at rt overnight. The Pd/C was filtered. Volatiles were evaporated, and the residue was purified by RP-HPLC (C18, A: ACN, B: 10 mM TEAA, 35% → 55% A in 40 min, flow rate = 5 mL/min, t_{R} = 26 min) to give **133** as a white powder (0.62 mg, 69%): ¹H NMR (400 MHz, MeOD) δ 8.76 (s, 1H), 8.45 (d, *J* = 2.0 Hz, 1H), 8.07 (d, *J* = 8.8 Hz, 1H), 8.03 - 7.98 (m, 3H), 7.94 (dd, *J =* 8.9, 1.9 Hz, 1H), 7.81 (d, *J* = 8.1 Hz, 2H), 7.65 - 7.58 (m, 4H), 3.42 - 3.36 (m, 2H), 3.36 - 3.32 (m, 2H), 3.09 (d, *J =* 12.5 Hz, 5H), 2.28 (t, *J* = 9.7 Hz, 2H), 2.02 (dd, *J* = 7.1, 1.8 Hz, 2H). HRMS m/z [M+H]⁺ for C₃₁H₂₇NO₂F₃ calculated 502.1994, found 502.1995.

4-(4-((1S,2S,4S)-4-Amino-2-(hydroxymethyl)cyclopentyl)phenyl)-7-(4-(trifluoromethyl)phenyl)-2-naphthoic acid (**134**). Treatment of **185** (16 mg, 0.03 mmol) by using Procedure B gave **134** (10 mg, 66%) as a white powder: ¹H NMR (400 MHz, methanol-*d*₄) δ 8.57 (s, 1H), 8.34 (s, 1H), 8.01 (d, *J =* 2.5 Hz, 1H), 7.95 (t, *J =* 9.1 Hz, 3H), 7.76 (dd, *J* = 9.5, 4.5 Hz, 3H), 7.47 - 7.36 (m, 4H), 3.87 (d, *J* = 8.4 Hz, 1H), 3.64 (d, *J* = 10.8 Hz, 1H), 3.52 (d, *J* = 10.6 Hz, 1H), 2.54 (dd, *J =* 13.9, 7.1 Hz, 1H), 2.43 - 2.18 (m, 3H), 1.74 (q, *J =* 6.8 Hz, 1H). HRMS m/z [M+H]⁺ for C₃₀H₂₇NO₃F₃ calculated 506.1943, found 506.1948.

4-(4-((1*R*,2*R*,4*R*)-4-Amino-2-(hydroxymethyl)cyclopentyl)phenyl)-7-(4-(trifluoromethyl)phenyl)-2-naphthoic acid (**135**). Treatment of **301** (18.9 mg, 0.035 mmol) by using Procedure B gave **135** (12.5 mg, 71%) as a white powder: ¹H NMR (400 MHz, methanol-*d*₄) δ 8.57 (s, 1H), 8.34 (s, 1H), 8.01 (d, *J* = 2.2 Hz, 1H), 7.94 (t, *J* = 9.7 Hz, 3H), 7.76 (t, *J =* 6.9 Hz, 3H), 7.46 - 7.41 (m, 2H), 7.38 (d, *J* = 8.0 Hz, 2H), 3.87 (d, *J* = 7.5 Hz, 1H), 3.63 (d, *J* = 10.8 Hz, 1H), 3.52 (dd, *J =* 11.0, 5.2 Hz, 1H), 3.23 (q, *J =* 9.5 Hz, 1H), 2.55 (dt, *J =* 14.7, 7.8 Hz, 1H), 2.41 - 2.19 (m, 3H), 1.73 (dt, *J =* 13.8, 7.1 Hz, 1H). HRMS m/z [M+H]⁺ for C₃₀H₂₇NO₃F₃ calculated 506.1943, found 506.1948.

4-(4-((1*S*,2*R*,4*R*)-2-Amino-4-(hydroxymethyl)cyclopentyl)phenyl)-7-(4-(trifluoromethyl)phenyl)-2-naphthoic acid (**136**). Treatment of **190** (12 mg, with impurity TEA) by using Procedure B gave **136** (2.5 mg, overall yield% from **188:** 45%) as a white powder: ¹H NMR (400 MHz, methanol-*d*₄) δ 8.56 (s, 1H), 8.33 (d, *J* = 2.0 Hz, 1H), 8.00 (d, *J* = 1.6 Hz, 1H), 7.97 (s, 3H), 7.76 (dd, *J* = 8.7, 7.0 Hz, 3H), 7.50 (d, *J* = 8.3 Hz, 2H), 7.47 (d, *J* = 8.3 Hz, 2H), 3.74 - 3.63 (m, 1H), 3.63 - 3.58 (m, 2H), 3.14 (q, *J =* 9.4 Hz, 1H), 2.58 - 2.40 (m, 2H), 2.18 - 2.02 (m, 2H), 1.56 (dt, *J* = 12.4, 8.9 Hz, 1H). HRMS m/z [M+H]⁺ for C₃₀H₂₇NO₃F₃ calculated 506.1943, found 506.1949.

4-(4-((1*R*,2*S*,4*S*)-2-Amino-4-(hydroxymethyl)cyclopentyl)phenyl)-7-(4-(trifluoromethyl)phenyl)-2-naphthoic acid (**137**). Treatment of **306** (19.2 mg, with 20% TEA; 0.03 mmol) by using Procedure B gave **137** (7.7 mg, 50%) as a white powder: ¹H NMR (400 MHz, methanol-*d*₄) δ 8.64 (s, 1H), 8.37 (d, *J =* 2.0 Hz, 1H), 8.01 - 7.93 (m, 4H), 7.80 (td, *J =* 6.6, 3.1 Hz, 3H), 7.51 (s, 4H), 3.79 (td, *J =* 9.4, 6.8 Hz, 1H), 3.66 - 3.57 (m, 2H), 3.29 - 3.23 (m, 1H), 2.63 - 2.45 (m, 2H), 2.26 - 2.05 (m, 2H), 1.70 - 1.58 (m, 1H). HRMS m/z [M+H]⁺ for C₃₀H₂₇NO₃F₃ calculated 506.1943, found 506.1943.

4-(4-((1*S*,2*S*,4*S*)-4-Amino-2-carboxycyclopentyl)phenyl)-7-(4-(trifluoromethyl)phenyl)-2-naphthoic acid (**138**). Treatment of **186** (10.3 mg, 0.019 mmol) by using Procedure B (reaction time, overnight; RP-HPLC, A: ACN, B: 10 mM TEAA, 10% → 50% A in 40 min, flow rate = 5 mL/min, t_{R} = 39 min) gave **138** (7.5 mg, 77%) as a white powder: ¹H NMR (400 MHz, methanol-*d*₄) δ 8.57 (d, *J* = 1.6 Hz, 1H), 8.34 (d, *J* = 1.9 Hz, 1H), 8.01 (d, *J* = 1.6 Hz, 1H), 8.00 - 7.92 (m, 3H), 7.82 - 7.74 (m, 3H), 7.46 (s, 4H), 3.95 (dq, *J =* 6.2, 3.2, 2.8 Hz, 1H), 3.79 - 3.68 (m, 1H), 3.05 - 2.96 (m, 1H), 2.55 - 2.36 (m, 2H), 2.35 - 2.23 (m, 1H), 2.12 - 2.04 (m, 1H). HRMS m/z [M+H]⁺ for C₃₀H₂₅NO₄F₃ calculated 520.1736, found 520.1733.

4-(4-((1*R*,2*R*,4*R*)-4-Amino-2-carboxycyclopentyl)phenyl)-7-(4-(trifluoromethyl)phenyl)-2-naphthoic acid (**139**). Treatment of **302** (10.6 mg, 0.02 mmol) by using Procedure B (reaction time, overnight; RP-HPLC, A: ACN, B: 10 mM TEAA, 10% → 50% A in 40 min, flow rate = 5 mL/min, t_{R} = 39 min) gave **139** (8.1 mg, 78%) as a white powder: ¹H NMR (400 MHz, methanol-*d*₄) δ 8.58 (s, 1H), 8.35 (s, 1H), 8.04 - 7.95 (m, 4H), 7.85 - 7.76 (m, 3H), 7.51 - 7.46 (m, 4H), 3.95 (s, 1H), 3.79 - 3.70 (m, 1H), 3.02 (s, 1H), 2.55 - 2.37 (m, 2H), 2.36 - 2.24 (m, 1H), 2.09 (d, *J* = 14.5 Hz, 1H). HRMS m/z [M+H]⁺ for C₃₀H₂₅NO₄F₃ calculated 520.1736, found 520.1742.

4-(4-((1*S*,2*R*,4*R*)-2-Amino-4-carboxycyclopentyl)phenyl)-7-(4-(trifluoromethyl)phenyl)-2-naphthoic acid (**140**). Treatment of **191** (5.3 mg, 0.01 mmol) by using Procedure B (reaction time, overnight; RP-HPLC, A: ACN, B: 10 mM TEAA, 10% → 50% A in 40 min, flow rate = 5 mL/min, t_{R} = 39 min) gave **140** (3.8 mg, 73%) as a white powder: ¹H NMR (400 MHz, methanol-*d*₄) δ 8.59 (s, 1H), 8.35 (d, *J =* 2.0 Hz, 1H), 8.03 - 7.94 (m, 4H), 7.84 - 7.75 (m, 3H), 7.52 (d, *J* = 8.1 Hz, 2H), 7.48 (d, *J* = 8.1 Hz, 2H), 3.84 - 3.75 (m, 1H), 3.43 (q, *J* = 8.2 Hz, 1H), 3.15 - 3.08 (m, 1H), 2.58 (ddd, *J* = 11.7, 8.2, 3.0 Hz, 1H), 2.45 (dt, *J =* 15.0, 7.7 Hz, 1H), 2.26 (dt, *J =* 13.6, 9.1 Hz, 1H), 2.13 (dt, *J =* 13.9, 4.3 Hz, 1H). HRMS m/z [M+H]⁺ for C₃₀H₂₅NO₄F₃ calculated 520.1736, found 520.1733.

4-(4-((1*R*,2*S*,4*S*)-2-Amino-4-carboxycyclopentyl)phenyl)-7-(4-(trifluoromethyl)phenyl)-2-naphthoic acid (**141**). Treatment of **307** (5.5 mg, 0.01 mmol) by using Procedure B (reaction time, overnight; RP-HPLC, A: ACN, B: 10 mM TEAA, 10% → 50% A in 40 min, flow rate = 5 mL/min, t_{R} = 39 min) gave **141** (2.8 mg, 54%) as a white powder: ¹H NMR (400 MHz, methanol-*d*₄) δ 8.60 (s, 1H), 8.36 (d, *J =* 2.0 Hz, 1H), 8.03 - 7.94 (m, 4H), 7.85 - 7.75 (m, 3H), 7.53 (d, *J* = 8.0 Hz, 2H), 7.48 (d, *J* = 8.1 Hz, 2H), 3.79 (q, *J* = 7.0 Hz, 1H), 3.42 (q, *J* = 8.0 Hz, 1H), 3.18 - 3.09 (m, 1H), 2.63 - 2.53 (m, 1H), 2.45 (dt, *J* = 14.9, 7.8 Hz, 1H), 2.26 (dt, *J =* 13.6, 9.1 Hz, 1H), 2.18 - 2.10 (m, 1H). HRMS m/z [M+H]⁺ for C₃₀H₂₅NO₄F₃ calculated 520.1736, found 520.1730.

4-(4-Bromophenyl)-3-methylpiperidine (**143**). **Procedure C.** Bromine (0.5 mL) was added into a solution of 3-methyl-4-phenylpiperidine (**142**, 40 µL, 43.2 mg, 0.24 mmol) in acetic acid (1 mL). The resulting solution was stirred at rt for 10 min and then quenched with 2% aqueous sodium bisulfite solution to remove all the excess bromine added. The residue was basified with 6 N NaOH aqueous solution and extracted with dichloromethane three times. The organic layer was dried over anhydrous Na₂SO₄. Volatiles were evaporated, and the residue was purified by RP-HPLC (C18, A: ACN, B: 0.2% TFA, 30% → 60% A in 40 min, flow rate = 5 mL/min, t_{R} = 25 min) to give **143** (18.6 mg, 30%) as clear solid. The ¹H NMR spectrum with two groups of signals showed that the product is a mixture of diastereomers: ¹H NMR (400 MHz, CDCl₃) δ 7.42 (d, *J =* 7.7 Hz, 2H), 7.06 (d, *J =* 8.1 Hz, 0.8H), 7.02 (d, *J =* 8.1 Hz, 1.2H), 3.48 (s, 0.6H), 3.38 (s, 0.4H), 3.27 (s, 0.6H), 3.17 (s, 0.6H), 3.07 - 2.54 (m, 3H), 2.49 (dd, *J =* 13.7, 9.0 Hz, 1H), 2.20 - 1.93 (m, 2H), 1.07 (d, *J =* 5.7 Hz, 3H). MS m/z [M+H]⁺ for C₁₂H₁₇N⁷⁹Br calculated 254.0, found 254.1.

4-(4-Bromophenyl)-2-methylpiperidine (**145**). Treatment of 2-methyl-4-phenylpiperidine (**144**, 40 µL, 43.2 mg, 0.24 mmol) with bromine by using Procedure C gave **145** (36.3 mg, 62%) as clear solid. The ¹H NMR spectrum with two groups of signals showed that the product is a mixture of diastereomers: ¹H NMR (400 MHz, CDCl₃) δ 7.51 - 7.41 (m, 2H), 7.14 - 7.05 (m, 2H), 3.77 - 3.72 (m, 0.3H), 3.51 (d, *J* = 12.5 Hz, 0.7H), 3.28 - 3.21 (m, 1H), 3.09 - 2.96 (m, 1.3H), 2.83 - 2.71 (m, 0.7H), 2.29 - 2.18 (m, 0.3H), 2.11 - 1.92 (m, 2.7H), 1.91 - 1.76 (m, 1H), 1.48 (d, *J =* 6.6 Hz, 1H), 1.40 (d, *J=* 6.3 Hz, 2H). MS m/z [M+H]⁺ for C₁₂H₁₇N⁷⁹Br calculated 254.0, found 254.1.

*rac-*(1*R*,4*R*,5*R*)-5-(4-Bromophenyl)-2-azabicyclo[2.2.1]heptane (**147**). Treatment of 5-phenyl-2-azabicyclo[2.2.1]heptane **112a** (38.9 mg, 0.22 mmol) with bromine by using Procedure C gave racemate **147** as clear solid (32.6 mg, 59%): ¹H NMR (400 MHz, MeOD) δ 7.48 - 7.38 (m, 2H), 7.20 - 7.14 (m, 2H), 3.61 (s, 1H), 3.00 - 2.89 (m, 2H), 2.78 (dd, *J* = 9.9, 1.5 Hz, 1H), 2.58 - 2.52 (m, 1H), 2.09 (ddd, *J =* 12.2, 9.1, 2.6 Hz, 1H), 1.77 (ddd, *J =* 13.3, 5.4, 3.0 Hz, 1H), 1.73 - 1.66 (m, 1H), 1.56 - 1.47 (m, 1H). MS m/z [M+H]⁺ for C₁₂H₁₅N⁷⁹Br calculated 252.0, found 252.0.

6-(4-Bromophenyl)-3-azabicyclo[4.1.0]heptane (**149**). A 25 mL round-bottom flask was flamed dried under argon and charged with 4-(4-bromophenyl)-1,2,3,6-tetrahydropyridine (**148**, 95.2 mg, 0.4 mmol) and CH₂Cl₂ (4 mL). Into the solution cooled in ice bath was added diethylzinc (1M in hexane, 1 mL, 1 mmol). The mixture was stirred at 0 °C for 30 min and then diiodomethane (161.1 µL, 535.7 mg, 2 mmol) was added. The resulting mixture was stirred at 0 °C for 1.5 h and then at rt overnight. The reaction was quenched with saturated NH₄Cl solution and then basified with NaHCO₃ solution and extracted with CH₂Cl₂ three times. The combined organic layer was dried with Na₂SO₄. The volatiles were evaporated, and the residue was column chromatographed (CH₂Cl₂/MeOH/TEA, 100:0:1→795:5:1) to give **149** as transparent oil (62.8 mg, 62%): ¹H NMR (400 MHz, CDCl₃) δ 7.38 (d, *J* = 8.1 Hz, 2H), 7.20 (d, *J =* 8.1 Hz, 2H), 5.96 (s, 1H), 3.57 (dd, *J =* 13.1, 6.5 Hz, 1H), 3.12 (d, *J =* 13.2 Hz, 1H), 3.07 - 2.92 (m, 1H), 2.77 - 2.66 (m, 1H), 2.26 - 2.05 (m, 2H), 1.33 (q, *J* = 7.3, 5.7 Hz, 1H), 1.06 (dd, *J* = 9.3, 5.2 Hz, 1H), 0.92 (t, *J =* 5.5 Hz, 1H). HRMS m/z [M+H]⁺ for C₁₂H₁₅⁷⁹BrN calculated 252.0388, found 252.0387.

N-Boc-7,7-difluoro-6-(4-bromophenyl)-3-azabicyclo[4.1.0]heptane (**151**). To a solution of **150** (169 mg, 0.5 mmol) in THF (3 mL) in a pressure tube was added NaI (37.5 mg, 0.25 mmol) and TMSCF₃ (369 µl, 710 mg, 2.5 mmol). The reaction vessel was sealed and heated at 65 °C overnight. The volatiles were evaporated, and the residue was column chromatographed (hexane/EtOAc, 90:10->85:15) to give **151** as a white solid (50.5 mg, 26%): ¹H NMR (400 MHz, CDCl₃) δ 7.48 (d, *J =* 8.3 Hz, 2H), 7.15 (d, *J =* 8.3 Hz, 2H), 4.26-4.09 (m, 1H), 3.99-3.66 (m, 1H), 3.55-3.42 (m, 1H), 3.37-3.12 (m, 2H), 2.29-2.09 (m, 1H), 2.08-1.89 (m, 1H), 1.48 (s, 9H); ¹⁹F NMR (376 MHz, CDCl₃) δ -129.24 (dd, *J =* 153.0, 86.7 Hz, 1F), -145.25 (d, *J=* 154.0 Hz, 1F). HRMS m/z [M-*t*-butyl+2H]⁺ for C₁₃H₁₃⁷⁹BrF₂NO₂ calculated 332.0092, found 332.0103.

6-(4-Bromophenyl)-7,7-difluoro-3-azabicyclo[4.1.0]heptane (**152**). A TFA solution in THF (67%, 1.5 mL) was added to **151** (12.1 mg, 0.031 mmol) and the resulting mixture was stirred at rt for 0.5 h. Volatiles were evaporated, and the residue was purified by RP-HPLC (C18, A: ACN, B: 0.2% TFA, 30% → 60% A in 40 min, flow rate = 5 mL/min, t_{R} = 28 min) to give **152** as clear solid (6.7 mg, 75%): ¹H NMR (400 MHz, CDCl₃) δ 7.52 (d, *J =* 6.8 Hz, 2H), 7.35 (d, *J =* 7.7 Hz, 2H), 4.08 - 3.72 (m, 1H), 3.42 - 3.12 (m, 2H), 3.04 - 2.80 (m, 1H), 2.53 - 2.12 (m, 3H). HRMS m/z [M+H]⁺ for C₁₂H₁₃⁷⁹BrF₂N calculated 288.0199, found 288.0201.

*N*-Boc-4-(4-bromophenyl)-2-methoxypiperidine (**154**). Into an ElectraSyn vial (20 mL, IKA, Wilmington, NC) equipped with two Pt plate electrodes without diaphragm or reference electrode was added a solution of *N*-Boc-4-(4-bromophenyl)-piperidine **153** (625 mg, 1.84 mmol) and tetraethylammonium *p*-toluenesulfonate (200 mg, 0.667 mmol) in MeOH (10 mL). The solution was stirred at 0 °C while a constant voltage of 4.5 V was applied to the electrodes using an ElectraSyn 2.0 Electrochemistry Kit. After 2F/mol of electricity was passed through the solution, MeOH was removed. The residue was partitioned between dichloromethane and aqueous NaHCO₃. The organic layer was dried over anhydrous Na₂SO₄. The volatiles were evaporated to give crude **154** as a white powder (646 mg, 95%): ¹H NMR (400 MHz, CDCl₃) δ 7.45 - 7.37 (m, 2H), 7.07 (d, *J =* 8.2 Hz, 2H), 5.56 - 5.51 (m, 0.4H), 5.39 (s, 0.6H), 4.16 - 4.07 (m, 0.6H), 4.00 - 3.91 (m, 0.4H), 3.27 (s, 3H), 3.15 - 2.95 (m, 2H), 2.07 - 1.96 (m, 1H), 1.85 -1.56 (m, 3H), 1.49 (s, 9H).

N-Boc-4-(4-bromophenyl)-1,2,3,4-tetrahydropyridine (**155**). The crude **154** (626.2 mg, 1.69 mmol) was mixed well with ammonium chloride (45.2 mg, 0.84 mmol) in a flask and dried under high vacuum for 2 h. The flask was filled with argon. The resulting mixture was stirred under vacuum in a 110 °C oil bath for 5 h. The residue was column chromatographed (hexane/ethyl acetate = 100:0 → 90:10) to give **155** as a white powder (419.4 mg, 73%): ¹H NMR (400 MHz, CDCl₃) δ 7.48 - 7.38 (m, 2H), 7.11 (d, *J =* 8.1 Hz, 2H), 7.10 - 7.04 (m, 0.4H), 6.94 (d, *J* = 8.5 Hz, 0.6H), 4.95 - 4.88 (m, 0.4H), 4.84 - 4.76 (m, 0.6H), 3.66 - 3.41 (m, 3H), 2.14 - 2.07 (m, 1H), 1.81 - 1.70 (m, 1H), 1.51 (s, 9H). HRMS m/z [M - *t*-butyl+2H]⁺ for C₁₂H₁₃N⁸¹BrO₂ calculated 284.0109, found 284.0113.

*rac*-(1*S*,5*R*,6*S*)-5-(4-Bromophenyl)-2-azabicyclo[4.1.0]heptane (**156**) and *rac-*(1*R*,5*R*,6*R*)-5-(4-bromophenyl)-2-azabicyclo[4.1.0]heptane (**157**). A 10 mL round-bottom flask was flamed dried under argon and charged with **155** (72 mg, 0.213 mmol) and CH₂Cl₂ (1.5 mL). Into the solution cooled in ice bath was added diethylzinc (1M in hexane, 0.532 mL, 0.532 mmol). The mixture was stirred at 0 °C for 30 min and then diiodomethane (85.8 µL, 285.2 mg, 1 mmol) was added. The resulting mixture was stirred at 0 °C for 1.5 h and then at rt overnight. The reaction was quenched with saturated NH₄Cl solution and then basified with NaHCO₃ solution and extracted with CH₂Cl₂ three times. The combined organic layer was dried with Na₂SO₄. The volatiles were evaporated, and the residue was column chromatographed (CH₂Cl₂/MeOH/TEA, 100:0:1→90:10:1) to give **156** as transparent solid (16.9 mg, 31%) in addition to **157** (15.8 mg, 29%). **156** has: ¹H NMR (400 MHz, CDCl₃) δ 7.49 - 7.44 (m, 2H), 7.29 - 7.24 (m, 2H), 3.17 (t, *J =* 7.3 Hz, 1H), 2.91 - 2.82 (m, 2H), 2.74 - 2.68 (m, 1H), 1.95 - 1.84 (m, 1H), 1.63 - 1.53 (m, 1H), 1.22 - 1.14 (m, 1H), 1.04 - 0.89 (m, 1H), 0.87 - 0.78 (m, 1H). HRMS m/z [M+H]⁺ for C₁₂H₁₅⁷⁹BrN calculated 252.0388, found 252.0390. **157** has: ¹H NMR (400 MHz, CDCl₃) δ 7.46 - 7.42 (m, 2H), 7.29 - 7.25 (m, 2H), 3.15 - 3.06 (m, 1H), 2.75 - 2.69 (m, 1H), 2.65 - 2.60 (m, 1H), 2.55 - 2.47 (m, 1H), 1.77 - 1.68 (m, 1H), 1.46 - 1.37 (m, 1H), 1.05 - 0.97 (m, 1H), 0.79 - 0.72 (m, 1H), 0.48 - 0.42 (m, 1H). HRMS m/z [M+H]⁺ for C₁₂H₁₅⁷⁹BrN calculated 252.0388, found 252.0385.

(1*R*,3*r*,5*S*)-8-Benzyl-3-(4-bromophenyl)-8-azabicyclo[3.2.1]octan-3-ol (**159**). A mixture of 1,4-dibromobenzene (1.4 g, 6 mmol) and magnesium turnings (109.4 mg, 4.5 mmol) in THF (9 mL) was sonicated for 4 h. The solution was cooled to 0 °C. Into the Grignard reagent was added the solution of *N*-benzyl nortropinone **158** (646 mg, 3 mmol) in THF (15 mL). The resulting mixture was stirred at °C for 2 h. The residue was partitioned between ethyl acetate and aqueous NaHCO₃. The organic layer was dried over anhydrous Na₂SO₄. Volatiles were evaporated, and the residue was column chromatographed (hexane/ethyl acetate = 100:0 --7 40:60) to give **159** as a white powder (1.08 g; 42% of the desired bromo-product in addition to 58% byproduct from debromination of product), which could be used for a Suzuki coupling without further purification. **59** has ¹H NMR (400 MHz, CDCl₃) δ 7.54 - 7.21 (m, 9H), 3.61 (s, 2H), 3.31 - 3.25 (m, 2H), 2.34 (dd, *J =* 14.5, 3.8 Hz, 2H), 2.25 - 2.19 (m, 2H), 2.10 - 2.01 (m, 2H), 1.79 (d, *J* = 2.1 Hz, 1H), 1.75 (d, *J =* 2.0 Hz, 1H). HRMS m/z [M+H]⁺ for C₂₀H₂₃⁷⁹BrNO calculated 372.0963, found 372.0960.

(1*R*,5*S*,8*r*)-3-Benzyl-8-(4-bromophenyl)-3-azabicyclo[3.2.1]octan-8-ol (**161**). A mixture of 1,4-dibromobenzene (1.4 g, 6 mmol) and magnesium turnings (109 mg, 4.5 mmol) in THF (10 mL) was sonicated for 3 h. The solution was cooled to 0 °C. Into the Grignard reagent was added the solution of *N*-benzyl-3-azabicyclo[3.2.1]octan-8-one **160** (646 mg, 3 mmol) in THF (15 mL). The resulting mixture was stirred at 0 °C for 2 h. The residue was partitioned between ethyl acetate and aqueous NaHCO₃. The organic layer was dried over anhydrous Na₂SO₄. Volatiles were evaporated, and the residue was column chromatographed (hexane/ethyl acetate = 90: 10 → 0: 100) to give **161** as a white powder (582.5 mg; 86% of the desired bromo-product in addition to 14% byproduct from debromination of product), which could be used for a Suzuki coupling without further purification. **161** has ¹H NMR (400 MHz, CDCl₃) δ 7.58 - 7.28 (m, 9H), 3.72 - 3.67 (m, 2H), 3.00 - 2.89 (m, 2H), 2.77 - 2.65 (m, 2H), 2.49 - 2.35 (m, 2H), 1.93 - 1.86 (m, 2H), 1.55 - 1.41 (m, 2H). HRMS m/z [M+H]⁺ for C₂₀H₂₃⁷⁹BrNO calculated 372.0963, found 372.0959.

3-Benzyl-8-(4-bromophenyl)-3-azabicyclo[3.2.1]octane (**162**). A solution of **161** (37 mg, 0.1 mmol) in concentrated sulfuric acid (1 mL) was stirred at 80 °C for 30 min. The reaction was cooled to rt and quenched with aqueous NaHCO₃ solution. The residue was partitioned between ethyl acetate and aqueous NaHCO_{3.} The organic layer was dried over anhydrous Na₂SO₄. Volatiles were evaporated, and the residue was purified with preparative-TLC (hexane/ethyl acetate/TEA = 80:20:1) to give **162** as a white powder (5.3 mg, 15%): ¹H NMR (400 MHz, CD₂Cl₂) δ 7.50 - 7.13 (m, 9H), 3.31 (s, 2H), 2.97 (t, *J* = 4.4 Hz, 1H), 2.67 - 2.59 (m, 2H), 2.45 (dd, *J* = 10.8, 3.8 Hz, 2H), 2.35 (d, *J* = 10.8 Hz, 2H), 2.05 - 1.95 (m, 2H), 1.90 - 1.81 (m, 2H). HRMS m/z [M+H]⁺ for C₂₀H₂₃⁷⁹BrN calculated 356.1014 found 356.1014.

Ethyl 4-(4-(3-methylpiperidin-4-yl)phenyl)-7-(4-(trifluoromethyl)phenyl)-2-naphthoate (**165**). **Procedure D.** A flame-dried microwave tube (5 mL) was charged with argon, **164** (20 mg, 0.042 mmol), **143** (10.6 mg, 0.042 mmol), tetrakis(triphenylphosphine)palladium(O) (9.6 mg, 0.0084 mmol), potassium carbonate (17.4 mg, 0.126 mmol) and DMF (1 mL). The resulting mixture was degassed with argon for 10 min and stirred at 150 °C for 30 min in microwave. The volatiles were evaporated, and the residue was column chromatographed (CH₂Cl₂/MeOH/TEA, 100:0:1→90:10:1) to give **165** (13.2 mg, 61%) of sufficient purity to be used in next step: HRMS m/z [M+H]⁺ for C₃₂H₃₁NO₂F₃ calculated 518.2307, found 518.2306.

Ethyl 4-(4-(2-methylpiperidin-4-yl)phenyl)-7-(4-(trifluoromethyl)phenyl)-2-naphthoate (**166**): Treatment of **145** (10.6 mg, 0.042 mmol) by using Procedure D gave **166** (11 mg, 50%) of sufficient purity to be used in next step: HRMS m/z [M+H]⁺ for C₃₂H₃₁NO₂F₃ calculated 518.2307, found 518.2307.

*rac*-Ethyl 4-(4-((1*S*,4*S*,5*S*)-2-azabicyclo[2.2.1]heptan-5-yl)phenyl)-7-(4-(trifluoromethyl)phenyl)-2-naphthoate (**167**). Treatment of **147** (8 mg, 0.032 mmol) by using Procedure D gave **167** (9.3 mg, 43%) of sufficient purity to be used in next step: HRMS m/z [M+H]⁺ for C₃₂H₂₉NO₂F₃ calculated 516.2150, found 516.2156.

Ethyl 4-(4-(3-azabicyclo[4.1.0]heptan-6-yl)phenyl)-7-(4-(trifluoromethyl)phenyl)-2-naphthoate (**168**). Treatment of **149** (10.6 mg, 0.042 mmol) by using Procedure D gave **168** (8 mg, 37%) ¹H NMR (400 MHz, CDCl₃) δ 8.69 - 8.64 (m, 1H), 8.22 (d, *J* = 1.9 Hz, 1H), 8.06 - 8.00 (m, 2H), 7.82 (d, *J =* 8.1 Hz, 2H), 7.79 - 7.71 (m, 3H), 7.49 (d, *J =* 8.3 Hz, 2H), 7.45 (d, *J* = 8.3 Hz, 2H), 4.45 (q, *J =* 7.1 Hz, 2H), 3.68 - 3.56 (m, 1H), 3.23 - 3.13 (m, 1H), 3.05 - 2.98 (m, 1H), 2.82 - 2.65 (m, 1H), 2.35 (ddd, *J* = 13.6, 7.9, 5.3 Hz, 1H), 2.24 (dt, *J =* 14.4, 5.6 Hz, 1H), 1.54 - 1.39 (m, 4H), 1.23 - 1.09 (m, 1H), 1.00 (t, *J =* 5.4 Hz, 1H). HRMS m/z [M+H]⁺ for C₃₂H₂₉NO₂F₃ calculated 516.2150, found 516.2158.

Ethyl 4-(4-(7,7-difluoro-3-azabicyclo[4.1.0]heptan-6-yl)phenyl)-7-(4-(trifluoromethyl)phenyl)-2-naphthoate (**169**). Treatment of **152** (6.7 mg, 0.023 mmol) by using Procedure D. **169** (5.6 mg, 44%) of sufficient purity to be used in next step: ¹H NMR (400 MHz, CDCl₃) δ 8.71 (d, *J* = 1.7 Hz, 1H), 8.25 (d, *J* = 1.9 Hz, 1H), 8.06 (d, *J* = 2.0 Hz, 1H), 7.86 - 7.76 (m, 4H), 7.72 - 7.66 (m, 4H), 7.52 - 7.48 (m, 2H), 4.48 (q, *J =* 7.1 Hz, 2H), 3.51 - 3.41 (m, 1H), 3.37 - 3.29 (m, 1H), 2.93 - 2.75 (m, 2H), 2.34 - 2.22 (m, 1H), 2.02 - 1.97 (m, 2H), 1.47 (t, *J* = 7.1 Hz, 3H). HRMS m/z [M+H]⁺ for C₃₂H₂₇NO₂F₅ calculated 552.1962, found 552.1962.

*rac*-Ethyl 4-(4-((1*S*,5*R*,6*S*)-2-azabicyclo[4.1.0]heptan-5-yl)phenyl)-7-(4-(trifluoromethyl)phenyl)-2-naphthoate (**170**). A flame-dried flask (5 mL) was charged with argon, **164** (37.6 mg, 0.08 mmol), **156** (16.9 mg, 0.067 mmol), Tetrakis(triphenylphosphine)palladium(0) (15.5 mg, 0.0134 mmol), saturated sodium carbonate aqueous solution (169 µL) and DME/EtOH (85:15, 1 mL). The resulting mixture was degassed with argon for 10 min and stirred at 80 °C for 30 min. The volatiles were evaporated, and the residue was column chromatographed (CH₂Cl₂/MeOH/TEA, 100:0:1→795:5:1) to give **170** (9.8 mg, 25%) of sufficient purity to be used in next step: HRMS m/z [M+H]⁺ for C₃₂H₂₉NO₂F₃ calculated 516.2150, found 516.2148.

*rac*-Ethyl 4-(4-((1*R*,5*R*,6*R*)-2-azabicyclo[4.1.0]heptan-5-yl)phenyl)-7-(4-(trifluoromethyl)phenyl)-2-naphthoate (**170**). A flame-dried flask (5 mL) was charged with argon, **164** (35.4 mg, 0.075 mmol), **157** (15.8 mg, 0.063 mmol), tetrakis(triphenylphosphine)palladium(0) (14.6 mg, 0.0126 mmol), saturated sodium carbonate aqueous solution (158 µL) and DME/EtOH (85:15, 1 mL). The resulting mixture was degassed with argon for 10 min and stirred at 80 °C for 30 min. The volatiles were evaporated, and the residue was column chromatographed (CH₂Cl₂/MeOH/TEA, 100:0:1→795:5:1) to give **71** (15.4 mg, 40%) of sufficient purity to be used in next step: HRMS m/z [M+H]⁺ for C₃₂H₂₉NO₂F₃ calculated 516.2150, found 516.2147.

Ethyl 4-(4-((1*R*,3*r*,5*S*)-8-benzyl-3-hydroxy-8-azabicyclo[3.2.1]octan-3-yl)phenyl)-7-(4-(trifluoromethyl)phenyl)-2-naphthoate (**172**). Treatment of **159** (47 mg, 0.126 mmol) by using Procedure D gave **172** (61.2 mg, 76%) of sufficient purity to be used in next step: ¹H NMR (400 MHz, CDCl₃) δ 8.71 - 8.67 (m, 1H), 8.23 (d, *J =* 1.9 Hz, 1H), 8.06 (s, 1H), 7.85 - 7.79 (m, 3H), 7.79 - 7.76 (m, 2H), 7.68 (d, *J =* 8.2 Hz, 2H), 7.53 - 7.46 (m, 4H), 7.34 (d, *J =* 7.5 Hz, 2H), 7.30 - 7.18 (m, 2H), 4.48 - 4.43 (m, 2H), 3.67 (s, 2H), 3.35 (s, 2H), 2.52 (dd, *J* = 14.9, 3.5 Hz, 2H), 2.35 - 2.22 (m, 2H), 2.16 - 2.06 (m, 2H), 1.93 (d, *J* = 2.3 Hz, 1H), 1.90 (s, 1H), 1.48 - 1.44 (m, 3H). HRMS m/z [M+H]⁺ for C₄₀H₃₈NO₃F₃ calculated 636.272, found 636.2734.

Ethyl 4-(4-((1*R*,5*S*,8*r*)-3-benzyl-8-hydroxy-3-azabicyclo[3.2.1]octan-8-yl)phenyl)-7-(4-(trifluoromethyl)phenyl)-2-naphthoate (**173**). Treatment of **161** (31.3 mg, 0.084 mmol) by using Procedure D gave **173** (32.8 mg, 61%) as a white powder: ¹H NMR (400 MHz, CDCl₃) δ 8.69 (d, *J =* 1.7 Hz, 1H), 8.23 (d, *J =* 2.0 Hz, 1H), 8.08 - 8.03 (m, 2H), 7.82 (d, *J =* 8.2 Hz, 2H), 7.79 - 7.72 (m, 3H), 7.67 (d, *J =* 8.0 Hz, 2H), 7.53 (d, *J =* 8.0 Hz, 2H), 7.43 (d, *J =* 7.5 Hz, 2H), 7.35 (t, *J =* 7.4 Hz, 2H), 7.31 - 7.21 (m, 1H), 4.47 (q, *J =* 7.1 Hz, 2H), 3.67 (s, 2H), 2.97 (d, *J =* 10.4 Hz, 2H), 2.71 (dd, *J =* 10.7, 3.5 Hz, 2H), 2.53 (s, 2H), 1.91 (q, *J* = 5.1 Hz, 2H), 1.80 (s, 1H), 1.62 - 1.53 (m, 2H), 1.46 (t, *J =* 7.1 Hz, 3H). HRMS m/z [M+H]⁺ for C₄₀H₃₇NO₃F₃ calculated 636.2726, found 636.2732.

Ethyl 4-(4-(3-benzyl-3-azabicyclo[3.2.1]octan-8-yl)phenyl)-7-(4-(trifluoromethyl)phenyl)-2-naphthoate (**174**). Treatment of **162** (5.3 mg, 0.0144 mmol) by using Procedure D (column chromatography; hexane/ethyl acetate/TEA = 100:0:1 → 95:5:1) gave **174** (5.3 mg, 59%) of sufficient purity to be used in next step: HRMS m/z [M+H]⁺ for C₄₀H₃₇NO₂F₃ calculated 620.2771, found 620.2778.

Ethyl 4-(4-(pyrrolidin-3-yl)phenyl)-7-(4-(trifluoromethyl)phenyl)-2-naphthoate (**175**). Treatment of 3-(4-bromophenyl)pyrrolidine hydrochloride **163** (11 mg, 0.042 mmol) by using Procedure D gave **75** (15.7 mg, 76%) of sufficient purity to be used in next step: HRMS m/z [M+H]⁺ for C₃₀H₂₇NO₂F₃ calculated 490.1994, found 490.1995.

Regioisomeric mixture **177** of (1S,4S,SS)-5-(4-bromophenyl)-2-azabicyclo[2.2.1]heptan-3-one (**180**) and (1R,4R,6S)-6-(4-bromophenyl)-2-azabicyclo[2.2.1]heptan-3-one (**181**). Into a solution of (1R,4S)-2-azabicyclo[2.2.1]hept-5-en-3-one (**176**, 54.6 mg, 0.5 mmol), 1-bromo-4-iodobenzene (282.9 mg, 1 mmol), and (PPh₃)₂PdCl₂ (35 mg, 0.05 mmol) in THF (3 mL) was added TEA (209 µL, 151.8 mg, 1.5 mmol) and then formic acid (22.6 µL, 27.6 mg, 0.6 mmol) dropwise. The resulting solution was stirred at 65 °C overnight. The volatiles were evaporated, and the residue was column chromatographed (hexane/ethyl acetate = 100:0 → 70:30) to give **177** as a white solid (158.7mg, quantitative): ¹H NMR (400 MHz, CDCl₃) δ 7.45 - 7.39 (m, 2H), 7.15 - 7.05 (m, 2H), 6.72 (s, 0.4H), 6.58 (s, 0.6H), 4.04 - 3.98 (m, 0.6H), 3.88 - 3.83 (m, 0.4H), 3.29 - 3.16 (m, 1H), 2.83 - 2.79 (m, 1H), 2.25 (ddd, *J =* 12.0, 8.9, 2.5 Hz, 0.6H), 2.15 (ddd, *J =* 13.0, 8.8, 2.3 Hz, 0.4H), 2.05 - 1.90 (m, 2H), 1.77 - 1.66 (m, 1H).

*tert*-Butyl (1*S*,4*S*,5*S*)-5-(4-bromophenyl)-3-oxo-2-azabicyclo[.2.1]heptane-2-carboxylate (**178**) and *tert*-butyl (1*R*,4*R*,6*S*)-6-(4-bromophenyl)-3-oxo-2-azabicyclo[2.2.1]heptane-2-carboxylate (**179**). To a solution of **177** (138.5 mg, 0.52 mmol), TEA (253.7 µL, 184.2 mg, 1.82 mmol), DMAP (64 mg, 0.52 mmol) in CH₂Cl₂ (7 mL) was added (Boc)₂O (340 mg, 1.56 mmol). The resulting solution was stirred at rt overnight. The volatiles were evaporated, and the residue was column chromatographed (hexane/EtOAc, 100:0→80:20) to give **178** (87.6, 46%) and **179** (68.5, 36%) as a white solids. **178** has: ¹H NMR (400 MHz, CDCl₃) δ 7.49 - 7.40 (m, 2H), 7.14 - 7.06 (m, 2H), 4.63 (t, *J =* 2.1 Hz, 1H), 3.37 (dd, *J =* 9.0, 5.2 Hz, 1H), 2.94 (q, *J =* 1.7 Hz, 1H), 2.38 (ddd, *J =* 13.3, 8.9, 2.5 Hz, 1H), 1.99 (ddt, *J* = 12.5, 7.5, 2.3 Hz, 2H), 1.75 (dt, *J =* 10.4, 1.5 Hz, 1H), 1.54 (s, 9H). HRMS m/z [M+Na]⁺ for C₁₇H₂₀ ⁷⁹BrNO₃Na calculated 388.0524, found 388.0528. **179** has: ¹H NMR (400 MHz, CDCl₃) δ 7.49 - 7.44 (m, 2H), 7.16 - 7.12 (m, 2H), 4.51 - 4.44 (m, 1H), 3.37 - 3.30 (m, 1H), 2.95 (dd, *J =* 4.2, 2.0 Hz, 1H), 2.26 (ddd, *J =* 13.4, 8.8, 2.2 Hz, 1H), 2.15 (dt, *J =* 13.3, 4.8 Hz, 1H), 1.90 (dp, *J* = 10.4, 1.9 Hz, 1H), 1.65 (dt, *J =* 10.4, 1.5 Hz, 1H), 1.56 (s, 9H). HRMS m/z [M+Na]⁺ for C₁₇H₂₀⁷⁹BrNO₃Na calculated 388.0524, found 388.0526.

(1*S*,4*S*,5*S*)-5-(4-Bromophenyl)-2-azabicyclo[2.2.1]heptan-3-one (**180**)**.** To a solution of 178 (559 mg, 1.53 mmol) in DCM (8 mL) was added TFA (8 mL). The resulting mixture was stirred at rt for 1 h. Toluene (1 mL) was added and the volatiles were evaporated. The residue was column chromatographed (hexane/EtOAc/TEA, 20:80:1→0:100:1) to give **180** (357 mg, 88%) as a white solid: ¹H NMR (400 MHz, CDCl₃) δ 7.47 - 7.39 (m, 2H), 7.16 - 7.07 (m, 2H), 6.42 (s, 1H), 4.01 (q, *J =* 1.9 Hz, 1H), 3.25 (dd, *J* = 9.1, 5.0 Hz, 1H), 2.82 (s, 1H), 2.25 (ddd, *J =* 12.0, 8.9, 2.5 Hz, 1H), 2.05 - 1.92 (m, 2H), 1.74 (dd, *J =* 9.9, 1.7 Hz, 1H). HRMS m/z [M+H]⁺ for C₁₂H₁₃⁷⁹BrNO calculated 266.0181, found 266.0178.

(1R,4R,6S)-6-(4-Bromophenyl)-2-azabicyclo[2.2.1]heptan-3-one (**181**). To a solution of 179 (289 mg, 0.79 mmol) in DCM (4 mL) was added TFA (4 mL). The resulting mixture was stirred at rt for 1 h. Toluene (1 mL) was added and the volatiles were evaporated. The residue was column chromatographed (hexane/EtOAc/TEA, 20:80:1→0:100:1) to give **181** (200 mg, 95%) as a white solid: ¹H NMR (400 MHz, CDCl₃) ¹H NMR (400 MHz, CDCl₃) δ 7.47 - 7.38 (m, 2H), 7.18 - 7.07 (m, 2H), 3.89 - 3.83 (m, 1H), 3.24 - 3.15 (m, 1H), 2.81 - 2.75 (m, 1H), 2.19 - 1.97 (m, 2H), 1.97 - 1.87 (m, 1H), 1.75 - 1.67 (m, 1H). HRMS m/z [M+H]⁺ for C₁₂H₁₃⁷⁹BrNO calculated 266.0181, found 266.0185.

(1*S*,4*S*,5*S*)-5-(4-Bromophenyl)-2-azabicyclo[2.2.1]heptane (**182**) and ((1*S*,2*S*,4*S*)-4-amino-2-(4-bromophenyl)cyclopentyl)methanol (**183**). **Procedure E.** A dry flask was vacuumed and then then charged with argon, **180** (53.2 mg, 0.2 mmol), tris(perfluorophenyl)borane (102.4 mg, 0.2 mmol), borane ammonia complex (61.7 mg, 2 mmol), boron trifluoride diethyl etherate (24.7 µL, 28.4 mg, 0.2 mmol), 1,2-dichloroethane (4 mL). The resulting solution was stirred at 75 °C for 24 h. DCM and H₂O were added. The mixture was basified with 6 N NaOH aqueous solution and extracted with dichloromethane three times. The organic layer was dried over anhydrous Na₂SO₄. Volatiles were evaporated, and the residue was purified by RP-HPLC (C18, A: ACN, B: 0.2% TFA, 30% → 60% A in 40 min, flow rate = 5 mL/min) to give **182** (26.1 mg, 52%, t_{R} = 27 min) in addition to **183** (26.2 mg, 48%, t_{R} = 20 min). **182** has: ¹H NMR (400 MHz, methanol-*d*₄) δ 7.46 (d, *J =* 8.0 Hz, 2H), 7.21 (d, *J =* 8.0 Hz, 2H), 4.17 (s, 1H), 3.18 - 3.13 (m, 3H), 2.79 (s, 1H), 2.33 (dd, *J =* 14.6, 9.2 Hz, 1H), 2.03 - 1.89 (m, 2H), 1.74 (d, *J =* 11.7 Hz, 1H). HRMS m/z [M+H]⁺ for C₁₂H₁₅N⁷⁹Br calculated 252.0388, found 252.0386. **183** has: ¹H NMR (400 MHz, methanol-*d*₄) δ 7.46 - 7.40 (m, 2H), 7.19 (d, *J =* 8.1 Hz, 2H), 3.84 (p, *J =* 6.3 Hz, 1H), 3.54 (dd, *J =* 10.8, 3.5 Hz, 1H), 3.43 (dd, *J =* 10.8, 5.3 Hz, 1H), 3.14 - 3.07 (m, 1H), 2.54 - 2.45 (m, 1H), 2.30 - 2.16 (m, 3H), 1.75 - 1.62 (m, 1H). HRMS m/z [M+H]⁺ for C₁₂H₁₇NO⁷⁹Br calculated 270.0494, found 270.0493.

Ethyl 4-(4-((1*S*,4*S*,5*S*)-2-Azabicyclo[2.2.1]heptan-5-yl)phenyl)-7-(4-(trifluoromethyl)phenyl)-2-naphthoate (**184**). Method A. **Procedure F.** To a vacuum dried mixture of **182** (13 mg, 0.052 mmol), **164** (24.46 mg, 0.052 mmol), Pd(PPh₃)₄ (6 mg, 0.0052 mmol), and Na₂CO₃ (8.2 mg, 0.077 mmol) was added sonicated 1,2-dimethoxyethane-water (4:1, 2 mL). The reaction mixture was degassed with argon for 10 min and then stirred at 80 °C overnight. Volatiles were evaporated, and the residue was column chromatographed (CH₂Cl₂/MeOH/TEA, 100:0:1→90:10:1) to give **184** (18.1 mg, 68%). Method B. A dry flask was vacuumed and then then charged with argon, **186** (18 mg, 0.034 mmol), tris(perfluorophenyl)borane (17.4 mg, 0.034 mmol), borane ammonia complex (10.0 mg, 0.34 mmol), and 1,2-dichloroethane (2 mL). The resulting solution was stirred at 75 °C for 48 h. DCM and H₂O were added. The mixture was basified with 6 N NaOH aqueous solution and extracted with dichloromethane three times. The organic layer was dried over anhydrous Na₂SO₄. Volatiles were evaporated, and the residue was column chromatographed (CH₂Cl₂/MeOH/TEA, 100:0:1→90:10:1) to give 184 (7.87 mg, 45%) in addition to **185** (3.8, 21%). 184 has: ¹H NMR (400 MHz, CDCl₃) δ 8.68 (s, 1H), 8.23 (s, 1H), 8.02 - 7.94 (m, 2H), 7.85 - 7.79 (m, 3H), 7.76 (d, *J =* 8.3 Hz, 2H), 7.52 (d, *J =* 7.9 Hz, 2H), 7.31 (d, *J =* 8.0 Hz, 2H), 4.50 - 4.41 (m, 3H), 3.61 - 3.45 (m, 2H), 3.35 - 3.29 (m, 1H), 3.13 - 3.08 (m, 1H), 2.52 (ddd, *J =* 15.6, 8.7, 2.3 Hz, 1H), 2.29 - 2.16 (m, 2H), 1.97 (d, *J =* 12.1 Hz, 1H), 1.46 (t, *J =* 7.1 Hz, 3H). HRMS m/z [M+H]⁺ for C₃₂H₂₉NO₂F₃ calculated 516.2150, found 516.2145.

Ethyl 4-(4-((1S,2S,4S)-4-amino-2-(hydroxymethyl)cyclopentyl)phenyl)-7-(4-(trifluoromethyl)phenyl)-2-naphthoate (**185**). Treatment of **183** (23.4 mg, 0.087 mmol) by using Procedure F gave **185** (37.7 mg, 81%) as a white powder: ¹H NMR (400 MHz, CDCl₃) δ 8.61 (s, 1H), 8.16 (s, 1H), 8.00 (d, *J =* 7.4 Hz, 2H), 7.83 - 7.66 (m, 5H), 7.40 (q, *J* = 8.0 Hz, 4H), 4.41 (q, *J =* 7.2 Hz, 2H), 3.83 (s, 1H), 3.72 - 3.67 (m, 1H), 3.52 (dd, *J =* 13.7, 6.8 Hz, 1H), 3.29 (dt, *J =* 24.5, 6.4 Hz, 1H), 2.74 (q, *J =* 7.3 Hz, 8H), 2.51 - 2.34 (m, 2H), 2.25 (t, *J =* 10.4 Hz, 1H), 2.12 (dt, *J =* 17.4, 9.2 Hz, 1H), 1.84 (d, *J =* 12.6 Hz, 1H), 1.41 (t, *J* = 7.2 Hz, 3H). HRMS m/z [M+H]⁺ for C₃₂H₃₁NO₃F₃ calculated 534.2256, found 534.2248.

Ethyl 4-(4-((1*S*,4*S*,5*S*)-3-oxo-2-azabicyclo[2.2.1]heptan-5-yl)phenyl)-7-(4-(trifluoromethyl)phenyl)-2-naphthoate (**186**). Treatment of **180** (160 mg, 0.34 mmol) by using Procedure F (column chromatography; hexane/ethyl acetate = 50:50 → 0:100) gave **186** (168 mg, 93%) as a white powder: ¹H NMR (400 MHz, CDCl₃) δ 8.68 (s, 1H), 8.22 (d, *J =* 1.9 Hz, 1H), 8.07 - 8.00 (m, 2H), 7.81 (d, *J =* 8.2 Hz, 2H), 7.79 - 7.71 (m, 3H), 7.49 (d, *J =* 8.2 Hz, 2H), 7.43 (d, *J =* 8.1 Hz, 2H), 6.33 (s, 1H), 4.46 (q, *J =* 7.1 Hz, 2H), 4.10 (t, *J =* 3.5 Hz, 1H), 3.45 (dd, *J =* 8.9, 5.0 Hz, 1H), 2.99 (s, 1H), 2.35 (ddd, *J =* 11.8, 8.9, 2.4 Hz, 1H), 2.16 (ddd, *J =* 12.6, 5.0, 2.3 Hz, 1H), 2.11 - 2.01 (m, 1H), 1.96 - 1.89 (m, 1H), 1.45 (t, *J* = 7.1 Hz, 3H). HRMS m/z [M+H]⁺ for C₃₂H₂₇NO₃F₃ calculated 530.1943, found 530.1942.

(1*R*,4*R*,6*S*)-6-(4-Bromophenyl)-2-azabicyclo[2.2.1]heptane (**187**) and ((1*R*,3*R*,4*S*)-3-amino-4-(4-bromophenyl)cyclopentyl)methanol (**188**). Treatment of **181** (26.6 mg, 0.1 mmol) by using Procedure E gave **187** (6.4 mg, 25%, t_{R} = 27 min) and **188** (3.1 mg, 12%, t_{R} = 20 min). 87 has: ¹H NMR (400 MHz, methanol-*d*₄) δ 7.50 (dt, *J =* 8.3, 1.9 Hz, 2H), 7.27 - 7.18 (m, 2H), 4.08 (s, 1H), 3.30 - 3.24 (m, 1H), 3.20 (d, *J =* 10.8 Hz, 1H), 3.10 (d, *J =* 11.0 Hz, 1H), 2.85 (s, 1H), 2.16 - 2.06 (m, 1H), 2.05 - 1.92 (m, 2H), 1.73 (d, *J =* 11.8 Hz, 1H). HRMS m/z [M+H]⁺ for C₁₂H₁₅N⁷⁹Br calculated 252.0388, found 252.0385. **188** has: ¹H NMR (400 MHz, methanol-*d*₄) δ 7.52 (dt, *J* = 8.5, 2.0 Hz, 2H), 7.26 (dt, *J* = 8.5, 2.0 Hz, 2H), 3.64 (q, *J* = 8.2, 7.5 Hz, 1H), 3.56 (q, *J =* 2.3 Hz, 2H), 3.09 (q, *J =* 9.3 Hz, 1H), 2.51 - 2.36 (m, 2H), 2.14 - 2.03 (m, 1H), 1.97 (t, *J* = 10.8 Hz, 1H), 1.56 (td, *J =* 10.4, 7.3 Hz, 1H). HRMS m/z [M+H]⁺ for C₁₂H₁₇NO⁷⁹Br calculated 270.0494, found 270.0489.

Ethyl 4-(4-((1R,4R,6S)-2-azabicyclo[2.2.1]heptan-6-yl)phenyl)-7-(4-(trifluoromethyl)phenyl)-2-naphthoate **(189).** Treatment of **187** (3.0 mg, 0.012 mmol) by using Procedure F gave **189** (3.5 mg, 56%) of sufficient purity to be used in next step: ¹H NMR (400 MHz, CDCl₃) δ 8.67 (s, 1H), 8.21 (d, *J* = 1.9 Hz, 1H), 8.02 - 7.96 (m, 2H), 7.82 (d, *J* = 8.2 Hz, 2H), 7.79 - 7.72 (m, 3H), 7.49 - 7.43 (m, 2H), 7.37 (d, *J =* 8.1 Hz, 2H), 4.45 (q, *J =* 7.2 Hz, 2H), 4.24 (s, 1H), 3.94 (t, *J =* 7.4 Hz, 1H), 3.39 - 3.32 (m, 1H), 3.23 - 3.19 (m, 1H), 2.83 (s, 1H), 2.25 - 2.22 (m, 1H), 2.06 - 2.03 (m, 2H), 1.96 - 1.94 (m, 1H), 1.45 - 1.43 (m, 3H). HRMS m/z [M+H]⁺ for C₃₂H₂₉NO₂F₃ calculated 516.2150, found 516.2141.

Ethyl 4-(4-((1*S*,2*R*,4*R*)-2-amino-4-(hydroxymethyl)cyclopentyl)phenyl)-7-(4-(trifluoromethyl)phenyl)-2-naphthoate **(190).** Treatment of **188** (3.1 mg, 0.011 mmol) by using Procedure F gave **190** (12 mg, with TEA) of sufficient purity to be used in next step: ¹H NMR (400 MHz, CDCl₃) δ 8.67 (s, 1H), 8.21 (d, *J* = 1.9 Hz, 1H), 8.02 - 7.97 (m, 2H), 7.81 (d, *J* = 8.0 Hz, 2H), 7.78 - 7.73 (m, 3H), 7.47 (d, *J* = 8.0 Hz, 2H), 7.40 (d, *J* = 8.0 Hz, 2H), 4.45 (q, *J* = 7.1 Hz, 2H). HRMS m/z [M+H]⁺ for C₃₂H₃₁NO₃F₃ calculated 534.2256, found 534.2263.

Ethyl 4-(4-((1*R*,4*R*,6*S*)-3-oxo-2-azabicyclo[2.2.1]heptan-6-yl)phenyl)-7-(4-(trifluoromethyl)phenyl)-2-naphthoate **(191).** Treatment of **181** (5.3 mg, 0.02 mmol) by using Procedure F (column chromatography; hexane/ethyl acetate = 50:50 → 0:100) gave **191** (10.56 mg, 99.7%) as a white powder: ¹H NMR (400 MHz, CDCl₃) δ 8.69 (d, *J =* 1.7 Hz, 1H), 8.24 (d, *J* = 2.0 Hz, 1H), 8.07 - 7.99 (m, 2H), 7.82 (d, J = 8.2 Hz, 2H), 7.80 - 7.73 (m, 3H), 7.51 (d, *J* = 8.3 Hz, 2H), 7.40 (d, *J =* 8.0 Hz, 2H), 5.91 (s, 1H), 4.47 (q, *J =* 7.1 Hz, 2H), 4.02 (s, 1H), 3.39 (dd, *J =* 8.8, 5.3 Hz, 1H), 2.91 (d, *J =* 3.8 Hz, 1H), 2.32 - 2.17 (m, 2H), 2.08 - 2.03 (m, 1H), 1.93 - 1.88 (m, 1H), 1.45 (t, *J =* 7.1 Hz, 3H). HRMS m/z [M+H]⁺ for C₃₂H₂₇NO₃F₃ calculated 530.1943, found 530.1948.

Regioisomeric mixture **193** of (1*R*,4*R*,5*R*)-5-(4-bromophenyl)-2-azabicyclo[2.2.1]heptan-3-one **(196)** and (1*S*,4*S*,6*R*)-6-(4-bromophenyl)-2-azabicyclo[2.2.1]heptan-3-one **(197).** Into a solution of (1*S*,4*R*)-2-azabicyclo[2.2.1]hept-5-en-3-one (**192,** 327 mg, 3 mmol), 1-bromo-4-iodobenzene (1.7 g, 6 mmol), and (PPh₃)₂PdCl₂ (210 mg, 0.3 mmol) in THF (12 mL) was added TEA (1.2 mL, 910.7 mg, 9 mmol) and then formic acid (135.7 µL, 165.6 mg, 3.6 mmol) dropwise. The resulting solution was stirred at 65 °C overnight. The volatiles were evaporated, and the residue was column chromatographed (hexane/ethyl acetate = 70:30 → 0:100) to give **193** as a white solid (798 mg, quantitative) of sufficient purity to be used in next step: HRMS m/z [M+H]⁺ for C₁₂H₁₃NO⁷⁹Br calculated 266.0181, found 266.0176.

*tert*-Butyl (1*R*,4*R*,5*R*)-5-(4-bromophenyl)-3-oxo-2-azabicyclo[2.2.1]heptane-2-carboxylate **(194)** and *tert*-butyl (1*S*,4*S*,6*R*)-6-(4-bromophenyl)-3-oxo-2-azabicyclo[2.2.1]heptane-2-carboxylate **(195).** To a solution of **193** (798 mg, 3 mmol), TEA (1.46 mL, 1 g, 10.5 mmol), DMAP (366.5 mg, 3 mmol) in CH₂Cl₂ (25 mL) was added (Boc)₂O (1.96 g, 9 mmol). The resulting solution was stirred at rt overnight. The volatiles were evaporated, and the residue was column chromatographed (hexane/EtOAc, 100:0→80:20) to give **194** (600 mg, 55%) and **195** (291 mg, 41%) as a white solids. **94** has: ¹H NMR (400 MHz, CDCl₃) δ 7.31 - 7.20 (m, 2H), 6.99 - 6.92 (m, 2H), 4.51 - 4.46 (m, 1H), 3.18 (dd, *J =* 9.1, 5.1 Hz, 1H), 2.78 - 2.73 (m, 1H), 2.24 (ddd, *J =* 13.2, 8.9, 2.3 Hz, 1H), 1.83 (ddt, *J* = 18.8, 10.6, 2.3 Hz, 2H), 1.64 - 1.56 (m, 1H), 1.38 (s, 9H). HRMS m/z [M+Na]⁺ for C₁₇H₂₀⁷⁹BrNO₃Na calculated 388.0524, found 388.0526. **95** has: ¹H NMR (400 MHz, CDCl₃) δ 7.41 - 7.34 (m, 2H), 7.08 (d, *J =* 8.5 Hz, 2H), 4.39 (d, *J =* 2.2 Hz, 1H), 3.31 - 3.23 (m, 1H), 2.87 (dd, *J =* 3.9, 2.0 Hz, 1H), 2.17 (ddd, *J =* 13.3, 8.7, 2.0 Hz, 1H), 2.13 - 2.01 (m, 1H), 1.86 - 1.78 (m, 1H), 1.63 - 1.55 (m, 1H), 1.49 (s, 9H). HRMS m/z [M+Na]⁺ for C₁₇H₂₀⁷⁹BrNO₃Na calculated 388.0524, found 388.0526. MS m/z [M-*t*-butyl+2H]⁺ for C₁₃H₁₃⁸¹BrNO₃ calculated 312.0, found 312.0.

(1*R*,4*R*,5*R*)-5-(4-Bromophenyl)-2-azabicyclo[2.2.1]heptan-3-one **(196).** To a solution of 194 (600 mg, 1.638 mmol) in DCM (8 mL) was added TFA (8 mL). The resulting mixture was stirred at rt for 1 h. Toluene (1 mL) was added and the volatiles were evaporated. The residue was column chromatographed (hexane/EtOAc/TEA, 30:70:0.5→0:100:0.5) to give **196** (341.5 mg, 78%) as a white solid: ¹H NMR (400 MHz, CDCl₃) δ 7.47 - 7.42 (m, 2H), 7.15 - 7.11 (m, 2H), 5.78 (s, 1H), 4.02 (d, *J =* 2.8 Hz, 1H), 3.27 (dd, *J =* 9.1, 5.0 Hz, 1H), 2.84 (s, 1H), 2.26 (ddd, *J =* 12.0, 8.9, 2.5 Hz, 1H), 2.05 - 1.97 (m, 2H), 1.76 (dd, *J =* 9.9, 1.7 Hz, 1H). HRMS m/z [M+H]⁺ for C₁₂H₁₃⁷⁹BrNO calculated 266.0181, found 266.0183.

(1S,4S,6R)-6-(4-Bromophenyl)-2-azabicyclo[2.2.1]heptan-3-one **(197).** To a solution of 195 (291 mg, 0.79 mmol) in DCM (4 mL) was added TFA (4 mL). The resulting mixture was stirred at rt for 1 h. Toluene (1 mL) was added and the volatiles were evaporated. The residue was column chromatographed (hexane/EtOAc/TEA, 30:70:0.5→0:100:0.5) to give **197** (167 mg, 79%) as a white solid: ¹H NMR (400 MHz, CDCl₃) δ 7.50 - 7.41 (m, 2H), 7.14 - 7.06 (m, 2H), 5.68 (s, 1H), 3.87 (s, 1H), 3.21 (dd, *J =* 9.0, 5.1 Hz, 1H), 2.84 (d, *J =* 4.0 Hz, 1H), 2.19 (ddd, *J* = 12.5, 8.7, 2.3 Hz, 1H), 2.07 - 1.94 (m, 2H), 1.78 - 1.70 (m, 1H). HRMS m/z [M+H]⁺ for C₁₂H₁₃⁷⁹BrNO calculated 266.0181, found 266.0180.

(1*R*,4*R*,5*R*)-5-(4-Bromophenyl)-2-azabicyclo[2.2.1]heptane **(198)** and ((1*R*,2*R*,4*R*)-4-amino-2-(4-bromophenyl)cyclopentyl)methanol **(199).** Treatment of **196** (53.2 mg, 0.2 mmol) by using Procedure E gave **198** (25.7 mg, 51%, t_{R} = 27 min) and **199** (23.7 mg, 44%, t_{R} = 20 min). 198 has: ¹H NMR (400 MHz, Methanol-*d*₄) δ 7.51 - 7.44 (m, 2H), 7.25 - 7.18 (m, 2H), 4.18 (s, 1H), 3.20 - 3.16 (m, 2H), 3.13 (t, *J =* 7.2 Hz, 1H), 2.80 (s, 1H), 2.36 - 2.25 (m, 1H), 2.05 - 1.91 (m, 2H), 1.73 (d, *J* = 11.7 Hz, 1H). HRMS m/z [M+H]⁺ for C₁₂H₁₅N⁷⁹Br calculated 252.0388, found 252.0390. 199 has ¹H NMR (400 MHz, methanol-*d*₄) δ 7.45 (d, *J* = 8.4 Hz, 2H), 7.24 - 7.17 (m, 2H), 3.84 (p, *J =* 6.5 Hz, 1H), 3.54 (dd, *J =* 10.7, 3.5 Hz, 1H), 3.43 (dd, *J =* 10.7, 5.3 Hz, 1H), 3.12 (q, *J* = 9.7 Hz, 1H), 2.50 (dt, *J =* 13.9, 7.6 Hz, 1H), 2.31 - 2.08 (m, 3H), 1.69 (ddd, *J =* 14.1, 8.8, 6.0 Hz, 1H). HRMS m/z [M+H]⁺ for C₁₂H₁₇NO⁷⁹Br calculated 270.0494, found 270.0491.

Ethyl 4-(4-((1*R*,4*R*,5*R*)-2-azabicyclo[2.2.1]heptan-5-yl)phenyl)-7-(4-(trifluoromethyl)phenyl)-2-naphthoate **(300)** and ethyl 4-(4-((1R,2R,4R)-4-amino-2-(hydroxymethyl)cyclopentyl)phenyl)-7-(4-(trifluoromethyl)phenyl)-2-naphthoate **(301).** Method A was used to prepare **300.** Treatment of **198** (7.75 mg, 0.031 mmol) by using Procedure F gave **300** (7.6 mg, 48%) of sufficient purity to be used in next step. Also, method B was used to prepare **300** and **301.** A dry flask was vacuumed and then then charged with argon, **102** (185 mg, 0.35 mmol), tris(perfluorophenyl)borane (179.2 mg, 0.35 mmol), borane ammonia complex (108 mg, 3.5 mmol), and 1,2-dichloroethane (20 mL). The resulting solution was stirred at 75 °C for 48 h. DCM and H₂O were added. The mixture was basified with 6 N NaOH aqueous solution and extracted with dichloromethane three times. The organic layer was dried over anhydrous Na₂SO₄. Volatiles were evaporated, and the residue was column chromatographed (CH₂Cl₂/MeOH/TEA, 100:0:1→90:10:1) to give **300** (31 mg, 17%) in addition to **301** (46.7, 25%) of sufficient purity to be used in next step. **300** has: ¹H NMR (400 MHz, chloroform-d) δ 8.68 (s, 1H), 8.23 (s, 1H), 8.05 - 7.98 (m, 2H), 7.82 (d, *J* = 8.2 Hz, 2H), 7.80 - 7.72 (m, 3H), 7.50 (dd, *J =* 8.1, 2.3 Hz, 2H), 7.36 (d, *J =* 7.7 Hz, 2H), 4.51 - 4.41 (m, 2H), 4.18 (s, 1H), 3.45 - 3.37 (m, 1H), 3.36 - 3.19 (m, 4H), 2.98 - 2.91 (m, 2H), 2.89 (s, 1H), 2.68 (dd, *J* = 14.4, 9.3 Hz, 1H), 1.48 - 1.42 (m, 4H). HRMS m/z [M+H]⁺ for C₃₂H₂₉NO₂F₃ calculated 516.2150, found 516.2142. **301** has HRMS m/z [M+H]⁺ for C₃₂H₃₁NO₃F₃ calculated 534.2256, found 534.2255.

Ethyl 4-(4-((1*R*,4*R*,5*R*)-3-oxo-2-azabicyclo[2.2.1]heptan-5-yl)phenyl)-7-(4-(trifluoromethyl)phenyl)-2-naphthoate **(302).** Treatment of **196** (109.1 mg, 0.41 mmol) by using Procedure F (column chromatography; hexane/ethyl acetate = 50:50 → 0:100) gave **302** (211.8 mg, 98%) as a white powder: ¹H NMR (400 MHz, CDCl₃) δ 8.71 - 8.66 (m, 1H), 8.23 (d, *J =* 1.9 Hz, 1H), 8.07 - 8.00 (m, 2H), 7.83 (d, *J =* 8.1 Hz, 2H), 7.81 - 7.74 (m, 3H), 7.52 - 7.48 (m, 2H), 7.43 (d, *J =* 8.2 Hz, 2H), 5.59 (s, 1H), 4.46 (q, *J =* 7.1 Hz, 2H), 4.09 (s, 1H), 3.46 (dd, *J* = 8.7, 5.0 Hz, 1H), 3.00 (s, 1H), 2.35 (ddd, *J* = 11.7, 8.7, 2.3 Hz, 1H), 2.17 (ddd, *J* = 12.8, 5.1, 2.3 Hz, 1H), 2.11 - 2.05 (m, 1H), 1.93 (dd, *J =* 9.8, 1.6 Hz, 1H), 1.45 (t, *J* = 7.1 Hz, 3H). HRMS m/z [M+H]⁺ for C₃₂H₂₇NO₃F₃ calculated 530.1943, found 530.1939.

(1S,4S,6R)-6-(4-Bromophenyl)-2-azabicyclo[2.2.1]heptane **(303)** and ((1S,3S,4R)-3-amino-4-(4-bromophenyl)cyclopentyl)methanol **(304).** Treatment of **97** (53.2 mg, 0.1 mmol) by using Procedure E gave **303** (11.2 mg, 22%, t_{R} = 27 min) and **304** (14.7 mg, 27%, t_{R} = 20 min). **303** has: ¹H NMR (400 MHz, methanol-*d*₄) δ 7.53 - 7.48 (m, 2H), 7.23 - 7.18 (m, 2H), 4.08 (s, 1H), 3.26 (t, *J =* 7.5 Hz, 1H), 3.23 - 3.16 (m, 1H), 3.10 (d, *J =* 11.0 Hz, 1H), 2.85 (s, 1H), 2.11 (ddd, *J =* 11.6, 9.0, 2.4 Hz, 1H), 2.05 - 1.92 (m, 2H), 1.77 - 1.69 (m, 1H). HRMS m/z [M+H]⁺ for C₁₂H₁₅N⁷⁹Br calculated 252.0388, found 252.0387. **304** has ¹H NMR (400 MHz, methanol-*d₄*) δ 7.54 - 7.49 (m, 2H), 7.26 (d, *J* = 8.3 Hz, 2H), 3.64 (q, *J* = 8.7 Hz, 1H), 3.56 (d, *J* = 5.6 Hz, 2H), 3.09 (q, *J* = 9.2 Hz, 1H), 2.50 - 2.39 (m, 2H), 2.09 (ddd, *J =* 13.6, 8.8, 4.6 Hz, 1H), 1.96 (dt, *J =* 13.7, 9.5 Hz, 1H), 1.62 - 1.50 (m, 1H). MS m/z [M+H]⁺ for C₁₂H₁₇NO⁷⁹Br calculated 270.0, found 270.0.

Ethyl 4-(4-((1S,4S,6R)-2-azabicyclo[2.2.1]heptan-6-yl)phenyl)-7-(4-(trifluoromethyl)phenyl)-2-naphthoate **(305).** Treatment of **303** (12.6 mg, 0.05 mmol) by using Procedure F gave **305** (23 mg, with 17% TEA; yield 74%) of sufficient purity to be used in next step: ¹H NMR (400 MHz, CDCl₃) δ 8.65 (s, 1H), 8.20 (d, *J =* 2.0 Hz, 1H), 8.02 - 7.93 (m, 2H), 7.80 (d, *J* = 8.2 Hz, 2H), 7.77 - 7.69 (m, 3H), 7.46 (d, *J* = 7.8 Hz, 2H), 7.36 (d, *J* = 7.9 Hz, 2H), 4.44 (q, *J =* 7.3 Hz, 2H), 4.23 (s, 1H), 3.83 (t, *J* = 7.4 Hz, 1H), 3.34 (dt, *J =* 10.8, 3.1 Hz, 1H), 3.23 - 3.16 (m, 1H), 2.86 (s, 1H), 2.23 (dd, *J =* 13.0, 8.9 Hz, 1H), 2.11- 1.96 (m, 3H), 1.47 - 1.41 (m, 3H). HRMS m/z [M+H]⁺ for C₃₂H₂₉NO₂F₃ calculated 516.2150, found 516.2145.

Ethyl 4-(4-((1*R*,2*S*,4*S*)-2-amino-4-(hydroxymethyl)cyclopentyl)phenyl)-7-(4-(trifluoromethyl)phenyl)-2-naphthoate **(306).** Treatment of **304** (16 mg, 0.06 mmol) by using Procedure F gave **306** (19.2 mg, with 20% TEA; yield 48%) of sufficient purity to be used in next step: ¹H NMR (400 MHz, CDCl₃) δ 8.61 (s, 1H), 8.16 (d, *J =* 2.0 Hz, 1H), 8.02 - 7.89 (m, 2H), 7.84 - 7.66 (m, 5H), 7.41 (s, 4H), 4.49 - 4.36 (m, 2H), 3.75 (dt, *J* = 17.8, 4.6 Hz, 3H), 2.70 (s, 1H), 2.51 - 2.40 (m, 1H), 2.36 - 2.16 (m, 2H), 1.98 (d, *J =* 15.5 Hz, 1H), 1.47 - 1.41 (m, 3H). HRMS m/z [M+H]⁺ for C₃₂H₃₁NO₃F₃ calculated 534.2256, found 534.2258.

Ethyl 4-(4-((1*S*,4*S*,6*R*)-3-oxo-2-azabicyclo[2.2.1]heptan-6-yl)phenyl)-7-(4-(trifluoromethyl)phenyl)-2-naphthoate **(307).** Treatment of **197** (5.3 mg, 0.02 mmol) by using Procedure F (column chromatography; hexane/ethyl acetate = 50:50 → 0:100) gave **307** (10.9 mg, quantitative) as a white powder: ¹H NMR (400 MHz, CDCl₃) δ 8.69 (s, 1H), 8.24 (d, *J =* 1.9 Hz, 1H), 8.07 - 7.99 (m, 2H), 7.82 (d, *J* = 8.2 Hz, 2H), 7.80 - 7.73 (m, 3H), 7.51 (d, *J* = 8.1 Hz, 2H), 7.40 (d, *J =* 7.9 Hz, 2H), 5.84 (s, 1H), 4.47 (q, *J =* 7.1 Hz, 2H), 4.02 (s, 1H), 3.39 (dd, *J =* 8.5, 5.6 Hz, 1H), 2.94 - 2.88 (m, 1H), 2.31 - 2.17 (m, 2H), 2.08 - 2.03 (m, 1H), 1.93 - 1.88 (m, 1H), 1.46 (t, *J =* 7.1 Hz, 3H). HRMS m/z [M+H]⁺ for C₃₂H₂₇NO₃F₃ calculated 530.1943, found 530.1945.

Ethyl 4-(4-((1S,4S,SS)-2-(methylsulfonyl)-2-azabicyclo[2.2.1]heptan-5-yl)phenyl)-7-(4-(trifluoromethyl)phenyl)-2-naphthoate **(202).** Methanesulfonyl chloride (0.6 µL, 0.0075 mmol) was added into a solution of **184** (1.1 mg, 0.002 mmol) and *N,N*-Diisopropylethylamine (1.7 µL, 0.01 mmol) in DCM (0.5 mL). The resulting solution was stirred at rt for 3 h. Volatiles were removed and the residue was column chromatographed (hexane/EtOAc, 70:30→50:50) to give **202** (1.2 mg, quantitative): ¹H NMR (400 MHz, Chloroform-*d*) δ 8.69 (s, 1H), 8.23 (d, *J* = 1.9 Hz, 1H), 8.06 - 7.99 (m, 2H), 7.83 (d, *J =* 8.1 Hz, 2H), 7.80 - 7.73 (m, 3H), 7.51 - 7.46 (m, 2H), 7.38 (d, *J =* 8.0 Hz, 2H), 4.46 (q, *J =* 7.1 Hz, 2H), 4.35 (s, 1H), 3.42 (dd, *J =* 8.7, 3.4 Hz, 1H), 3.31 (d, *J* = 8.8 Hz, 1H), 3.27 - 3.19 (m, 1H), 2.92 (s, 3H), 2.90 (s, 1H), 2.59 - 2.48 (m, 1H), 2.00 - 1.87 (m, 2H), 1.73 (d, *J =* 10.7 Hz, 1H), 1.45 (t, *J =* 7.1 Hz, 3H). HRMS m/z [M+H]⁺ for C₃₃H₃₁NO₄F₃³²S calculated 594.1926, found 594.1927.

*tert*-Butyl (1*S*,4*S*,5*S*)-5-(4-bromophenyl)-2-azabicyclo[2.2.1]heptane-2-carboxylate **(211).** To a solution of **182** (23 mg, 0.09 mmol), TEA (44.5 µL, 32.3 mg, 0.32 mmol), DMAP (11 mg, 0.09 mmol) in CH₂Cl₂ (1 mL) was added (Boc)₂O (59 mg, 0.27 mmol). The resulting solution was stirred at rt overnight. The volatiles were evaporated, and the residue was column chromatographed (hexane/EtOAc, 100:0→80:20) to give **211** (19.3, 61%): ¹H NMR (400 MHz, Chloroform-*d*) δ 7.41 (d, *J =* 8.4 Hz, 2H), 7.07 (d, *J =* 8.1 Hz, 2H), 4.36 (s, 0.46H), 4.23 (s, 0.54H), 3.37 - 3.27 (m, 1H), 3.21 - 3.07 (m, 1H), 2.99 - 2.91 (m, 1H), 2.61 (s, 1H), 2.34 - 2.16 (m, 1H), 1.76 - 1.65 (m, 2H), 1.54 - 1.49 (m, 1H), 1.47 (s, 9H). HRMS m/z [M - *t*-butyl+2H]⁺ for C₁₃H₁₅⁷⁹BrNO₂ calculated 296.0286, found 296.0284.

*tert*-Butyl (1*S*,4*S*,5*S*)-5-(4-(3-(ethoxycarbonyl)-6-(4-(trifluoromethyl)phenyl)naphthalen-1-yl)phenyl)-2-azabicyclo[2.2.1]heptane-2-carboxylate **(212).** Treatment of **212** (19.3 mg, 0.055 mmol) by using Procedure F gave **212** (34 mg, quantitative): ¹H NMR (400 MHz, Chloroform-d) δ 8.68 (d, *J =* 1.7 Hz, 1H), 8.23 (d, *J =* 2.0 Hz, 1H), 8.08 - 8.01 (m, 2H), 7.82 (d, *J* = 8.1 Hz, 2H), 7.79 - 7.74 (m, 3H), 7.48 (d, *J* = 7.9 Hz, 2H), 7.37 (d, *J =* 8.0 Hz, 2H), 4.51 - 4.39 (m, 2H), 4.44 (s, 0.47H), 4.32 (s, 0.53H), 3.45 - 3.35 (m, 1H), 3.24 (dd, *J =* 29.8, 9.7 Hz, 1H), 3.18 - 3.10 (m, 1H), 2.77 (s, 1H), 2.45 - 2.26 (m, 1H), 1.95 - 1.84 (m, 1H), 1.84 - 1.77 (m, 1H), 1.75 - 1.68 (m, 1H), 1.52 (s, 9H), 1.45 (t, *J* = 7.1 Hz, 3H).

4-(4-((1*S*,4*S*,5*S*)-2-(tert-Butoxycarbonyl)-2-azabicyclo[2.2.1]heptan-5-yl)phenyl)-7-(4-(trifluoromethyl)phenyl)-2-naphthoic acid **(213).** Treatment of **212** (34 mg, 0.055 mmol) by using Procedure B gave **213** (22.7 mg, 64%) as a white solid: ¹H NMR (400 MHz, Methanol-*d*₄) δ 8.65 (d, *J* = 1.8 Hz, 1H), 8.29 (d, *J* = 1.9 Hz, 1H), 7.96 - 7.86 (m, 4H), 7.81 - 7.71 (m, 3H), 7.40 - 7.30 (m, 4H), 4.30 (d, *J* = 8.5 Hz, 1H), 3.39 - 3.33 (m, 1H), 3.19 (d, *J* = 9.6 Hz, 1H), 3.08 (t, *J =* 7.2 Hz, 1H), 2.69 (s, 1H), 2.31 - 2.18 (m, 1H), 1.91 - 1.82 (m, 1H), 1.82 - 1.76 (m, 1H), 1.70 - 1.60 (m, 1H), 1.50 (s, 9H).

*tert*-Butyl (1*S*,4*S*,5*S*)-5-(4-(3-(hydroxycarbamoyl)-6-(4-(trifluoromethyl)phenyl)naphthalen-1-yl)phenyl)-2-azabicyclo[2.2.1]heptane-2-carboxylate **(215).** Carbonyldiimidazole (32 mg, 0.2 mmol) was added to a solution of **213** (5.9 mg, 0.01 mmol) in THF (1 mL). The resulting solution was stirred at rt overnight to give intermediate **214** (MS m/z [M+H]⁺ for C₃₈H₃₅N₃O₃F₃ calculated 638.2, found 638.2). Into the reaction residue was added hydroxylamine hydrochloride (14 mg, 0.2 mmol) and the reaction mixture was stirred at rt for 2 h to give **215** (HRMS m/z [M - *t*-butyl+2H]⁺ for C₃₁H₂₆N₂O₄F₃ calculated 547.1845, found 547.1840) as single product. **215** was acid-labile and decomposed during column chromatography.

*tert*-Butyl (1S,4S,SS)-5-(4-(3-((2-(dimethylamino)-2-oxoethoxy)carbonyl)-6-(4-(trifluoromethyl)phenyl)naphthalen-1-yl)phenyl)-2-azabicyclo[2.2.1]heptane-2-carboxylate **(217).** To a solution of **213** (164 mg, 0.280 mmol) in DMF (10 mL) was added K₂CO₃ (116 mg, 0.840 mmol). 2-Chloro-*N,N*-dimethylacetamide (68 mg, 0.560 mmol) was added to the reaction mixture under N₂ atmosphere, and the reaction was stirred at 45 °C for 1 h under N₂ atmosphere. The reaction mixture was partitioned ethyl acetate (10 mL) and water (10 mL), and the aqueous phase was extracted with ethyl acetate (2 × 10 mL). The combined organic layers were dried over Na₂SO₄, filtered, and concentrated under pressure. The residue was purified by silica gel chromatography (hexane:ethyl acetate = 3:1) to afford compound **217** (170 mg, 90%) as a white solid; ¹H NMR (400 MHz, methanol-*d*₄) δ 8.74 (s, 1H), 8.42 (s, 1H), 8.04 - 7.95 (m, 4H), 7.95 - 7.88 (m, 1H), 7.80 (d, *J* = 8.0 Hz, 2H), 7.56 - 7.46 (m, 4H), 5.06 (s, 2H), 4.25 (s, 1H), 3.35 - 3.26 (m, 3H), 2.98 (s, 6H), 2.96 (s, 1H), 2.46 - 2.35 (m, 1H), 2.18 (d, *J =* 15.1 Hz, 1H), 2.11 (d, *J* = 11.7 Hz, 1H), 1.81 (d, *J =* 11.8 Hz, 1H), 1.42 (s, 9H). HRMS m/z [M+H]⁺ for C₃₉H₄₀N₂O₅F₃ calculated 673.1943, found 673.1945.

2-(Dimethylamino)-2-oxoethyl 4-(4-((1S,4S,SS)-2-azabicyclo[2.2.1]heptan-5-yl)phenyl)-7-(4-(trifluoromethyl)phenyl)-2-naphthoate **(218).** A TFA solution in THF (67%, 1.5 mL) was added to **217** (100.0 mg, 0.148 mmol) and the resulting mixture was stirred at rt for 0.5 h. The solvent was evaporated with toluene under reduced pressure. The residue was purified by silica gel column chromatography (dichloromethane:methanol = 95:5) to afford **218** (75 mg, 88%) the compound as a white solid. ¹H NMR (400 MHz, methanol-*d*₄) δ 8.74 (s, 1H), 8.42 (s, 1H), 8.04 - 7.95 (m, 4H), 7.95 - 7.88 (m, 1H), 7.80 (d, *J* = 8.0 Hz, 2H), 7.56 - 7.46 (m, 4H), 5.06 (s, 2H), 4.25 (s, 1H), 3.35 - 3.26 (m, 3H), 2.98 (s, 6H), 2.96 (s, 1H), 2.46 - 2.35 (m, 1H), 2.18 (d, *J =* 15.1 Hz, 1H), 2.11 (d, *J =* 11.7 Hz, 1H), 1.81 (d, *J =* 11.8 Hz, 1H). HRMS m/z [M+H]⁺ for C₃₄H₃₂N₂O₃F₃ calculated 573.1943, found 573.1945.

(7-(Diethylamino)-2-oxo-2*H*-chromen-4-yl)methyl 4-(4-((1*S*,4*S*,5*S*)-2-azabicyclo[2.2.1]heptan-5-yl)phenyl)-7-(4-(trifluoromethyl)phenyl)-2-naphthoate **(220).** *N,N'-*Dicyclohexylcarbodiimide (4.54 mg, 0.022 mmol) was added to a stirred solution of **213** (12.3 mg, 0.02 mmol), 7-(diethylamino)-4-(hydroxymethyl)coumarin (5.44 mg, 0.022 mmol), and 4-dimethylaminopyridine (2.7 mg, 0.022 mmol) in anhydrous DCM (1 mL) at 0 °C. After 10 min stirring at 0 °C, the reaction mixture was warmed to rt and stirred at rt overnight to give **219** (HRMS m/z [M - *t*-butyl+2H]⁺ for C₄₅H₄₀N₂O₆F₃ calculated 761.2838, found 761.2847). Into the reaction mixture was added TFA (1 mL). The resulting mixture was stirred at rt for 1 h. Toluene (1 mL) was added to mixture and the volatiles were removed. The residue was column chromatographed (DCM/MeOH/TEA, 100:0: 1-80:20: 1) to give **220** (14 mg, 98%): ¹H NMR (400 MHz, Chloroform-d) δ 8.76 (s, 1H), 8.28 (d, *J =* 2.0 Hz, 1H), 8.09 - 8.01 (m, 2H), 7.86 - 7.80 (m, 3H), 7.76 (d, *J* = 7.7 Hz, 2H), 7.50 (d, *J* = 7.9 Hz, 2H), 7.41 - 7.36 (m, 3H), 6.61 (dd, *J* = 9.0, 2.6 Hz, 1H), 6.54 (d, *J =* 2.6 Hz, 1H), 6.32 (s, 1H), 5.55 (s, 2H), 4.14 (s, 1H), 3.42 (q, *J*= 7.1 Hz, 4H), 3.30 (q, *J =* 6.9 Hz, 2H), 3.20 (d, *J =* 10.3 Hz, 1H), 2.90 (d, *J =* 3.3 Hz, 1H), 2.68 (dd, *J =* 14.1, 9.2 Hz, 1H), 1.98 - 1.88 (m, 3H), 1.22 (t, *J =* 7.1 Hz, 6H). HRMS m/z [M+H]⁺ for C₄₄H₄₀N₂O₄F₃ calculated 717.2940, found 717.2944.

### EXAMPLE 2

This example demonstrates inhibition of fluorescent antagonist **(2)** binding in hP2Y₁₄R-expressing CHO cells (X, Y, Z = CH, unless noted). The results are set forth in Tables 1 and 2.

**Table 1**

| 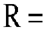 | | | |
|---|---|---|---|
| 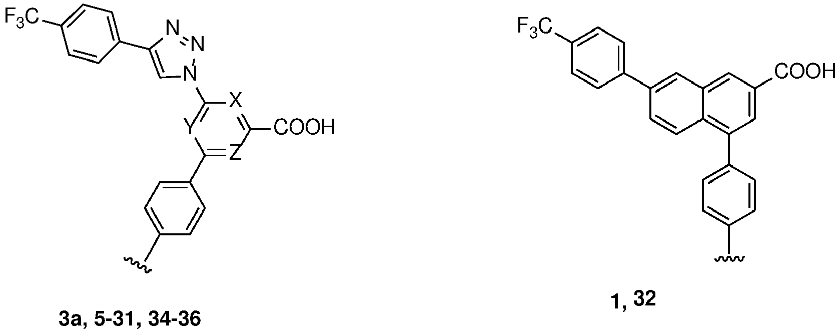 | | | |
| **Compound** | **Structure^{a} =** | **IC₅₀, hP2Y₁₄R (µM, mean±SEM)** | **cLogP^{c}** |
| *Comparative compounds* | | | |
| **1a^{b}** | 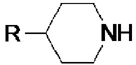 | 0.00796±0.0035 | 6.18 |
| **1b^{b}** | 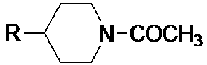 | 0.0276±0.0043 | 6.47 |
| **1c** | 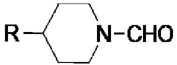 | 0.0366±0.0109 | 5.99 |
| **1d** | 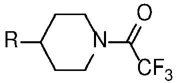 | | |
| **3a^{b}** | 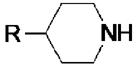 | 0.0317±0.0080 | 4.64 |
| **3b** | 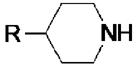 | 60.5±6.9 | 3.89 |
| **5^{b}** | R-CONH₂ | 0.269±0.121 | 3.63 |
| **6^{b}** | 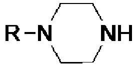 | 0.233±0.026 | 3.95 |

| *Phenyl-CO or NH* | | | |
|---|---|---|---|
| **7** | R-NH₂ | 1.48±0.16 | 4.03 |
| **8** | R-NH-Ac | 0.811±0.095 | 4.07 |
| c | R-NH-CO-phenyl | 1.86±0.54 | 5.19 |
| **10** | R-NH-Boc | 3.16±0.31 | 4.98 |
| **11** | 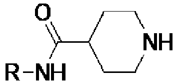 | 0.197±0.029 | 3.85 |
| **12** | 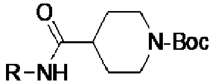 | 22.5±3.7 | 4.85 |
| **13** | R-COOH | 0.632±0.023 | 3.88 |
| **14** | R-CONH(CH₂)₃NH₂ | 0.588±0.043 | 0.92 |

| *Phenyl-halo or acyclic* | | | |
|---|---|---|---|
| **15** | R-Br | >10 | 4.71 |
| **16** | R-CH₂CONH₂ | 1.29±0.32 | 3.65 |
| **17** | R-(CH₂)₂-CN | 17.8±2.9 | 4.74 |
| **18** | R-(CH₂)₃-NH₂ | 0.292±0.067 | 1.60 |
| **19** | R-C□CCH₂-NH₂ | 0.308±0.063 | 1.47 |
| **20** | R-(CH₂)₃-NH-Boc | 42.5±27.0 | 5.37 |
| **21** | R-(CH₂)₄-OH | 9.22±1.22 | 4.84 |

| *Phenyl-cyclic* | | | |
|---|---|---|---|
| **22** | 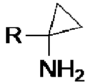 | 14.1±0.9 | 1.36 |
| **23** | 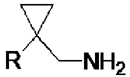 | 8.91± 0.75 | 1.57 |
| **24** | 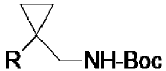 | 18.8±2.0 | 5.41 |
| **25** | 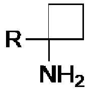 | 2.00±0.18 | 1.70 |
| **26** | 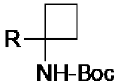 | >10 | 5.41 |
| **27** | 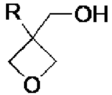 | 2.86±0.44 | 4.73 |
| **28** | 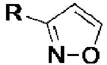 | 2.23±0.38 | 4.98 |
| **29** | 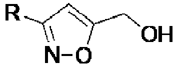 | 0.296±0.013 | 4.44 |
| **30** | 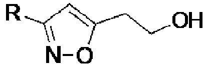 | 0.389±0.042 | 4.59 |
| **31** | 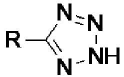 | 0.895±0.122 | 3.65 |

| | | | |
|---|---|---|---|
| | | | |

| *Alternative scaffolds* | | | |
|---|---|---|---|
| **32** | 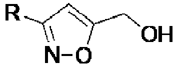 | 0.0150±0.0044 | 5.77 |
| **33** | 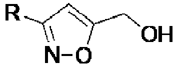 | 7.40±1.05 | 4.36 |

| *Aza-scan of **3a*** | | | |
|---|---|---|---|
| **34** | 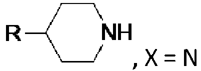 | 1.69±0.28 | 4.16 |
| **35** | 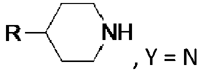 | 4.90±0.54 | 4.10 |
| **36** | 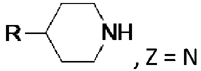 | 2.92±0.31 | 4.01 |

| | | | |
|---|---|---|---|
| a. *p*-Phenyl substituted, unless noted. IC₅₀ values are mean ± SEM for 3-4 separate determinations, unless noted. b. Reported in Yu et al.¹, Jung et al.² and Mufti et al.⁷ c. cLogP calculated using ALOGPS 2.1 program (www.vcclab.org/lab/alogps/).⁵⁷ | | | |

**Table 2**

| 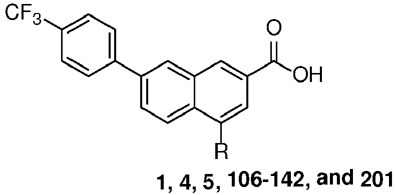 | | | | | | |
|---|---|---|---|---|---|---|
| No. | R= | IC₅₀ (nM) | Solubility (µg/mL) | | Log D_{7.4}^{e} | cLog P^{c} |
| | | | pH 4 | pH 7.4 | | |
| **1** | 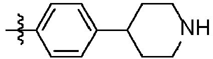 | 7.96±0.35 | 1.10±0.07 | 0.36±0.03 | 1.86±0.02 | 6.18 |
| **4** | 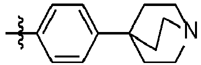 | 20.0±4.4 | 1.50±0.04 | 0.55±0.02 | 4.18±0.85^{f} | 6.32 |
| **105** ^{b} | 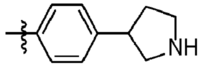 | 31.5±4.2 | 1.32±0.11 | 0.47±0.08 | 1.89±0.07^{f} | 5.79 |
| **106** | 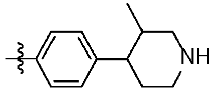 | 20.9±3.6 | 1.36±0.21 | 0.03±0.02 | 2.79±0.09^{f} | 6.48 |
| **107** | 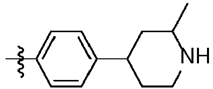 | 17.8±2.0 | 0.31±0.04 | 0.16±0.03 | 2.05±0.11^{f} | 6.55 |
| **108** ^{b} | 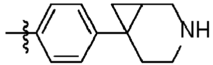 | 45.5±7.2 | 1.32±0.17 | 0.17±0.01 | 2.44±0.06^{f} | 5.99 |
| **109** ^{b} | 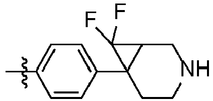 | 211±36 | 1.13±0.34 | 0.25±0.01 | 2.71±0.33^{f} | 5.97 |
| **110** ^{b} | 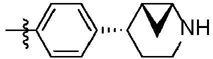 | 42.4±11.7 | 0.10±0.03 | 0.51±0.23 | 2.59±0.20^{f} | 5.67 |
| **111** ^{b} | 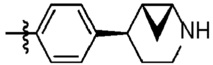 | 61.8±14.1 | 0.24±0.02 | 0.05±0.04 | 1.75±0.06^{f} | 5.67 |
| **112** ^{b} | 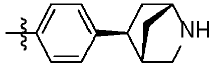 | 5.90±0.70 | 2.73±0.33 | 0.47±0.06 | 1.95+0.01^{f} | 5.95 |
| **113** ^{b} | 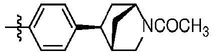 | 20.4±2.4 | 0.13±0.04 | 2.53±0.43 | 0.921±0.034 | 6.48 |
| **114** | 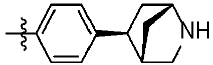 | 3.11±0.62 | 1.22±0.05 | 0.77±0.21 | 1.92±0.09 | 5.95 |
| **115** | 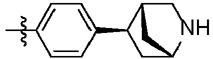 | 8.62±2.11 | 1.22±0.05^{g} | 0.77±0.21^{g} | 1.92±0.09^{g} | 5.95 |
| **116** | 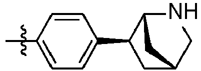 | 9.8±0.3 | 1.45±0.05 | 0.40±0.13 | 2.11±0.06 | 5.90 |
| **142** | 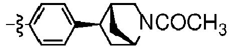 | 64.1±18.8 | 0±0.03^{g} | 1.86±0.05^{g} | 0.545±0 | 6.48 |
| **117** | 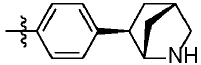 | 25.9±7.6 | 1.45±0.05^{g} | 0.40±0.13^{g} | 2.11±0.06^{g} | 5.90 |
| **118** | 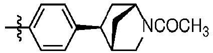 | 107±13 | 0±0.03 | 1.86±0.05 | 0.921±0.034 | 6.48 |
| **119** | 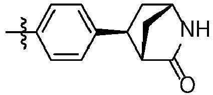 | 101±70 | 0.31±0.14^{g} | 3.42±0.46^{g} | 0.490±0.030^{g} | 5.99 |
| **120** | 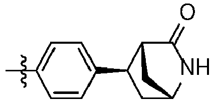 | 39.5±9.8 | 0.31±0.14 | 3.42±0.46 | 0.490±0.030 | 5.99 |
| **121** | 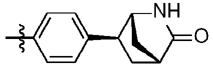 | 37.3±6.0 | 0.71±0.15 | 2.93±0.33 | 0.508±0.026 | 5.91 |
| **122** | 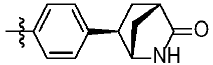 | 58.6±12.9 | 0.71±0.15^{g} | 2.93±0.33^{g} | 0.508±0.026^{g} | 5.91 |
| **123** | 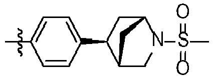 | 497±92 | ND | ND | ND | 5.70 |
| **125 a** | 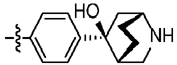 | 114±42 | 2.66±0.29 | 2.19±0.10 | 0.74±0.05 | 5.14 |
| **125 b** | 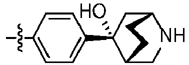 | 15.6±7.0 | 1.55±0.11 | 0.74±0.06 | 0.96±0.02 | 5.14 |
| **125 c** | 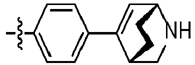 | 23.9±4.4 | 2.95±0.07 | 0.47±0.08 | 1.94±0.02 | 6.20 |
| **126 ^{b}** | 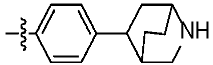 | 25.1±3.9 | 0.80±0.05 | 0.49±0.06 | 1.74±0.02 | 6.22 |
| **127** | 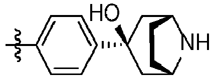 | 117±38 | 1.83±0.28 | 0.75±0.03 | 1.16±0.08^{f} | 5.02 |
| **128** | 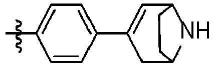 | 34.9±3.8 | 2.36±0.01 | 0.35±0.04 | 2.33±0.04^{f} | 6.02 |
| **129** | 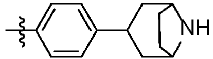 | 38.3±2.0 | 0.38±0.04 | 0.13±0.00 | 2.33±0.09^{f} | 6.27 |
| **130** | 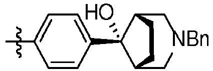 | 466±100 | 0.48±0.03 | 0.14±0.01 | 3.26±0.01^{f} | 7.61 |
| **131** | 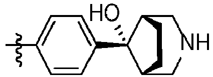 | 21.3±1.1 | 2.32±0.25 | 0.54±0.09 | 1.20+0.01^{f} | 5.12 |
| **132** | 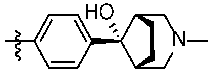 | 44.6 ±1.7 | 1.81±0.05 | 0.88±0.09 | 1.37±0.02^{f} | 6.41 |
| **133** | 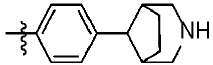 | 696±100 | 0.32±0.01 | 0.16±0.01 | 2.97±0.02^{f} | 6.17 |
| **142** | 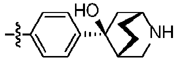 | 114±42 | 2.66±0.29 | 2.19±0.10 | 0.74±0.05 | 5.14 |
| **134** | 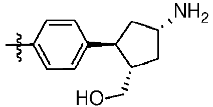 | 10.8±2.1 | 4.97±0.11^{g} | 0.66±0.02^{g} | 0.957±0.046^{g} | 2.33 |
| **135** | 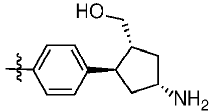 | 12.6±1.9 | 4.97±0.11 | 0.66±0.02 | 0.957±0.046 | 2.33 |
| **136** | 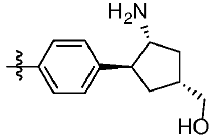 | 100±19 | 3.74±0.09 | 0.93±0.20 | 1.06±0.05 | 2.35 |
| **137** | 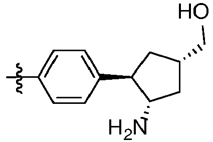 | 125±45 | 3.74±0.09^{g} | 0.93±0.20^{g} | 1.06±0.05^{g} | 2.35 |
| **138** | 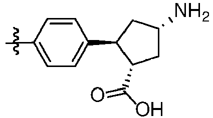 | 49.6±13.5 | 1.05±0.20^{g} | 5.52±0.21^{g} | -0.568±0.038^{g} | 2.26 |
| **139** | 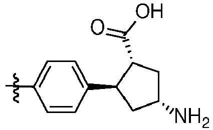 | 102±25 | 1.05±0.20 | 5.52±0.21 | -0.568±0.038 | 2.26 |
| **140** | 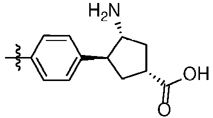 | 165±51 | 0.34±0.07 | 3.92±0.10 | -0.478±0.050 | 2.31 |
| **141** | 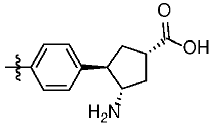 | 269±83 | 0.34±0.07^{g} | 3.92±0.10^{g} | -0.478±0.050^{g} | 2.31 |
| **201** ^{d} | 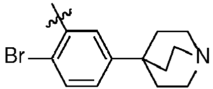 | 8900± 1160 | ND | ND | ND | 6.73 |
| **508** | 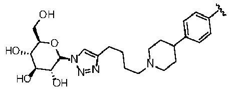 | 15.7±2.5 | ND | ND | ND | 4.74 |

| | | | | | | |
|---|---|---|---|---|---|---|
| *^{a}* IC₅₀ for inhibition of hP2Y₁₄R antagonist binding, determined using flow cytometry of whole hP2Y₁₄R-CHO cells in the presence of a fixed concentration (20 nM) of fluorescent hP2Y₁₄R antagonist **3** (mean±SEM, n = 3 - 6). *^{b}* Racemate. *^{c}* cLogP calculated using ALOGPS 2.1 program (www.vcclab.org/lab/alogps/).¹⁶ *^{d}* Upon prolonged storage, some decomposition was observed. *^{e}* HPLC-based lipophilicity. HPLC column: Agilent Eclipse XDB-C18 (5 µm); Mobile phase: A: Acetonitrile, B: 10 mM TEAA, 10%-100% A in 20 min; Flow rate: 1 mL/min. ^{f} Log D_{7.4} Calculated from retention factor difference between ligands and 1 (*k* = 3.41). ND, not determined. ^{g} Data taken from compound's enantiomer. | | | | | | |

### EXAMPLE 3

This Example illustrates a method of preparing compounds of formula (III). The synthesis includes a Grignard reaction of ketone starting materials for the generation of bromophenyl intermediates, Suzuki coupling of bromophenyl intermediates with boronic acid pinacol ester to obtain the phenylnaphthalenecarboxylate core structure, hydrolysis of esters to free carboxylic acid, Boc/benzyl deprotection to free amine, and conversion of analogues to their related ligands, as illustrated herein. Compound **405** with azetidine was prepared via Suzuki coupling between commercially available 3-(4-bromophenyl)azetidine **421** and naphthalenyl boronic acid pinacol ester **422** followed by ester hydrolysis. 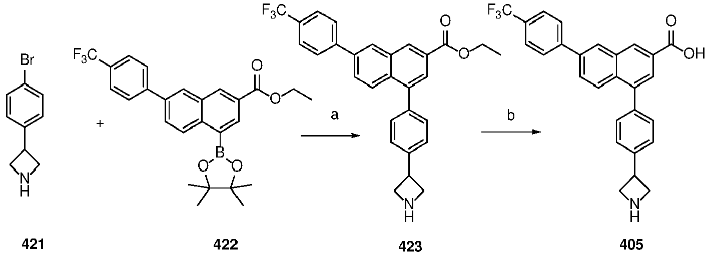 ^{a}Reagents and conditions: (a) **422,** Na₂CO₃, Pd(PPh₃)₄, DME-H₂O (4:1), 80°C, overnight, 57%; (b) LiOH, THF-MeOH-H₂O (3:1:1), rt, 3 h, 47%.

### EXAMPLE 4

This Example illustrates a method of preparation of PPTN analogues with 2-azaspiro[3.3]heptane, octahydrocyclopenta[c]pyrrole (B), 3-azabicyclo[3.1.1]heptane (C), 1-azacycloheptane (D), and azocane (E) were summarized in Scheme 2. In a one-pot reaction, treatment of ketones **424, 428, 432, 436, and 440** with in-situ generated Grignard reagent 4-bromophenylmagnesium bromide provided bromophenyl intermediates **425, 429, 433, 437,** and **441,** respectively. Derived from a racemic starting material **428,** intermediate **429** is a racemic mixture of left-handed and right-handed helix enantiomers. Stereoselective Grignard reaction of **432** yielded enantiomer **433** with hydroxyl group and one-carbon bridge identified as *trans* to each other.

The Suzuki coupling of naphthalenyl boronic acid pinacol ester **422** with the 4-(4-bromo-phenyl) intermediates **425, 429, 433, 437,** and **441** gave their corresponding esters of analogues **426, 430, 434, 438,** and **442.** The ester hydrolysis of **426, 430, 434, 438,** and **442** provided the derivatives **427, 431, 435, 439,** and **443.** Boc deprotection of **427** with TFA in DCM offered product 408 in addition to unexpected dehydrated product **407.** Compound **408** was unstable in the reaction residue in the presence of methanol and partially reacted with methanol and methanol-d4 to give *O*-methylated ligand **409.** Thus, methanol should be avoided if methylated by-product is not needed. Similarly, boc deprotection of **443** gave product **420** and dehydrated compound **419.** Nevertheless, boc deprotection of **439** meanwhile dehydrated the hydroxyl group to yield alkene mixture **417.** Boc deprotection of compound **435** simply gave ligand **415.** Pd-catalyzed debenzylation of **431** with H₂ afforded ligand **411,** which was further dehydrated by refluxing in TFA to give **412.** Reduction of **407, 412, 417,** and **419** by Pd-catalyzed hydrogenation offered PPTN analogues **406** with 2-azaspiro[3.3]heptane, **413** with octahydrocyclopenta[c]pyrrole (B), **416** with 1-azacycloheptane (D), and **418** with azocane (E), respectively. Stereoselective deoxygenation of **415** with sodium borohydride in sulfuric acid afforded **414.** Thus, hygroscopic sulfuric acid was employed to readily generate tertiary benzylic carbocation, which was further reduced by sodium borohydride to yield deoxygenated product.

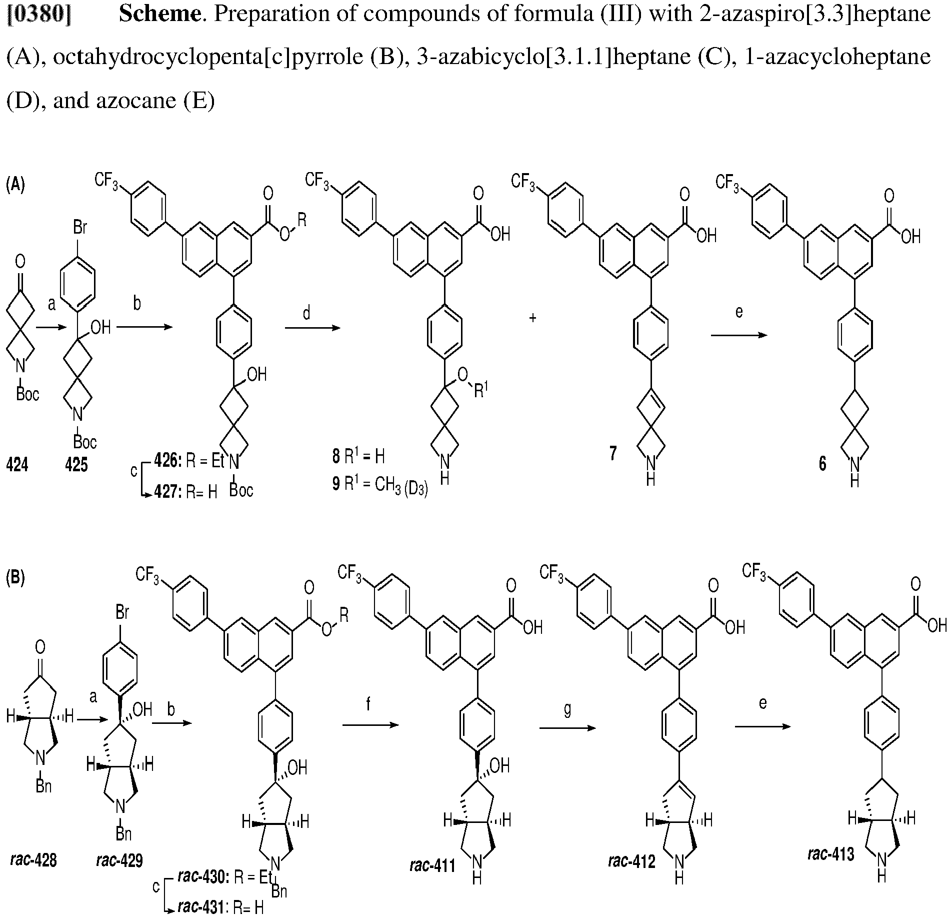

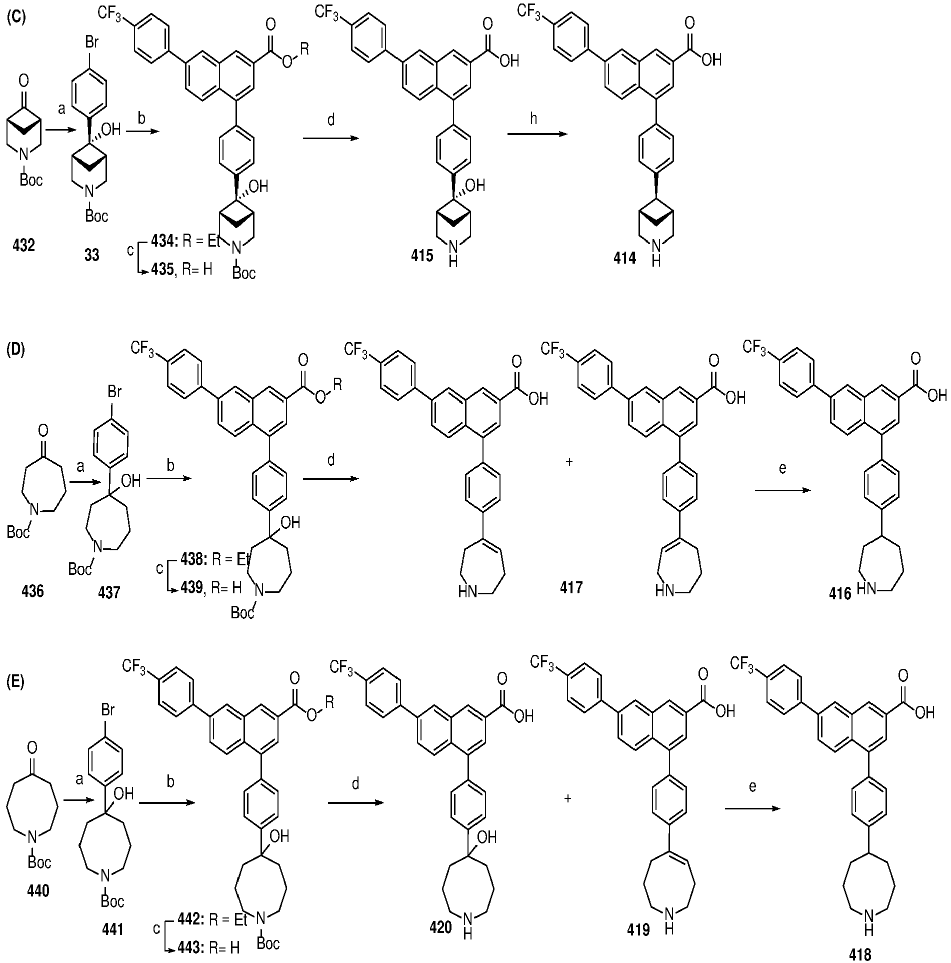

^{a}Reagents and conditions: (a) 4-bromophenylmagnesium bromide (in-situ generated: dibromobenzene, magnesium, THF, sonication, rt, 2 h), THF, 0 °C, 4 h; (b) boronic acid pinacol ester 4**22,** Na₂CO₃, Pd(PPh₃)₄, DME-H₂O (4:1), 85 °C, overnight; (c) LiOH, THF-MeOH-H₂O (3:1:1), rt, 3 h; (d) TFA, DCM, rt, 1 h; (e) H₂, Pd/C, DMF, 3 h; (f) H₂, Pd/C, DMF, 5 h; (g) TFA, 90°C, 2 h; (h) NaBH₄, THF, H₂SO₄, rt, 30 min;

### Chemical synthesis

**General Information.** *Materials and Methods.* All chemicals and anhydrous solvents were obtained directly from commercial sources. All reactions were carried out under argon atmosphere using anhydrous solvents unless specified otherwise. Room temperature (rt) refers to 25 ± 5 °C. Silica-gel precoated with F254 on aluminum plates were used for TLC. The spots were examined under ultraviolet light at 254 nm and further visualized by anisaldehyde or cerium ammonium molybdate stain solution. Column chromatography was performed on silica gel (40-63 µm, 60 Å). NMR spectra were recorded on a Bruker 400 MHz spectrometer. Chemical shifts are given in ppm (δ), calibrated to the residual solvent signal peaks of CDCl₃ (7.26 ppm), CD₃OD (3.31 ppm), or DMSO-*d*₆ (2.50 ppm) for ¹H NMR with coupling constant (*J*) values reported in Hz. High resolution mass (HRMS) measurements were performed on a proteomics optimized Q-TOF-2 (Micromass-Waters). The RP-HPLC was performed using Phenomenex Luna 5 µm C18(2)100A, AXIA, 21.2 mm × 250 mm column. Antagonist purity was determined as ≥ 95% using Agilent ZORBAX SB-Aq, 5 µm, 4.6 mm × 150 mm column attached to Agilent 1100 HPLC system. The HPLC traces for compounds tested in vivo (xxx) were included in Supporting Information.

4-(4-(Azetidin-3-yl)phenyl)-7-(4-(trifluoromethyl)phenyl)-2-naphthoic acid **(405). Procedure A.** LiOH (7.4 mg, 0.3 mmol) was added to a solution of **423** (6.25 mg, 0.013 mmol) in THF-H₂O-MeOH (3:1:1, 1 mL). The resulting mixture was sonicated for 30 s and stirred at rt for 3 h. The reaction was quenched and neutralized by 1 M HCl. Volatiles were evaporated, and the residue was purified by RP-HPLC (C18, A: ACN, B: 10 mM TEAA, 45% → 60% A in 40 min, flow rate = 5 mL/min, t_{R} = 17.2 min) to give **405** as a white powder (2.73 mg, 47%): ¹H NMR (400 MHz, MeOD) δ 8.76 (s, 1H), 8.44 (d, *J =* 2.0 Hz, 1H), 8.04 - 7.96 (m, 4H), 7.93 (dd, *J =* 8.8, 1.9 Hz, 1H), 7.81 (d, *J* = 8.1 Hz, 2H), 7.60 (s, 4H), 4.53 - 4.31 (m, 5H). HRMS m/z [M+H]⁺ for C₂₇H₂₁NO₂F₃ calculated 448.1524, found 448.1520.

4-(4-(2-Azaspiro[3.3]heptan-6-yl)phenyl)-7-(4-(trifluoromethyl)phenyl)-2-naphthoic acid **(406). Procedure B.** Pd/C (10%, 3 mg) was added to a solution of **407** (0.0016 mmol) in DMF (0.3 mL). The resulting mixture was bubbled with H₂ at rt for 5 h. The Pd/C was filtered. Volatiles were evaporated, and the residue was purified by RP-HPLC (C18, A: ACN, B: 10 mM TEAA, 45% → 60% A in 40 min, flow rate = 5 mL/min, t_{R} = 20 min) to give **406** as a white powder (0.4 mg, 51%): ¹H NMR (400 MHz, MeOD) δ 8.74 (s, 1H), 8.43 (d, *J* = 2.0 Hz, 1H), 8.01 (dd, *J* = 8.7, 3.6 Hz, 3H), 7.96 - 7.89 (m, 2H), 7.81 (d, *J* = 8.1 Hz, 2H), 7.48 (d, *J =* 8.0 Hz, 2H), 7.42 (d, *J =* 8.0 Hz, 2H), 4.30 (s, 2H), 4.07 (s, 2H), 3.62 - 3.60 (m, 1H), 2.83 - 2.75 (m, 2H), 2.51 (td, *J =* 9.7, 2.9 Hz, 2H). HRMS m/z [M+H]⁺ for C₃₀H₂₅NO₂F₃ calculated 488.1837, found 488.1833.

4-(4-(2-Azaspiro[3.3]hept-5-en-6-yl)phenyl)-7-(4-(trifluoromethyl)phenyl)-2-naphthoic acid **(407),** 4-(4-(6-Hydroxy-2-azaspiro[3.3]heptan-6-yl)phenyl)-7-(4-(trifluoromethyl)phenyl)-2-naphthoic acid **(408),** and 4-(4-(6-Methoxy-2-azaspiro[3.3]heptan-6-yl)phenyl)-7-(4-(trifluoromethyl)phenyl)-2-naphthoic acid and 4-(4-(6-(methoxy-d3)-2-azaspiro[3.3]heptan-6-yl)phenyl)-7-(4-(trifluoromethyl)phenyl)-2-naphthoic acid (6:4, **409**). A TFA solution in THF (67%, 4.5 mL) was added to **427** (all residue from hydrolysis of **426,** 0.057 mmol). The resulting mixture was stirred at rt for 1 h. The stir bar was washed with MeOH. Volatiles were evaporated, and the residue was dissolved in MeOD for crude NMR. MeOD was removed and the residue was purified by RP-HPLC (C18, A: ACN, B: 10 mM TEAA, 45% → 60% A in 40 min, flow rate = 5 mL/min) to give **407** (1.8 mg, 7%, t_{R} = 42.4 min), **408** (2.54 mg, 9%, t_{R} = 33.0 min), and **409** (10.5 mg, 36%, t_{R} = 40.6 min). **407** has: ¹H NMR (400 MHz, MeOD) δ 8.76 (s, 1H), 8.43 (d, *J =* 1.9 Hz, 1H), 8.04 - 7.97 (m, 4H), 7.97 - 7.91 (m, 1H), 7.81 (d, *J* = 8.1 Hz, 2H), 7.62 (d, *J* = 7.9 Hz, 2H), 7.56 (d, *J =* 8.0 Hz, 2H), 6.55 (s, 1H), 4.42 - 4.31 (m, 4H), 3.20 (s, 2H). HRMS m/z [M+H]⁺ for C₃₀H₂₃NO₂F₃ calculated 486.1681, found 486.1682. **408** has: ¹H NMR (400 MHz, MeOD) δ 8.72 (s, 1H), 8.41 (d, *J =* 1.9 Hz, 1H), 7.99 (d, *J =* 8.7 Hz, 4H), 7.90 (dd, *J =* 8.9, 1.9 Hz, 1H), 7.80 (d, *J =* 8.1 Hz, 2H), 7.62 (d, *J =* 8.0 Hz, 2H), 7.54 (d, *J =* 7.9 Hz, 2H), 4.30 (s, 2H), 4.09 (s, 2H), 2.94 (d, *J =* 13.0 Hz, 2H), 2.66 (d, *J =* 12.5 Hz, 2H). HRMS m/z [M+H]⁺ for C₃₀H₂₅NO₃F₃ calculated 504.1787, found 504.1796. **409** has: ¹H NMR (600 MHz, MeOD) δ 8.77 - 8.74 (m, 1H), 8.44 (d, *J =* 1.9 Hz, 1H), 8.05 - 8.00 (m, 4H), 7.94 (dd, *J =* 8.8, 2.0 Hz, 1H), 7.83 (d, *J =* 8.2 Hz, 2H), 7.63 - 7.55 (m, 4H), 4.27 (s, 2H), 4.08 (s, 2H), 3.07 (s, 2H), 2.91 - 2.84 (m, 2H), 2.80 - 2.74 (m, 2H). HRMS m/z [M+H]⁺ for C₃₁H₂₇NO₃F₃ calculated 518.1943, found 518.1939; for C₃₁H₂₄D₃NO₃F₃ calculated 521.2117, found 521.2126.

rac-4-(4-((3*aR*,6*aR*)-5-hydroxyoctahydrocyclopenta[c]pyrrol-5-yl)phenyl)-7-(4-(trifluoromethyl)phenyl)-2-naphthoic acid **(411).** Pd/C (10%, 53 mg) was added to a solution of **431** (10.7 mg, 0.0177 mmol) in DMF (5 mL). The resulting mixture was bubbled with H₂ at rt for 5 h. The Pd/C was filtered. The filtrate was purified by RP-HPLC (C18, A: ACN, B: 10 mM TEAA, 30% -> 50% A in 40 min, flow rate = 5 mL/min, t_{R} = 35 min) to give **411** as a white powder (3.18 mg, 35% overall yield from **430**): ¹H NMR (400 MHz, MeOD) δ 8.75 (s, 1H), 8.43 (d, *J=* 2.0 Hz, 1H), 8.05 - 7.96 (m, 4H), 7.93 (dd, *J* = 9.0, 2.0 Hz, 1H), 7.81 (d, *J* = 8.1 Hz, 2H), 7.69 (d, *J* = 8.2 Hz, 2H), 7.54 (d, *J* = 8.0 Hz, 2H), 3.56 - 3.46 (m, 2H), 2.98 (t, *J* = 11.2 Hz, 2H), 2.76 - 2.65 (m, 1H), 2.60 - 2.45 (m, 2H), 2.22 (dd, *J* = 12.6, 5.4 Hz, 1H), 2.06 - 1.95 (m, 2H). HRMS m/z [M+H]⁺ for C₃₁H₂₇NO₃F₃ calculated 518.1943, found 518.1944.

*rac*-4-(4-((3*aR*,6*aR*)-1,2,3,3a,4,6a-Hexahydrocyclopenta[c]pyrrol-5-yl)phenyl)-7-(4-(trifluoromethyl)phenyl)-2-naphthoic acid **(412).** A solution of **411** (0.012 mmol, crude reaction residue without HPLC purification) in TFA (2 mL) was refluxed in a 90 °C oil bath for 2 h. Volatiles were evaporated, and the residue was purified by RP-HPLC (C18, A: ACN, B: 10 mM TEAA, 45% -> 60% A in 40 min, flow rate = 5 mL/min, t_{R} = 21 min) to give **412** as a white powder (1.9 mg, 33% overall yield from **431**): ¹H NMR (400 MHz, MeOD) δ 8.75 (s, 1H), 8.43 (d, *J =* 2.0 Hz, 1H), 8.07 - 7.96 (m, 4H), 7.94 (dd, *J =* 8.9, 2.0 Hz, 1H), 7.81 (d, *J =* 8.1 Hz, 2H), 7.68 (d, *J =* 8.1 Hz, 2H), 7.53 (d, *J =* 8.0 Hz, 2H), 6.61 (s, 1H), 3.62 (d, *J =* 3.3 Hz, 1H), 3.53 (dd, *J* = 10.2, 6.5 Hz, 1H), 3.26 - 3.15 (m, 1H), 3.04 (d, *J =* 7.0 Hz, 2H), 2.99 (dd, *J =* 14.4, 6.0 Hz, 1H), 2.68 - 2.55 (m, 1H), 2.40 (dq, *J* = 11.9, 5.9 Hz, 1H). HRMS m/z [M+H]⁺ for C₃₁H₂₅NO₂F₃ calculated 500.1837, found 500.1834.

*rac*-4-(4-((3*aR*,6*aR*)-Octahydrocyclopenta[c]pyrrol-5-yl)phenyl)-7-(4-(trifluoromethyl)phenyl)-2-naphthoic acid **(413).** Treatment of **412** (0.021 mmol, crude reaction residue without HPLC purification) by using Procedure B gave **413** (0.57 mg, 5% overall yield from **431**) as a white powder: ¹H NMR (400 MHz, MeOD) δ 8.74 (s, 1H), 8.42 (d, *J =* 1.9 Hz, 1H), 8.06 - 7.95 (m, 4H), 7.93 (d, *J =* 8.4 Hz, 1H), 7.81 (d, *J =* 8.0 Hz, 2H), 7.49 (s, 4H), 4.01 (d, *J =* 9.2 Hz, 1H), 3.48 (p, *J =* 6.4 Hz, 2H), 3.03 - 2.86 (m, 2H), 2.59 (dd, *J =* 12.8, 6.4 Hz, 1H), 2.47 - 2.29 (m, 2H), 2.17 - 1.98 (m, 2H), 1.64 (q, *J =* 11.0 Hz, 1H). HRMS m/z [M+H]⁺ for C₃₁H₂₇NO₂F₃ calculated 502.1994, found 502.1986.

4-(4-((1*R*,5*S*,6*r*)-3-Azabicyclo[3.1.1]heptan-6-yl)phenyl)-7-(4-(trifluoromethyl)phenyl)-2-naphthoic acid **(414).** Concentrated sulfuric acid (95-98%, 0.9 mL) was added to a mixture of **415** (9 mg, 0.015 mmol) and NaBH₄ (5.7 mg, 0.15 mmol) in THF (0.1 mL) under argon (caution, running the reaction under argon is required to avoid fire/explosion). The resulting solution was stirred at rt for 30 min. The mixture was basified with cold 6 N NaOH aqueous solution and extracted with ethyl acetate three times. The organic layer was dried over anhydrous Na₂SO₄. Volatiles were evaporated, and the residue was purified by RP-HPLC (C18, A: ACN, B: 10 mM TEAA, 35% → 55% A in 40 min, flow rate = 5 mL/min, t_{R} = 38.8 min) to give **414** as a white powder (0.8 mg, 11%): ¹H NMR (400 MHz, MeOD) δ 8.76 (s, 1H), 8.44 (d, *J =* 2.0 Hz, 1H), 8.07 - 7.96 (m, 4H), 7.96 - 7.90 (m, 1H), 7.81 (d, *J =* 8.1 Hz, 2H), 7.56 (d, *J =* 7.8 Hz, 2H), 7.38 (d, *J =* 7.7 Hz, 2H), 4.09 (q, *J* = 9.6 Hz, 1H), 3.62 (t, *J* = 7.2 Hz, 1H), 3.42 - 3.33 (m, 3H), 3.23 (dd, *J =* 16.7, 7.1 Hz, 2H), 2.74 (q, *J =* 9.1 Hz, 1H), 2.33 (q, *J =* 10.5 Hz, 1H). HRMS m/z [M+H]⁺ for C30H₂₄F3NO₂ calculated 488.1837, found 488.1829.

4-(4-((1*R*,5*S*,6*r*)-6-Hydroxy-3-azabicyclo[3.1.1]heptan-6-yl)phenyl)-7-(4-(trifluoromethyl)phenyl)-2-naphthoic acid **(415). Procedure C.** A TFA solution in THF (67%, 2 mL) was added to **435** (0.022 mmol, reaction residue). The resulting mixture was stirred at rt for 1 h. Volatiles were evaporated, and the residue was purified by RP-HPLC (C18, A: ACN, B: 10 mM TEAA, 35% → 55% A in 40 min, flow rate = 5 mL/min, t_{R} = 27.7 min) to give **415** (4.6 mg, 42%) as a white solid: ¹H NMR (400 MHz, DMSO) δ 8.76 (s, 1H), 8.65 (d, *J =* 2.0 Hz, 1H), 8.06 (t, *J =* 8.2 Hz, 3H), 7.98 (d, *J =* 8.9 Hz, 1H), 7.92 - 7.85 (m, 3H), 7.73 (d, *J =* 7.9 Hz, 2H), 7.59 (d, *J =* 7.8 Hz, 2H), 3.69 (d, *J =* 11.7 Hz, 2H), 3.47 (d, *J* = 11.9 Hz, 2H), 2.89 (d, *J* = 4.5 Hz, 2H), 1.71 (s, 2H). HRMS m/z [M+H]⁺ for C₃₀H₂₅NO₃F₃ calculated 504.1787, found 504.1793.

4-(4-(Azepan-4-yl)phenyl)-7-(4-(trifluoromethyl)phenyl)-2-naphthoic acid **(416).** Treatment of **417** (0.038 mmol, crude reaction residue without HPLC purification) by using Procedure B gave **416** (4 mg, 22% overall yield from **438**) as a white powder: ¹H NMR (400 MHz, MeOD) δ 8.77 - 8.72 (m, 1H), 8.42 (d, *J* = 1.9 Hz, 1H), 8.05 - 7.95 (m, 4H), 7.92 (dd, J = 8.9, 1.9 Hz, 1H), 7.81 (d, *J =* 8.1 Hz, 2H), 7.48 (q, *J =* 8.1 Hz, 4H), 3.54 - 3.35 (m, 4H), 3.02 (t, *J =* 10.9 Hz, 1H), 2.22 (d, *J =* 15.9 Hz, 4H), 2.09 - 1.86 (m, 2H). HRMS m/z [M+H]⁺ for C₃₀H₂₇NO₂F₃ calculated 490.1994, found 490.1992.

Mixture of 4-(4-(2,3,6,7-Tetrahydro-1*H*-azepin-4-yl)phenyl)-7-(4-(trifluoromethyl)phenyl)-2-naphthoic acid and 4-(4-(2,5,6,7-tetrahydro-1H-azepin-4-yl)phenyl)-7-(4-(trifluoromethyl)phenyl)-2-naphthoic acid **(417).** Treatment of **439** (all residue from hydrolysis of **438,** 0.057 mmol) by using Procedure C gave **417** (5.08 mg, 18%) as a mixture of two constitutional isomers (1:1): ¹H NMR (400 MHz, MeOD) δ 8.76 (d, *J =* 2.8 Hz, 1H), 8.43 (d, *J =* 2.4 Hz, 1H), 8.05 - 7.97 (m, 4H), 7.97 - 7.90 (m, 1H), 7.81 (d, *J =* 8.1 Hz, 2H), 7.66 - 7.49 (m, 4H), 6.35 (t, *J =* 6.2 Hz, 0.5H), 6.21 (t, *J =* 6.5 Hz, 0.5H), 3.99 (d, *J =* 6.5 Hz, 1H), 3.59 - 3.49 (m, 1H), 3.48 - 3.41 (m, 1H), 3.39 - 3.32 (m, 1H), 3.14 - 3.05 (m, 1H), 3.06 - 2.99 (m, 1H), 2.74 (d, *J =* 5.5 Hz, 1H), 2.14 (d, *J =* 5.7 Hz, 1H). HRMS m/z [M+H]⁺ for C₃₀H₂₅NO₂F₃ calculated 488.1837, found 488.1835.

4-(4-(1,2,3,4,7,8-Hexahydroazocin-5-yl)phenyl)-7-(4-(trifluoromethyl)phenyl)-2-naphthoic acid **(419)** and 4-(4-(5-hydroxyazocan-5-yl)phenyl)-7-(4-(trifluoromethyl)phenyl)-2-naphthoic acid **(420).** Treatment of **443** (all residue from hydrolysis of **442,** 0.049 mmol) by using Procedure C gave **419** (4.52 mg, 18%) in addition to **420** (4.67 mg, 18%). **419** has: ¹H NMR (400 MHz, MeOD) δ 8.72 (s, 1H), 8.40 (d, *J =* 2.0 Hz, 1H), 8.03 - 7.95 (m, 5H), 7.90 (dd, *J =* 9.0, 2.0 Hz, 1H), 7.78 (d, *J =* 8.1 Hz, 3H), 7.66 (d, *J =* 8.0 Hz, 3H), 7.51 (d, *J* = 8.0 Hz, 2H), 6.28 (t, *J* = 8.3 Hz, 1H), 3.35 - 3.28 (m, 2H), 3.25 - 3.21 (m, 2H), 2.90 (t, *J* = 6.3 Hz, 2H), 2.72 (dd, *J* = 12.0, 7.5 Hz, 2H), 2.08 (s, 2H). HRMS m/z [M+H]⁺ for C₃₁H₂₆NO₂F₃ calculated 502.1994, found 502.2001. **20** has: ¹H NMR (400 MHz, MeOD) δ 8.75 (s, 1H), 8.42 (d, *J =* 1.9 Hz, 1H), 8.00 - 7.94 (m, 4H), 7.91 (dd, *J =* 8.9, 1.9 Hz, 1H), 7.78 (d, *J =* 8.1 Hz, 2H), 7.70 (d, *J* = 8.4 Hz, 2H), 7.65 (d, *J* = 8.5 Hz, 2H), 3.85 (dt, *J* = 13.3, 7.0 Hz, 2H), 3.41 (dt, *J* = 12.5, 6.7 Hz, 2H), 2.70 (dt, *J* = 13.5, 6.8 Hz, 2H), 2.47 (dt, *J* = 13.5, 6.7 Hz, 2H), 2.35 (dp, *J* = 13.7, 6.8 Hz, 2H), 2.24 (dq, *J* = 13.5, 6.8 Hz, 2H). HRMS m/z [M - OH⁻]⁺ for C₃₁H₂₇NO₂F₃ calculated 502.1994, found 502.1993.Ethyl 4-(4-(azetidin-3-yl)phenyl)-7-(4-(trifluoromethyl)phenyl)-2-naphthoate **(423). Procedure D.** To a vacuum dried mixture of **421** (5.8 mg, 0.023 mmol), **422** (11 mg, 0.023 mmol), Pd(PPh₃)₄ (2.7 mg, 0.0023 mmol), and Na₂CO₃ (6 mg, 0.057 mmol) was added sonicated 1,2-dimethoxyethane-water (4:1, 1 mL). The reaction mixture was degassed with argon for 10 min and then stirred at 80°C overnight. Volatiles were evaporated, and the residue was column chromatographed (CH₂Cl₂/MeOH/TEA, 100:0:1→90:10:1) to give **423** (6.25 mg, 57%): ¹H NMR (400 MHz, CDCl₃) δ 8.68 (s, 1H), 8.22 (d, *J =* 1.9 Hz, 1H), 8.01 (d, *J =* 1.7 Hz, 1H), 7.97 (d, *J =* 8.8 Hz, 1H), 7.86 - 7.77 (m, 3H), 7.75 (d, *J* = 8.3 Hz, 2H), 7.61 (d, *J* = 7.9 Hz, 2H), 7.55 (d, *J* = 8.0 Hz, 2H), 4.57 - 4.41 (m, 5H), 3.15 (q, *J =* 7.3 Hz, 2H), 1.44 (td, *J =* 7.2, 4.1 Hz, 3H). HRMS m/z [M+H]⁺ for C₃₂H₂₉NO₂F₃ calculated 516.2150, found 516.2145. HRMS m/z [M+H]⁺ for C₂₉H₂₅NO₂F₃ calculated 476.1837, found 476.1837.

*tert*-Butyl 6-(4-bromophenyl)-6-hydroxy-2-azaspiro[3.3]heptane-2-carboxylate **(425). Procedure E.** A mixture of 1,4-dibromobenzene (2.9 g, 12.5 mmol) and magnesium turnings (60.8 mg, 2.5 mmol) in THF (5 mL) was sonicated for 2 h. The solution was cooled to 0 °C. Into the Grignard reagent was added the solution of **424** (105.6 mg, 0.5 mmol) in THF (10 mL). The resulting mixture was stirred at 0 °C to rt overnight. The residue was partitioned between ethyl acetate and aqueous NaHCO₃. The organic layer was dried over anhydrous Na₂SO₄. Volatiles were evaporated, and the residue was column chromatographed (hexane/ethyl acetate = 80:20 → 40:60) to give **425** (72.3 mg; 39%): ¹H NMR (400 MHz, CDCl₃) δ 7.50 - 7.44 (m, 2H), 7.29 - 7.24 (m, 2H), 4.05 (s, 2H), 3.78 (s, 2H), 2.72 - 2.66 (m, 2H), 2.52 (d, *J =* 12.7 Hz, 2H), 1.41 (s, 9H). HRMS m/z [M - *t*-butyl+2H]⁺ for C₁₃H₁₅N⁷⁹BrO₃ calculated 312.0235, found 312.0234.

*tert*-Butyl 6-(4-(3-(ethoxycarbonyl)-6-(4-(trifluoromethyl)phenyl)naphthalen-1-yl)phenyl)-6-hydroxy-2-azaspiro[3.3]heptane-2-carboxylate **(426).** Treatment of **425** (25.6 mg, 0.07 mmol) by using Procedure D gave **426** (36.12 mg, 82%): ¹H NMR (400 MHz, CDCl₃) δ 8.68 (s, 1H), 8.22 (d, *J =* 1.8 Hz, 1H), 8.06 - 7.96 (m, 2H), 7.81 (d, *J* = 8.1 Hz, 2H), 7.76 (td, *J =* 6.8, 3.3 Hz, 3H), 7.57 (d, *J =* 8.0 Hz, 2H), 7.52 (d, *J =* 8.1 Hz, 2H), 4.46 (q, *J =* 7.1 Hz, 2H), 4.13 (s, 2H), 3.90 (s, 2H), 2.87 (d, *J =* 12.8 Hz, 2H), 2.64 (d, *J =* 12.6 Hz, 2H), 1.44 (d, *J =* 9.7 Hz, 12H). HRMS m/z [M+Na]⁺ for C₃₇H₃₆NO₅F₃Na calculated 654.2443, found 654.2446.

4-(4-(2-(*tert*-Butoxycarbonyl)-6-hydroxy-2-azaspiro[3.3]heptan-6-yl)phenyl)-7-(4-(trifluoromethyl)phenyl)-2-naphthoic acid **(427).** Treatment of **426** (36 mg, 0.057 mmol) by using Procedure A (without HPLC purification) gave **427** of sufficient purity to be used in next step: ¹H NMR (400 MHz, MeOD) δ 8.50 (s, 1H), 8.21 (s, 1H), 7.91 - 7.80 (m, 4H), 7.67 (dd, *J =* 14.7, 9.8 Hz, 3H), 7.52 - 7.45 (m, 2H), 7.37 (t, *J* = 5.3 Hz, 2H), 3.98 (s, 2H), 3.76 (s, 2H), 2.73 (d, *J =* 12.1 Hz, 2H), 2.52 - 2.45 (m, 2H), 1.32 (d, *J* = 3.8 Hz, 9H). HRMS m/z [M+Na]⁺ for C₃₅H₃₂NO₅F₃Na calculated 626.2130, found 626.2136.

*rac*-(3*aR*,6*aR*)-2-Benzyl-5-(4-bromophenyl)octahydrocyclopenta[c]pyrrol-5-ol **(29).** Treatment of **428** (125.9 mg, 0.5 mmol) by using Procedure E (column chromatography; DCM/MeOH, 100:0→95:5) gave **429** (101.18 mg, 54%): ¹H NMR (400 MHz, CDCl₃) δ 7.48 - 7.40 (m, 2H), 7.40 - 7.20 (m, 7H), 3.94 - 3.78 (m, 2H), 2.88 (dq, *J* = 9.6, 4.5 Hz, 2H), 2.80 (s, 1H), 2.50 (tdt, *J =* 16.7, 11.3, 8.9 Hz, 3H), 2.29 (dd, *J =* 12.7, 7.1 Hz, 1H), 2.18 (dtd, *J =* 16.1, 11.4, 5.6 Hz, 1H), 1.97 (dd, *J =* 12.6, 4.7 Hz, 1H), 1.76 - 1.61 (m, 2H). HRMS m/z [M+H]⁺ for C₂₀H₂₃NO⁷⁹Br calculated 372.0963, found 372.0966.

*rac*-Ethyl 4-(4-((3aR,6aR)-2-benzyl-5-hydroxyoctahydrocyclopenta[c]pyrrol-5-yl)phenyl)-7-(4-(trifluoromethyl)phenyl)-2-naphthoate **(30).** Treatment of **429** (26.1 mg, 0.07 mmol) by using Procedure D gave **430** (33.7 mg, 76%): ¹H NMR (400 MHz, CDCl₃) δ 8.65 (s, 1H), 8.20 (d, *J =* 1.9 Hz, 1H), 8.04 - 7.95 (m, 2H), 7.79 (d, *J =* 8.2 Hz, 2H), 7.76 - 7.68 (m, 3H), 7.60 (d, *J* = 8.1 Hz, 2H), 7.54 (d, *J* = 7.4 Hz, 2H), 7.48 (d, *J* = 8.0 Hz, 2H), 7.36 (dt, *J* = 13.7, 7.2 Hz, 3H), 4.44 (q, *J* = 7.1 Hz, 2H), 4.14 (s, 2H), 3.32 - 3.17 (m, 2H), 2.99 - 2.76 (m, 3H), 2.57 - 2.42 (m, 2H), 2.20 (dd, *J* = 12.6, 5.2 Hz, 1H), 1.94 (dt, *J* = 17.9, 12.1 Hz, 2H), 1.44 (t, *J* = 7.1 Hz, 3H). HRMS m/z [M+H]⁺ for C₄₀H₃₇NO₃F₃ calculated 636.2726, found 636.2724.

*rac*-4-(4-((3*aR*,6*aR*)-2-Benzyl-5-hydroxyoctahydrocyclopenta[c]pyrrol-5-yl)phenyl)-7-(4-(trifluoromethyl)phenyl)-2-naphthoic acid **(31).** Treatment of **430** (33.7 mg, 0.053 mmol) by using Procedure A (without HPLC purification) gave **431** of sufficient purity to be used in next step: ¹H NMR (400 MHz, MeOD) δ 8.59 (s, 1H), 8.30 (s, 1H), 8.00 (s, 1H), 7.92 (dd, *J =* 8.5, 5.3 Hz, 3H), 7.74 (d, *J* = 7.5 Hz, 3H), 7.65 (dd, *J* = 15.8, 6.5 Hz, 4H), 7.51 - 7.39 (m, 5H), 4.62 - 4.50 (m, 2H), 3.54 (d, *J* = 12.9 Hz, 2H), 3.22 (q, *J* = 13.9 Hz, 2H), 2.94 - 2.66 (m, 2H), 2.47 (dd, *J* = 13.2, 7.1 Hz, 1H), 2.18 (dd, *J* = 12.8, 5.0 Hz, 1H), 1.97 (q, *J =* 11.3 Hz, 2H). HRMS m/z [M+H]⁺ for C₃₈H₃₃NO₃F₃ calculated 608.2413, found 608.2408.

*tert*-Butyl (1*R*,5*S*,6*r*)-6-(4-bromophenyl)-6-hydroxy-3-azabicyclo[3.1.1]heptane-3-carboxylate **(433).** Treatment of **432** (105.65 mg, 0.5 mmol) by using Procedure E gave **33** (99.36 mg, 54%): ¹H NMR (400 MHz, CDCl₃) δ 7.50 (dd, *J =* 8.7, 2.3 Hz, 2H), 7.39 (d, *J =* 8.5 Hz, 2H), 3.82 - 3.66 (m, 4H), 2.82 - 2.76 (m, 1H), 2.71 - 2.63 (m, 1H), 1.55 (dt, *J =* 11.4, 6.2 Hz, 1H), 1.45 (d, *J* = 2.5 Hz, 9H), 1.33 (d, *J =* 10.1 Hz, 1H). HRMS m/z [M *- t-*butyl+2H]⁺ for C₁₃H₁₅N⁷⁹BrO₃ calculated 312.0235, found 312.0234.

*tert*-Butyl (1R,5S,6r)-6-(4-(3-(ethoxycarbonyl)-6-(4-(trifluoromethyl)phenyl)naphthalen-1-yl)phenyl)-6-hydroxy-3-azabicyclo[3.1.1]heptane-3-carboxylate **(434).** Treatment of **433** (25.8 mg, 0.07 mmol) by using Procedure D gave **434** (41 mg, 93%): ¹H NMR (400 MHz, CDCl₃) δ 8.69 (d, *J =* 1.6 Hz, 1H), 8.23 (d, *J =* 1.9 Hz, 1H), 8.07 - 8.01 (m, 2H), 7.81 (d, *J =* 8.2 Hz, 2H), 7.79 - 7.73 (m, 3H), 7.71 (d, *J =* 8.0 Hz, 2H), 7.56 (d, *J =* 8.0 Hz, 2H), 4.45 (q, *J =* 7.1 Hz, 2H), 3.94 - 3.77 (m, 4H), 3.02 - 2.94 (m, 1H), 2.88 (d, *J =* 6.7 Hz, 1H), 1.74 (dt, *J =* 11.4, 6.1 Hz, 1H), 1.52 (s, 9H), 1.48 - 1.40 (m, 4H). HRMS m/z [M - Boc - O]⁺ for C₃₂H₂₇NO₂F₃ calculated 514.1994, found 514.2001.

4-(4-((1*R*,5*S*,6*r*)-3-(*tert*-Butoxycarbonyl)-6-hydroxy-3-azabicyclo[3.1.1]heptan-6-yl)phenyl)-7-(4-(trifluoromethyl)phenyl)-2-naphthoic acid **(435).** Treatment of **434** (41 mg, 0.065 mmol) by using Procedure A (without HPLC purification) gave **435** of sufficient purity to be used in next step: ¹H NMR (400 MHz, MeOD) δ 8.58 (s, 1H), 8.31 (d, *J =* 1.9 Hz, 1H), 8.04 (d, *J =* 1.6 Hz, 1H), 7.97 (dd, *J =* 8.6, 3.2 Hz, 3H), 7.81 - 7.74 (m, 3H), 7.72 (d, *J =* 7.9 Hz, 2H), 7.53 (t, *J =* 6.6 Hz, 2H), 3.84 - 3.69 (m, 4H), 2.96 - 2.82 (m, 2H), 1.69 (dt, *J =* 11.1, 6.0 Hz, 1H), 1.36 (d, *J* = 9.7 Hz, 1H). MS m/z [M - Boc - O]⁺ for C₃₀H₂₃NO₂F₃ calculated 486.2, found 486.2.

*tert*-Butyl 4-(4-bromophenyl)-4-hydroxyazepane-1-carboxylate **(437).** Treatment of **436** (106.65 mg, 0.5 mmol) by using Procedure E gave **37** (135 mg, 73%): ¹H NMR (400 MHz, CDCl₃) δ 7.45 (d, *J =* 8.0 Hz, 2H), 7.32 (d, *J =* 8.3 Hz, 2H), 3.92 - 3.47 (m, 2H), 3.32 (d, *J =* 11.3 Hz, 2H), 2.27 - 1.67 (m, 7H), 1.47 (d, *J =* 4.1 Hz, 9H).

tert-Butyl 4-(4-(3-(ethoxycarbonyl)-6-(4-(trifluoromethyl)phenyl)naphthalen-1-yl)phenyl)-4-hydroxyazepane-1-carboxylate **(438).** Treatment of **437** (38.9 mg, 0.105 mmol) by using Procedure D gave **438** (36.4 mg, 82%): ¹H NMR (400 MHz, CDCl₃) δ 8.67 (s, 1H), 8.22 (s, 1H), 8.03 (d, *J =* 9.4 Hz, 2H), 7.81 (d, *J =* 8.1 Hz, 2H), 7.74 (d, *J =* 8.0 Hz, 3H), 7.62 (d, *J =* 8.1 Hz, 2H), 7.55 - 7.45 (m, 2H), 4.46 (q, *J =* 7.1 Hz, 2H), 3.93 - 3.53 (m, 2H), 3.45 - 3.34 (m, 2H), 2.35 - 2.05 (m, 3H), 1.99 - 1.68 (m, 4H), 1.51 (s, 9H), 1.45 (t, *J =* 7.2 Hz, 3H). HRMS m/z [M + Na]⁺ for C₃₇H₃₈NO₅F₃Na calculated 656.2600, found 656.2589.

4-(4-(1-(*tert*-Butoxycarbonyl)-4-hydroxyazepan-4-yl)phenyl)-7-(4-(trifluoromethyl)phenyl)-2-naphthoic acid **(439).** Treatment of **438** (36.4 mg, 0.057 mmol) by using Procedure A (without HPLC purification) gave **439** of sufficient purity to be used in next step: ¹H NMR (400 MHz, MeOD) δ 8.34 (s, 1H), 8.08 (s, 1H), 7.80 (s, 1H), 7.74 (d, *J* = 8.1 Hz, 3H), 7.55 (d, *J* = 8.0 Hz, 3H), 7.39 (d, *J* = 8.1 Hz, 2H), 7.25 (d, *J* = 7.2 Hz, 2H), 3.46 (s, 2H), 3.23 - 3.16 (m, 2H), 2.16 - 1.51 (m, 6H), 1.27 (s, 9H). MS m/z [M - Boc - O]⁺ for C₃₀H₂₅NO₂F₃ calculated 488.1837, found 488.1840.

*tert*-Butyl 5-(4-bromophenyl)-5-hydroxyazocane-1-carboxylate **(441).** Treatment of **440** (113.65 mg, 0.5 mmol) by using Procedure E gave **441** (155 mg, 81%): ¹H NMR (400 MHz, CDCl₃) δ 7.44 - 7.39 (m, 2H), 7.37 - 7.32 (m, 2H), 3.46 - 3.24 (m, 4H), 2.06 - 1.77 (m, 6H), 1.58 - 1.45 (m, 2H), 1.44 (s, 9H). HRMS m/z [M - t-butyl+H - OH⁻]⁺ for C₁₄H₁₇N⁷⁹BrO₂ calculated 310.0443, found 310.0437.

*tert*-Butyl 5-(4-(3-(ethoxycarbonyl)-6-(4-(trifluoromethyl)phenyl)naphthalen-1-yl)phenyl)-5-hydroxyazocane-1-carboxylate **(442).** Treatment of **441** (26.9 mg, 0.07 mmol) by using Procedure D gave **442** (35.5 mg, 78%): ¹H NMR (400 MHz, CDCl₃) δ 8.68 (s, 1H), 8.22 (d, *J =* 1.9 Hz, 1H), 8.07 - 8.00 (m, 2H), 7.81 (d, *J =* 8.1 Hz, 2H), 7.78 - 7.71 (m, 3H), 7.66 (d, *J =* 8.1 Hz, 2H), 7.54 - 7.47 (m, 2H), 4.46 (q, *J =* 7.1 Hz, 2H), 3.56 - 3.37 (m, 5H), 2.27 - 2.06 (m, 2H), 2.00 (d, *J* = 14.6 Hz, 4H), 1.71 (s, 2H), 1.50 (s, 9H), 1.45 (t, *J* = 7.1 Hz, 3H). MS m/z [M - Boc - O]⁺ for C₃₃H₃₁NO₂F₃ calculated 530.2, found 530.2.

4-(4-(1-(*tert*-Butoxycarbonyl)-5-hydroxyazocan-5-yl)phenyl)-7-(4-(trifluoromethyl)phenyl)-2-naphthoic acid **(443).** Treatment of **442** (32 mg, 0.049 mmol) by using Procedure A (without HPLC purification) gave **443** of sufficient purity to be used in next step: ¹H NMR (400 MHz, MeOD) δ 8.57 (s, 1H), 8.32 - 8.28 (m, 1H), 8.05 - 8.01 (m, 1H), 7.96 (d, *J* = 8.3 Hz, 3H), 7.82 - 7.73 (m, 3H), 7.67 (d, *J* = 7.9 Hz, 2H), 7.46 (d, *J* = 7.9 Hz, 2H), 3.56 (q, *J =* 11.3 Hz, 2H), 3.45 - 3.28 (m, 2H), 2.27 - 1.87 (m, 6H), 1.67 - 1.53 (m, 2H), 1.49 (s, 9H). MS m/z [M - Boc - O]⁺ for C₃₁H₂₇NO₂F₃ calculated 502.1994, found 502.1990.

### EXAMPLE 5

This Example illustrates the affinity of compounds of formula (III). The affinity was determined using a whole cell fluorescent binding assay, which was shown to underestimate the actual affinity.

| No. | MRS # | R= | IC₅₀ (nM) |
|---|---|---|---|
| 4-membered rings | | | |
| **405** | MRS4827 | 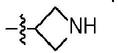 | 65.9±19.3 |
| **406** | MRS4838 | 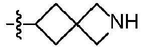 | 46.6±7.1 |
| **407** | MRS4821 | 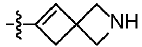 | 9.69±3.56 |
| **408** | MRS4820 | 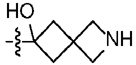 | 20.8±5.6 |
| **409** | MRS4829 | 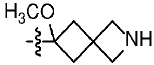 Mix^{c} of OCH₃ and OCD₃ | 46.5±10.0 |

| 5-membered rings | | | |
|---|---|---|---|
| **410** | MRS4664^{d} | 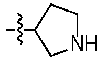 | 31.5±4.2 |
| **411** | MRS4822^{a} | 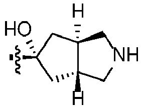 | 9.48±1.66 |
| **412** | MRS4823^{a} | 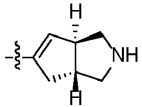 | 16.6±3.2 |
| **413** | MRS4824^{a} | 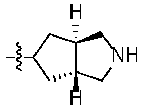 | 32.0±11.2 |

| 6-membered rings | | | |
|---|---|---|---|
| **421** | PPTN^{d} | 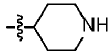 | 7.96±0.35 |
| **422** | MRS4608^{d} | 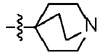 | 20.0±4.4 |
| **423** | MRS4738^{d} | 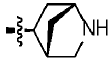 | 3.11±0.62 |
| **424** | MRS4837 | 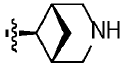 | 524±104 |
| **425** | MRS4833 | 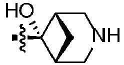 | 5.92±0.55 |

| 7-membered rings | | | |
|---|---|---|---|
| **426** | MRS4826 | 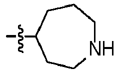 | 9.58±5.14 |
| **427** | MRS4825^{b} | 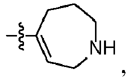 | 11.9±3.6 |
| | | 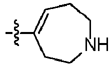 | |

| 8-membered rings | | | |
|---|---|---|---|
| **428** | MRS4835 | 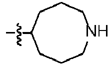 | TBD |
| **429** | MRS4832 | 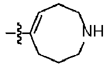 | 58.4±16.4 |
| **430** | MRS4831 | 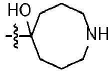 | 1780±440 |

### EXAMPLE 6

This example demonstrates the species dependence of binding of various P2Y₁₄R antagonists, as determined in a fluorescent antagonist binding assay in whole cells (N = 3-4). As is apparent from the results set forth in Table 2, compound **29** had slightly enhanced affinity (IC₅₀ 184 nM) at the mP2Y₁₄R vs the human homologue, while **11, 19** and **32** had nearly the same affinity in the two species. Desirably, receptor affinity is maintained across species, facilitating performance in *in vivo* preclinical testing in small animals, with the mouse being the most commonly used species in initial evaluation.

**Table 2**

| Compound | mP2Y₁₄R, IC₅₀ (nM)^{a} | hP2Y₁₄R, IC₅₀ (nM)^{a} |
|---|---|---|
| **1b**^{b} | 29.7±9.3 | 27.6±4.3 |
| **3a**^{b} | 142±58 | 31.7±8.0 |
| **4**^{b} | 384±88 | 169±42 |
| **5**^{b} | 902±344 | 269±121 |
| **6**^{b} | 499±57 | 233±26 |
| **11** | 155±79 | 197±29 |
| **19** | 375±175 | 308±63 |
| **29** | 184±24 | 296±13 |
| **32** | 18.6±8.4 | 15.0±4.4 |

| | | |
|---|---|---|
| ^{a} Fluorescent binding assays using 2 (20 nM, for a 45 min incubation) as tracer. The hP2Y₁₄R was stably expressed in CHO cells, and the mP2Y₁₄R was stably expressed in HEK293 cells. ^{b} Data from Jung et al.² and Mufti et al.⁷ | | |

### EXAMPLE 7

This example demonstrates some properties of amidomethyl ester prodrugs **37a-c**.^{a} The results are set forth in Table 3. The triazole-containing compound **37a** was the most rapidly cleaved, while the naphthalene-containing compounds **37b** and **37c** did not reach 50% cleavage after 72 h at 37 °C. All of the compounds were stable for 24 h upon aqueous incubation in the absence of PLE.

**Table 3**

| Compound (molecular charge, pH 7) | MW, TPSA (Å²) | cLogP^{b} | Parent drug | *t*_{1/2} (min, mean±SEM) for esterase cleavage^{c} |
|---|---|---|---|---|
| **37a** (+1) | 577.2, 86.6 | 4.59 | **3a** | 20.2±1.9 |
| **37b** (uncharged) | 574.6, 88.4 | 5.65 | **32** | >72 (32.6±0.9%)^{d} |
| **37c** (uncharged) | 602.7, 66.9 | 6.27 | **1b** | >72 (27.2±0.3%)^{d} |

| | | | | |
|---|---|---|---|---|
| a. Structures are shown in Scheme 7. b. cLogP calculated using ALOGPS 2.1 program (www.vcclab.org/lab/alogps/).⁵⁷ ND, not determined. c. At pH 7.3 in aqueous medium in the presence of PLE. d. Percent cleavage at 72 h in parentheses. | | | | |

### EXAMPLE 8

This examples demonstrates some in vitro and in vivo ADMET data for compound **32** compared to known P2Y₁₄R antagonist **5**.^{a} The compound was stable in simulated gastric and intestinal fluids, in plasma of three species, and in human and mouse liver microsomes.

**Table 4**

| Test | **5**^{b} | **32** |
|---|---|---|
| Simulated intestinal fluid (t_{1/2}, min) | >240 | >240 |
| Simulated gastric fluid (t_{1/2}, min) | >240 | 161 |
| CYP1A2 (IC₅₀, µM) | >30 | >30 |
| CYP2C9 (IC₅₀, µM) | >30 | 10.4 |
| CYP2C19 (IC₅₀, µM) | >30 | >30 |
| CYP2D6 (IC₅₀, µM) | >30 | >30 |
| CYP3A4 (IC₅₀, µM) | >30 | >30 |
| Plasma stability (3 species)d (t_{1/2}, min) | >240 (h); >240 (r); >240 (m) | TBD (h); >240 (r); >240 (m) |
| Microsomal stability (t_{1/2}, min) | 145 (m), 108 (r), 145 (h) | >240 (h), 47.7 (r), >240 (m) |
| hERG, IC₅₀ (µM) | 0.166 (fluorescent) | >30 (patch clamp) |
| t_{1/2} (h)^{e} | 0.20 | 0.689 |
| aqueous solubility^{c} (pH 7.4, µg/mL) | 138±4 | 7.63±1.93 |
| aqueous solubility^{c} (pH 4.0, µg/mL) | ND | 0.14±0.02 |

| | | |
|---|---|---|
| ^{a} Procedures are in Yu et al.¹ ^{b} Data determined in Yu et al.¹ ^{c} Mean ± SD, pION method. ^{d} Species tested for plasma stability were human, rat and mouse; species as indicated for microsomal stability. ^{e} Method noted in parentheses. ^{e} By i.v. administration in rat: 0.5 mg/kg dose. See Table 5 for complete results. ND, not determined. | | |

### EXAMPLE 9

This example demonstrates some pharmacokinetic parameters^{a} for compound **32** administered by intravenous and intraperitoneal routes. The results are set forth in Table 5.

**Table 5**

| Dose (mg/kg) | Cₘₐₓ (ng/mL) | Tₘₐₓ (h) | AUC₀₋ₗₐₛₜ (h* ng/mL) | AUC_{0-∞} (h*ng/mL) | T_{1/2} (h) | MRTₗₐₛₜ (h) | Vd (mL/kg) | Kel (1/h) | F (%) | Cl (mL/h/kg) |
|---|---|---|---|---|---|---|---|---|---|---|
| i.v. (0.5) | 359 | 0.083 | 234 | 238 | 0.689 | 0.703 | 2090 | 1.01 | 100 | 2110 |
| i.p. (1.0) | 88.4 | 2.0 | 650 | 700 | 7.23 | 6.24 | 14,900 | 0.096 | 139 | 1430 |
| i.p. (3.0) | 315 | 2.0 | 2060 | 2120 | 4.68 | 5.68 | 9570 | 0.148 | 147 | 1420 |
| i.p. (10) | 786 | 4.0 | 4900 | 4920 | 2.98 | 5.02 | 8730 | 0.233 | 105 | 2030 |

| | | | | | | | | | | |
|---|---|---|---|---|---|---|---|---|---|---|
| ^{a} In healthy adult male Wistar rats; with 3 per group. Abbreviations: Cₘₐₓ, maximal concentration; Tₘₐₓ, time at which maximal concentration observed; AUC₀₋ₗₐₛₜ, area under the plasma drug concentration-time curve up to the last quantifiable time-point; AUC_{0-∞}, area under the plasma drug concentration-time curve to infinite time; T_{1/2}, terminal half-life; MRTₗₐₛₜ, mean residence time; Vd, volume of distribution; Kel, elimination rate constant; F, biovailability; Cl, total body clearance. | | | | | | | | | | |

### EXAMPLE 10

This example demonstrates a binding comparison of various P2Y₁₄R antagonists at mP2Y₁₄R and hP2Y₁₄R. The results are set forth in Table 6.

**Table 6**

| Compound | mP2Y₁₄R, IC₅₀ (nM)^{a} | hP2Y₁₄R, IC₅₀ (nM)^{a} |
|---|---|---|
| **100**^{b} PPTN | 21.6±7.0 | 7.96±0.35 |
| **102a**^{b} | 142±58 | 31.7±8.0 |
| **114** | 33.9±4.6 | 3.11±0.62 |
| **117** | | 25.9±7.6 |
| **118** | 102±39 | 107±13 |
| **120** | 26.2±10.7 | 39.5±9.8 |
| **121** | 42.9±7.0 | 37.3±6.0 |
| **131** | 15.5±3.3 | 21.3±1.1 |
| **134** | 17.9±2.6 | 10.8±2.1 |
| **137** | | 125±45 |
| **138** | 40.9±6.3 | 49.6±13.5 |

| | | |
|---|---|---|
| *^{a}*IC₅₀ for inhibition of fluorescent hP2Y₁₄R antagonist **(3)** binding was determined as in Table 1. IC₅₀ for inhibition of mP2Y₁₄R binding was determined using flow cytometry of whole mP2Y₁₄R-HEK cells in the presence of a fixed concentration (200 nM) of **3** (mean±SEM, n = 3 - 6). *^{b}*IC₅₀ values from literature (Yu, J. et al., J. Med. Chem. 2018, 61, 4860-4882, Jung, Y. H. et al., J. Med. Chem. 2020, 63, 9563-9589) | | |

### EXAMPLE 11

This example demonstrates ADMET values for two P2Y₁₄R antagonists in this study, compared to reference quinuclidine **4.**

**Table 7**

| Test | **4**^{a} | **114** | **121** |
|---|---|---|---|
| Simulated intestinal fluid (% remaining at 120 min) | 99.7 | 99.1 | 100 |
| Simulated gastric fluid (% remaining at 120 min) | 74.2 | 100e | 59.3 |
| CYP1A2 (IC₅₀, µM) | 8.69 | >30 | >30 |
| CYP2C9 (IC₅₀, µM) | >30 | >30 | >30 |
| CYP2C19 (IC₅₀, µM) | >30 | >30 | >30 |
| CYP2D6 (IC₅₀, µM) | >30 | >30 | >30 |
| CYP3A4 (IC₅₀, µM) | 3.29 | >30 | >30 |
| Plasma stability (3 species)^{c} (% remaining at 120 min) | 100 (h); 79.9 (r); 100 (m) | 82.8 (r); 100 (m) | 74.7 (r); 74.1 (m) |
| Microsomal stability (t_{1/2}, min) | 67.2 (m), 80.0 (r), >240 (h) | 243 (m), 91.0 (r), 280 (h) | >1000 (m), 89.4 (r), 851 (h) |
| hERG, IC₅₀ (µM) | 16.4 | >30 | >30 |
| HepG2 cell toxicity, IC₅₀ (µM) | 21.6 | 11.0 | 9.34 |
| t_{1/2} (h), administration mode^{d} | 5.1 (1 mg/kg, i.p.); 3.46 (i.v.) | 8.94; 7.24 | 4.59; 2.11 |
| aqueous solubility^{b} (pH 7.4, µg/mL) | 0.44±0.06 | | |
| aqueous solubility^{c} (pH 4.0, µg/mL) | 14.3±0.5 | | |

| | | | |
|---|---|---|---|
| ^{a} Data determined in Jung, Y. H. et al., J. Med. Chem. 2020, 63, 9563-9589 ^{b} Mean ± SD, pION method. ^{e} Species tested for plasma stability were human, rat and mouse; species as indicated for microsomal stability. ^{d} By i.v. administration: 0.5 mg/kg dose. ^{e} hERG inhibition was measured using a fluorescent method. Aqueous acid stability was determined by NMR. | | | |

### EXAMPLE 12

This example demonstrates IC₅₀ values of hERG ion channel block by selected P2Y₁₄R antagonists and positive control (cisapride) determined in a patch clamp assay. Value in parentheses indicates EC₅₀ value for stimulation of hERG channel activity. None of the compounds inhibited hERG, but one antagonist **20** stimulated hERG activity with an EC₅₀ of 18 µM.

**Table 8**

| Compound | **IC₅₀/(EC₅₀), µM** |
|---|---|
| PPTN | >30 |
| **114** MRS4738 | >30 |
| **115** MRS4742 | >30 |
| **116** MRS4743 | >30 |
| **120** MRS4745 | (18.1) |
| **131** MRS4679 | >30 |
| **134** MRS4748 | >30 |
| **135** MRS4749 | >30 |
| Cisapride | 0.019 |

### EXAMPLE 13

This example illustrates the preparation of prodrugs and the Plasma Protein Binding (PPB) of selected derivatives in three Species, including three active drugs and two prodrugs.

**Scheme 6.** Preparation of Amidomethyl Esters of **114, 121** and **138,** as well as Reported Ester of **1,** i.e. **147**,¹³ and the Double Prodrug of **115,** i.e. **143***^{a}* 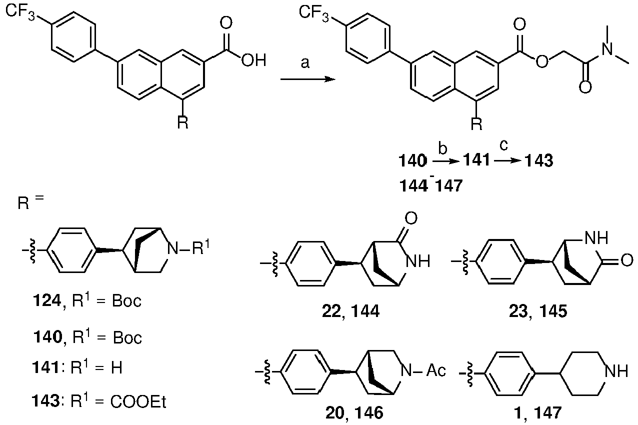 *^{a}*Reagents and conditions: (a) 2-chloro-*N,N*-dimethylacetamide, Cs₂CO₃, DMF, 45 °C, 1 h, 76-98%; (b) TFA, DCM, rt, 1 h, 99%; (c) ethyl chloroformate, *N,N*-diisopropylethylamine, DMF, rt, overnight, 71%.

The results obtained are set forth in Table 9.

**Table 9. Plasma Protein Binding (PPB) of selected derivatives**

| Compound | Mouse, % bound (±SD), or *% remaining* | Rat, % bound (±SD), or % *remaining* | Human, % bound (±SD) |
|---|---|---|---|
| *Active drugs* | | | |
| **114** | 99.76±0.03 | 99.79±0.02 | 87.98±10.29 |
| **121** | 99.74±0.04 | 96.18±0.32 | 98.89±0.50 |
| **138** | 100.00±0 | 100.00±0 | 100.00±0 |

| *Ester prodrugs* | | | |
|---|---|---|---|
| **141** | *0%*^{a} | *0.72*±*0.04%*^{a} | 99.70±0.07 |
| **145** | *0%*^{a} | *0%*^{a} | 99.79±0.18 |

^{a} For the ester prodrugs extensive compound loss was observed, and the percent remaining in mouse and rat plasma at 5 h is given in italics.

### EXAMPLE 14

This example illustrates the evaluation of compound **114** in Chronic Constriction Injury Mouse Model of Neuropathic Pain.

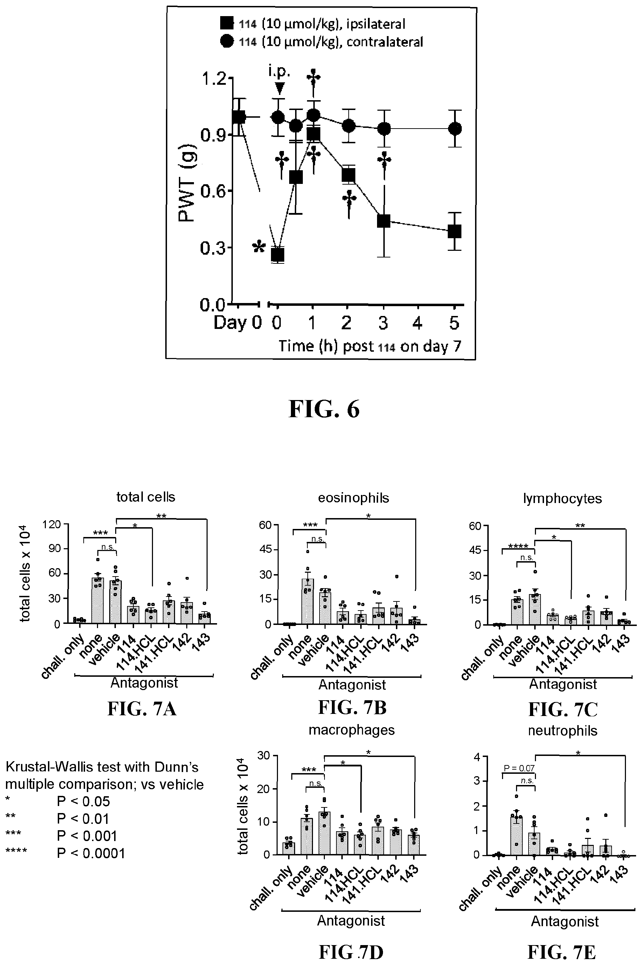

Compound **114** was evaluated in a validated model of chronic neuropathic pain in the mouse, i.e. following chronic constriction of the sciatic nerve (CCI). This compound was administered by the i.p. route (10 µmol/kg). The reversal of CCI-induced mechano-allodynia by the compound was nearly complete (89.3±10.1%) at 1 h post-injection, and significant partial protection was maintained through 3 h but was absent at 5 h (Figure 6). No obvious side effects were observed in the mice.

### EXAMPLE 15

This example illustrates the antagonist potency of prodrugs **142** and **143.**

The carbamate prodrug **142** and the double prodrug **143** having carbamate and ester moieties were derived from the most potent antagonist **114.** Prodrugs **142** and **143** were tested in the mouse asthma model in comparison to the HCl salt form of the parent drug **114.** The N,N-dimethylamidomethyl ester prodrug **141** and the carbamate prodrug **142** reduced allergic inflammation more effectively than the parent drug 15. Intriguingly, the double prodrug **143** displayed an even stronger potency, dramatically reducing the recruitment of neutrophils, eosinophils, and lymphocytes into the airway. The results are depicted in Fig. 7. These promising results suggest that double prodrug **143** could be a candidate for further drug development and therapeutic treatment of asthma and other inflammatory diseases.

### EXAMPLE 16

### Experimental procedures

### Chemical synthesis

**General Information.** *Materials and Methods.* All chemicals and anhydrous solvents were obtained directly from commercial sources. All reactions were carried out under argon atmosphere using anhydrous solvents unless specified otherwise. Room temperature (rt) refers to 25 ± 5 °C. Silica-gel precoated with F254 on aluminum plates were used for TLC. The spots were examined under ultraviolet light at 254 nm and further visualized by anisaldehyde or cerium ammonium molybdate stain solution. Column chromatography was performed on silica gel (40-63 µm, 60 Å). NMR spectra were recorded on a Bruker 400 MHz spectrometer. Chemical shifts are given in ppm (δ), calibrated to the residual solvent signal peaks of CDCl₃ (7.26 ppm), CD₃OD (3.31 ppm), or DMSO-*d*₆ (2.50 ppm) for ¹H NMR with coupling constant (*J*) values reported in Hz. High resolution mass (HRMS) measurements were performed on a proteomics optimized Q-TOF-2 (Micromass-Waters). The RP-HPLC was performed using Phenomenex Luna 5 µm C18(2)100A, AXIA, 21.2 mm × 250 mm column. Antagonist purity was determined as ≥95% using Agilent ZORBAX SB-Aq, 5 µm, 4.6 mm × 150 mm column attached to Agilent 1100 HPLC system. The HPLC traces for compounds tested in vivo **(xxxx)** were included in Supporting Information.

(2*R*,3*R*,4*S*,5*R*,6*R*)-2-(Acetoxymethyl)-6-azidotetrahydro-2*H*-pyran-3,4,5-triyl triacetate **(523).** A mixture of **522** (205.6 mg, 0.5 mmol) and sodium azide (65 mg, 1 mmol) in DMF (4 mL) was stirred at rt for 2 h. The volatiles were evaporated, and the residue was extracted with DCM three times. The combined organic layer was dried with Na₂SO₄ to give **23** as a white powder (184.7 mg, 99%): ¹H NMR (400 MHz, CDCl₃) δ 5.14 (t, *J =* 9.5 Hz, 1H), 5.01 (t, *J =* 9.7 Hz, 1H), 4.86 (t, *J =* 9.2 Hz, 1H), 4.59 (d, *J =* 8.9 Hz, 1H), 4.18 (dd, *J* = 12.5, 4.8 Hz, 1H), 4.07 (dd, *J* = 12.5, 2.3 Hz, 1H), 3.74 (ddd, *J =* 10.1, 4.8, 2.3 Hz, 1H), 2.00 (s, 3H), 1.98 (s, 3H), 1.94 (s, 3H), 1.91 (s, 3H). HRMS m/z [M+Na]⁺ for C₁₄H₁₉N₃O₉Na calculated 396.1019, found 396.1017.

4-(4-(1-(Hex-5-yn-1-yl)piperidin-4-yl)phenyl)-7-(4-(trifluoromethyl)phenyl)-2-naphthoic acid **(525).** A mixture of **502** (11.9 mg, 0.025 mmol), 6-Iodo-1-hexyne (7.3 µL, 11.4 mg, 0.055 mmol), and K₂CO₃ (13.8 mg, 0.1 mmol) in DMF (1 mL) was stirred at rt overnight. THF-H₂O-MeOH (3:1:1, 1 mL) and LiOH (7.4 mg, 0.31 mmol) were added into the solution. The resulting mixture was sonicated for 30 s and stirred at rt for 2 h to hydrolyze the ester byproduct to give product. The reaction was neutralized by 1 M HCl. The volatiles were evaporated, and the residue was column chromatographed (DCM/MeOH/AcOH, 100:0:0.1→95:5:0.1) to give **525** (6 mg, 43%): ¹H NMR (400 MHz, MeOD) δ 8.69 (d, *J =* 1.7 Hz, 1H), 8.38 (d, *J=* 1.9 Hz, 1H), 7.97 - 7.93 (m, 4H), 7.86 (dd, *J =* 8.8, 1.9 Hz, 1H), 7.76 (d, *J =* 8.1 Hz, 2H), 7.47 (t, *J =* 6.8 Hz, 4H), 3.71 (d, *J =* 12.1 Hz, 2H), 3.25 - 3.11 (m, 4H), 3.09 - 2.98 (m, 1H), 2.33 - 2.28 (m, 2H), 2.22 (d, *J =* 13.9 Hz, 2H), 2.07 (d, *J =* 13.0 Hz, 2H), 1.94 (d, *J =* 10.8 Hz, 3H), 1.62 (t, *J =* 7.6 Hz, 2H); HRMS m/z [M+H]⁺ for C₃₅H₃₃NO₂F₃ calculated 556.2463, found 556.2466.

4-(4-(1-(4-(1-((2*R*,3*R*,4*S*,5*R*,6*R*)-3,4,5-Triacetoxy-6-(acetoxymethyl)tetrahydro-2*H*-pyran-2-yl)-1*H*-1,2,3-triazol-4-yl)butyl)piperidin-4-yl)phenyl)-7-(4-(trifluoromethyl)phenyl)-2-naphthoic acid **(504).** Sodium ascorbate (5.9 mg, 0.03 mmol) was added into a mixture of **525** (8.3 mg, 0.015 mmol), **523** (11.2 mg, 0.03 mmol), and CuSO₄.5H₂O (3.7 mg, 0.015 mmol) in DMF/H₂O (9:1, 2 mL). the resulting mixture was stirred at 90 °C overnight. The volatiles were evaporated, and the residue was column chromatographed (DCM/MeOH/AcOH, 100:0:0.2->90:10:0.2) to give **504** (13 mg, quantitative): ¹H NMR (400 MHz, MeOD) δ 8.63 (s, 1H), 8.32 (s, 1H), 8.09 (s, 1H), 7.89 (q, *J* = 14.2 Hz, 4H), 7.72 (d, *J* = 7.8 Hz, 3H), 7.35 (s, 4H), 6.07 (d, *J* = 8.1 Hz, 1H), 5.50 (td, *J* = 16.9, 8.2 Hz, 2H), 5.22 (t, *J =* 9.3 Hz, 1H), 4.28 (dd, *J =* 12.4, 4.6 Hz, 1H), 4.20 (d, *J =* 9.8 Hz, 1H), 4.12 (d, *J* = 12.4 Hz, 1H), 3.66 (s, 2H), 3.21 - 3.06 (m, 4H), 2.99 - 2.89 (m, 1H), 2.79 (s, 2H), 2.09 (s, 4H), 2.00 (d, *J =* 5.7 Hz, 6H), 1.95 (d, *J =* 2.5 Hz, 6H), 1.81 (s, 4H). HRMS m/z [M+H]⁺ for C₄₉H₅₂N₄O₁₁F₃ calculated 929.3585, found 929.3578.

7-(4-(Trifluoromethyl)phenyl)-4-(4-(1-(4-(1-((2R,3R,4S,SS,6R)-3,4,5-trihydroxy-6-(hydroxymethyl)tetrahydro-2*H*-pyran-2-yl)-1*H*-1,2,3-triazol-4-yl)butyl)piperidin-4-yl)phenyl)-2-naphthoic acid **(508).** NaOH (3 M, 1 mL) was added into a solution of **504** (13 mg, 0.015 mmol) in MeOH (2 mL). The solution was stirred at rt overnight. Volatiles were evaporated, and the residue was purified by RP-HPLC (C18, A: ACN, B: 10 mM TEAA, 35% → 60% A in 40 min, flow rate = 5 mL/min, t_{R} = 24.9 min) to give **508** as a white powder (9 mg, 79% overall yield from **525**): ¹H NMR (400 MHz, DMSO) δ 8.61 (s, 1H), 8.52 (s, 1H), 8.02 (d, *J* = 8.4 Hz, 3H), 7.89 (d, *J* = 15.3 Hz, 3H), 7.83 (d, *J* = 8.1 Hz, 2H), 7.40 (d, *J =* 2.8 Hz, 4H), 5.42 (d, *J =* 9.3 Hz, 1H), 3.75 - 3.61 (m, 2H), 3.43 - 3.13 (m, 4H), 2.99 (d, *J =* 10.5 Hz, 2H), 2.62 (t, *J =* 7.4 Hz, 2H), 2.58 - 2.52 (m, 1H), 2.34 (s, 2H), 2.02 (t, *J =* 11.4 Hz, 2H), 1.84 - 1.78 (m, 2H), 1.70 (d, *J =* 11.8 Hz, 2H), 1.60 (d, *J* = 7.3 Hz, 2H), 1.51 (s, 2H). HRMS m/z [M+H]⁺ for C₄₁H₄₄N₄O₇F₃ calculated 761.3162, found 761.3154.

The invention provides the following aspects:
1. A compound of formula (I) or formula (II): 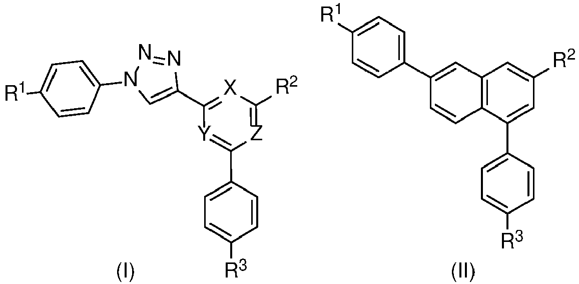
   wherein X, Y, Z are CH or N,
   R¹ is halo or CF₃,
   R² is selected from COOH, COOR¹², CONHOH, CH₂CON(R¹¹)₂, CHR⁷OCOR⁸, (CH₂)ₒNR⁹, tetrazolyl, CH₂OPO(OH)₂, and 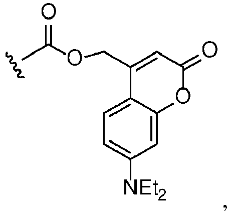
   R³ is selected from NHR⁴, COOR⁵, (C=C)ₙ(CH₂)ₘR⁶, CONH(CH₂)_{q}NH₂, COCF₃, 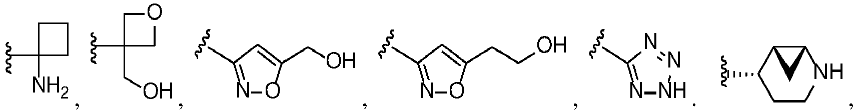 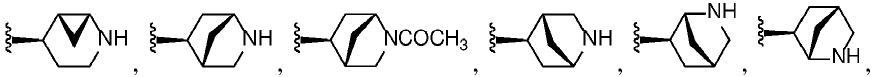 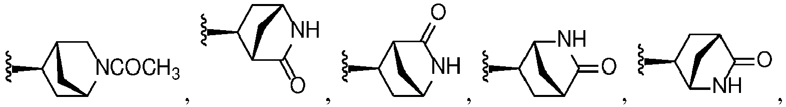 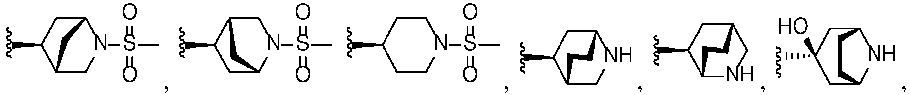 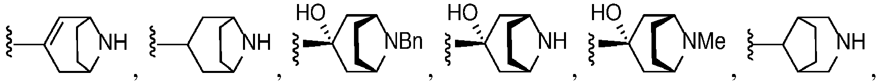 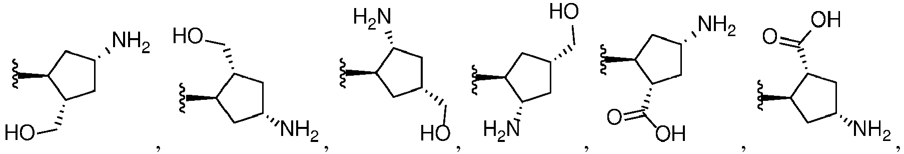 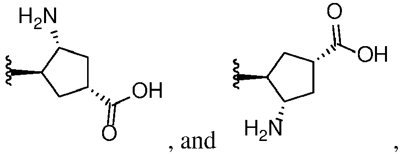
      R⁴ is H or COR⁶ wherein R⁶ is C₁-C₆ alkyl, C₆-C₁₀ aryl, or 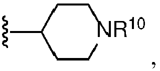
      R⁵ is C₁-C₆ alkyl, C₃-C₈ cycloalkyl, or benzyl,
      R⁶ is NH₂, or CONH₂,
      R⁷-R⁹ are independently hydrogen or C₁-C₆ alkyl,
      R¹⁰ is H, C₁-C₁₀ alkylcarbonyl, or C₁-C₁₀ alkyloxycarbonyl,
      R¹¹ is hydrogen or C₁-C₆ alkyl,
      R¹² is C₁-C₆ alkyl, C₃-C₈ cycloalkyl, or benzyl,
      m, o, and q are independently integers of 1 to about 10, and
      n is zero or an integer of 1 to about 10,
      or a pharmaceutically acceptable salt thereof, or stereoisomers thereof.
2. The compound or salt or stereoisomers thereof of aspect 1, wherein R¹ is CF₃.
3. The compound or salt or stereoisomers thereof of aspect 1 or 2, wherein the compound is of formula (I).
4. The compound or salt or stereoisomers thereof of any one of aspects 1-3, wherein R³ is selected from NH₂, NHCOCH₃, NHCOC₆H₅, NHCOC(CH₃)₃, 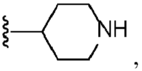 and 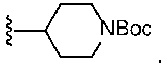
5. The compound or salt or stereoisomers thereof of any one of aspects 1-3, wherein R³ is COOR⁵.
6. The compound or salt or stereoisomers thereof of any one of aspects 1-3, wherein R³ is (C≡C)ₙ(CH₂)ₘR⁶ or CONH(CH₂)_{q}NH₂.
7. The compound or salt or stereoisomers thereof of aspect 6, wherein R³ is selected from CONH(CH₂)₃NH₂, CH₂CONH₂, (CH₂)₃NH₂, and C≡CCH₂NH₂.
8. The compound or salt or stereoisomers thereof of any one of aspects 1-3, wherein R³ is selected from Br, (CH₂)₂-CN, 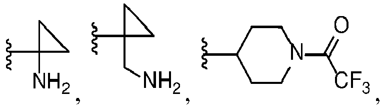 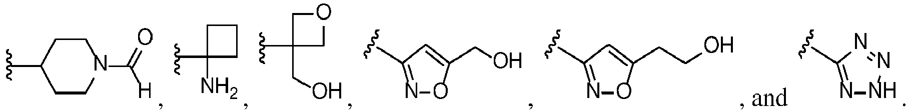
9. The compound or salt or stereoisomers thereof of aspect 1 or 2, wherein the compound is of formula (II).
10. The compound or salt or stereoisomers thereof of aspect 9, wherein R³ is selected from Br, (CH₂)₂-CN, 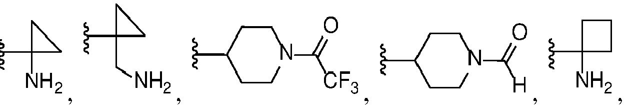 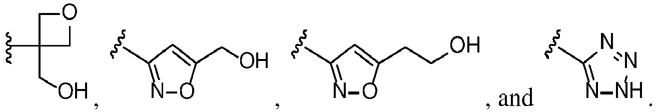
11. The compound or salt or stereoisomers thereof of aspect 9, wherein R³ is 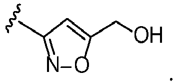
11. The compound or salt or stereoisomers thereof of any one of aspects 1-10, wherein R² is H.
12. The compound or salt or stereoisomers thereof of any one of aspects 1-10, wherein R² is COOR¹², CH₂CON(R¹¹)₂, CHR⁷OCOR⁸, (CH₂)ₒNR⁹, tetrazolyl, CH₂OPO(OH)₂, or 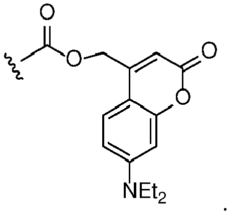
13. The compound or salt or stereoisomers thereof of aspect 1, wherein the compound is: 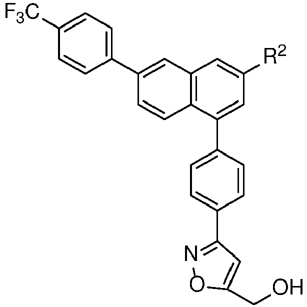 wherein R² is COOH or COOCH₂CON(CH₃)₂.
14. The compound or salt or stereoisomers thereof of aspect 9, wherein R³ is selected from 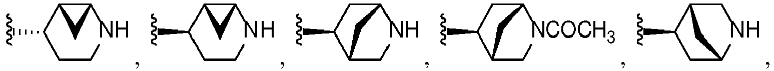 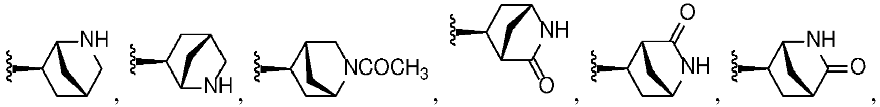 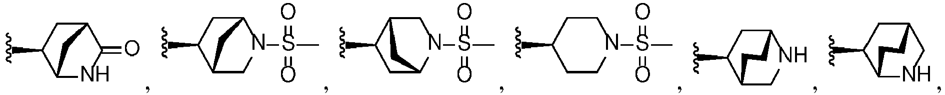 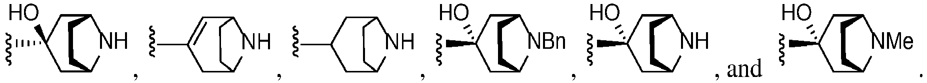
15. The compound or salt or stereoisomers thereof of aspect 14, wherein R³ is 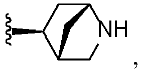 and wherein the configuration of the three chiral centers is (S,S,S).
16. The compound or salt or stereoisomers thereof of aspect 9, wherein R³ is selected from 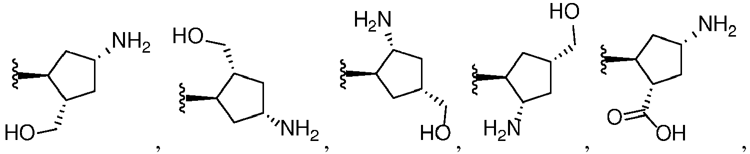 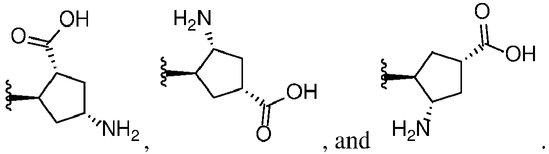
17. The compound or salt or stereoisomers thereof of any one of aspects 14-16, wherein R² is H.
18. The compound or salt or stereoisomers thereof of any one of aspects 14-16, wherein R² is COOR¹², CH₂CON(R¹¹)₂, CHR⁷OCOR⁸, (CH₂)ₒNR⁹, tetrazolyl, CH₂OPO(OH)₂, or 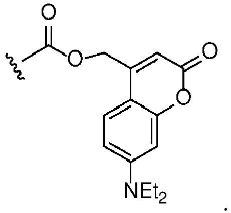
19. The compound or salt or stereoisomers thereof of aspect 1, wherein the compound is: 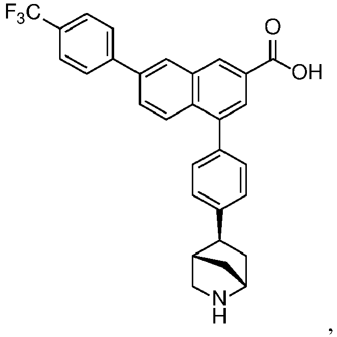 wherein the configuration about the three chiral centers is (S,S,S).
20. A pharmaceutical composition comprising a compound or salt or stereoisomers thereof of any one of aspects 1-19 and a pharmaceutically acceptable carrier.
21. A compound or salt or stereoisomers thereof of any one of aspects 1-19, for use in antagonizing a P2Y₁₄R receptor in a mammal in need thereof.
22. A compound or salt or stereoisomers thereof of any one of aspects 1-19, for use in treating or preventing an inflammatory condition in a mammal in need thereof.
23. The compound for use according to aspect 22 wherein the inflammatory condition is selected from the group consisting of asthma, cystic fibrosis, and sterile inflammation of the kidney.
24. A compound or salt or stereoisomers thereof of any one of aspects 1-19, for use in treating pain in a mammal in need thereof.
25. A method of antagonizing a P2Y₁₄R receptor in a mammal in need thereof, comprising administering to the mammal an effective amount of a compound or salt or stereoisomers thereof of any one of aspects 1-19.
26. A method of treating or preventing an inflammatory condition in a mammal in need thereof, comprising administering to the mammal an effective amount of a compound or salt or stereoisomers thereof of any one of aspects 1-19.
27. The method according to aspect 26, wherein the inflammatory condition is selected from the group consisting of asthma, cystic fibrosis, and sterile inflammation of the kidney.
28. A method of treating pain in a mammal in need thereof, comprising administering to the mammal an effective amount of a compound or salt or stereoisomers thereof of any one of aspects 1-19.
28. The method according to aspect 27, wherein the inflammatory condition is selected from the group consisting of asthma, cystic fibrosis, and sterile inflammation of the kidney.
29. A method of treating pain in a mammal in need thereof, comprising administering to the mammal an effective amount of a compound or salt or stereoisomers thereof of any one of aspects 1-20.

### References

1. Yu, J.; Ciancetta, A.; Dudas, S.; Duca, S.; Lottermoser, J.; Jacobson, K.A. Structure-guided modification of heterocyclic antagonists of the P2Y14 receptor. J. Med. Chem. 2018, 61, 4860-4882, doi: 10.1021/acs.jmedchem.8b00168.
2. Jung, Y. H.; Yu, J.; Wen, Z.; Salmaso, V.; Phung, N. B.; Karcz, T.; Chen, Z.; Duca, S.; Bennett, J. M.; Dudas, S.; Cook, D. N.; Salvemini, D.; Gao, Z. G.; Jacobson, K. A. Exploration of alternative scaffolds for P2Y14 receptor antagonists containing a biaryl core. J. Med. Chem. 2020, 63, 9563-9589.
3. Battistone, M. A.; Mendelsohn, A. C.; Spallanzani, R. G.; Allegretti, A. S.; Liberman, R. N.; Sesma, J.; Kalim, S.; Wall, S. M.; Bonventre, J. V.; Lazarowski, E. R.; Brown, D.; Breton, S. Pro-inflammatory P2Y14 receptor inhibition protects against ischemic acute kidney injury in mice. J. Clin. Invest. 2020, 130, 3734-3749.
4. Xu, J.; Morinaga, H.; Oh, D.; Li, P.; Chen, A.; Talukdar, S.; Lazarowski, E.; Olefsky, J. M.; Kim, J. J. GPR105 ablation prevents inflammation and improves insulin sensitivity in mice with diet-induced obesity. J. Immunol. 2012, 189(4), 1992-1999.
5. Jain, S.; Jacobson, K. A. Adipocyte specific ablation of P2Y14R improves glucose metabolism in mice with diet-induced obesity. American Diabetes Assoc., 80th Scientific Session, Abstract, 2020.
6. Karcz, T. P.; Whitehead, G. S.; Nakano, H.; Williams, J. G.; Deterding, L. J.; Jacobson, K. A.; Cook, D. N. UDP-glucose and P2Y14 receptor amplify allergen-induced airway eosinophilia. Submitted.
7. Mufti, F.; Jung, Y. H.; Giancotti, L. A.; Yu, J.; Chen, Z..; Phung, N. B.; Jacobson, K. A.; Salvemini, D. P2Y14 receptor antagonists reverse chronic neuropathic pain in a mouse model. ACS Med. Chem. Lett. 2020, 11, 1281-1286.
8. Lin, J.; Liu, F.; Zhang, Y-Y.; Song, N.; Liu, M-K.; Fang, X-y.; Liao, D-Q.; Zhou, C.;Wang, H.; Shen, J-F. P2Y14 receptor is functionally expressed in satellite glial cells and mediates interleukin-1β and chemokine CCL2 secretion. J. Cell. Physiol. 2019, 234, 21199- 21210.
9. Jacobson, K. A.; Delicado, E. G.; Gachet, C.; Kennedy, C.; von Kiigelgen, I.; Li, B.; Miras-Portugal, T.; Novak, I.; Schöneberg, T.; Perez-Sen, R.; Thor, D.; Wu, B.; Yang, Z.; Müller, C. E. Update of P2Y receptor pharmacology: IUPHAR review:"27". Br. J. Pharmacol. 2020, 177, 2413-2433.
10. Kinoshita, M.; Nasu-Tada, K.; Fujishita, K.; Sato, K.; Koizumi, S. Secretion of matrix metalloproteinase-9 from astrocytes by inhibition of tonic P2Y14-receptor-mediated signal(s). Cell. Mol. Neurobiol. 2013, 33, 47-58. DOI: 10.1007/s10571-012-9869-4
11. Zhang, Z.; Hao, K.; Li, H.; Lu, R.; Liu, C.; Zhou, M.; Li, B.; Meng, Z.; Hu, Q.; Jiang, C. Design, synthesis and anti-inflammatory evaluation of 3-amide benzoic acid derivatives as novel P2Y14 receptor antagonists. Eur. J. Med. Chem. 2019, 181, 111564.
12. Hamel, M.; Henault, M.; Hyjazie, H.; Morin, N.; Bayly, C.; Skorey, K.; Therien, A.G.; Mancini, J.; Brideau, C.; Kargman, S. Discovery of novel P2Y14 agonist and antagonist using conventional and nonconventional methods. J. Biomol. Screening 2011, 16(9), 1098-1105.
13. Wang, W.; Liu, C.; Li, H.; Tian, S.; Liu, Y.; Wang, N.; Yan, D.; Li, H.; Hu, Q. Discovery of novel and potent P2Y14R antagonists via structure-based virtual screening for the treatment of acute gouty arthritis. J. Adv. Res. 2020, 23, 133-142. doi: https://doi.org/10.1016/j.jare.2020.02.007
14. Junker, A.; Balasubramanian, R.; Ciancetta, A.; Uliassi, E.; Kiselev, E.; Martiriggiano, C.; Trujillo, K.; Mtchedlidze, G.; Birdwell, L.; Brown, K. A.; Harden, T. K.; Jacobson, K. A. Structure-based design of 3-(4-aryl-1H-1,2,3-triazol-1-yl)-biphenyl derivatives as P2Y14 receptor antagonists. J. Med. Chem. 2016, 59, 6149-6168.
15. Zhang, K.; Zhang, J.; Gao, Z. G.; Paoletta, S.; Zhang, D.; Han, G. W.; Li, T.; Ma, L.; Zhang, W.; Müller, C. E.; Yang, H.; Jiang, H.; Cherezov, V.; Katritch, V.; Jacobson, K. A.; Stevens, R. C.; Wu, B.; Zhao, Q. Agonist-bound structure of the human P2Y12R receptor. Nature 2014, 509, 119-122.
16. Kiselev, E.; Barrett, M.; Katritch, V.; Paoletta, S.; Weitzer, C. D.; Hammes, E.; Yin, A. L.; Zhao, Q.; Stevens, R. C.; Harden, T. K.; Jacobson, K. A. Exploring a 2-naphthoic acid template for the structure-based design of P2Y14 receptor antagonist molecular probes. ACS Chem. Biol. 2014, 9, 2833-2842.
17. Barrett, M. O.; Sesma, J. I.; Ball, C. B.; Jayasekara, P. S.; Jacobson, K. A.; Lazarowski, E. R.; Harden, T. K. A selective high affinity antagonist of the P2Y14 receptor inhibits UDP-glucose-stimulated chemotaxis of human neutrophils. Mol. Pharmacol. 2013, 84, 41-49.
18._Robichaud, J.; Fournier, J.-F.; Gagné, S.; Gauthier, J. Y.; Hamel, M.; Han, Y.; Hénault, M.; Kargman, S.; Levesque, J. F.; Mamane, Y.; Mancini, J.; Morin, N.; Mulrooney, E.; Wu, J.; Black, W. C. Applying the pro-drug approach to afford highly bioavailable antagonists of P2Y14. Bioorg. Med. Chem. Lett. 2011, 27(14), 4366-4368.
19. Mazák, K.; Noszál, B. Physicochemical properties of zwitterionic drugs in therapy. ChemMedChem 2020, 15, 1102-1.
20. Sabbadin, D.; Ciancetta, A.; Moro, S. Bridging molecular docking to membrane molecular dynamics to investigate GPCR-ligand recognition: The human A2A adenosine receptor as a key study. J. Chem. Inf. Model. 2014, 54 (1), 169-183.
21. Strupczewski, J. T.; Allen, R. C.; Gardner, B. A.; Schmid, B. L.; Stache, U.; Glamkowski, E. J.; Jones, M. C.; Ellis, D. B.; Huger, F. P.; Dunn, R. W. Synthesis and neuroleptic activity of 3-(1-substituted-4-piperidinyl)-1,2-benzisoxazoles, J. Med. Chem. 1985, 28, 761-769.
22. Ballesteros, J. A.; Weinstein, H. Integrated methods for the construction of three dimensional models and computational probing of structure function relations in G protein-coupled receptors. Methods Neurosci. 1995, 25, 366-428.
23. Pennington, L. D.; Moustakas, D. T. The necessary nitrogen atom: A versatile high-impact design element for multiparameter optimization. J. Med. Chem. 2017, 60(9), 3552-3579.
24. Gardell, S.J.; Hopf, M.; Khan, A.; Dispagna M, Hampton Sessions. E.; Falter, R.; Kapoor, N.; Brooks, J.; Culver, J.; Petucci, C; Ma, C. T.; Cohen, S. E.; Tanaka, J.; Burgos, E. S.; Hirschi, J. S.; Smith, S. R.; Sergienko, E.; Pinkerton, A. B. Boosting NAD+ with a small molecule that activates NAMPT. Nat. Commun. 2019, 10, 3241.
25. Kiselyuk, A. S. HNF4 alpha bioactive ligands discovered by a high- throughput screen for modulators of the human insulin promoter. (Doctoral dissertation, pp. 65). University of California, San Diego, 2011. (https://escho!arship.org/content/qt64k4q3gr/qt64k4q3gr.pdf)
26. Yan, Z.; Caldwell, G. Optimization in Drug Discovery: In Vitro Methods (pp. 58). Humana Press.
27. Valk6, K.; Application of high-performance liquid chromatography based measurements of lipophilicity to model biological distribution. J. Chromatogr. A 2004, 1037, 299-310.
28. Besnard, J.; Ruda, G. F.; Setola, V.; Abecassis, K.; Rodriguiz, R. M.; Huang, X. P.; Norval, S.; Sassano, M. F.; Shin, A. I.; Webster, L. A.; Simeons, F. R.; Stojanovski, L.; Prat, A.; Seidah, N. G.; Constam, D. B.; Bickerton, G. R.; Read, K. D.; Wetsel, W. C.; Gilbert, I. H.; Roth, B. L.; Hopkins, A. L. Automated design of ligands to polypharmacological profiles. Nature 2012, 492, 215-220.
29. Wible, B. A.; Hawryluk, P.; Ficker, E.; Kuryshev, Y. A.; Kirsch, G.; Brown, A. M. HERG-Lite: a novel comprehensive high-throughput screen for drug-induced hERG risk. J. Pharmacol. Toxicol. Methods 2005, 52(1), 136-145.
30. Piper, D. R.; Duff, S. R.; Eliason, H. C.; Frazee, W. J.; Frey, E. A.; Fuerstenau-Sharp, M.; Jachec, C.; Marks, B. D.; Pollok, B. A.; Shekhani, M. S.; Thompson, D. V.; Whitney, P.; Vogel, K. W.; Hess, S. D. Development of the predictor HERG fluorescence polarization assay using a membrane protein enrichment approach. Assay Drug Dev. Technol. 2008, 6, 213-223, DOI: 10.1089/adt.2008.137
31. Perez C, Daniel KB, Cohen SM. Evaluating prodrug strategies for esterase-triggered release of alcohols. ChemMedChem, 2013, 8, 1662-1667. doi: 10.1002/cmdc.201300255.
32. Bennett, G. J.; Xie, Y. K. A peripheral mononeuropathy in rat that produces disorders of pain sensation like those seen in man. Pain 1988, 33, 87-107.
33. Whitehead, G. S.; Thomas, S. Y.; Shalaby, K. H.; Nakano, K.; Moran, T. P.; Ward, J. M.; Flake, G. P.; Nakano, H.; Cook, D. N. TNF is required for TLR ligand-mediated but not protease-mediated allergic airway inflammation. J. Clin. Invest. 2017, 127, 3313-3326.
34. Mignani, S.; Rodrigues, J.; Tomas, H.; Jalal, R.; Singh, P. P.; Majoral, J.-P.; Vishwakarma, R. A. Present drug-likeness filters in medicinal chemistry during the hit and lead optimization process: how far can they be simplified? Drug Disc. Today, 2018, 23(3), 605-615.
35. Dalvie, D. K.; Kalgutkar, A. S.; Khojasteh-Bakht, S. C.; Obach, R. S.; O'Donnell, J. P. Biotransformation reactions of five-membered aromatic heterocyclic rings. Chem. Res. Toxicol. 2002, 15(3), 269-299. https://doi.org/10.1021/tx015574b
36. Zhu, J.; Mo, J.; Lin, H.; Chen, Y.; Sun, H. The recent progress of isoxazole in medicinal chemistry. Bioorg. Med. Chem. 2018, 26(12), 3065-3075.
37. Sun, H.; Shi, M.; Zhang, W.; Zheng, Y.-M.; Xu, Y.-Z.; Shi, J.-J.; Liu, T.; Gunosewoyo, H.; Pang, T.; Gao, Z. B.; Yang, F.; Tang, J.; Yu, L.-F. Development of novel alkoxyisoxazoles as sigma-1 receptor antagonists with antinociceptive efficacy. J. Med. Chem. 2016, 59, 6329-6343.
38. Ratnatilaka Na Bhuket, P.; Jithavech, P.; Ongpipattanakul, B.; Rojsitthisak, P. Interspecies differences in stability kinetics and plasma esterases involved in hydrolytic activation of curcumin diethyl disuccinate, a prodrug of curcumin. RSC Advances, 2019, 9(8), 4626-4634.
39. Schrödinger Release 2019-3: Maestro, Schrödinger, LLC, New York, NY, 2019.
40. Sastry, G. M.; Adzhigirey, M.; Day, T.; Annabhimoju, R.; Sherman, W. Protein and ligand preparation: Parameters, protocols, and influence on virtual screening enrichments. J. Comput. Aided Mol. Des. 2013, 27 (3), 221-234.
41. Harder, E.; Damm, W.; Maple, J.; Wu, C.; Reboul, M.; Xiang, J. Y.; Wang, L.; Lupyan, D.; Dahlgren, M. K.; Knight, J. L.; Kaus, J. W.; Cerutti, D. S.; Krilov, G.; Jorgensen, W. L.; Abel, R.; Friesner. R. A. OPLS3: A force field providing broad coverage of drug-like small molecules and proteins. J. Chem. Theory Comput. 2016, 12 (1), 281-296.
42. Friesner, R. A.; Banks, J. L.; Murphy, R. B.; Halgren, T. A.; Klicic, J. J.; Mainz, D. T.; Repasky, M. P.; Knoll, E. H.; Shelley, M.; Perry, J. K.; Shaw, D. E.; Francis, P.; Shenkin, P. S. Glide: A new approach for rapid, accurate docking and scoring. 1. Method and assessment of docking accuracy. J. Med. Chem. 2004, 47 (7), 1739-1749.
43. Doerr, S.; Harvey, M. J.; Noé, F.; De Fabritiis, G. HTMD: High-throughput molecular dynamics for molecular discovery. J. Chem. Theory Comput. 2016, 12 (4), 1845-1852.
44. Humphrey, W.; Dalke, A.; Schulten, K. VMD: Visual molecular dynamics. J. Mol. Graph. 1996, 14 (1), 33-38, 27.
45. Lomize, M. A.; Pogozheva, I. D.; Joo, H.; Mosberg, H. I.; Lomize, A. L. OPM Database and PPM web server: Resources for positioning of proteins in membranes. Nucleic Acids Res. 2012, 40 (Database issue), D370-6.
46. Jorgensen, W. L.; Chandrasekhar, J.; Madura, J. D.; Impey, R. W.; Klein, M. L. Comparison of simple potential functions for simulating liquid water. J. Chem. Phys. 1983, 79 (2), 926.
47. Harvey, M. J.; Giupponi, G.; Fabritiis, G. D. ACEMD: Accelerating biomolecular dynamics in the microsecond time scale. J. Chem. Theory Comput. 2009, 5 (6), 1632-1639.
48. Best, R. B.; Zhu, X.; Shim, J.; Lopes, P. E. M.; Mittal, J.; Feig, M.; Mackerell, A. D. Optimization of the additive CHARMM all-atom protein force field targeting improved sampling of the backbone ϕ, ψ and side-chain χ1 and χ2 dihedral angles. J. Chem. Theory Comput. 2012, 8 (9), 3257-3273.
49. Klauda, J. B.; Venable, R. M.; Freites, J. A.; O'Connor, J. W.; Tobias, D. J.; Mondragon-Ramirez, C.; Vorobyov, I.; MacKerell, A. D.; Pastor, R. W. Update of the CHARMM all-atom additive force field for lipids: Validation on six lipid types. J. Phys. Chem. B 2010, 114 (23), 7830-7843.
50. Vanommeslaeghe, K.; MacKerell, A. D. Automation of the CHARMM general force field (CGenFF) I: Bond perception and atom typing. J. Chem. Inf. Model. 2012, 52 (12), 3144-3154.
51. Vanommeslaeghe, K.; Raman, E. P.; MacKerell, A. D. Automation of the CHARMM general force field (CGenFF) II: Assignment of bonded parameters and partial atomic charges. J. Chem. Inf. Model. 2012, 52 (12), 3155-3168.
52. CHARMM General Force Field (CGenFF) program, https://cgcnff.umaryland.cdu/
53. Kräutler, V.; van Gunsteren, W. F.; Hünenberger, P. H. A fast SHAKE algorithm to solve distance constraint equations for small molecules in molecular dynamics simulations. J. Comput. Chem. 2001, 22 (5), 501-508.
54. Essmann, U.; Perera, L.; Berkowitz, M. L.; Darden, T.; Lee, H.; Pedersen, L. G. A smooth particle mesh Ewald method. J. Chem. Phys. 1995, 103 (19), 8577.
55. Phillips, J. C.; Braun, R.; Wang, W.; Gumbart, J.; Tajkhorshid, E.; Villa, E.; Chipot, C.; Skeel, R. D.; Kalé, L.; Schulten, K. Scalable molecular dynamics with NAMD. J. Comput. Chem. 2005, 26 (16), 1781-1802.
56. Williams, T.; Kelley, C. Gnuplot 5.0, http://www.gnuplot.info
57. Tetko, I. V.; Tanchuk, V. Y. Application of associative neural networks for prediction of lipophilicity in ALOGPS 2.1 program, J. Chem. Inf. Comput. Sci. 2002, 42, 1136-1145.
58. Gauthier, J. Y.; Belley, M.; Deschênes, D.; Fournier, J. F.; Gagné, S.; Gareau, Y.; Hamel, M.; Hénault, M.; Hyjazie, H.; Kargman, S.; Lavallée, G.; Levesque, J. F.; Li, L.; Mamane, Y.; Mancini, J.; Morin, N.; Mulrooney, E.; Robichaud, J.; Thérien, M.; Tranmer, G.; Wang, Z.; Wu, J.; Black, W. C. The identification of 4,7-disubstituted naphthoic acid derivatives as UDP-competitive antagonists of P2Y14. Bioorg. Med. Chem. Lett. 2011, 21, 2836-2839.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred aspects of this invention are described herein, including the best mode known to the inventors for carrying out the invention. Variations of those preferred aspects may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A compound of formula (II): 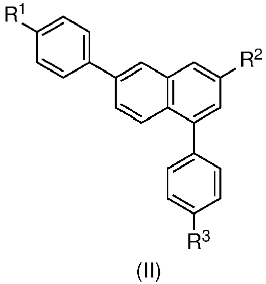
wherein R¹ is halo or CF₃,
R² is selected from the group consisting of COOH, COOR¹², CONHOH, CH₂CON(R¹¹)₂, CHR⁷OCOR⁸, (CH₂)ₒNR⁹, tetrazolyl, CH₂OPO(OH)₂, and 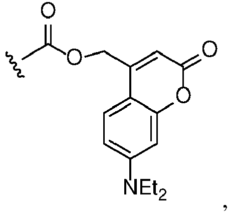
R³ is selected from the group consisting of NHR⁴, COOR⁵, (C≡C)ₙ(CH₂)ₘR⁶, CONH(CH₂)_{q}NH₂, COCF₃, 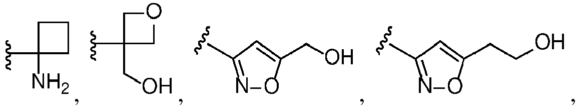 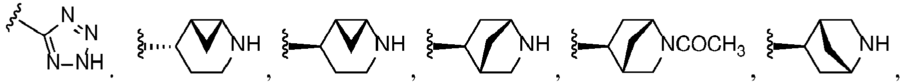 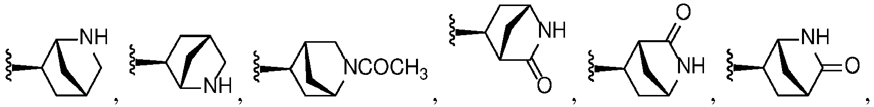 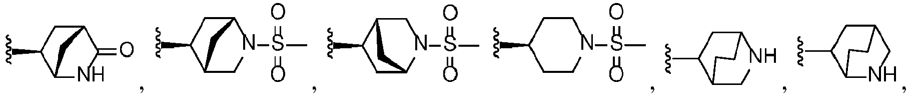 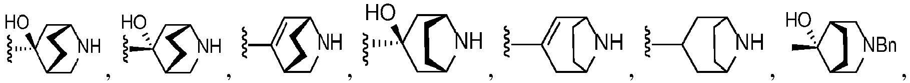 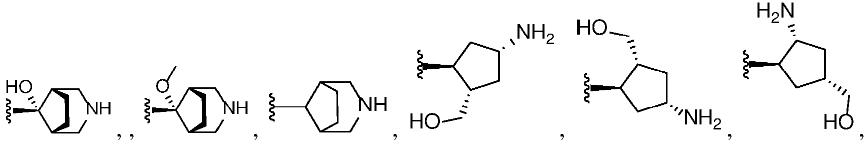 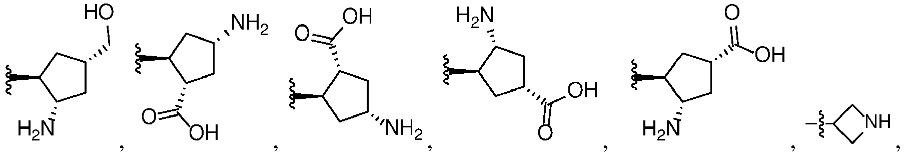 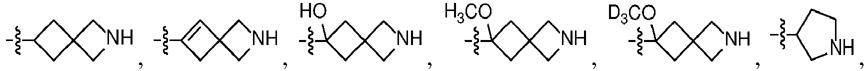 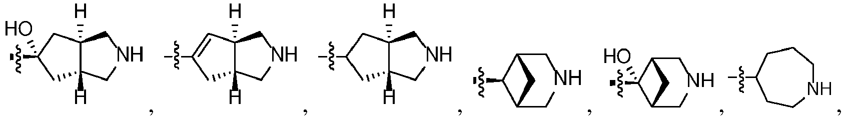 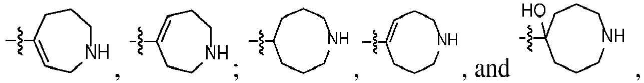
R⁴ is H or COR⁶ wherein R⁶ is C₁-C₆ alkyl, C₆-C₁₀ aryl, or 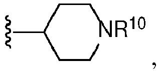
R⁵ is C₁-C₆ alkyl, C₃-C₈ cycloalkyl, or benzyl,
R⁶ is NH₂, or CONH₂,
R⁷-R⁹ are independently hydrogen or C₁-C₆ alkyl,
R¹⁰ is H, C₁-C₁₀ alkylcarbonyl, or C₁-C₁₀ alkyloxycarbonyl,
R¹¹ is hydrogen or C₁-C₆ alkyl,
R¹² is C₁-C₆ alkyl, C₃-C₈ cycloalkyl, or benzyl,
m, o, and q are independently integers of 1 to about 10, and
n is zero or an integer of 1 to about 10; or
a compound of formula (III) 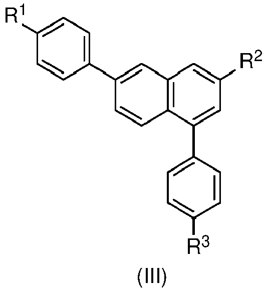 wherein R¹ is CF₃, R² is COOH, and R³ is a 4-8 membered heterocyclic ring having at least one nitrogen atom, wherein the heterocyclic ring comprises 1, 2, or 3 rings, fused or linked at one or more atoms, and wherein the heterocyclic ring is saturated or unsaturated; and wherein the heterocyclic ring is optionally substituted with one or more of substituents selected from the group consisting of alkyl, alkoxy, and hydroxy;
or a pharmaceutically acceptable salt thereof, or a stereoisomer thereof.

2. The compound, salt, or stereoisomer of claim 1, wherein R¹ is CF₃.

3. The compound, salt, or stereoisomer of claim 1 or 2, wherein the compound is of formula (II).

4. The compound, salt, or stereoisomer of any one of claims 1-3, wherein R² is H.

5. The compound, salt, or stereoisomer of any one of claims 1-3, wherein R² is COOR¹², CH₂CON(R¹¹)₂, CHR⁷OCOR⁸, (CH₂)ₒNR⁹, tetrazolyl, CH₂OPO(OH)₂, or

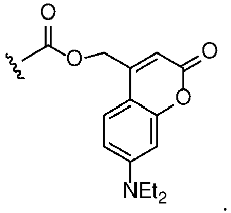

6. The compound, salt, or stereoisomer of claim 1, wherein R³ is selected from the group consisting of 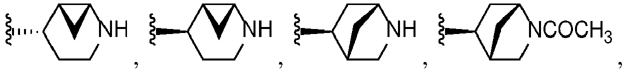 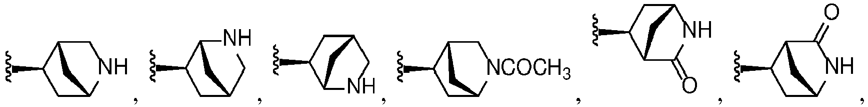 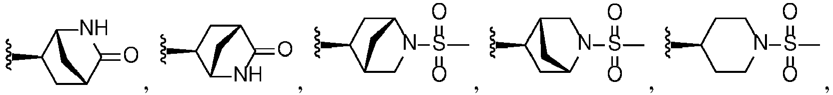 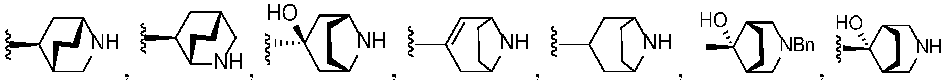 , and 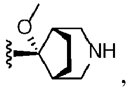 preferably R³ is

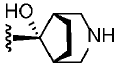

7. The compound, salt, or stereoisomer of claim 6, wherein R³ is 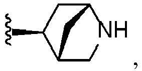 and wherein the configuration of the three chiral centers is (S,S,S).

8. The compound, salt, or stereoisomer of claim 6, wherein R³ is selected from the group consisting of

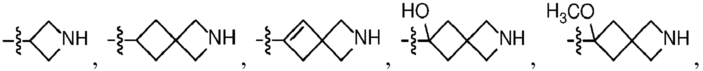

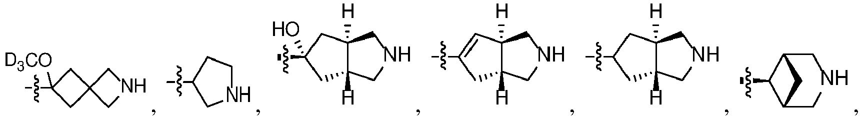

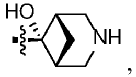

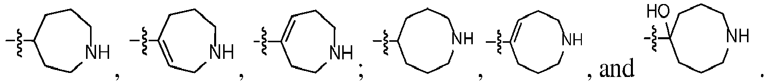

9. The compound, salt, or stereoisomer of any one of claims 6-8, wherein R² is H.

10. The compound, salt, or stereoisomer of any one of claims 6-8, wherein R² is COOR¹², CH₂CON(R¹¹)₂, CHR⁷OCOR⁸, (CH₂)ₒNR⁹, tetrazolyl, CH₂OPO(OH)₂, or

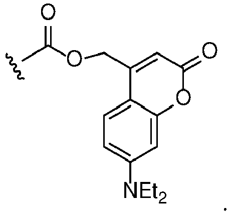

11. A pharmaceutical composition comprising a compound, salt, or stereoisomer of any one of claims 1-10 and a pharmaceutically acceptable carrier.

12. A compound, salt, or stereoisomer of any one of claims 1-10, or the pharmaceutical composition of claim 11, for use in antagonizing a P2Y₁₄R receptor in a mammal in need thereof.

13. A compound, salt, or stereoisomer of any one of claims 1-10, or the pharmaceutical composition of claim 11, for use in treating or preventing an inflammatory condition in a mammal in need thereof.

14. The compound, salt, or stereoisomer for use according to claim 13, wherein the inflammatory condition is selected from the group consisting of asthma, cystic fibrosis, and sterile inflammation of the kidney.

15. A compound, salt, or stereoisomer of any one of claims 1-10, or the pharmaceutical composition of claim 11, for use in treating pain in a mammal in need thereof.
